# FASCICULE DE BREVET EUROPEEN

(11) **EP 1 246 824 B1**
(45) Date de publication et mention de la délivrance du brevet: **09.06.2004**
(21) Numéro de dépôt: 00990087.9
(22) Date de dépôt: 21.12.2000
(51) Int. Cl.: C07D 487/04, A61K 31/551, C07K 5/02

(54) **DERIVES DE DIAZEPINE CARBOXAMIDE, LEUR PROCEDE DE PREPARATION, LEUR APPLICATION A TITRE DE MEDICAMENTS, COMPOSITIONS PHARMACEUTIQUES ET LEUR UTILISATION**
DIAZEPINECARBOXAMIDDERIVATE, VERFAHREN ZU IHRER HERSTELLUNG ,IHRE VERWENDUNG ALS ARZNEIMITTEL, PHARMAZEUTISCHE ZUBEREITUNGEN UND IHRE VERWENDUNG
CARBOXAMIDE DIAZEPIN DERIVATIVES, PREPARATION METHOD, USE AS MEDICINES, PHARMACEUTICAL COMPOSITIONS AND USE THEREOF

(30) Priorité: 28.12.1999 FR 9916567
(43) Date de publication de la demande: 09.10.2002
(73) Titulaire: Aventis Pharma S.A., 92160 Antony (FR)
(72) Inventeur: BHATNAGAR, Neerja, F-91600 Savigny sur Orge (FR); MAUGER, Jacques, F-75011 Paris (FR)
(74) Mandataire: Vieillefosse, Jean-Claude
(86) Numéro de dépôt international: PCT/FR2000/003622
(87) Numéro de publication internationale: WO 2001/047930

(56) Documents cités:
- WO-A-97/22619
- US-A- 5 552 400
- US-A- 5 847 135
- ROLAND E. DOLLE ET AL.: "Pyridazinodiazepines as a High-Affinity, P2-P3 Peptidomimetic Class of Interleukin-1beta-Converting Enzyme Inhibitor" JOURNAL OF MEDICINAL CHEMISTRY, vol. 40, no. 13, 20 juin 1997 (1997-06-20), pages 1942-1946, XP002039102

## Description

L'invention a ainsi pour objet de nouveaux dérivés de 9(S)-amino-6,10-dioxo-1,2,3,4,7,8,9,10-octahydro-6H-pyridazino[1,2-a][1,2]diazépine 1(S)-carboxamide, leur procédé de préparation, leur application à titre de médicaments, les compositions pharmaceutiques les renfermant et la nouvelle utilisation de tels dérivés.

La présente invention a notamment pour objet la préparation d'une librairie d'inhibiteurs d'enzymes utilisant le 9(S)-amino-6,10-dioxo-1,2,3,4,7,8,9,10-octahydro-6H-pyridazino[1,2-a][1,2]diazépine 1(S)-carboxamide.
Les enzymes métaboliques telles que des protéases ou des kinases sont des enzymes largement distribuées dans le règne animal. A titre d'exemples non exhaustifs, on peut citer comme références bibliographiques pour les protéases, le document : 'Methods in Enzymology XLII (1975)' et 'Journal of Medicinal Chemistry' vol. 43 n° 3 ('D. Leung, G. Abbenante et D.P. Fairlie') et pour les kinases, le document : 'Methods in Enzymology, Vol 80(1981)(Academic Press Inc.)'

Parmi les proteases capables de catalyser sélectivement l'hydrolyse des laisons polypeptidiques, on peut citer les quatre principales classes : protéase aspartique, sérine, cystéine et metallo-protéase.

Comme protéase aspartique on peut citer notamment la HIV-1 protéase, la rénine, les plasmepsines, la cathepsine D.

Comme sérine protéase on peut citer notamment la thrombine, le facteur Xa, l'élastase, la tryptase, les "complement de convertases", la protéase NS3 l'hépatite C.

Parmi les cystéine-protéases, il existe trois groupes structurellement distincts, le groupe papaine et les cathepsines, le groupe ICE (les caspases) et le groupe picorna-viral (semblable aux sérine-protéases dans lesquelles la sérine est remplacée par une cystêïne).

Ainsi, on peut citer notamment la cathepsine K, la cathepsine B, la cathepsine L, la cathepsine S, les caspases, le rhinovirus 3C protéase et les calpaines.

Comme metalloprotéase, on peut citer notamment l'enzyme de conversion de l'Angiotensine, l'Endopeptidase neutre et le mélange des deux, la matrix metalloprotéase ainsi que la Tumor-necrosis Factor-α-Converting Enzyme.

On peut citer particulièrement la cathepsine K, la cathepsine B, la cathepsine S, la cathepsine L et la papaine et plus particulièrement la cathepsine K, la cathepsine B et la papaine.

Ces enzymes kinases ou protéases sont impliquées dans des processus de catabolisation et de communication inter et intracellulaire : elles jouent un rôle important dans un grand nombre de maladies de domaines différents tels que notamment le domaine cardiovasculaire, l'oncologie, le système nerveux central, l'inflammation, l'ostéoporose et également les maladies infectieuses, parasitaires, fongiques ou virales. C'est pourquoi ces protéines sont des cibles de grand intérêt pour la recherche pharmaceutique. Dans le cadre de la présente invention, partant d'un intermédiaire peptidomimétique, une chimiothèque dirigée d'inhibiteurs potentiels de ces enzymes a été conçue et construite : un intermédiaire peptidomimétique correspond ici au produit de formule (II) tel que défini ci-après. Les produits de la présente invention tels que définis ci-dessus et ci-après possèdent des propriétés inhibitrices d'enzymes métaboliques telles que définies ci-dessus notamment de kinases ou de protéases comme notamment les cystéine protéases ou sérine protéases.
Les produits de la présente invention peuvent ainsi notamment être utiles dans la prévention ou le traitement de maladies dans lesquelles de telles enzymes métaboliques sont impliquées comme certaines maladies cardiovasculaires, maladies du système nerveux central, maladies inflammatoires, maladies de l'os telles que par exemple l'ostéoporose, maladies infectieuses nécessitant notamment pour leur thérapie des anti-infectieux ou encore certains cancers.

La présente invention a ainsi pour objet les produits de formule (I) : 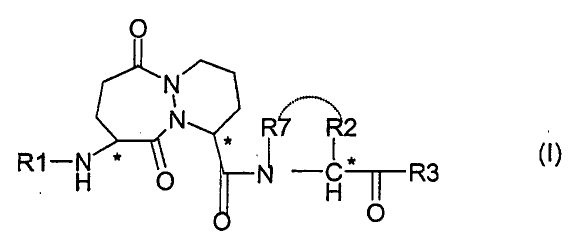 dans laquelle :
R₁ représente le radical -C(O)-R5, -SO2-R5 ou -C(O)-NR6R5 dans lesquels R6 représente un atome d'hydrogène ou un radical alkyle linéaire ou ramifié renfermant au plus 4 atomes de carbone et R5 représente un radical alkyle linéaire ou ramifié renfermant au plus 6 atomes de carbone, cycloalkyle renfermant au plus 6 atomes de carbone, phényle, naphtyle ou un radical hétérocyclique monocyclique à 5 ou 6 chaînons saturé ou insaturé contenant un ou plusieurs hétéroatomes identiques ou différents choisis parmi O, N ou S,
les radicaux alkyle, phényle et hétérocyclique tels que définis ci-dessus pour R1 , étant éventuellement substitués par un ou plusieurs radicaux choisis parmi les atomes d'halogène et les radicaux alkyle ou alcoxy linéaires ou ramifiés renfermant au plus 4 atomes de carbone, hydroxyle, acyle renfermant au plus 7 atomes de carbone, trifluorométhyle, cyano, thiényle, phényle, phenoxy, et isoxazolyle ;
R2 et R7 sont tels que
   soit R7 représente un atome d'hydrogène et
   R2 est tel que le groupement 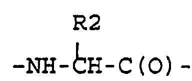 dans les produits de formule (I) représente le reste d'un acide aminé dit naturel ou dit non naturel de structure 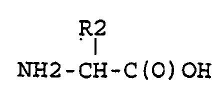 à l'exception de l'acide aspartique ,
   soit R2 et R7 forment un cycle ensemble avec les atomes d'azote et de carbone auxquels ils sont liés de telle manière que le groupement ainsi formé dans les produits de formule (I) : 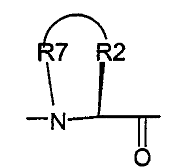 représente le reste d'un acide aminé dit naturel ou dit non naturel de structure : 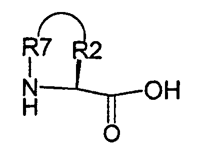
R3 représente le radical -CH=N2 ou le radical -CH2-L-R4 dans lequel L représente une simple liaison ou un radical divalent choisi parmi -O-, -O-C(O)-, -NH- et -S-(CH2)n-, avec n représente un entier de 0 à 6,
et R4 représente un radical alkyle linéaire ou ramifié renfermant au plus 6 atomes de carbone, un radical carbocyclique ou hétérocyclique monocyclique ou bicyclique renfermant de 5 à 10 chaînons, saturé ou insaturé, contenant un ou plusieurs hétéroatomes identiques ou différents choisis parmi O, N, NH ou S et pouvant contenir un chaînon -C(O),
les radicaux alkyle, carbocyclique et hétérocyclique tels que définis ci-dessus pour R4, étant éventuellement substitués par un ou plusieurs radicaux choisis parmi les atomes d'halogène ; les radicaux hydroxyle; carboxy libre, salifié ou estérifié ; -C(O)-NH2 , -C(O)-NH(alkyl), - C(O)-N(alkyl)(alkyl), -NH-C(O)-(alkyl), -N(alkyl)-C(O)-(alkyl) ; thiényle; phényle ; alkylphényle ; alkyle et alcoxy eux-mêmes éventuellement substitués par un ou plusieurs radicaux choisis parmi les radicaux acyl renfermant au plus 7 atomes de carbone, cyano, -NH2, -NH(alkyl), -N(alkyl)(alkyl) et phényle, étant entendu que dans les radicaux ci-dessus, les radicaux alkyle et alcoxy sont linéaires ou ramifiés et renferment au plus 4 atomes de carbone,
   le signe * indiquant les atomes de carbone qui peuvent être sous configuration S ou R,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

Dans les produits de formule (I) et dans ce qui suit :
- le terme radical alkyle linéaire ou ramifié désigne les radicaux méthyle, éthyle, propyle, isopropyle, butyle, isobutyle, sec-butyle, tert-butyle, pentyle, isopentyle, hexyle, isohexyle et également heptyle, octyle, nonyle et décyle ainsi que leurs isomères de position linéaires ou ramifiés : parmi ces radicaux alkyle, on peut choisir de préférence les radicaux alkyle qui renferment au plus 6 atomes de carbone ou ceux qui renferment au plus 4 atomes de carbone
- le terme radical alcoxy linéaire ou ramifié désigne les radicaux méthoxy, éthoxy, propoxy, isopropoxy, butoxy linéaire, secondaire ou tertiaire, pentoxy ou hexoxy ainsi que leurs isomères de position linéaires ou ramifiés : parmi ces radicaux alcoxy, on peut choisir de préférence les radicaux alcoxy qui renferment au plus 6 atomes de carbone ou ceux qui renferment au plus 4 atomes de carbone
- le terme atome d'halogène désigne les atomes de chloré, de brome, d'iode ou de fluor et de préférence l'atome de chlore, de brome ou d'iode
- le terme radical cycloalkyle désigne les radicaux cyclopropyle, cyclobutyle, cyclopentyle et cyclohexyle et tout particulièrement les radicaux cyclopentyle et cyclohexyle,
- le terme radical hétérocyclique monocyclique désigne un radical saturé ou insaturé constitué de 5 ou 6 chaînons tel que l'un ou plusieurs des chaînons représente un atome d'oxygène, de soufre ou d'azote : un tel radical hétérocyclique désigne ainsi un radical carbocyclique interrompu par un ou plusieurs hétéroatomes choisis parmi les atomes d'oxygène, d'azote ou de soufre étant entendu que les radicaux hétérocycliques peuvent renfermer un ou plusieurs hétéroatomes choisis parmi les atomes d'oxygène, d'azote ou de soufre et que lorsque ces radicaux hétérocycliques comportent plus d'un hétéroatome, les hétéroatomes de ces radicaux hétérocycliques peuvent être identiques ou différents. On peut citer notamment le radical dioxolane, dioxane, dithiolane, thiooxolane, thiooxane, morpholinyle, pipérazinyle, pipérazinyle substitué par un radical alkyle, linéaire ou ramifié, renfermant au plus 4 atomes de carbone, pipéridyle, thiényle tel que 2-thiényle et 3-thiényle, furyle tel que 2- furyle, pyrimidinyle, pyridyle tel que 2-pyridyle, 3-pyridyle et 4-pyridyle pyrimidyle, pyrrolyle, thiazolyle, isothiazolyle, diazolyle, thiadiazolyle, triazolyle, tétrazolyle libre ou salifié thiadiazolyle, thiatriazolyle, oxazolyle, oxadiazolyle, 3- ou 4-isoxazolyle. On peut citer tout particulièrement les radicaux morpholinyle, thiényle tel que 2-thiényle et 3-thiényle, furyle tel que 2-furyle , tétrahydrofuryle, thiényle, tétrahydrothienyle, pyrrolyle, pyrrolinyle, pyridyle et pyrrolidinyle.
- le terme radical hétérocyclique bicyclique désigne un radical saturé ou insaturé constitué de 8 à 12 chaînons tel que l'un ou plusieurs des chaînons représente un atome d'oxygène, de soufre ou d'azote et notamment des groupes hétérocycliques condensés contenant au moins un hétéroatome choisi parmi le soufre, l'azote et l'oxygène, par exemple benzothiényle tel que 3-benzothiényle, benzothiazolyle, quinolyle, tetralone, benzofuryle, benzopyrrolyle, benzimidazolyle, benzoxazolyle, thionaphtyle, indolyle ou purinyle.
- Le terme radical carbocyclique ou hétérocyclique monocyclique ou bicyclique renfermant de 5 à 10 chaînons, saturé ou insaturé, contenant un ou plusieurs hétéroatomes identiques ou différents choisis parmi O, N, NH ou S, et pouvant contenir un chaînon -C(O), regroupe les définitions indiquées ci-dessus d'une part pour le terme radical hétérocyclique monocyclique et d'autre part pour le terme radical hétérocyclique bicyclique, tous ces radicaux étant éventuellement substitués. On peut noter qu'un radical carbocyclique tel que défini ci-dessus est notamment le radical phényle et qu'un radical carbocyclique contenant un chaînon -C(O) est par exemple le radical tétralóne.
- le terme alkylphényle désigne un radical phényle substitué par un ou plusieurs radicaux alkyle tels que définis ci-dessus linéaires ou ramifiés de préférence renfermant au plus 4 atomes de carbone
   les termes NH(alk) et N(alk)(alk) désigne un radical amino substitués respectivement par un ou deux radicaux alkyle, de tels radicaux alkyle étant linéaires ou ramifiés et renfermant de préférence au plus 4 atomes de carbone le terme acylamino désigne les radicaux -C(O)-NH2, -C(O)-NH(alk) et -C(O)-N(alk)(alk) dans lesquels les radicaux NH(alk) et N(alk)(alk) ont la signification indiquée ci-dessus
- le terme acyle désigne un radical R-C(O)- dans lequel R représente un radical choisi parmi l'atome d'hydrogène, les radicaux alkyle linéaire ou ramifié renfermant au plus 6 atomes de carbone, un radical phényle ou un radical pyrrolidinyle : le terme acyle désigne ainsi notamment les radicaux formyle, les radicaux acétyle, propionyle, butanoyie, pentanoyl, hexanoyl, benzoyle et pyrrolidinylcarbonyle
   le terme alkényle désigne des radicaux linéaire ou ramifié renfermant au plus 6 atomes de carbone : on peut citer notamment les radicaux vinyle, 1-propényle, allyle, butényle, 3-méthyle-2-butényle
- le terme alkylthio désigne des radicaux linéaire ou ramifié renfermant au plus 6 atomes de carbone tels que notamment les radicaux méthylthio, éthylthio, propylthio, isopropylthio, butylthio, isobutylthio, sec-butylthio, tert-butylthio, pentylthio, isopentylthio, hexylthio ou encore isohexylthio ainsi que leurs isomères de position linéaires ou ramifiés : parmi ces radicaux alkylthio, on choisit de préférence parmi ceux cités ci-dessus, ceux qui renferment au plus 4 atomes de carbone

Les acides aminés dits naturels ou dits non naturels sont connus de l'homme du métier et peuvent être trouvés dans des documents usuels tels que par exemple le document 'Amino Acids (1999), 16(3-4), 345-379.
Le ou les radicaux carboxy des produits de formule (1) peuvent être salifiés ou estérifiés par les groupements divers connus de l'homme du métier parmi lesquels on peut citer, par exemple :
- parmi les composés de salification, des bases minérales telles que, par exemple, un équivalent de sodium, de potassium, de lithium, de calcium, de magnésium ou d'ammonium ou des bases organiques telles que, par exemple, la méthylamine, la propylamine, la triméthylamine, la diéthylamine, la triéthylamine, la N,N-diméthyléthanolamine, le tris (hydroxyméthyl)amino méthane, l'éthanolamine, la pyridine, la picoline, la dicyclohexylamine, la morpholine, la benzylamine, la procaïne, la lysine, l'arginine, l'histidine, la N-méthylglucamine,
- parmi les composés d'estérification, les radicaux alkyle pour former des groupes alcoxy carbonyle tel que, par exemple, méthoxycarbonyle, éthoxycarbonyle, tert-butoxycarbonyle ou benzyloxycarbonyle, ces radicaux alkyles pouvant être substitués par des radicaux choisis par exemple parmi les atomes d'halogène, les radicaux hydroxyle, alcoxy, acyle, acyloxy, alkylthio, amino ou aryle comme, par exemple; dans les groupements chlorométhyle, hydroxypropyle, méthoxyméthyle, propionyloxyméthyle, méthylthiométhyle, diméthylaminoéthyle, benzyle ou phénéthyle.
Les sels d'addition avec les acides minéraux ou organiques des produits de formule (1) peuvent être, par exemple, les sels formés avec les acides chlorhydrique, bromhydrique, iodhydrique, nitrique, sulfurique, phosphorique, propionique, acétique, trifluoroacétique, formique, benzoïque, maléique, fumarique, succinique, tartrique, citrique, oxalique, glyoxylique, aspartique, ascorbique, les acides alcoylmonosulfoniques tels que par exemple l'acide méthanesulfonique, l'acide éthanesulfonique, l'acide propanesulfonique, les acides alcoyldisulfoniques tels que par exemple l'acide méthanedisulfonique, l'acide alpha, bêta-éthanedisulfonique, les acides arylmonosulfoniques tels que l'acide benzènesulfonique et les acides aryldisulfoniques. On peut rappeler que la stéréoisomérie peut être définie dans son sens large comme l'isomérie de composés ayant mêmes formules développées, mais dont les différents groupes sont disposés différemment dans l'espace, tels que notamment dans des cyclohexanes monosubstitués dont le substituant peut être en position axiale ou équatoriale, et les différentes conformations rotationnelles possibles des dérivés de l'éthane. Cependant, il existe un autre type de stéréoisomérie, dû aux arrangements spatiaux différents de substituants fixés, soit sur des doubles liaisons, soit sur des cycles, que l'on appelle souvent isomérie géométrique ou isomérie cis-trans. Le terme stéréoisomères est utilisé dans la présente demande dans son sens le plus large et concerne donc l'ensemble des composés indiqués ci-dessus.
On peut noter que l'on préfère les produits de formule (I) telle que définie ci-dessus dans laquelle les atomes de carbone indiqués par le signe * sont sous la configuration stéréochimique S.
0n peut noter que dans les produits de formule (I) telle que définie ci-dessus, le troisième atome de carbone indiqué par le signe * sur la formule des produits de formule (I), ne possède évidemment pas de stéréochimie particulière R ou S lorsque R2 représente l'atome d'hydrogène.

La présente invention a ainsi plus particulièrement pour objet les produits de formule (I) telle que définie ci-dessus dans laquelle R1 et R3 ont les significations indiquées ci-dessus et R2 représente le reste d'un acide aminé dit naturel à l'exception de l'acide aspartique,
lesdits produits de formule (I) étant sous toutes les formes. isomères possibles racémiques, énantiomères et diastéréoisomères, ainsi que les sels d'addition avec les acides minéraux et organiques ou avec les bases minérales et organiques desdits produits de formule (I).

La présente invention a ainsi encore plus particulièrement pour objet les produits de formule (I) telle que définie ci-dessus dans laquelle R1 et R3 ont les significations indiquées ci-dessus et R2 et R7 sont tels que :
soit R7 représente un atome d'hydrogène et R2 représente un atome d'hydrogène ou un radical alkyle linéaire ou ramifié renfermant au plus 6 atomes de carbone éventuellement substitué par un ou plusieurs radicaux choisis parmi les radicaux amino ; acylamino; NH=C(NH2)-NH- ; mercapto ;alkylthio linéaire ou ramifié renfermant au plus 4 atomes de carbone ; hydroxyle ; alcoxy linéaire ou ramifié renfermant au plus 4 atomes de carbone ; imidazolyle ; indolyle ; phényle lui-même éventuellement substitué par un ou plusieurs radicaux choisis parmi les atomes d'halogène, le radical hydroxyle, un radical alcoxy linéaire ou ramifié renfermant au plus 4 atomes de carbone et le radical phénoxy lui-même éventuellement substitué par un ou plusieurs radicaux choisis parmi les atomes d'iode et le radical hydroxyle ; et le radical -C(O)-O-R8 dans lequel R8 représente un atome d'hydrogène ou un radical alkyle ou alkényle linéaire ou ramifié renfermant au plus 6 atomes de carbone,
soit R2 et R7 forment ensemble avec les atomes d'azote et de carbone auxquels ils sont liés un cycle comprenant 5 à 7 chaînons éventuellement substitué par un ou plusieurs radicaux hydroxyle ou alcoxy linéaire ou ramifié renfermant au plus 4 atomes de carbone,
lesdits produits de formule (I) étant sous toutes les formes isomères possibles racémiques, énantiomères et diastéréoisomères, ainsi que les sels d'addition avec les acides minéraux et organiques ou avec les bases minérales et organiques. desdits produits de formule (I).

La présente invention a notamment pour objet les produits de formule (I) telle que définie ci-dessus dans laquelle R1 et R3 ont les significations indiquées ci-dessus et R2 et R7 sont tels que :
soit R7 représente un atome d'hydrogène et R2 a la signification indiquée ci-dessus
soit R2 et R7 forment ensemble avec les atomes d'azote et de carbone un cycle pyrrolidinyle éventuellement substitué par un ou plusieurs radicaux hydroxyle ou alcoxy linéaire ou ramifié renfermant au plus 4 atomes de carbone,
lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

On peut citer notamment les produits de formule (I) telle que définie ci-dessus dans lesquels R2 et R7 forment ensemble avec les atomes d'azote et de carbone auxquels ils sont liés, un cycle pyrrolidinyle, le reste d'acide aminé étant alors la proline, ou un cycle pyrrolidinyle substitué par un radical hydroxyle éventuellement protégé, le reste d'acide aminé étant alors l'hydroxyproline.

La présente invention a notamment pour objet les produits de formule (I) telle que définie ci-dessus répondant à la formule (Ia): 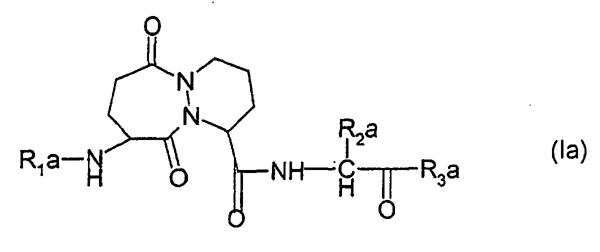 dans laquelle :
R₁ₐ représente le radical -C(O)-R5a, -SO2-R5a ou -C(O)-NR6aR5a dans lesquels R6a représente un atome d'hydrogène ou un radical alkyle linéaire ou ramifié renfermant au plus 4 atomes de carbone et R5a représente un radical choisi parmi les radicaux alkyle linéaire ou ramifié renfermant au plus 6 atomes de carbone ; cycloalkyle renfermant au plus 6 atomes de carbone ; phényle ; naphtyle ; furyle ; morpholinyle ; thiényle ; pyridyle,
   les radicaux alkyle, phényle, thiényle et pyridyle étant éventuellement substitués par un ou plusieurs radicaux choisis parmi les atomes d'halogène et les radicaux alkyle ou alcoxy linéaires ou ramifiés renfermant au plus 4 atomes de carbone, hydroxyle, acyle renfermant au plus 7 atomes de carbone, phénoxy, trifluorométhyle, cyano, thiényle, phényle et isoxazolyle ;
R2a a la signification indiquée ci-dessus lorsque R7 représente un atome d'hydrogène,
R3a représente le radical -CH=N2 ou le radical -CH2-La-R4a dans lequel La représente une simple liaison ou un radical divalent choisi parmi -O-, -O-C(O)- et -S-(CH2)na-, avec na représente un entier de 0 à 3,
et R4a représente un radical choisi parmi les radicaux; alkyle linéaire ou ramifié renfermant au plus 6 atomes de carbone ; phényle ; tétrazolyle ; pipérazinyle ; pipéridyle ; pyridyle ; benzothiazolyle ; quinolyle ; thiadiazolyle ; pyrimidinyle ; tétralone ;
   les radicaux alkyle, phényle, tétrazolyle, pipérazinyle et pipéridyle tels que définis ci-dessus pour R4a, étant éventuellement substitués par un ou plusieurs radicaux choisis parmi les atomes d'halogène ; les radicaux hydroxyle; carboxy libre, salifié ou estérifié ; -C(O)-NH2 ; -C(O)-NH(alkyl) ; -C(O)-N(alkyl)(alkyl) ; -NH-C(O)-(alkyl) ; -N(alkyl)-C(O)-(alkyl) ; thiényle; phényle ; alkylphényle ; alkyle et alcoxy eux-mêmes éventuellement substitués par un ou plusieurs radicaux choisis parmi les radicaux acyl renfermant au plus 7 atomes de carbone, cyano, -NH2, -NH(alkyl), -N(alkyl)(alkyl) et phényle,
étant entendu que dans les radicaux ci-dessus, les radicaux alkyle et alcoxy sont linéaires ou ramifiés et renferment au plus 4 atomes de carbone,
lesdits produits de formule (Ia) étant sous toutes les formes isomères possibles racémiques, énantiomères et diastéréoisomères, ainsi que les sels d'addition avec les acides minéraux et organiques ou avec les bases minérales et organiques desdits produits de formule (Ia).

La présente invention a plus particulièrement pour objet les produits de formule (I) telle que définie ci-dessus répondant à la formule (Ib): 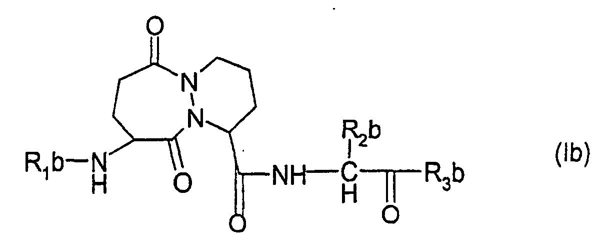 dans laquelle :
R_{1b} représente le radical -C(O)-R5b, -SO2-R5b ou -C(O)-NR6bR5b dans lesquels R6b représente un atome d'hydrogène ou un radical méthyle et R5b représente un radical choisi parmi les radicaux alkyle linéaire ou ramifié renfermant au plus 4 atomes de carbone éventuellement substitué par un radical thiényle ; cyclohexyle ; phényle éventuellement substitué par un radical alcoxy linéaire ou ramifié renfermant au plus 4 atomes de carbone, un radical phénoxy ou trifluorométhyle ; naphtyle ; furyle ; morpholinyle ; thiényle éventuellement substitué par un radical isoxazolyle ; pyridyle éventuellement substitué par un radical trifluorométhyle
R2b représente un atome d'hydrogène ou un radical alkyle linéaire ou ramifié renfermant au plus 6 atomes de carbone éventuellement substitué soit par le radical phényle lui-même éventuellement substitué par les radicaux hydroxyle ou alcoxy linéaire ou ramifié renfermant au plus 4 atomes de carbone, soit par le radical -C(O)-O-R8b dans lequel R8b représente un radical alkyle ou alkényle linéaire ou ramifié renfermant au plus 6 atomes de carbone,
R3b représente le radical -CH=N2 ou le radical -CH2-Lb-R4b dans lequel Lb représente une simple liaison ou un radical divalent choisi parmi -O-, -O-C(O)- et -S-(CH2)nb-, avec nb représente l'entier 0 ou 1,
et R4b représente un radical choisi parmi les radicaux pyridyle ; benzothiazolyle ; quinolyle ; thiadiazolyle ; pyrimidinyle ; tétralone ; alkyle linéaire ou ramifié renfermant au plus 4 atomes de carbone éventuellement substitué par un radical carboxy libre, salifié ou estérifié ou thiényle ; phényle éventuellement substitué par un ou plusieurs radicaux choisis parmi les atomes d'halogène, les radicaux carboxy libre, salifié ou estérifié, les radicaux alkyle et alcoxy linéaires ou ramifiés renfermant au plus 4 atomes de carbone, cyanoalkyle, alkylphényle, -NH-C(O)-CH3, -C(O)-N(alkyl)(alkyl) ; tétrazolyle éventuellement substitué par un radical phényle ; pipérazinyle éventuellement substitué sur le second atome d'azote par un radical phényle ou alkyle linéaire ou ramifié renfermant au plus 4 atomes de carbone lui-même éventuellement substitué par un radical acyl (pyrrolidinylcarbonyl), un ou deux radicaux phényle ou un radical -NH2, -NH(alkyl) ou -N(alkyl)(alkyl) ; pipéridyle éventuellement substitué par un radical benzyle ou -C(O)- N(alkyl) (alkyl) , étant entendu que dans les radicaux ci-dessus, les radicaux alkyle et alcoxy sont linéaires ou ramifiés et renferment au plus 4 atomes de carbone,
lesdits produits de formule (Ib) étant sous toutes les formes isomères possibles racémiques, énantiomères et diastéréoisomères, ainsi que les sels d'addition avec les acides minéraux et organiques ou avec les bases minérales et organiques desdits produits de formule (Ib).

La présente invention a spécialement pour objet les produits de formule (I) telle que définie ci-dessus dans laquelle R1 représente un radical choisi parmi les radicaux suivants : 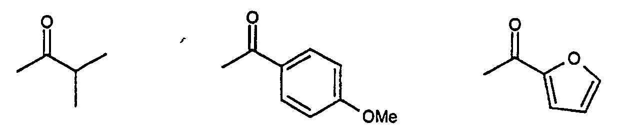 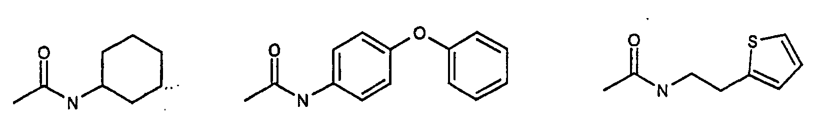 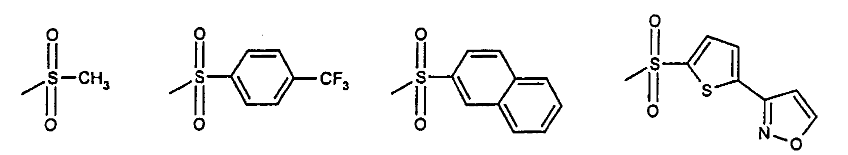 R2, R3 et R7 ayant les valeurs indiquées ci-dessus, ceux dans lesquels R7 représente un atome d'hydrogène et R2 représente un radical choisi parmi les radicaux suivants : 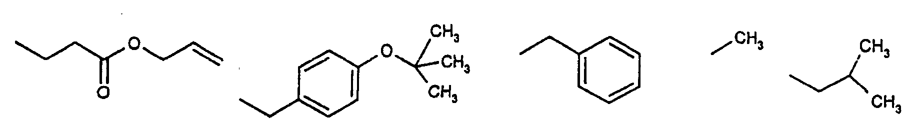 R1 et R3 ayant les valeurs indiquées ci-dessus et ceux dans lesquels R3 représente un radical choisi parmi les radicaux suivants : 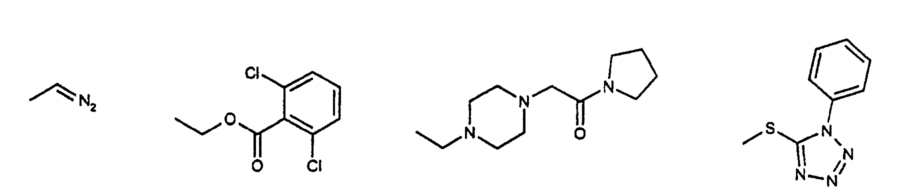 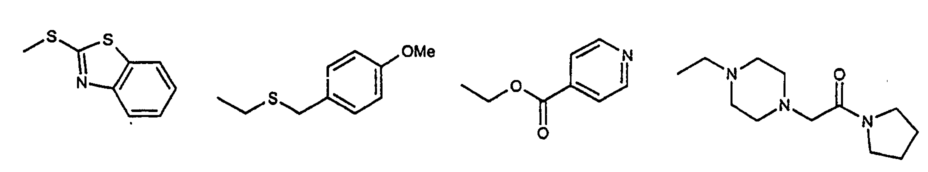 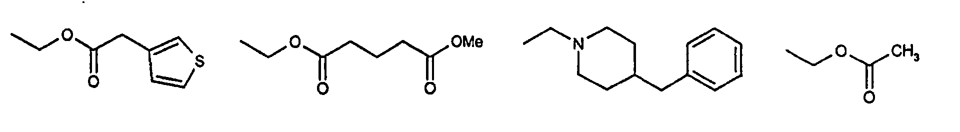 R1, R2 et R7 ayant les valeurs indiquées ci-dessus, lesdits produits de formule (Ib) étant sous toutes les formes isomères possibles racémiques, énantiomères et diastéréoisomères, ainsi que les sels d'addition avec les acides minéraux et organiques ou avec les bases minérales et organiques desdits produits de formule (Ib).

Dans les produits de formule (I) de la présente invention, R₂ représente notamment un reste d'acide aminé choisi parmi les acides aminés naturels : GLU, GLY, ALA, VAL, LEU, PHE, TYR ou TYR(OtBU). TYR(OtBU) représente l'acide aminé TYR dans lequel le radical hydroxyle est protégé en radical terbutoxy.

La présente invention a tout particulièrement pour objet les produits de formule (I) telle que définie ci-dessus, répondant aux formules suivantes :
- le 3-[ 9(S)- benzoylamino -6,10-dioxo-1,2,3,4,7,8,9,10-octahydro -6H-pyridazino[1,2-a][1,2]diazepine-1(S)-carboxamide]-5-méthyl-1-benzoyloxy-hexane-2-one
- le 3-[ 9(S)- (2-furanoyl)amino -6,10-dioxo-1,2,3,4,7,8,9,10- octahydro -6H-pyridazino[1,2-a][1,2]diazepine-1(S)-carboxamide]-5-méthyl-1-(2,6-dichlorobenzoyloxy-hexane-2-one
- le 3(S)-[ 9(S)- (2-furanoyl)amino -6,10-dioxo-1,2,3,4,7,8,9,10- octahydro -6H-pyridazino[1,2-a][1,2]diazepine-1(S)-carboxamide]-4-(4-hydroxyphényl)-1-(2,6-dichlorobenzoyloxy)-butane-2-one

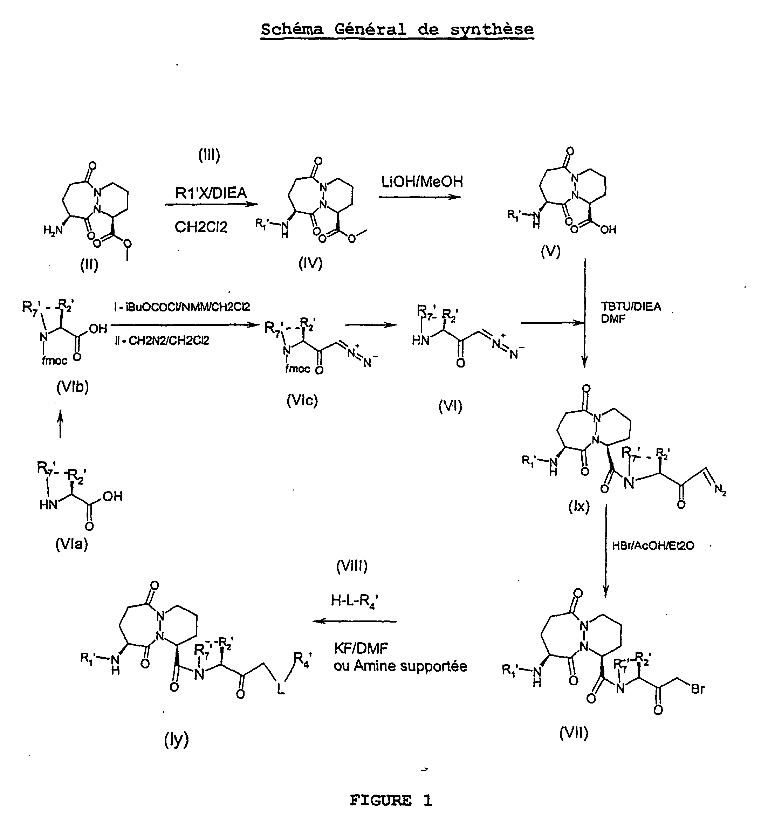

La présente invention a également tout particulièrement pour objet les produits de formule (I) telle que définie ci-dessus répondant aux formules suivantes :
- (4S) 6-diazo-4-[[[(1S,9S)-9-[(2-methyl-1-oxo-propyl)amino]-octahydro-6,10-dioxo-6H-pyridazino[1,2-a][1,2]diazepine-1-yl]carbonyl]amino]-5-oxo-Hexanoate de (2-propenyle)
- (4S) 6-diazo-4-[[[(1S,9S)-9-[(3-methoxybenzoyl)amino]-octahydro-6,10-dioxo-6H-pyridazino[1,2-a][1,2]diazepine-1-yl]carbonyl]amino]-5-oxo-Hexanoate de (2-propenyle)
- (4S) 6-diazo-4-[[[(1S,9S)-9-[[(2-furanyl)carbonyl] amino]-octahydro-6,10-dioxo-6H-pyridazino[1,2-a] [1,2]diazepine-1-yl]carbonyl]amino]-5-oxo-Hexanoate de(2-propenyle)
- (4S) 6-diazo-4-[[[(1S,9S)-9-[[(cyclohexylamino) carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-diazo-4-[[[(1S,9S)-9-[[[(4-phenoxyphenyl)amino] carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-diazo-4-[[[(1S,9S)-9-[[[[2-(p-2-yl)-ethyl]amino]carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-diazo-4-[[[(1S,9S)-9-[(methylsulfonyl)amino]-octahydro-6,10-dioxo-6H-pyridazino[1,2-a][1,2]diazepine-1-yl]carbonyl]amino]-5-oxo-Hexanoate de (2-propenyle)
- (4S) 6-diazo-4-[[[(1S,9S)-9-[[[4-(trifluoromethyl) phenyl]sulfonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-diazo-4-[[[(1S,9S)-9-[[(2-naphthalenyl) sulfonyl]amino]-octahydro-6,10-dioxo-6H-pyridazino[1,2-a][1,2]diazepine-1-yl]carbonyl]amino]-5-oxo-Hexanoate de(2-propenyle)
- (4S) 6-diazo-4-[[[(1S,9S)-9-[[[5-(isoxazol-3-yl)-2-thienyl]sulfonyl]amino]-octahydro-6,10-dioxo-6H-pyridazino[1,2-a][1,2]diazepine-1-yl]carbonyl]amino]-5-oxo-Hexanoate de (2-propenyle)
- 9-[(9S)-[[(4-phenoxyphenyl)amino]carbonyl]amino]-N-[(2S)-4-diazo-1-[4-[(1,1-dimethyl)ethoxy]phenyl]-3-oxo-2-butyl]-6H-(1S)-pyridazino[1,2-a][1,2]diazepine-1-carboxamide
- (4S) 6-[(2,6-dichlorobenzoyl)oxy] -4-[[[(1S,9S)-9-[[(2-furanyl)carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[(2,6-dichlorobenzoyl)oxy]-4-[[[(1S,9S)-9-[[[4-(trifluoromethyl)phenyl]sulfonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[4-[[2-oxo-2-(1-pyrrolidinyl)ethyl]piperazin-1-yl]-4-[[[(1S,9S)-9-[[[4-(trifluoromethyl)phenyl] sulfonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[4-[[2-oxo-2-(1-pyrrolidinyl)ethyl]piperazin-1-yl]-4-[[[(1S,9S)-9-[[(2-naphthalenyl)sulfonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[(2,6-dichlorobenzoyl)oxy] -4-[[[(1S,9S)-9-[[(cyclohexylamino)carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[(1-phenyl-1H-tetrazol-5-yl)thio]-4-[[[(1S,9S)-9-[[[(4-phenoxyphenyl)amino]carbonyl]amino]-octahydro-6,10-dioxo-6H-pyridazino[1,2-al[1,2]diazepine-1-yl]carbonyl]amino]-5-oxo-Hexanoate de (2-propenyle)
- (4S) 6-[(benzothiazol-2-yl)thio]-4-[[[(1S,9S)-9-[[[(4-phenoxyphenyl)amino]carbonyl]amino]-octahydro-6,10-dioxo-6H-pyridazino[1,2-a][1,2]diazepine-1-yl] carbonyl]amino]-5-oxo-Hexanoate de (2-propenyle)
- (4S) 6-[[(4-methoxyphenyl)methyl]thio]-4-[[[(1S,9S)-9-[[[(4-phenoxyphenyl)amino]carbonyl]amino]-octahydro-6,10-dioxo-6H-pyridazino[1,2-a][1,2]diazepine-1-yl] carbonyl]amino]-5-oxo-Hexanoate de (2-propenyle)
- (4S) 6-[[(4-pyridinyl)carbonyl]oxy]-4-[[[(1S,9S)-9-[[[(4-phenoxyphenyl)amino]carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[(2,6-dichlorobenzoyl)oxy]-4-[[[(1S,9S)-9-[[[(4-phenoxyphenyl)amino]carbonyl]amino]-octahydro-6,10-dioxo-6H-pyridazino[1,2-a][1,2]diazepine-1-yl] carbonyl]amino]-5-oxo-Hexanoate de (2-propenyle)
- ((4S) 6-acetyloxy-4-[[[(1S,9S)-9-[[[(4-phenoxyphenyl)amino]carbonyl]amino]-octahydro-6,10-dioxo-6H-pyridazino [1,2-a][1,2]diazepine-1-yl]carbonyl]amino] -5-oxo-Hexanoate de (2-propenyle)
- (4S) 6-[1-oxo-2-(thien-3-yl)ethoxy]-4-[[[(1S,9S)-9-[[[(4-phenoxyphenyl)amino]carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[(1-phenyl-1H-tetrazol-5-yl)thio]-4-[[[(1S,9S)-9-[[[[2-(thien-2-yl)-ethyl]amino]carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[(2,6-dichlorobenzoyl)oxy]-4-[[[(1S,9S)-9-[[[[2-(thien-2-yl)-ethyl]amino]carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[(2,6-dichlorobenzoyl)oxy]-4-[[[(1S,9S)-9-[(methylsulfonyl)amino]-octahydro-6,10-dioxo-6H-pyridazino[1,2-a] [1,2]diazepine-1-yl]carbonyl]amino]-5-oxo-Hexanoate de (2-propenyle)
- 9-[(9S) [[(4-phenoxyphenyl)amino]carbonyl]amino]-N-((2S)-4-diazo-1-phenyl-3-oxo-2-butyl)- octahydro-6,10-dioxo -6H-(1S)-pyridazino[1,2-a][1,2]diazepine-1-carboxamide
- 9-[(9S)-[[[2-(thien-2-yl)-ethyl]amino]carbonyl]amino]-N-((2S)-4-diazo-1-phenyl-3-oxo-2-butyl)- octahydro-6,10-dioxo-6H-(1S)-pyridazino[1,2-a][1,2]diazepine-1-carboxamide
- 9-[(9S)-[(2-naphthalenyl)sulfonyl]amino ]-N-((2S)-4-diazo-1-phenyl-3-oxo-2-butyl)- octahydro-6,10-dioxo-6H-(1S)-pyridazino[1,2-a] [1,2]diazepine-1-carboxamide
- 9-[(9S)-[[5-(isoxazol-3-yl)-2-thienyl]sulfonyl]amino]-N-((2S)-4-diazo-1-phenyl-3-oxo-2-butyl)- octahydro-6,10-dioxo-6H-(1S)-pyridazino[1,2-a][1,2]diazepine-1-carboxamide
- 9-[(9S)-[[(4-phenoxyphenyl)amino]carbonyl]amino]-N-((2S)-4-diazo-3-oxo-2-butyl)- octahydro-6,10-dioxo -6H-(1S)-pyridazino[1,2-a][1,2]diazepine-1-carboxamide
- 9-[(9S)-[[[3-(thien-2-yl)-ethyl]amino]carbonyl]amino]-N-((2S)-4-diazo-3-oxo-2-butyl)- octahydro-6,10-dioxo -6H-(1S)-pyridazino[1,2-a][1,2]diazepine-1-carboxamide
- 9-[(9S)-(3-methoxybenzoyl)amino]-N-((2S)-4-[(2,6-dichlorobenzoyl)oxy]-1-phenyl-3-oxo-2-butyl]- octahydro-6,10-dioxo-6H-(1S)-pyridazino[1,2-a][1,2] diazepine-1-carboxamide
- 9-[(9S)-[(2-furanyl)carbonyl]amino]-N-[(2S)-4-[(2,6-dichlorobenzoyl)oxy] -1-phenyl-3-oxo-2-butyl]- octahydro-6,10-dioxo-6H-(1S)-pyridazino[1,2-a][1,2] diazepine-1-carboxamide
- 9-[(9S)-[(cyclohexylamino)carbonyl]amino]-N-[(2S)-4-[(2,6-dichlorobenzoyl)oxy] -1-phenyl-3-oxo-2-butyl]- octahydro-6, 10-dioxo-6H-(1S)-pyridazino [1,2-a][1,2] diazepine-1-carboxamide
- 9-[(9S)-[[(4-phenoxyphenyl)amino]carbonyl]amino]-N-[(2S)-1-phenyl-4-[(1-phenyl-1H-tetrazol-5-yl)thio]-3-oxo-2-butyl]-octahydro-6,10-dioxo-6H-(1S)-pyridazino[1,2-a][1,2]diazepine-1-carboxamide
- 9- [(9S)-[[(4-phenoxyphenyl)amino] carbonyl]amino]-N-[(2S)-1-phenyl-4-[1-oxo-2-(3-thienyl)ethoxy]-3-oxo-2-butyl]-octahydro-6,10-dioxo-6H-(1S)-pyridazino[1,2-a][1,2]diazepine-1-carboxamide
- Pentanedioate de (methyle) [2-oxo-4-phenyl-3[(3S)-[[(4-phenoxyphenyl)amino]carbonyl]amino]- octahydro-6,10-dioxo-6H-(1S)-pyridazino [1,2-a][1,2]diazepin-1-yl]carbonyl]amino]butyle]
- 9-[(9S)-[[(4-phenoxyphenyl)amino]carbonyl]amino]-N-[(2S)-4-[(4-phenylmethyl)-1-piperidinyl ]-1-phenyl-3-oxo-2-butyl]-octahydro-6,10-dioxo-6H-(1S)-pyridazino[1,2-a][1,2]diazepine-1-carboxamide
- 9-[(9S)-[[[2-(thien-2-yl)-ethyl]amino]carbonyl]amino]-N-[(2S)-1-phenyl-4-[(1-phenyl-1H-tetrazol-5-y1)thio]-3-oxo-2-butyl]- octahydro-6,10-dioxo-6H-(1S)-pyridazino [1,2-a][1,2]diazepine-1-carboxamide
- 9-[(9S)-[[[2-(thien-2-yl)-ethyl]amino]carbonyl]amino]-N-[(2S)-1-phenyl-4-[(2-benzothiazolyl)thio]-3-oxo-2-butyl]-octahydro-6,10-dioxo-6H-(1S)-pyridazino[1,2-a][1,2]diazepine-1-carboxamide
- 9-[(9S)-[[[2-(thien-2-yl)-ethyl]amino]carbonyl]amino]-N-[(2S)-1-phenyl-4-[(-2,6-dichlorobenzoyl)oxy] -3-oxo-2-butyl]-octahydro-6,10-dioxo-6H-(1S)-pyridazino[1,2-a][1,2]diazepine-1-carboxamide
- 9-[(9S)-[[[2-(thien-2-yl)-ethyl]amino]carbonyl]amino]-N-[(2S)-1-phenyl-4-[4-[2-(1-pyrrolidinyl)-2-oxoethyl]piperazin-1-yl]] -3-oxo-2-butyl]- octahydro-6,10-dioxo-6H-(1S)-pyridazino[1,2-a][1,2]diazepine-1-carboxamide
- 9-[(9S)-(methylsulfonyl)amino]-N-[(2S)-1-phenyl-4-[(-2,6-dichlorobenzoyl)oxy] -3-oxo-2-butyl]- octahydro-6,10-dioxo-6H-(1S)-pyridazino[1,2-a][1,2]diazepine-1-carboxamide
- 9-[(9S)-[[[4-(trifluoromethyl)phenyl]sulfonyl]amino]-N-[(2S)-1-phenyl-4-[(1-phenyl-1H-tetrazol-5-yl)thio]-3-oxo-2-butyl]- octahydro-6,10-dioxo-6H-(1S)-pyridazino [1,2-a][1,2]diazepine-1-carboxamide
- 9-[(9S)-[[[4-(trifluoromethyl)phenyl]sulfonyl]amino]-N-[(2S)-1-phenyl-4-(2-benzothiazolyl)thio)-3-oxo-2-butyl]-octahydro-6,10-dioxo-6H-(1S)-pyridazino[1,2-a][1,2]diazepine-1-carboxamide
- 9-[(9S)-(methylsulfonyl)amino]-N-[(3S)-5-methyl-1-[(2,6-dichlorobenzoyl)oxy] -2-oxo-3-hexyl]- octahydro-6,10-dioxo-6H-(1S)-pyridazino[1,2-a][1,2]diazepine-1-carboxamide
- 9-[(9S)-[[[4-(trifluoromethyl)phenyl]sulfonyl]amino]]-N-[(3S)-5-methyl-1-[(2,6-dichlorobenzoyl)oxy]-2-oxo-3-hexyl]-octahydro-6,10-dioxo-6H-(1S)-pyridazino[1,2-a][1,2]diazepine-1-carboxamide
- 9-[(9S)-[(2-naphthalenyl)sulfonyl]amino]-N-[(3S)-5-methyl-1-[(2,6-dichlorobenzoyl)oxy]-2-oxo-3-hexyl]- octahydro-6,10-dioxo-6H-(1S)-pyridazino[1,2-a][1,2]diazepine-1-carboxamide
- 9-[(9S)-(2-methyl-1-oxo-propyl)amino]-N-((3S)-1-diazo-5-methyl-2-oxo-3-hexyl)- octahydro-6,10-dioxo -6H-(1S)-pyridazino[1,2-a][1,2]diazepine-1-carboxamide
- 9-[(9S)- (3-methoxybenzoyl)amino]-N-((3S)-1-diazo-5-methyl-2-oxo-3-hexyl)- octahydro-6,10-dioxo -6H-(1S)-pyridazino[1,2-a][1,2]diazepine-1-carboxamide
- 9-[(9S)-[(2-furanyl)carbonyl]amino]-N-((3S)-1-diazo-5-methyl-2-oxo-3-hexyl)- octahydro-6,10-dioxo -6H-(1S)-pyridazino[1,2-a][1,2]diazepine-1-carboxamide
- 9-[(9S)-[(cyclohexylamino)carbonyl]amino]-N-((3S)-1-diazo-5-methyl-2-oxo-3-hexyl)- octahydro-6,10-dioxo -6H-(1S)-pyridazino[1,2-a][1,2]diazepine-1-carboxamide
- 9-[(9S)-[[(4-phenoxyphenyl)amino]carbonyl]amino]-N-((3S)-1-diazo-5-methyl-2-oxo-3-hexyl)- octahydro-6,10-dioxo -6H-(1S)-pyridazino[1,2-a][1,2]diazepine-1-carboxamide
- 9-[(9S)-[[[2-(thien-2-yl)-ethyl]amino]carbonyl]amino]-N-((3S)-1-diazo-5-methyl-2-oxo-3-hexyl)- octahydro-6,10-dioxo-6H-(1S)-pyridazino[1,2-a][1,2]diazepine-1-carboxamide
- 9-[(9S)-(methylsulfonyl)amino]-N-((3S)-1-diazo-5-methyl-2-oxo-3-hexyl)- octahydro-6,10-dioxo -6H-(1S)-pyridazino[1,2-a][1,2]diazepine-1-carboxamide
- 9-[(9S)-[[4-(trifluoromethyl)phenyl]sulfonyl]amino]-N-((3S)-1-diazo-5-methyl-2-oxo-3-hexyl)- octahydro-6,10-dioxo-6H-(1S)-pyridazino[1,2-a][1,2]diazepine-1-carboxamide
- 9-[(9S)-[(2-naphthalenyl)sulfonyl]amino]-N-((3S)-1-diazo-5-methyl-2-oxo-3-hexyl)- octahydro-6,10-dioxo -6H-(1S)-pyridazino[1,2-a][1,2]diazepine-1-carboxamide
- 9-[(9S)- (3-methoxybenzoyl)amino]-N-[(3S)-1-[(benzothiazol-2-yl)thio]-4-methyl-2-oxo-3-pentyl]- octahydro-6,10-dioxo-6H-(1S)-pyridazino[1,2-a][1,2] diazepine-1-carboxamide
- 9-[(9S)-[(2-furanyl)carbonyl]amino]-N-[(3S)-1-[(benzothiazol-2-yl)thio]-4-methyl-2-oxo-3-pentyl]-octahydro-6,10-dioxo -6H-(1S)-pyridazino[1,2-a][1,2] diazepine-1-carboxamide
- 9-[(9S)-[(cyclohexylamino)carbonyl]amino]-N-[(3S)-1-[(benzothiazol-2-yl)thio]-4-methyl-2-oxo-3-pentyl]-octahydro-6,10-dioxo -6H-(1S)-pyridazino[1,2-a][1,2] diazepine-1-carboxamide
- 9-[(9S)-[[(4-phenoxyphenyl)amino]carbonyl]amino]-N-[(3S)-1-[(1-phenyl-1H-tetrazol-5-yl)thio]-4-methyl-2-oxo-3-pentyl]-octahydro-6,10-dioxo -6H-(1S)-pyridazino [1,2-a][1,2]diazepine-1-carboxamide
- 9-[(9S)-[[(4-phenoxyphenyl)amino]carbonyl]amino]-N-[3S)-1-[(benzothiazol-2-yl)thio]-4-methyl-2-oxo-3-pentyl]-octahydro-6,10-dioxo -6H-(1S)-pyridazino[1,2-a][1,2]diazepine-1-carboxamide
- 9-[(9S)-[[[2-(thien-2-yl)-ethyl]amino]carbonyl]amino]-N-[(3S)-1-[(1-phenyl-1H-tetrazol-5-yl)thio]-4-methyl-2-oxo-3-pentyl]- octahydro-6,10-dioxo -6H-(1S)-pyridazino [1,2-a][1,2]diazepine-1-carboxamide
- 9-[(9S)-[[[2-(thien-2-yl)-ethyl]amino]carbonyl]amino]-N-[(3S)-1-[(benzothiazol-2-yl)thio]-4-methyl-2-oxo-3-pentyl]-octahydro-6,10-dioxo -6H-(1S)-pyridazino[1,2-a][1,2]diazepine-1-carboxamide
- 9-[(9S)-[(cyclohexylamino)carbonyl]amino]-N-[(2S)-4-[(2,6-dichlorobenzoyl)oxy]-3-oxo-2-butyl]- octahydro-6,10-dioxo-6H-(1S)-pyridazino[1,2-a][1,2]diazepine-1-carboxamide
- 9-[(9S)-[(2-furanyl)carbonyl]amino]-N-[(2S)-4-[(2,6-dichlorobenzoyl)oxy]-3-oxo-2-butyl]- octahydro-6,10-dioxo-6H-(1S)-pyridazino[1,2-a][1,2]diazepine-1-carboxamide
- 9-[(9S)-[[(4-phenoxyphenyl)amino]carbonyl]amino]-N-[(2S)-4-[(benzothiazol-2-yl)thio]- 3-oxo-2-butyl]- octahydro-6,10-dioxo -6H-(1S)-pyridazino[1,2-a][1,2] diazepine-1-carboxamide
- 9-[(9S)-[[(4-phenoxyphenyl)amino]carbonyl]amino]-N-[(2S)-4-[(2,6-dichlorobenzoyl)oxy]-3-oxo-2-butyl]- octahydro-6,10-dioxo -6H-(1S)-pyridazino[1,2-a][1,2] diazepine-1-carboxamide
- 9-[(9S)- (3-methoxybenzoyl)amino]-N-[(2S)-4-[(2,6-dichlorobenzoyl)oxy]-3-oxo-2-butyl]- octahydro-6,10-dioxo-6H-(1S)-pyridazino[1,2-a][1,2]diazepine-1-carboxamide
- 9-[(9S)-[[[2-(thien-2-yl)-ethyl]amino]carbonyl]amino]-N-[(2S)-4-[(2,6-dichlorobenzoyl)oxy]-3-oxo-2-butyl]- octahydro-6,10-dioxo -6H-(1S)-pyridazino[1,2-a][1,2]diazepine-1-carboxamide
- 9-[(9S)-[[4-(trifluoromethyl)phenyl]sulfonyl]amino]-N-[(2S)-4-[(2,6-dichlorobenzoyl)oxy]-3-oxo-2-butyl]- octahydro-6,10-dioxo -6H-(1S)-pyridazino[1,2-a][1,2] diazepine-1-carboxamide
- 9-[(9S)-[(cyclohexylamino)carbonyl]amino]-N-[(2S)-4-[(benzothiazol-2-yl)thio]-1-[4-[(1,1-dimethyl) ethoxy]phenyl]-3-oxo-2-butyl]-6H-(1S)-pyridazino[1,2-a][1,2]diazepine-1-carboxamide.
Les produits de formule (I) selon la présente invention peuvent être préparés selon le schéma général de synthèse décrit à la figure 1 de la présente invention.

La présente invention a ainsi également pour objet un procédé de préparation des produits de formule (I), telle que définie ci-dessus, caractérisé en ce que l'on soumet le composé de formule (II) : 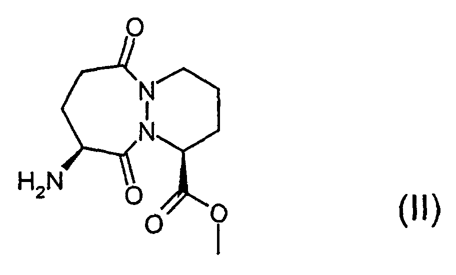 à une réaction avec un composé de formule (III) : ,

R1'-X (III)

dans laquelle X représente un atome d'halogène et R1' a la signification indiquée ci-dessus pour R1, dans laquelle les éventuelles fonctions réactives sont éventuellement protégées par des groupements protecteurs, pour obtenir le produit de formule (IV) : 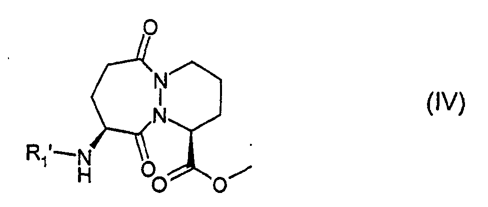 dans laquelle R1' a la signification indiquée ci-dessus, produit de formule (IV) que l'on soumet à une réaction de saponification pour obtenir le produit de formule (V) : 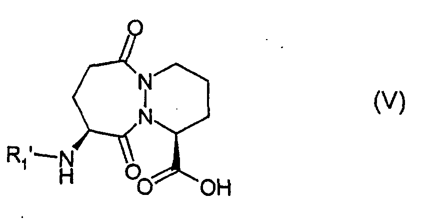 dans laquelle R1' a la signification indiquée ci-dessus, produit de formule (V) que l'on soumet à une réaction avec un produit de formule (VI) : 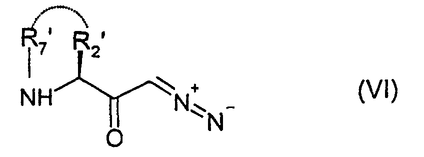 dans laquelle R2' et R7' ont les significations indiquées ci-dessus respectivement pour R2 et R7, dans lesquelles les éventuelles fonctions réactives sont éventuellement protégées par des groupements protecteurs,
pour obtenir un produit de formule (Ix) : 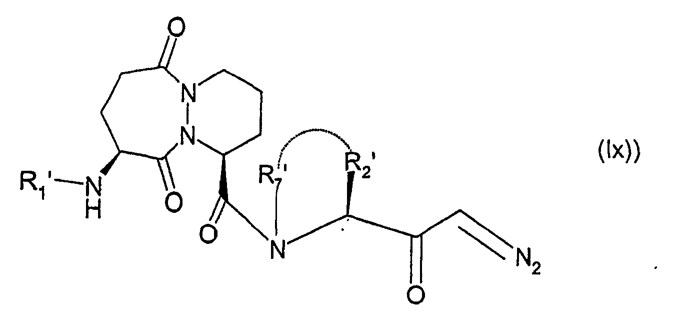 dans laquelle R1', R2' et R7' ont les significations indiquées ci-dessus,
produit de formule (Ix) que l'on peut soumettre à une réaction de bromation pour obtenir un produit de formule (VII) : 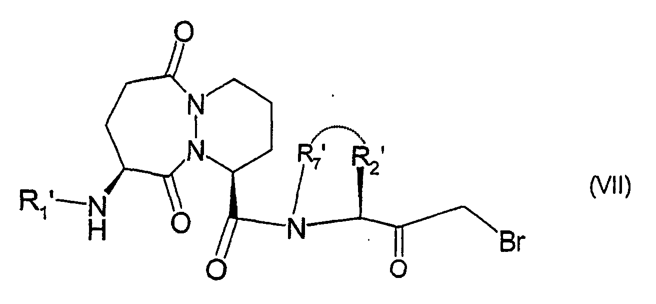 dans laquelle R1', R2' et R7' ont les significations indiquées ci-dessus,
produit de formule (VII) que l'on soumet à une réaction avec un produit de formule (VIII) :

H'-L-R4' (VIII)

dans laquelle L a la signification indiquée ci-dessus et R4' a la signification indiquée ci-dessus pour R4, dans laquelle les éventuelles fonctions réactives sont éventuellement protégées par des groupements protecteurs,
pour obtenir un produit de formule (Iy) : 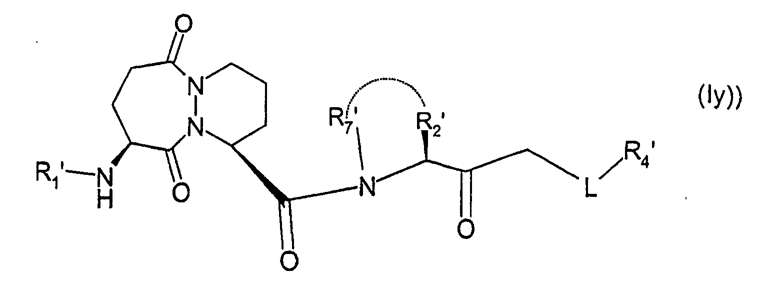 dans laquelle R1', R2', R4' et R7' ont les significations indiquées ci-dessus,
produits de formules (Ix) et (Iy) qui peuvent être des produits de formule (I) et que, pour obtenir des ou d'autres produits de formule (I), l'on peut soumettre, si désiré et si nécessaire, à l'une ou plusieurs des réactions de transformations suivantes, dans un ordre quelconque :
a) une réaction d'estérification de fonction acide,
b) une réaction de saponification de fonction ester en fonction acide,
c) une réaction d'oxydation de groupement alkylthio en sulfoxyde ou sulfone correspondant,
d) une réaction de transformation de fonction cétone en fonction oxime,
e) une réaction de réduction de la fonction carboxy libre ou estérifié en fonction alcool,
f) une réaction de transformation de fonction alcoxy en fonction hydroxyle, ou encore de fonction hydroxyle en fonction alcoxy,
g) une réaction d'oxydation de fonction alcool en fonction aldéhyde, acide ou cétone,
h) une réaction de transformation de radical nitrile en tétrazolyle,
i) une réaction d'élimination des groupements protecteurs que peuvent porter les fonctions réactives protégées,
j) une réaction de salification par un acide minéral ou organique ou par une base pour obtenir le sel correspondant,
k) une réaction de dédoublement des formes racémiques en produits dédoublés, lesdits produits de formule (I) ainsi obtenus étant sous toutes les formes isomères possibles racémiques, énantiomères et diastéréoisomères.

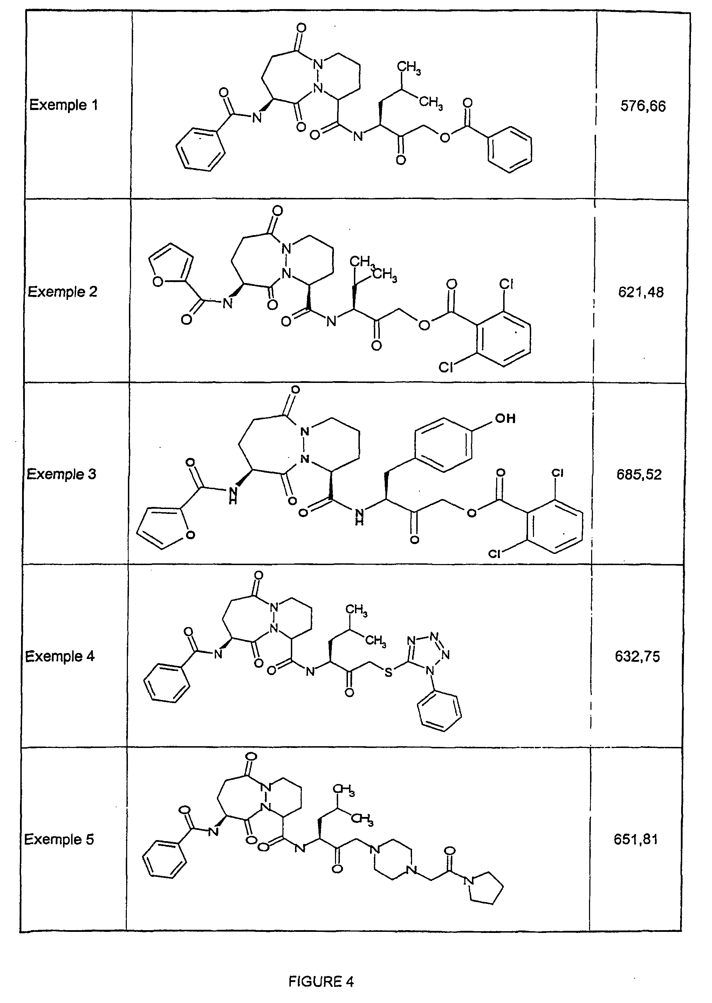

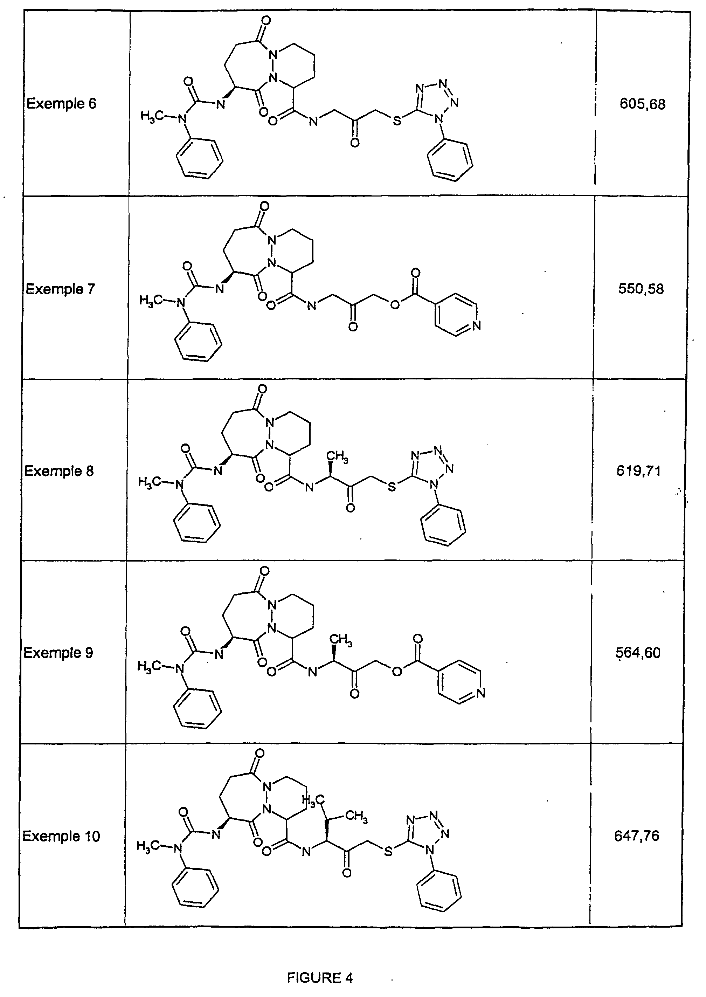

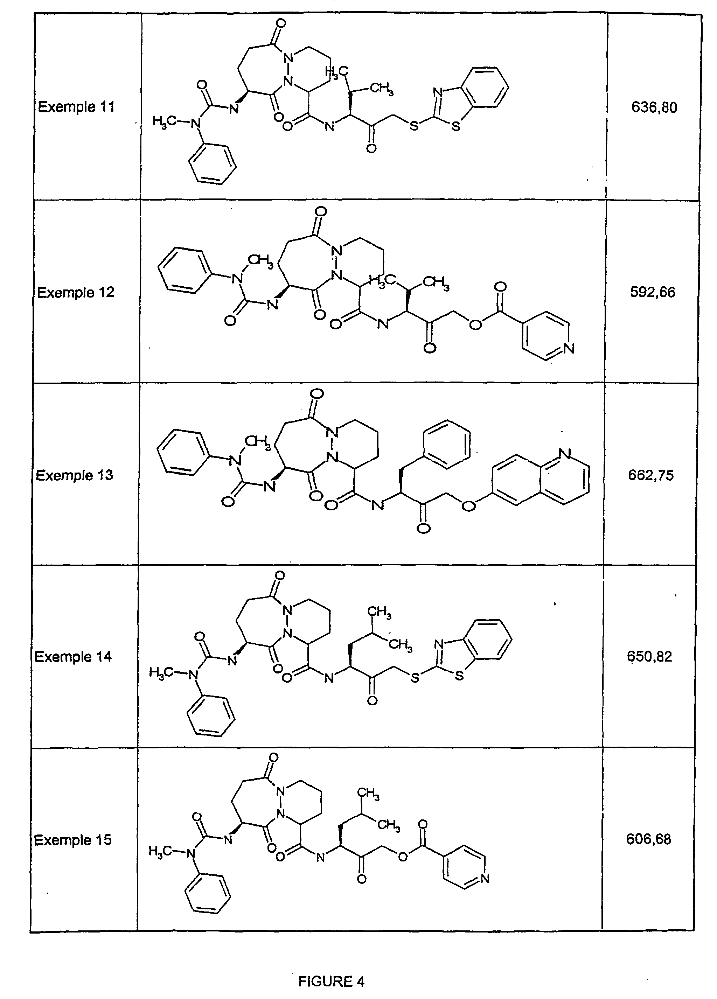

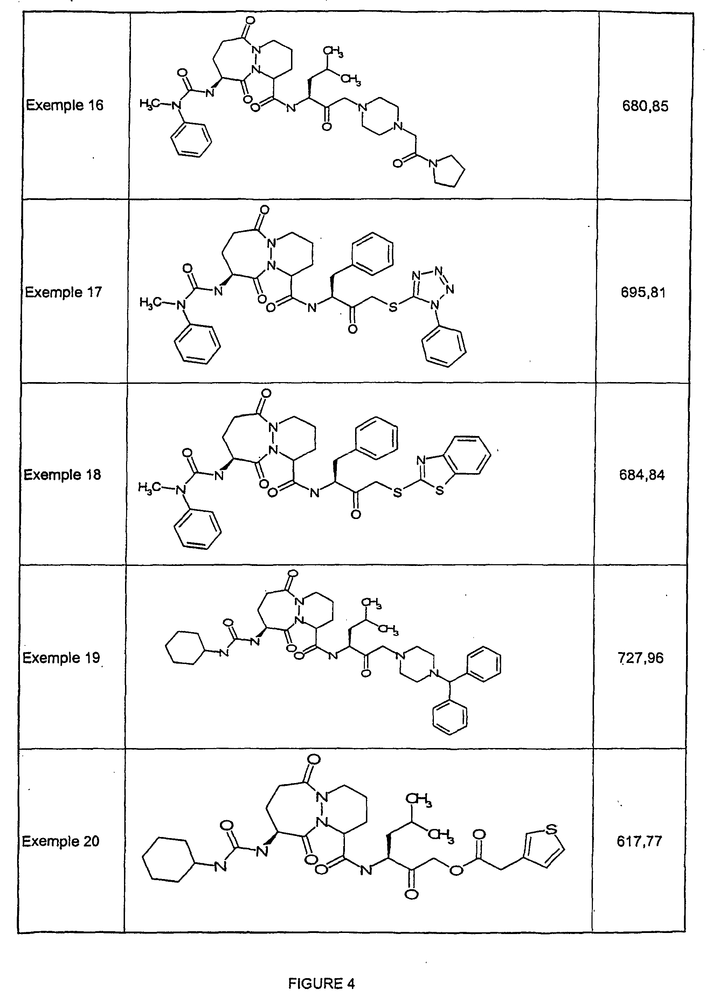

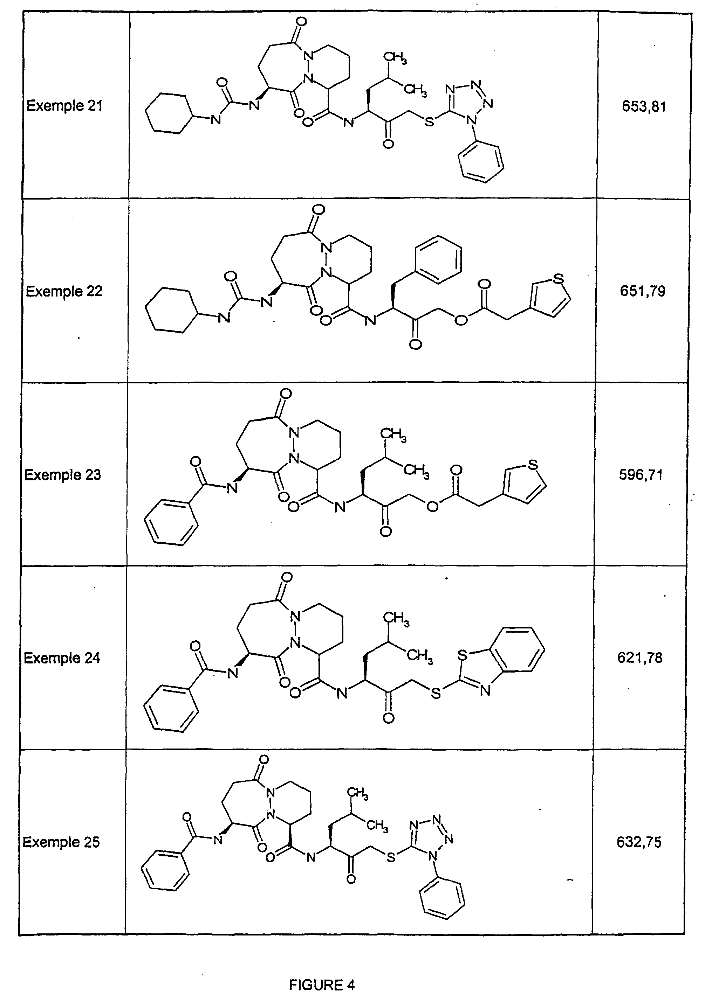

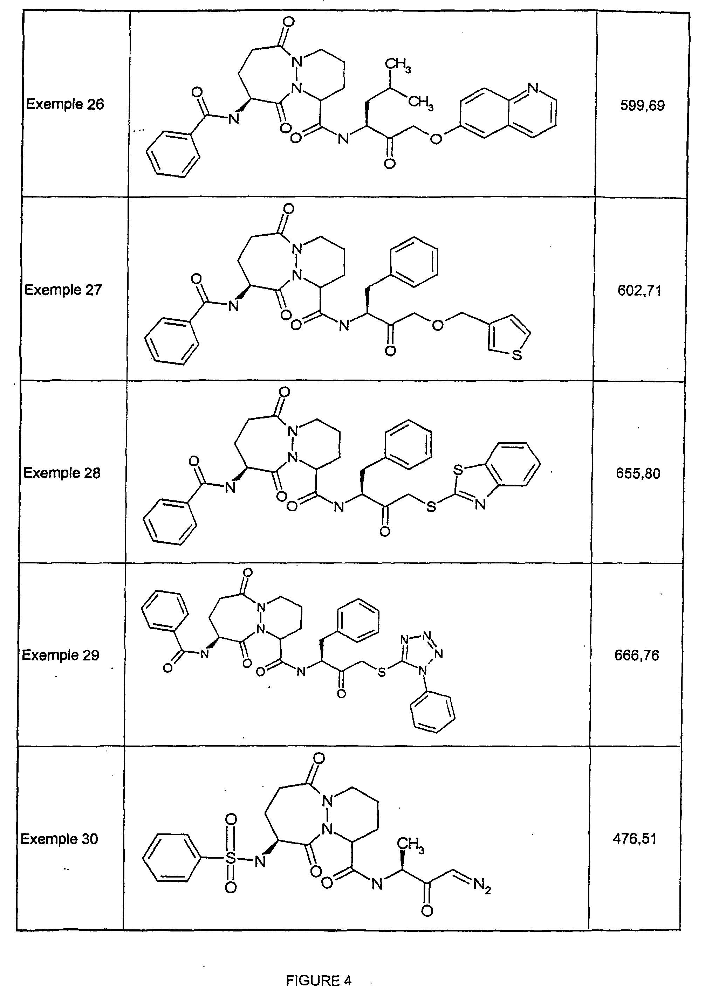

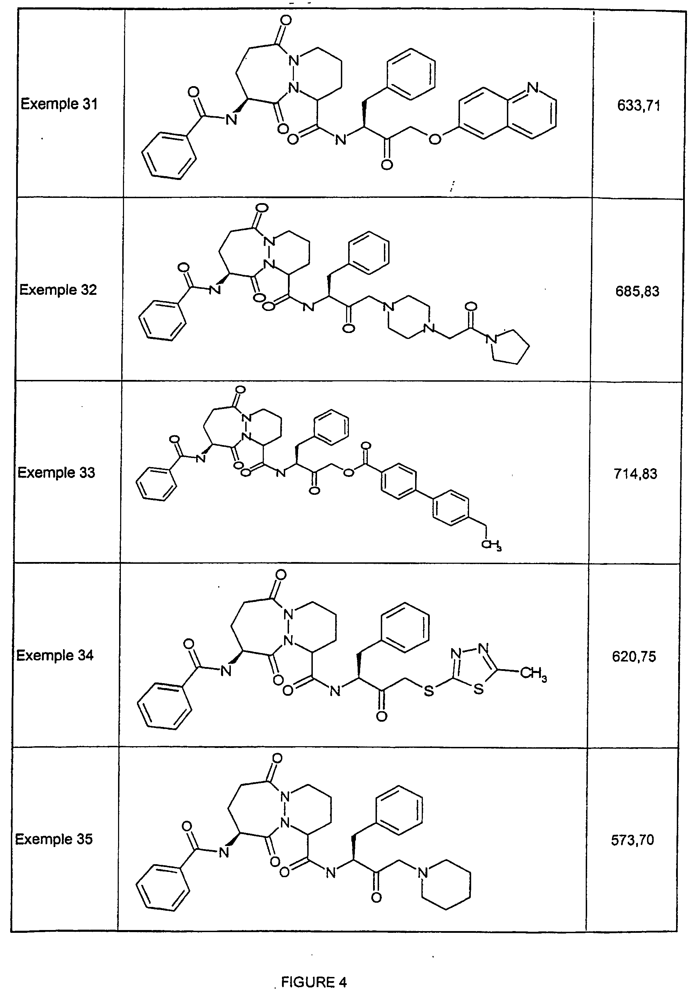

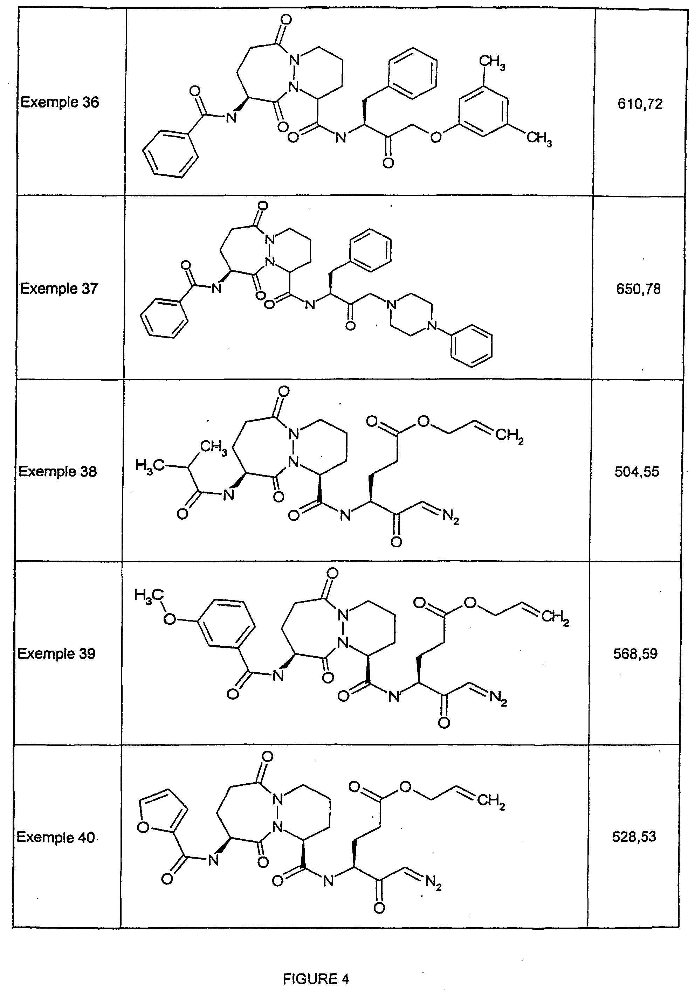

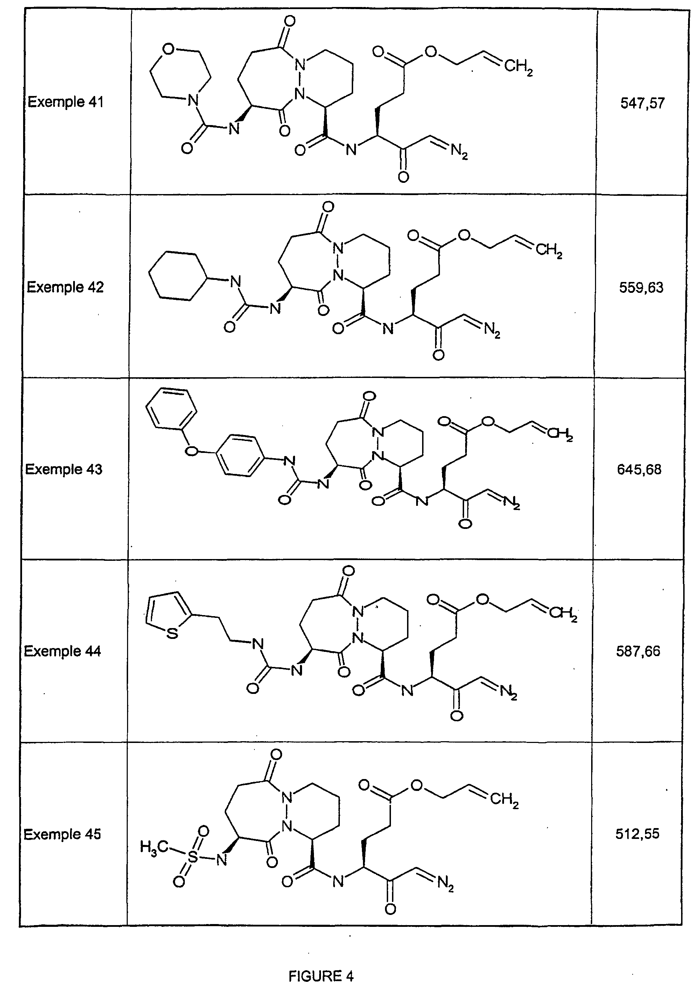

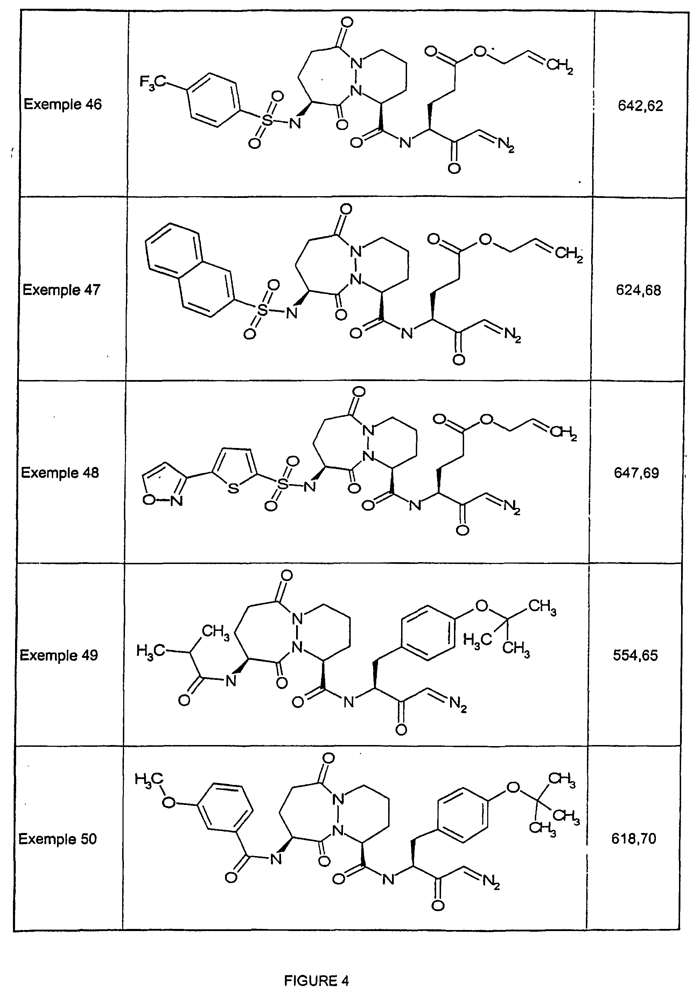

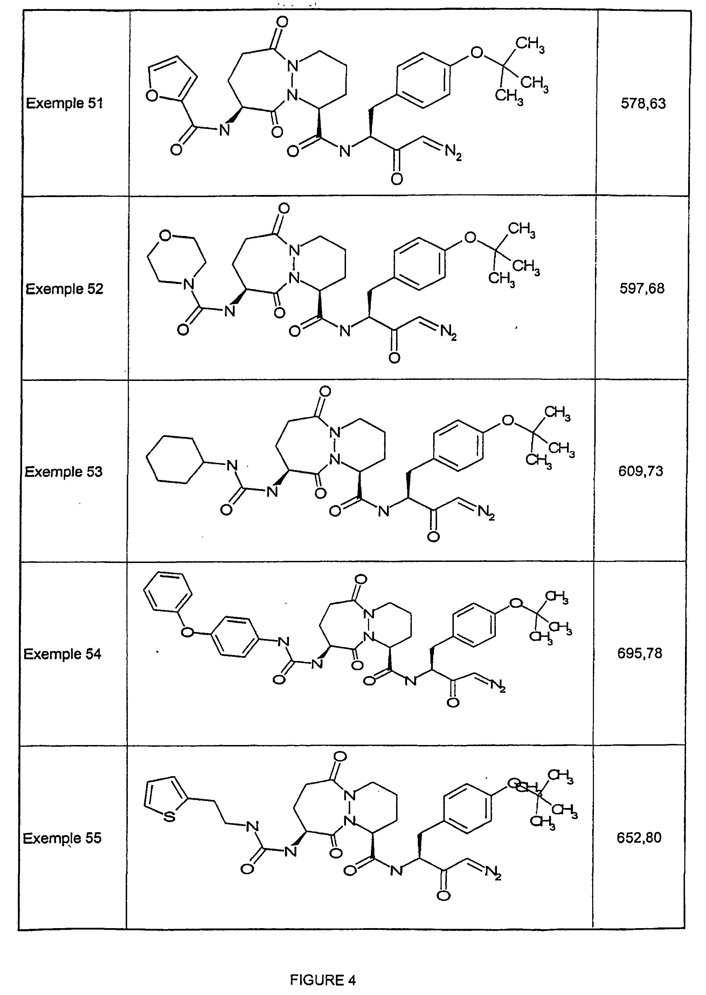

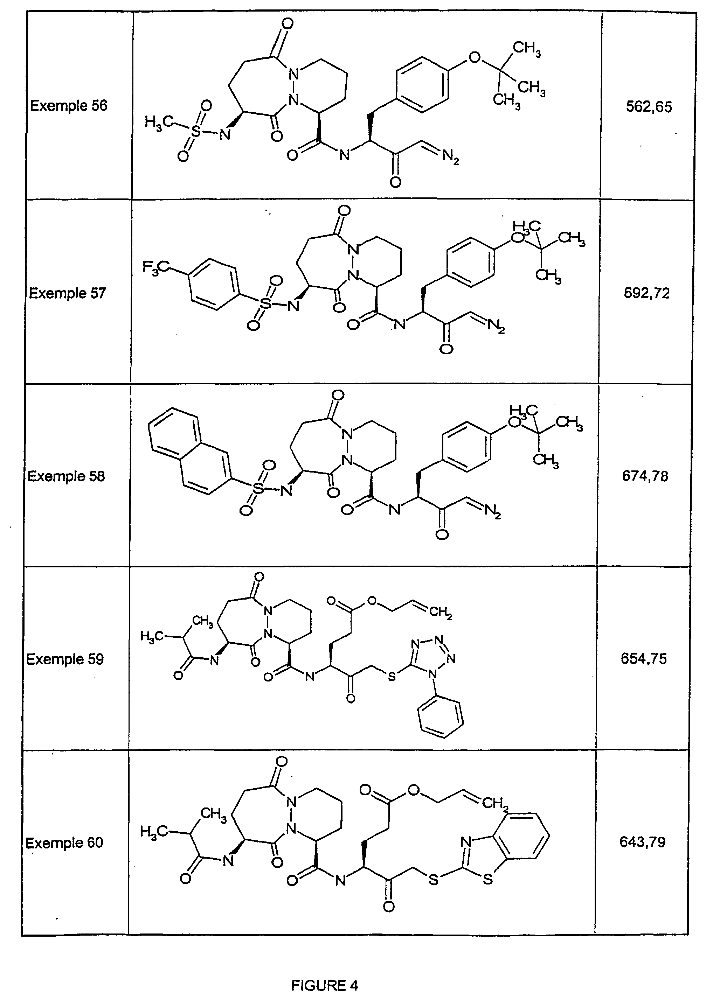

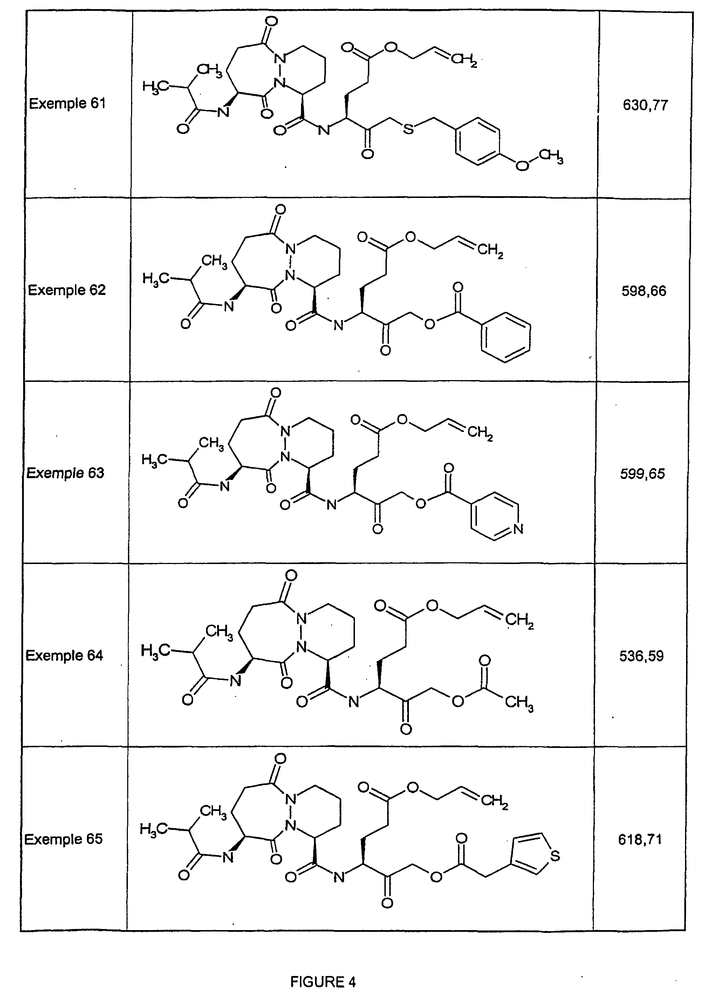

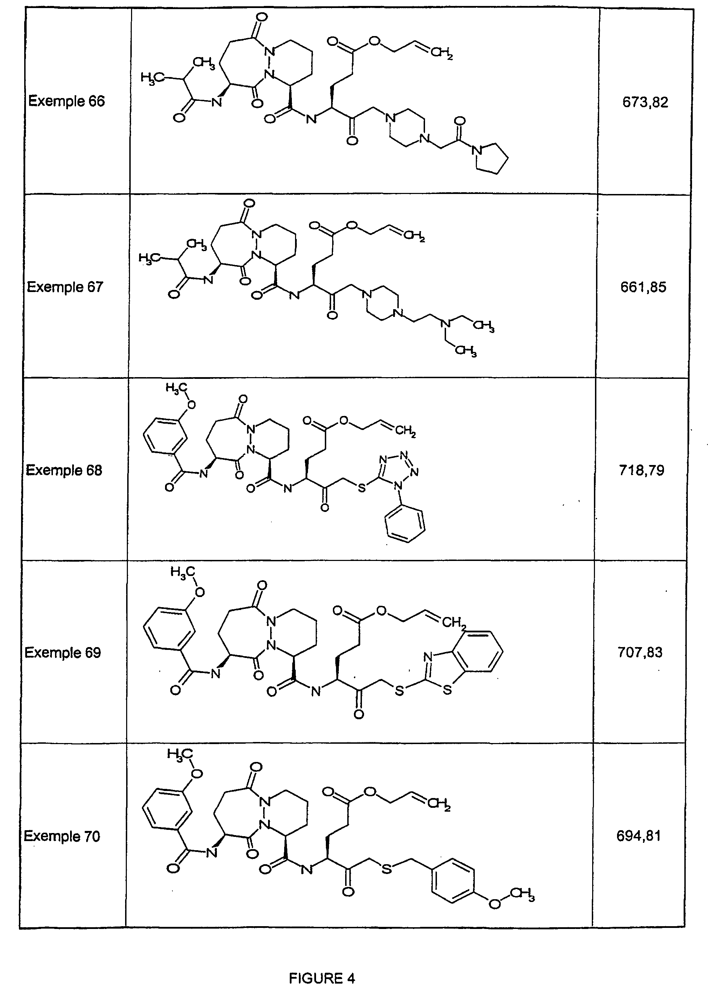

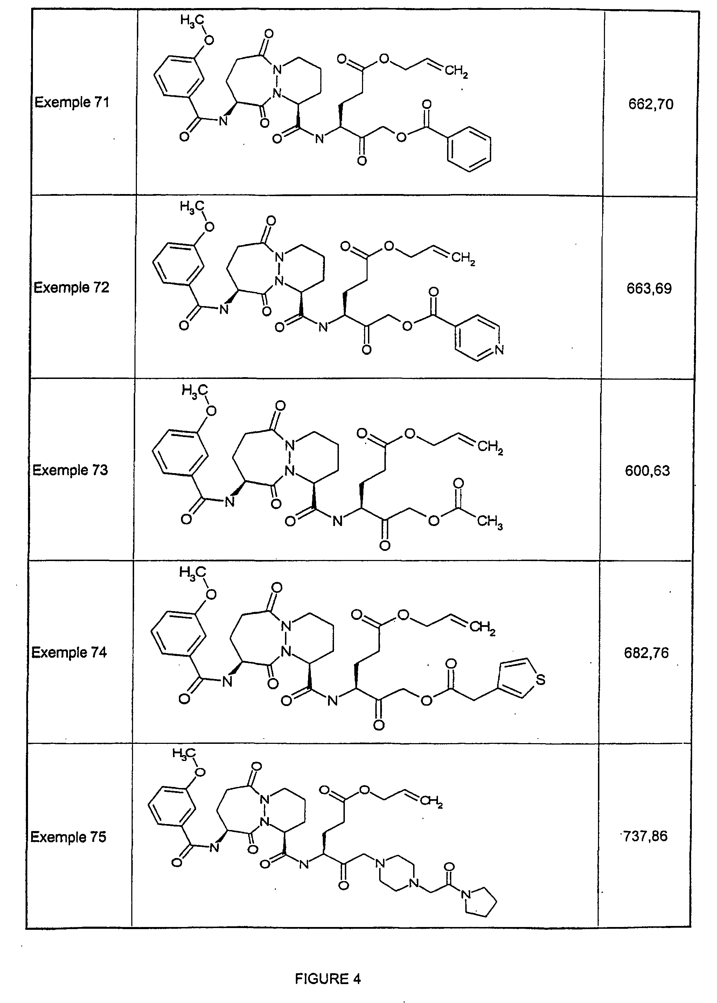

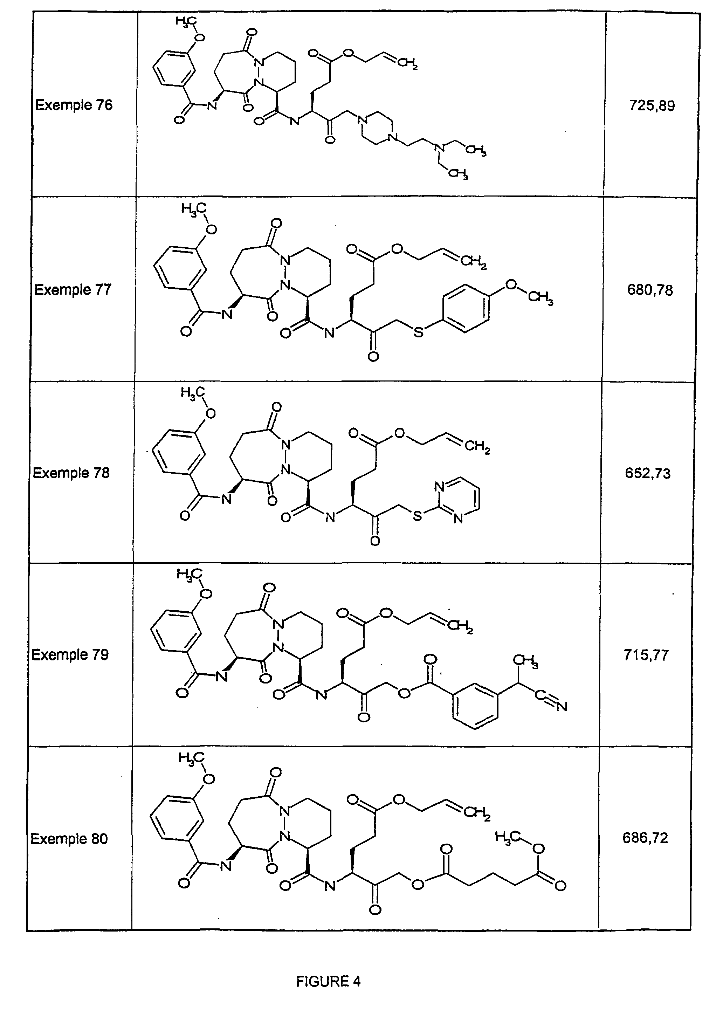

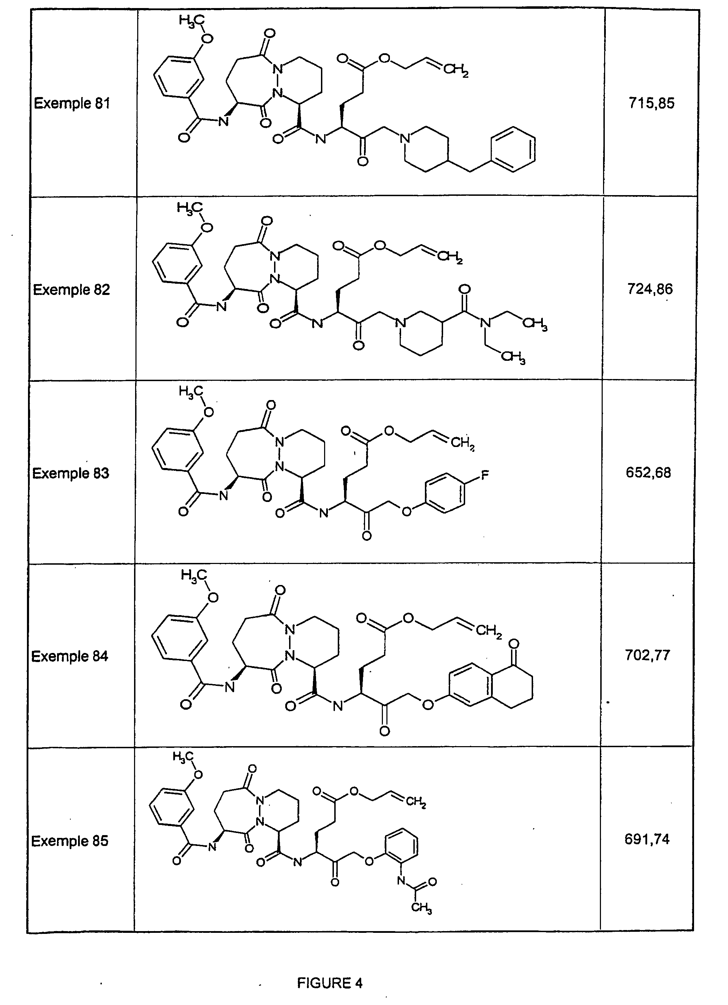

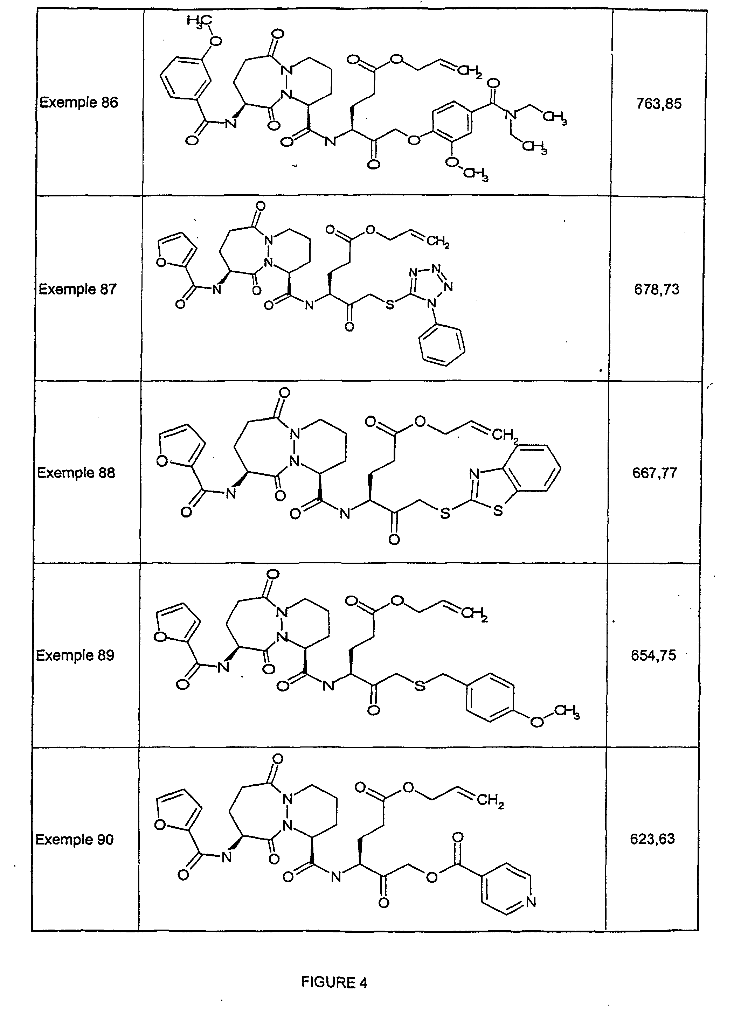

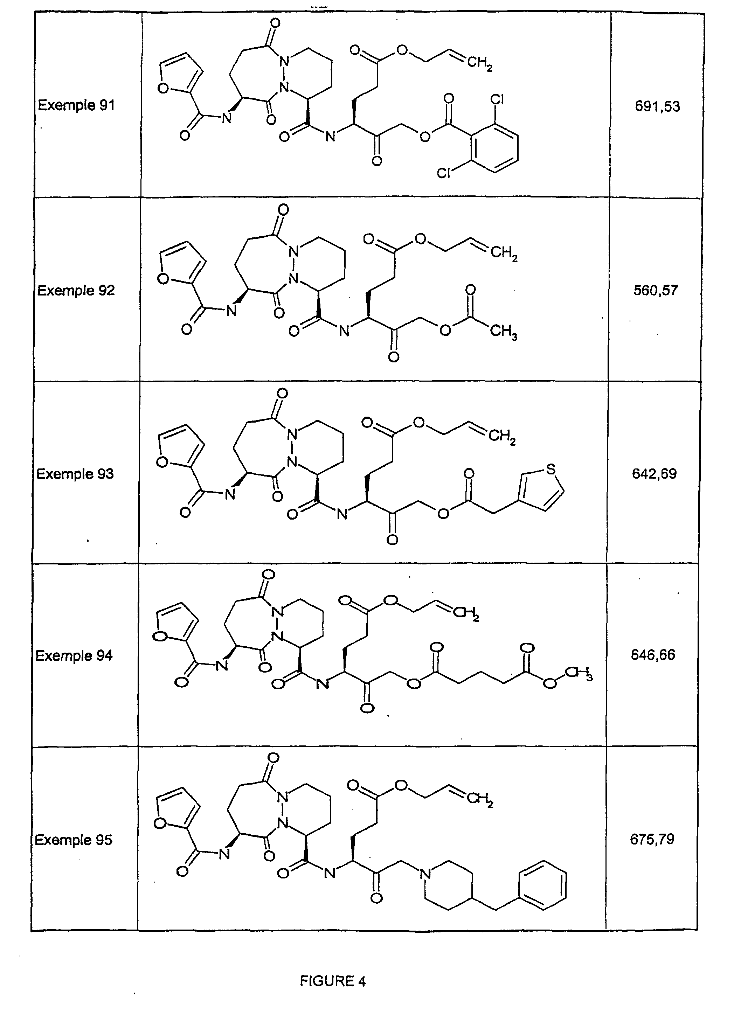

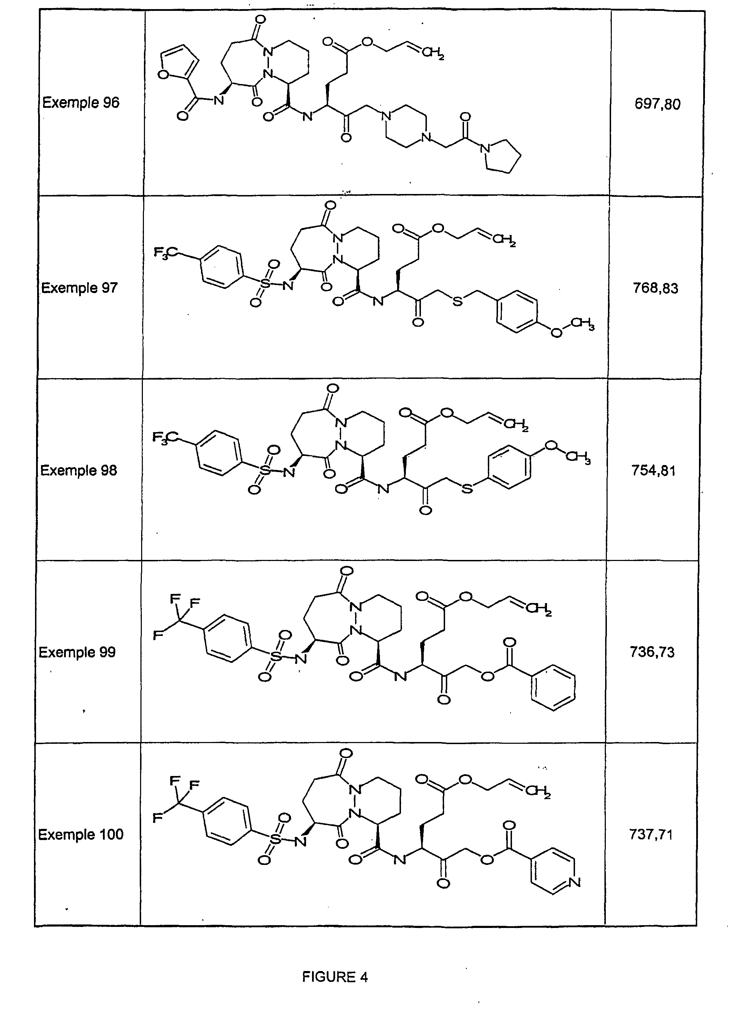

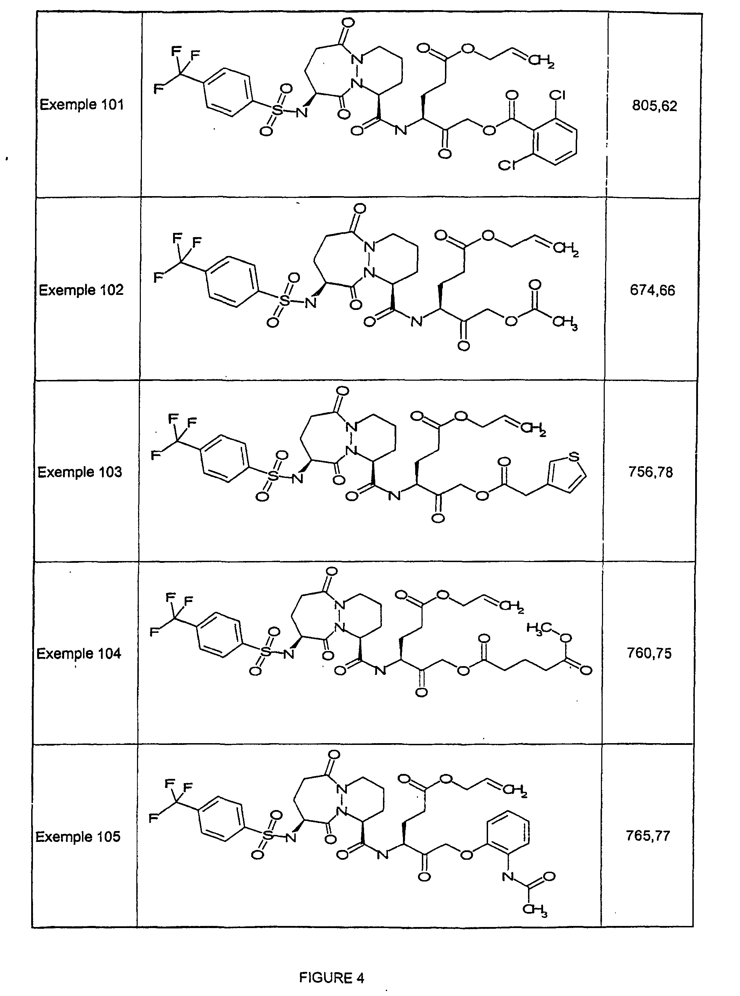

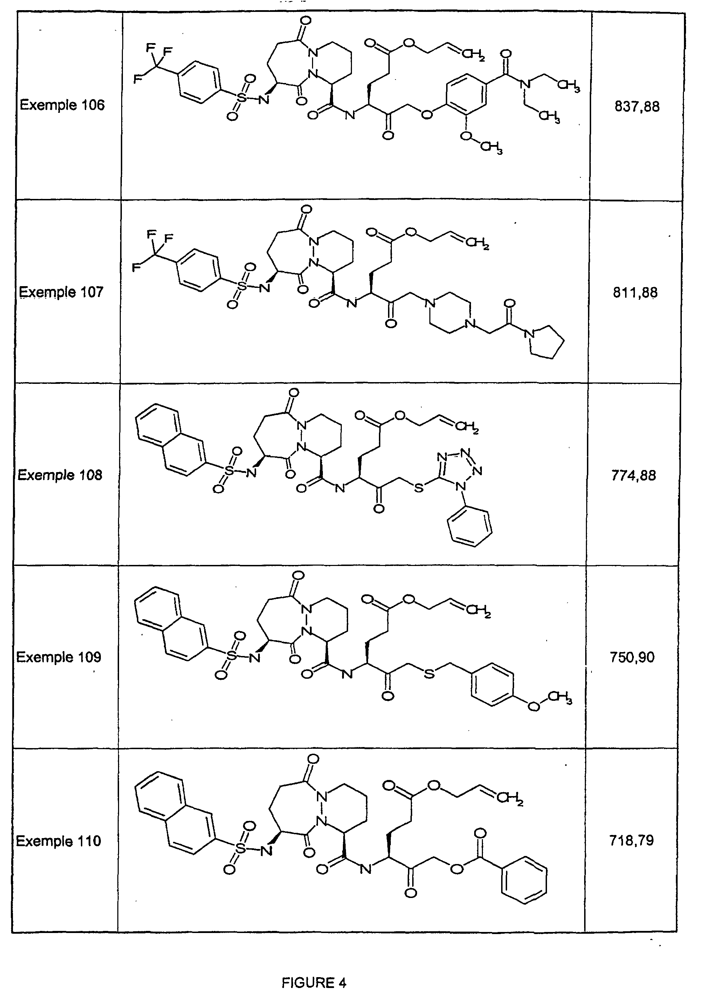

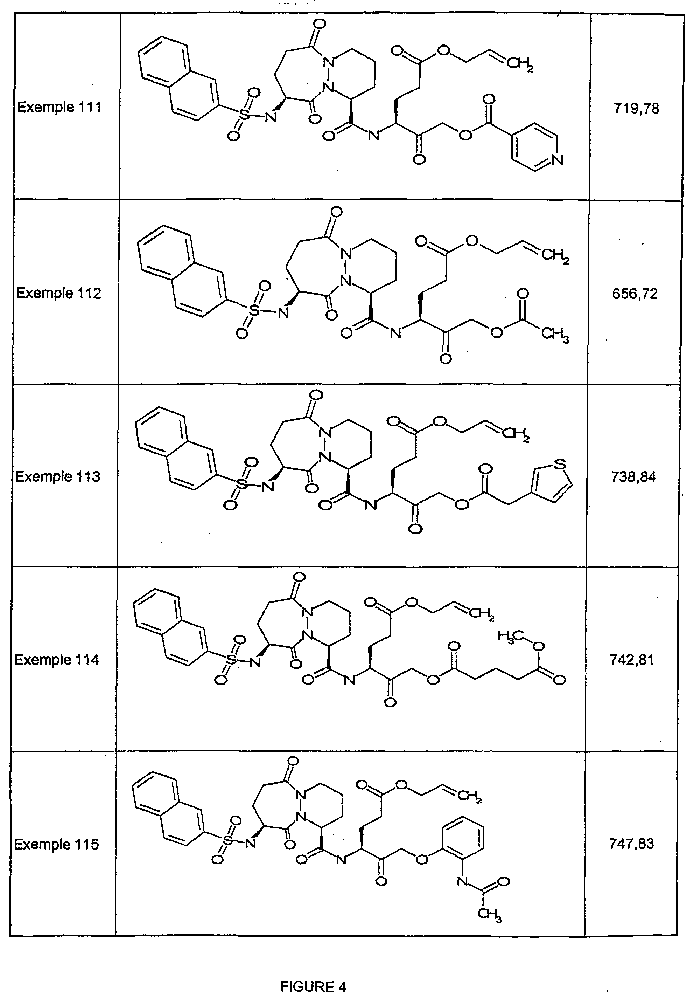

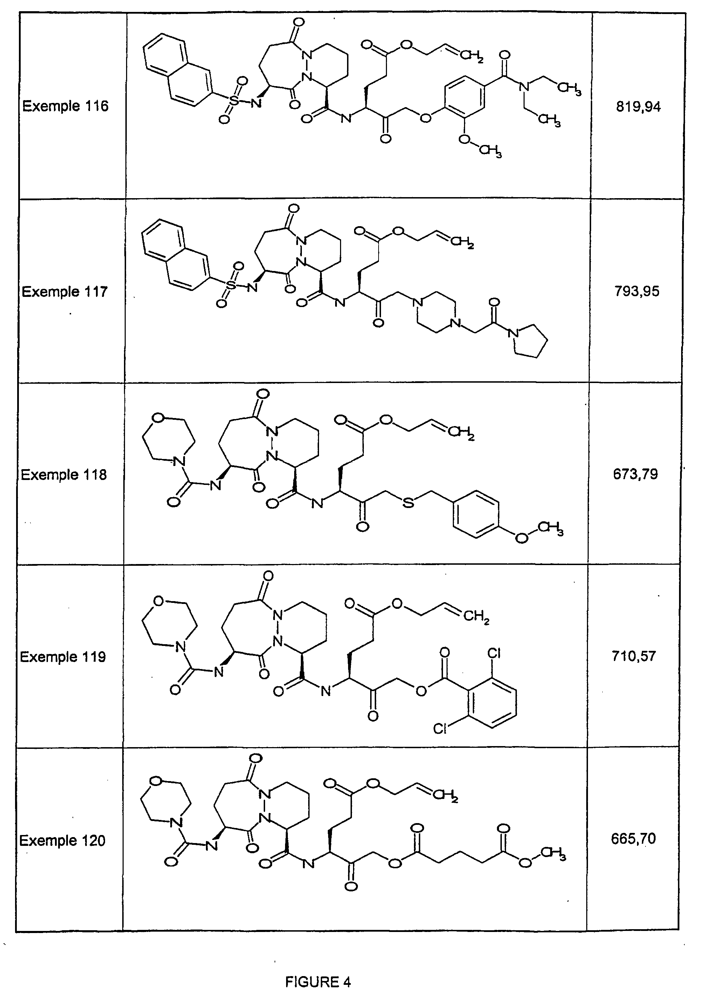

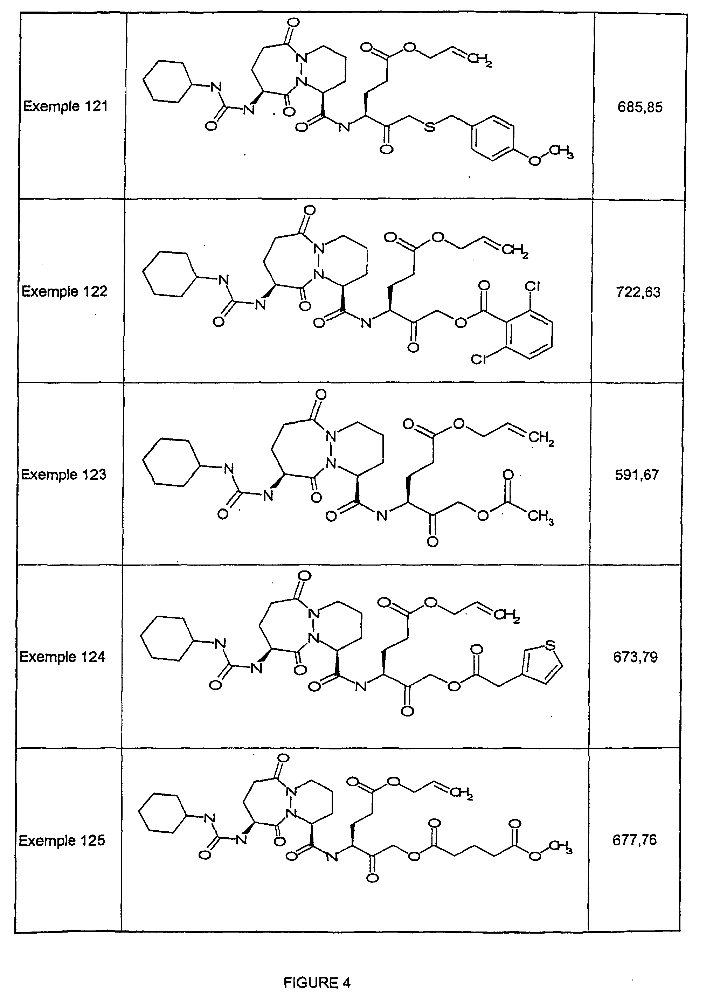

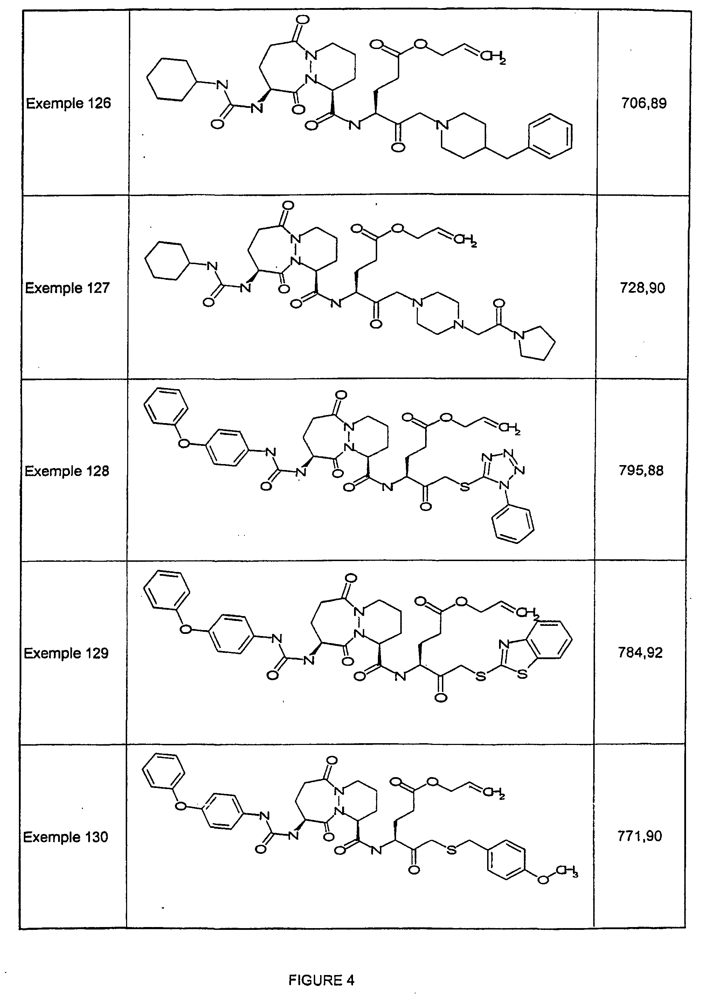

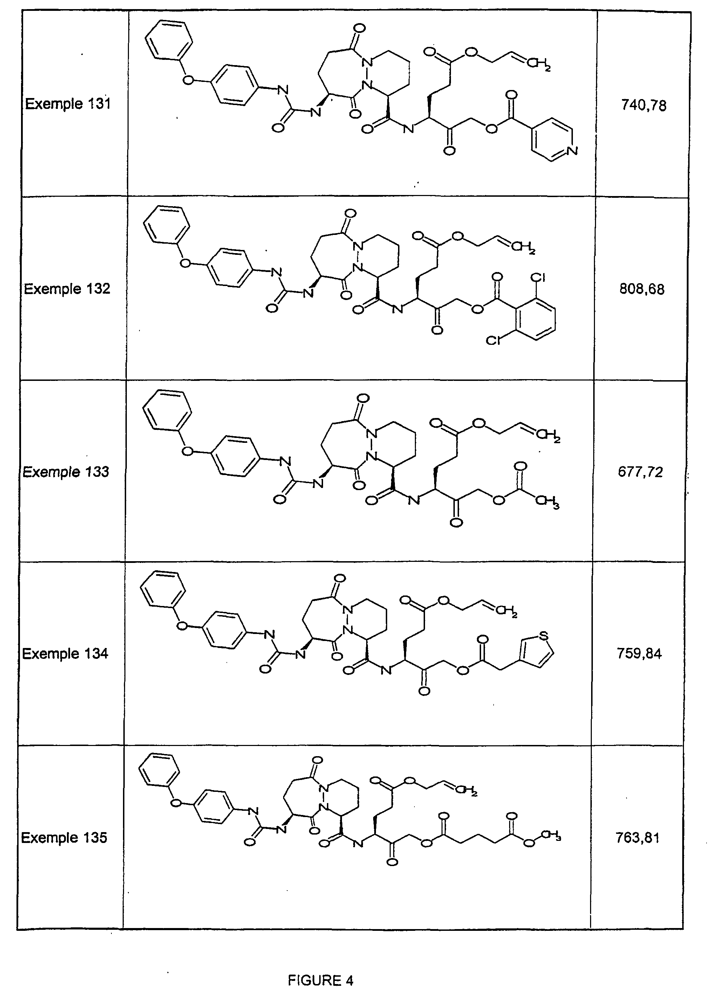

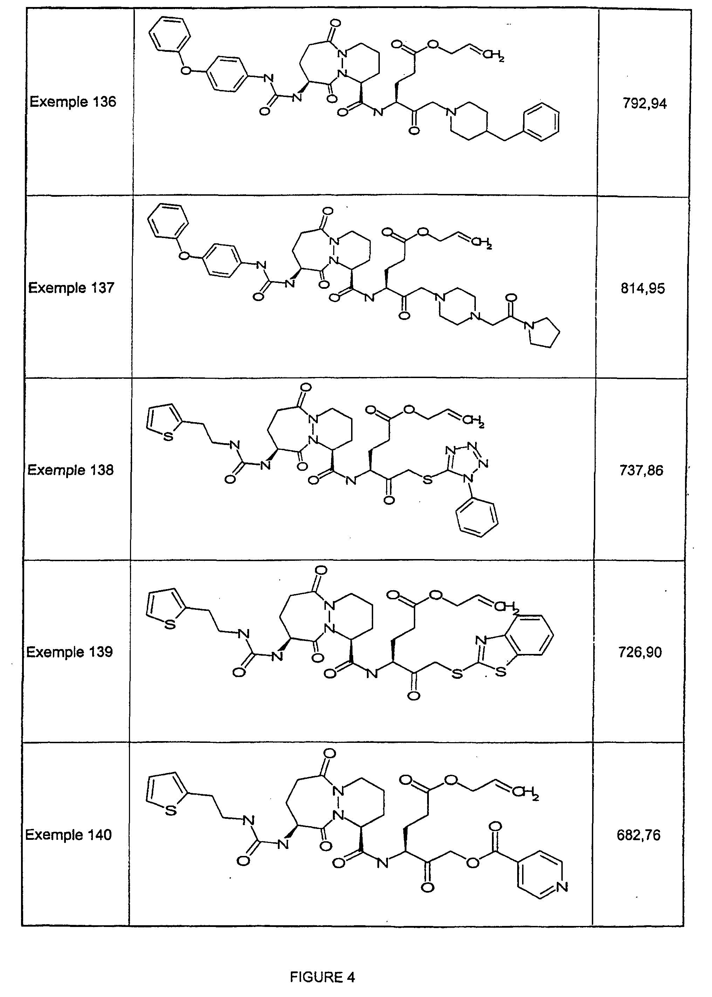

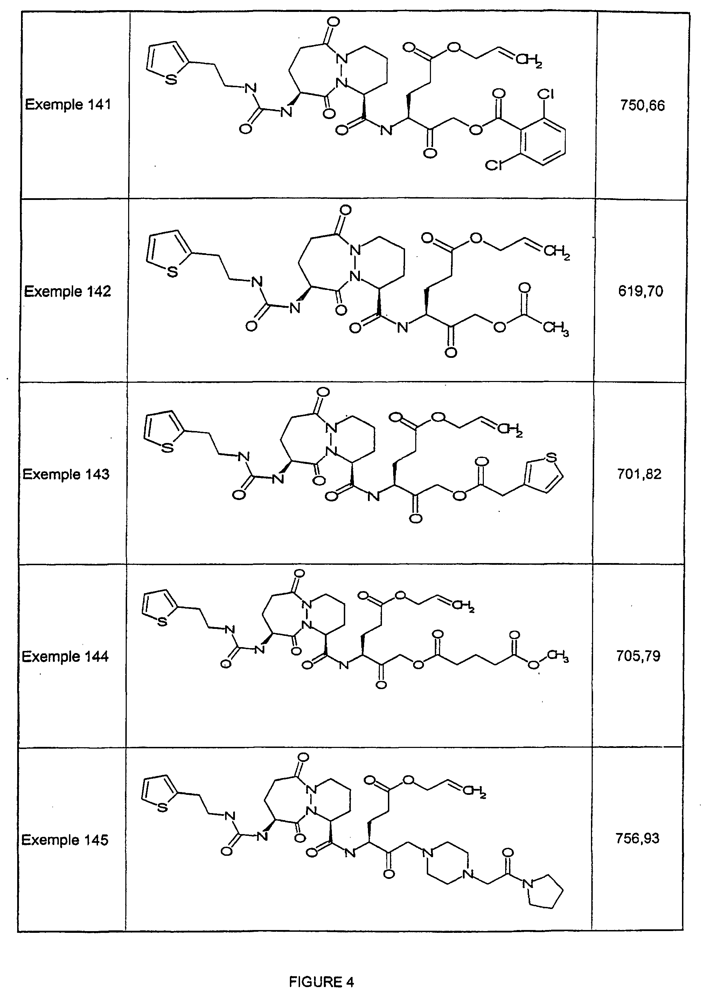

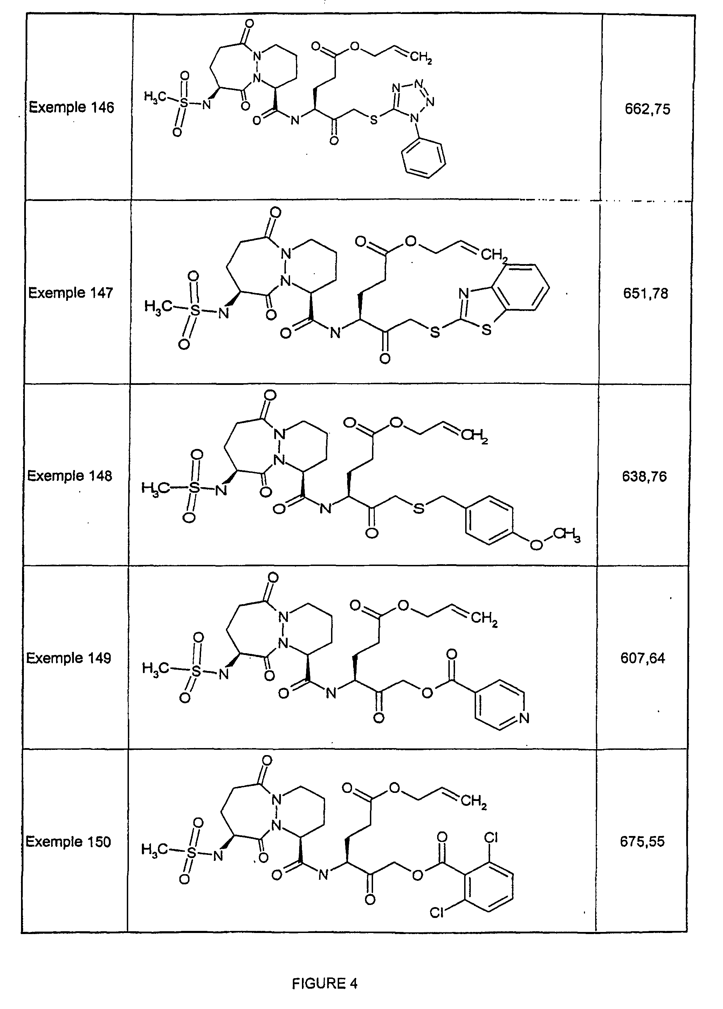

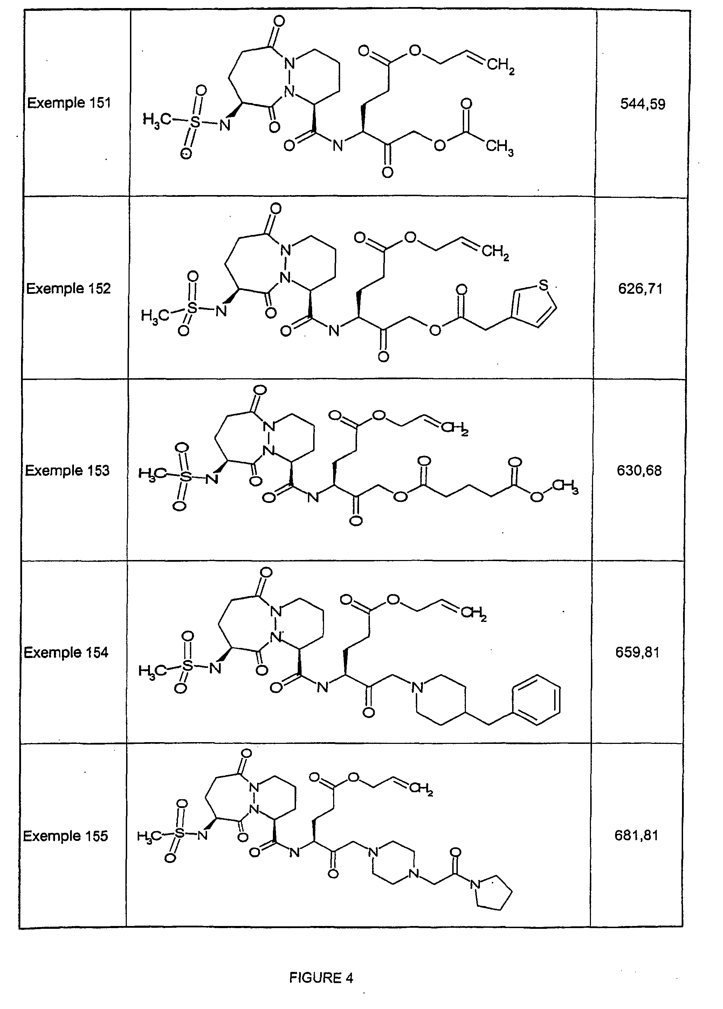

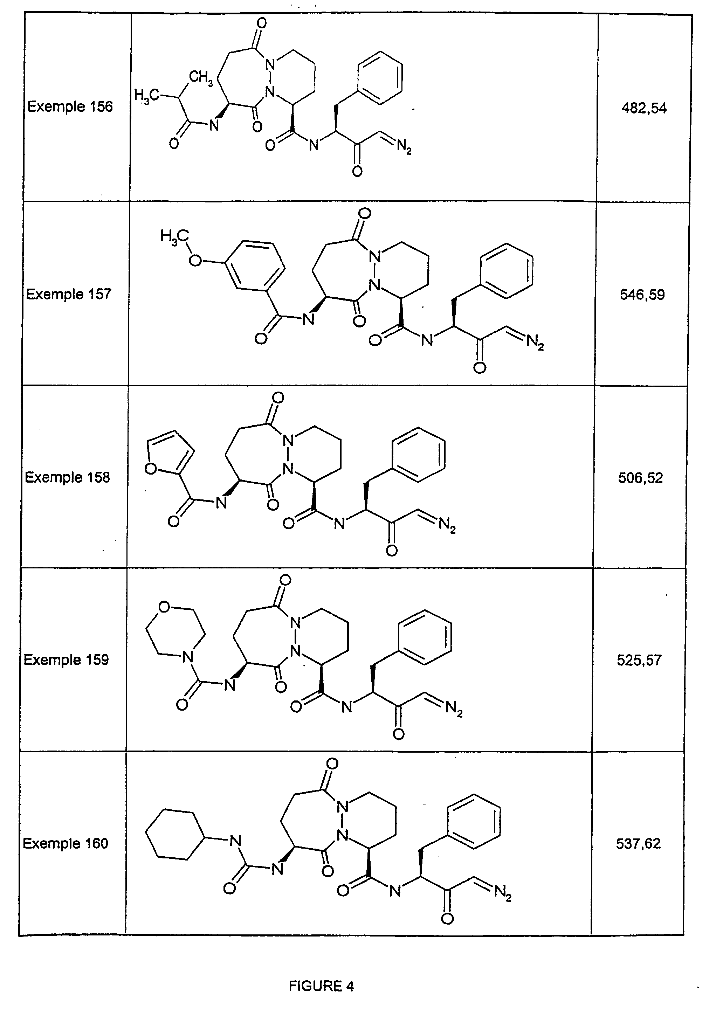

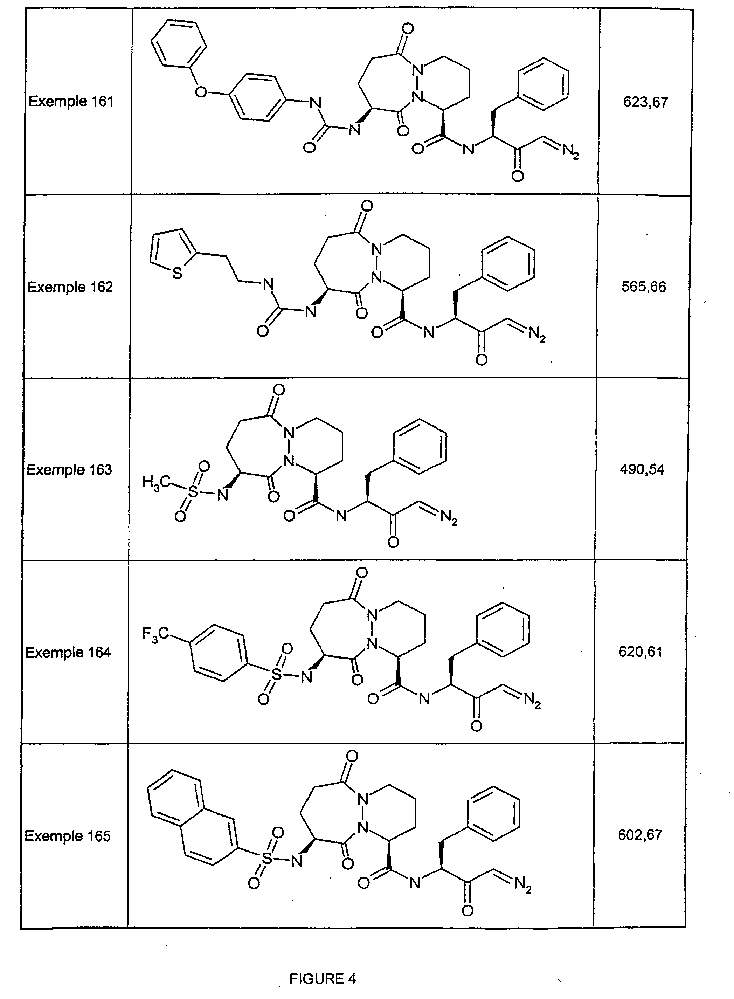

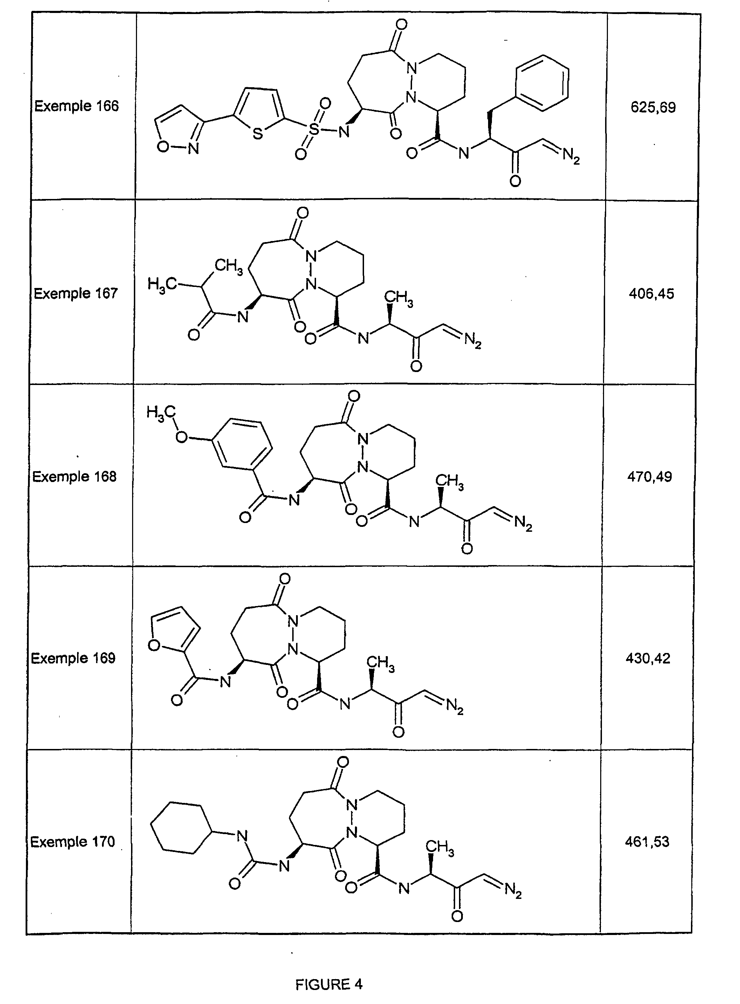

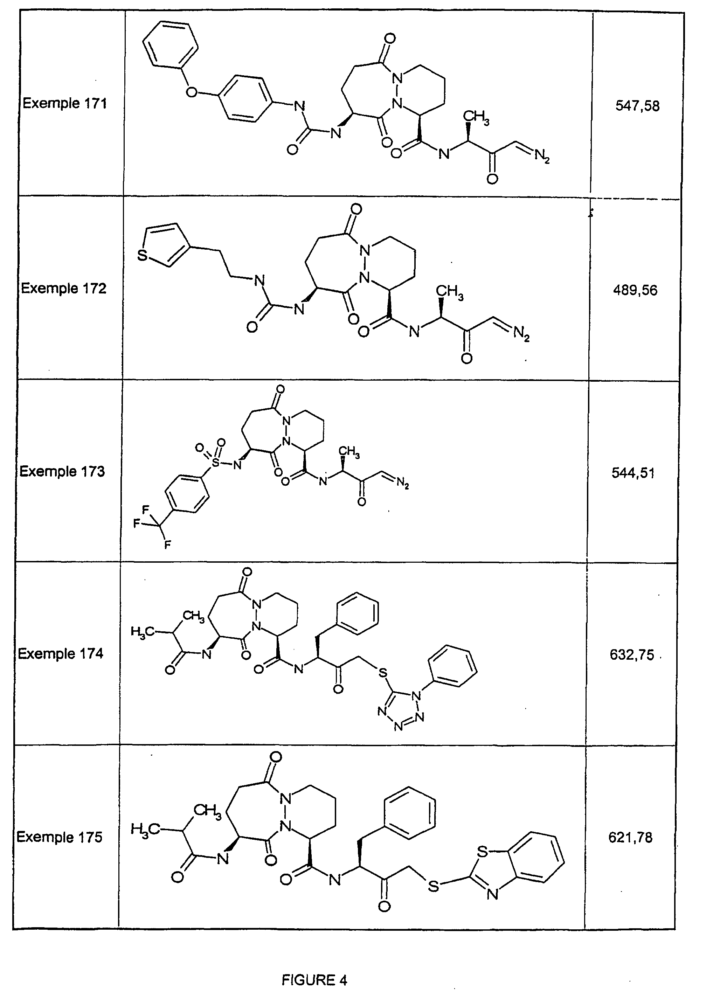

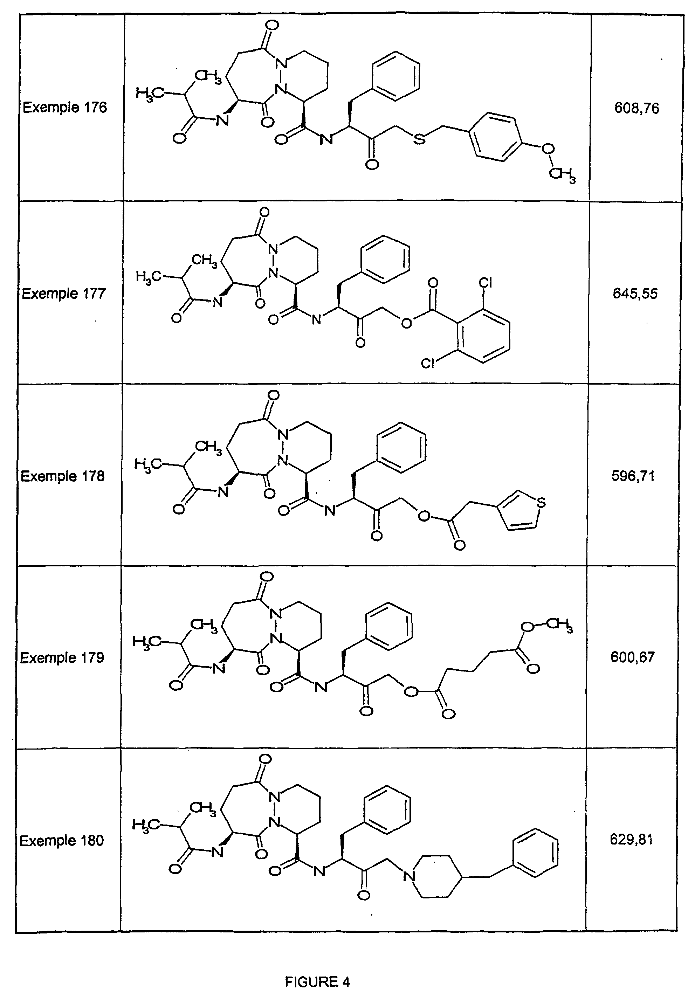

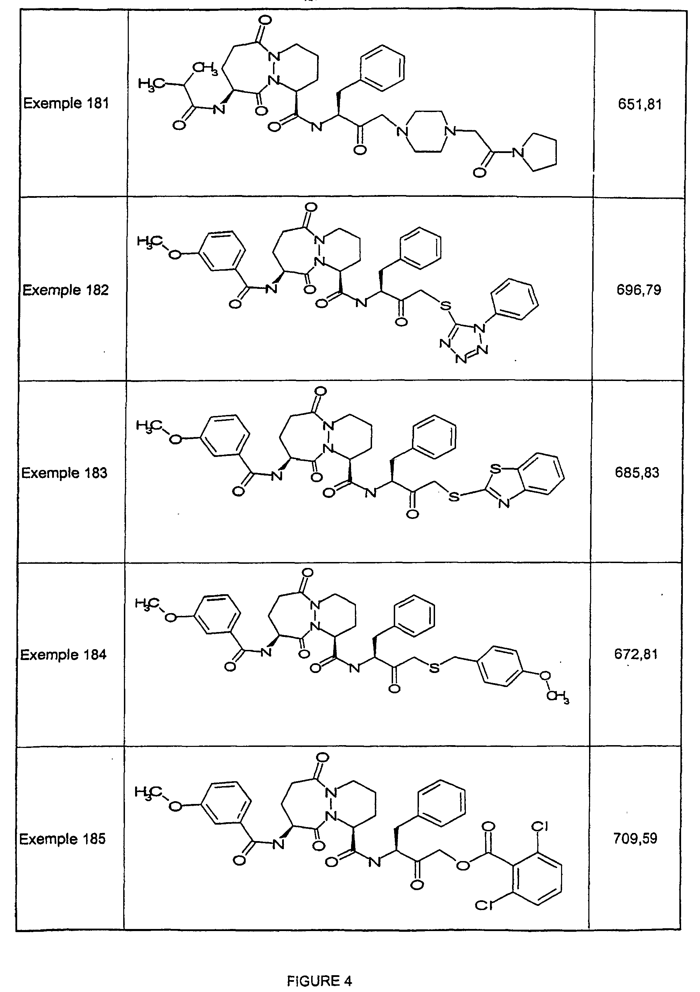

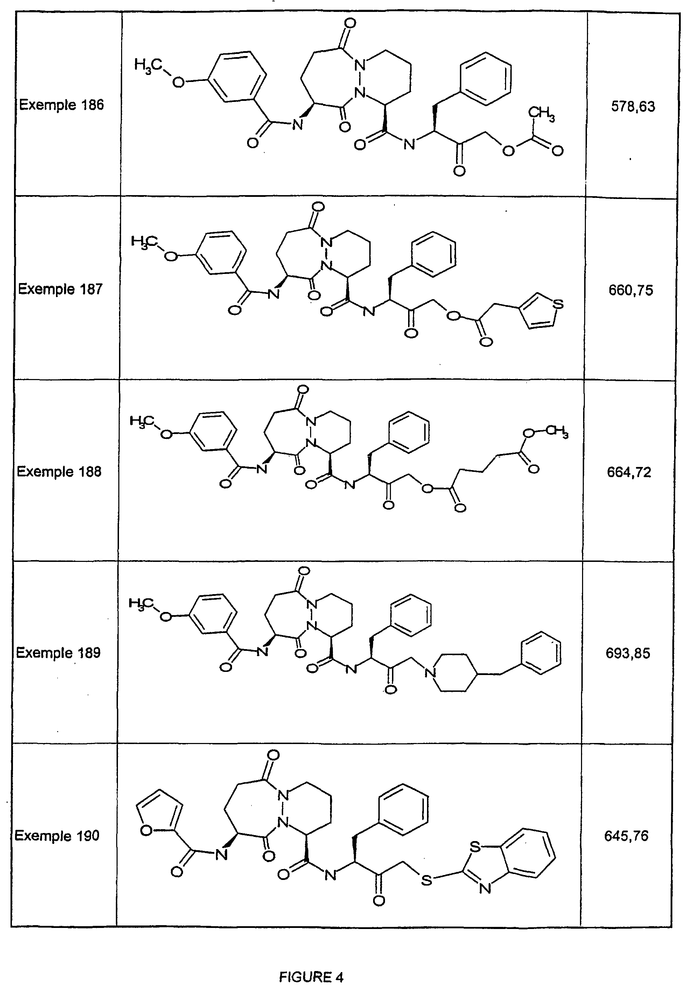

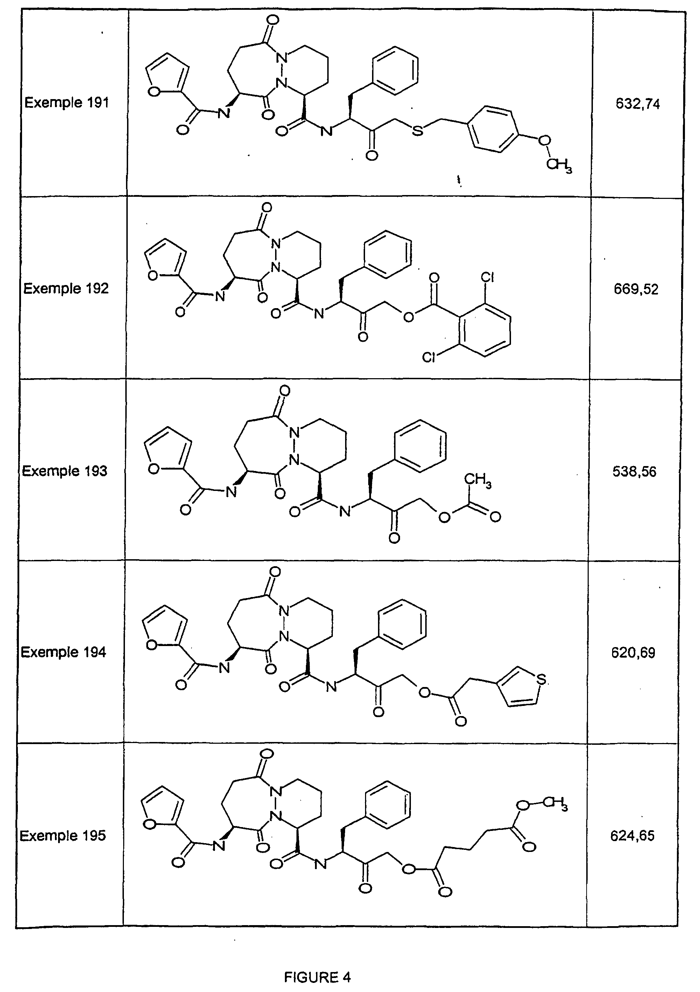

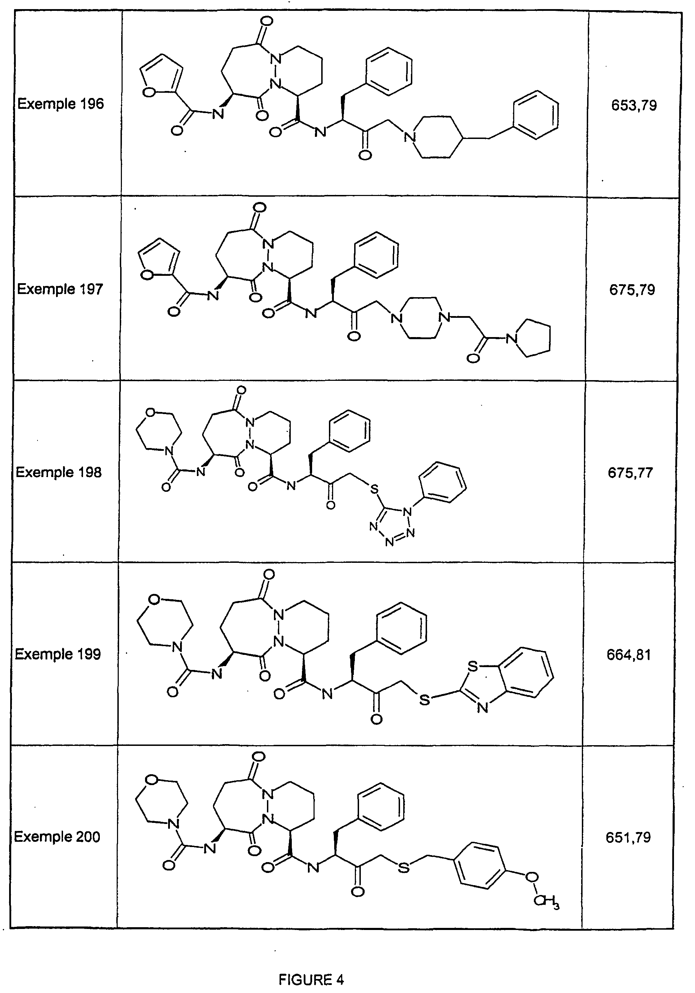

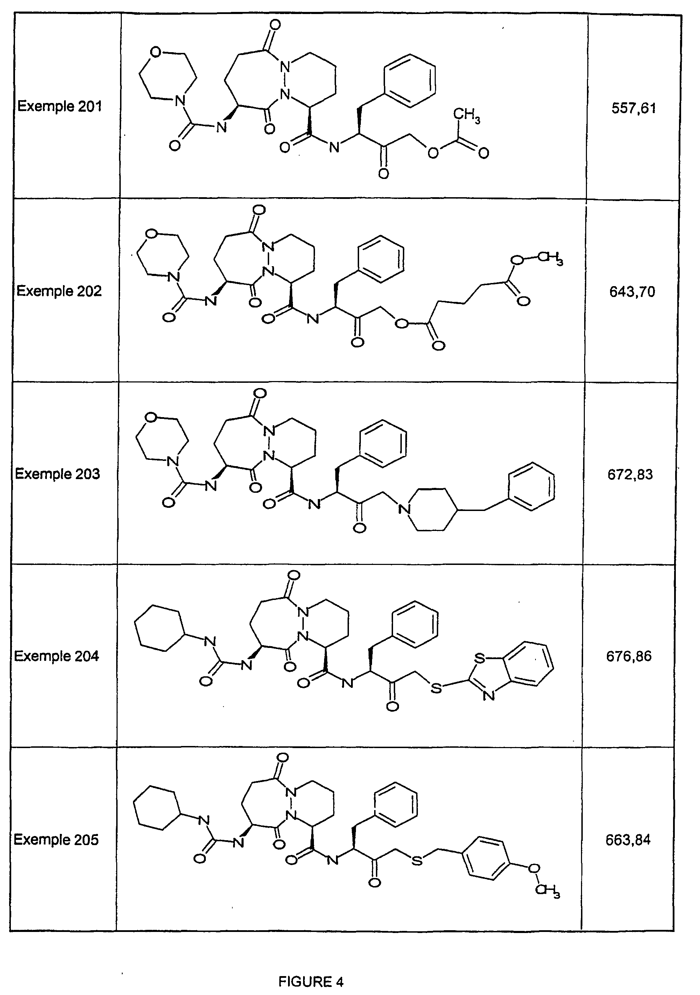

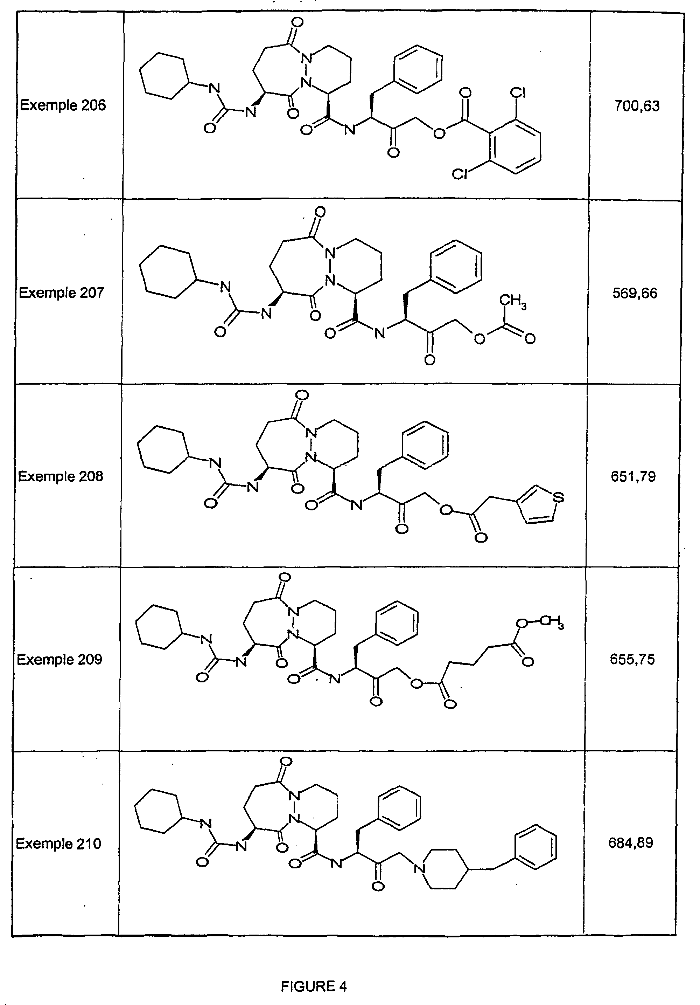

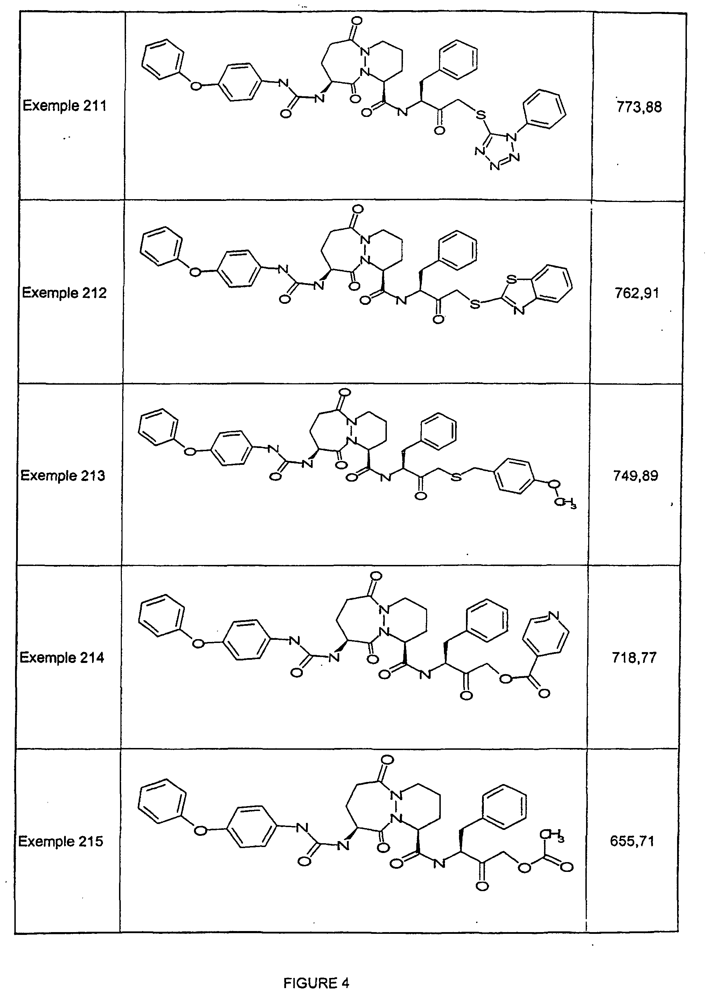

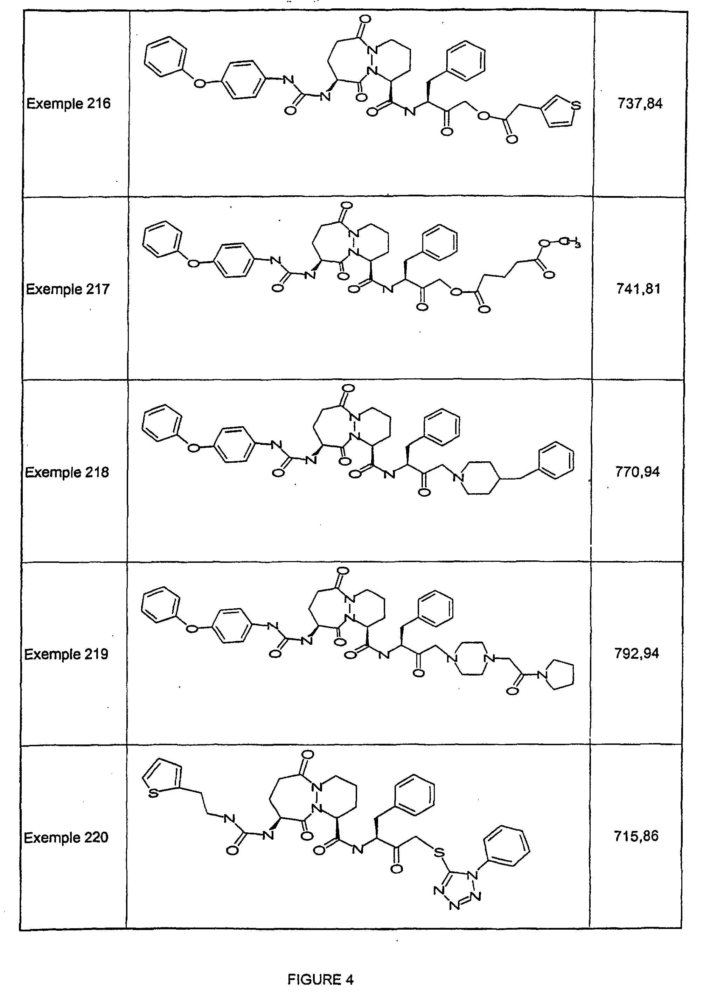

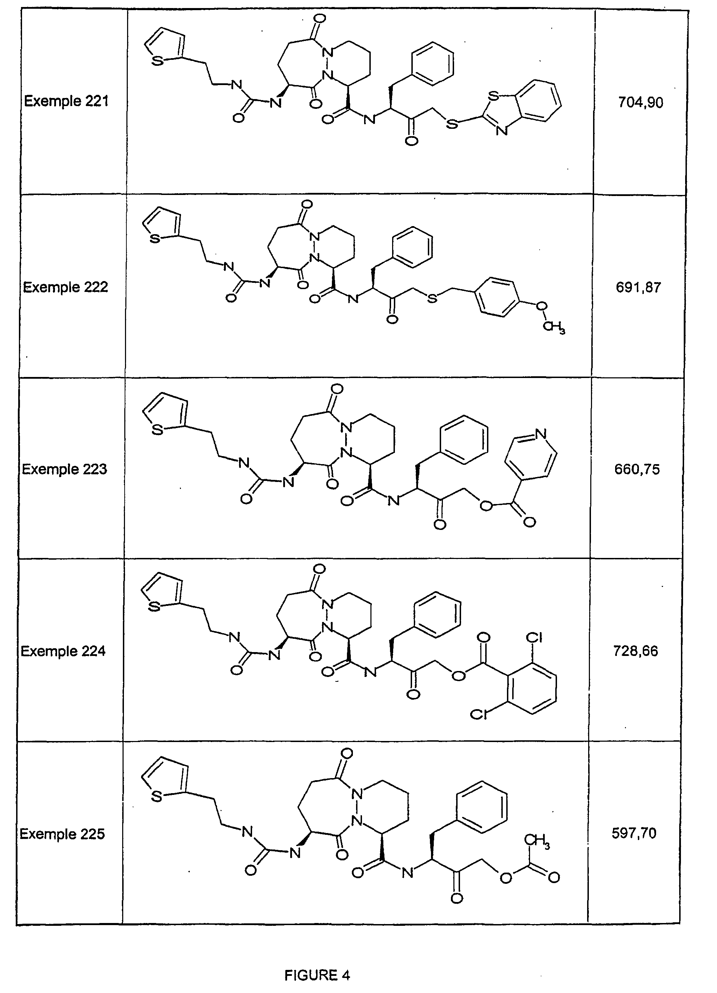

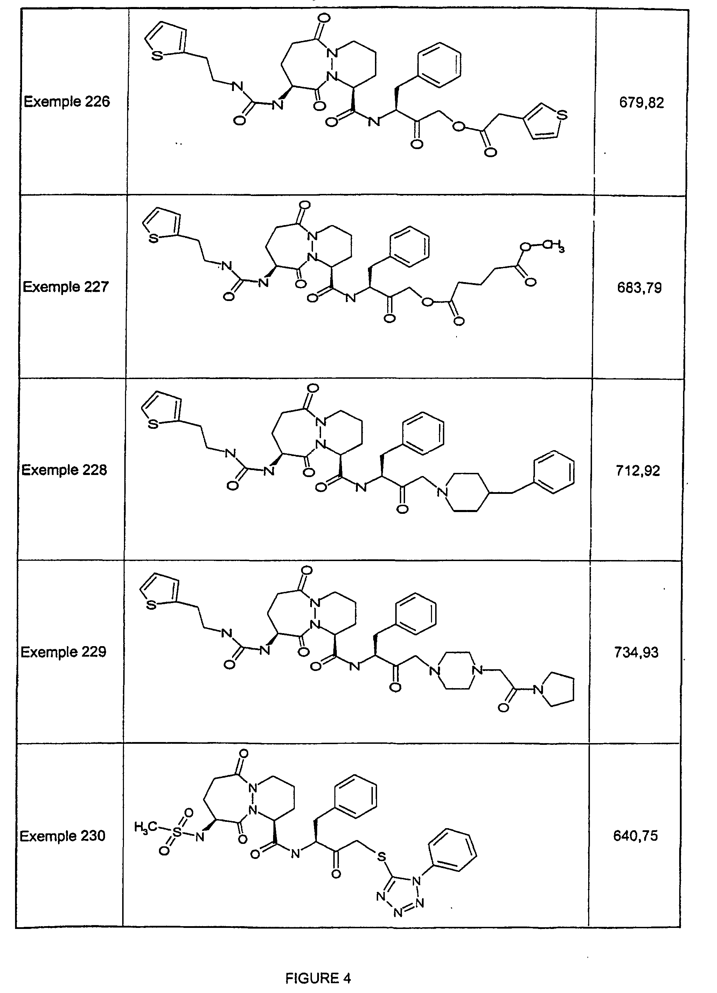

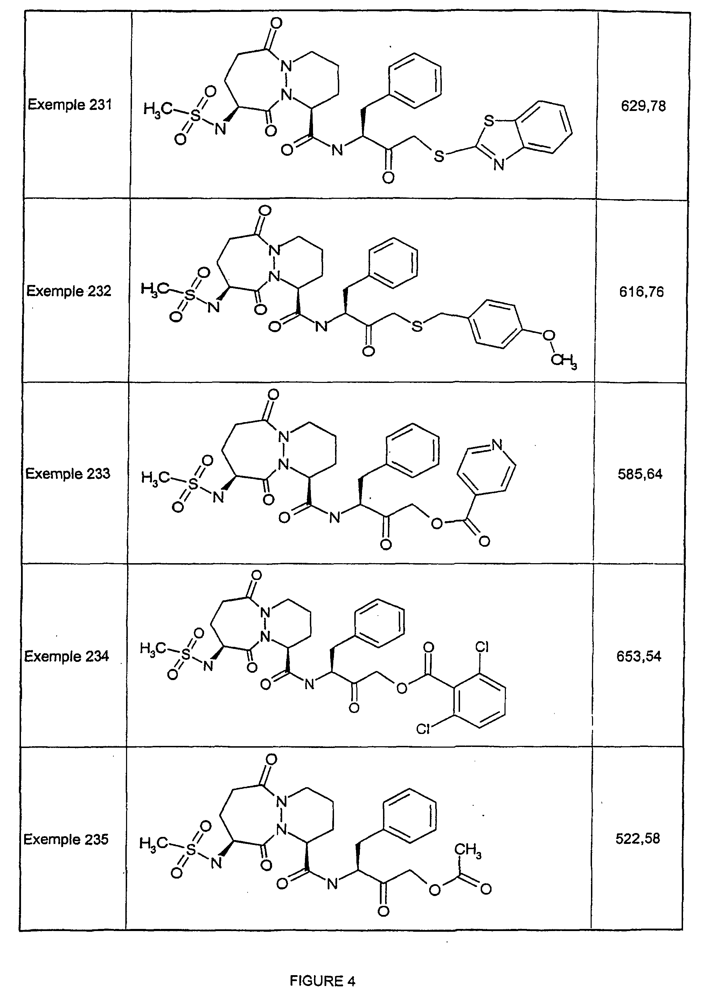

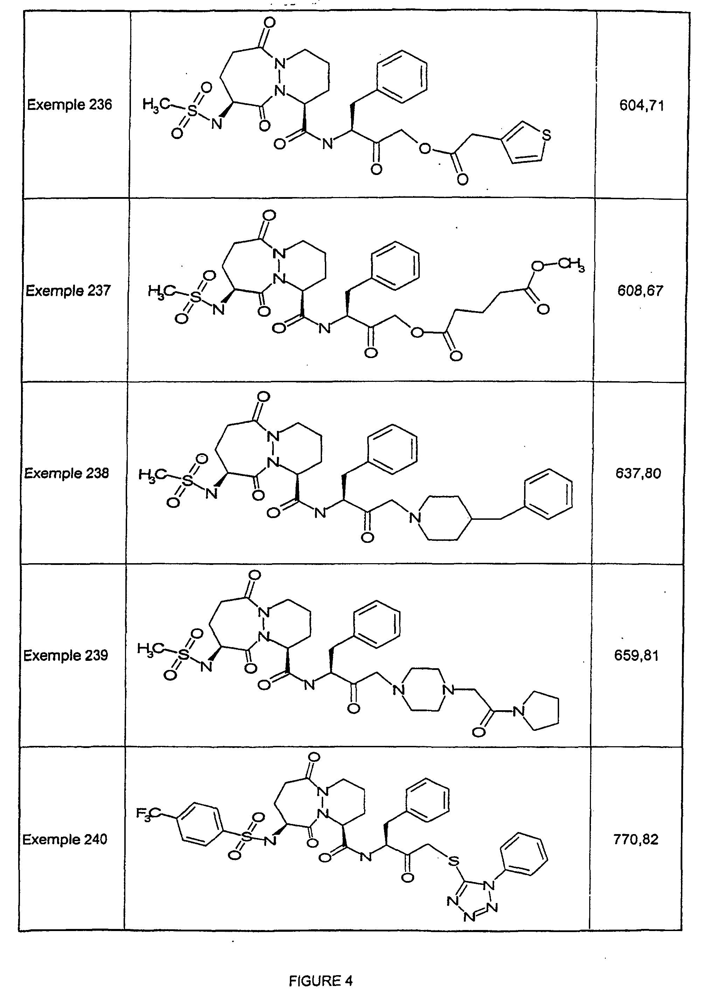

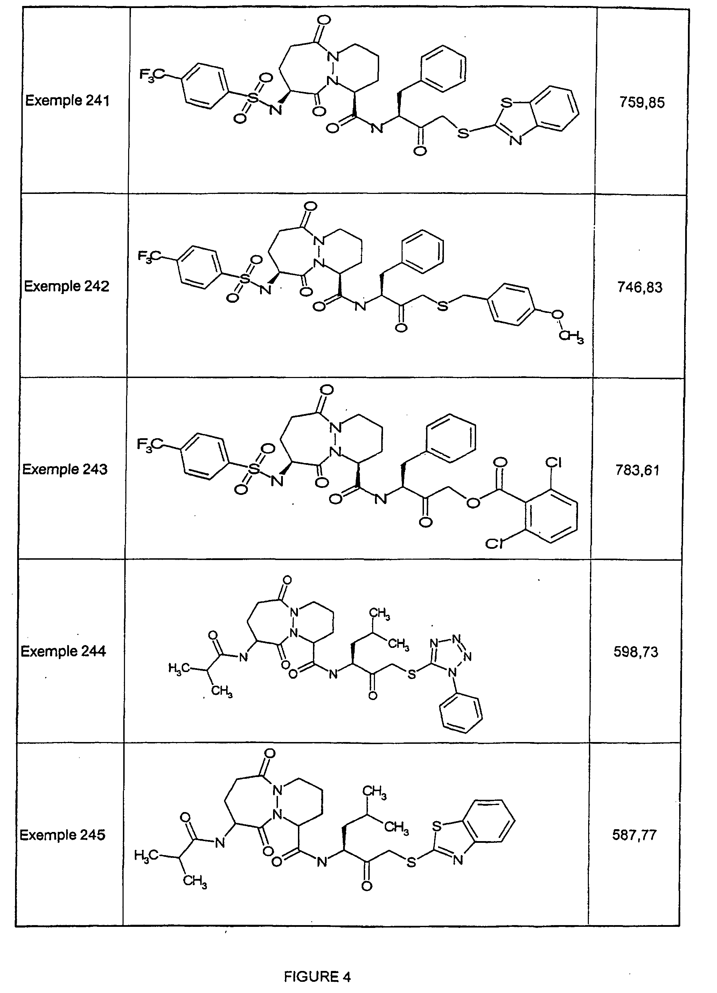

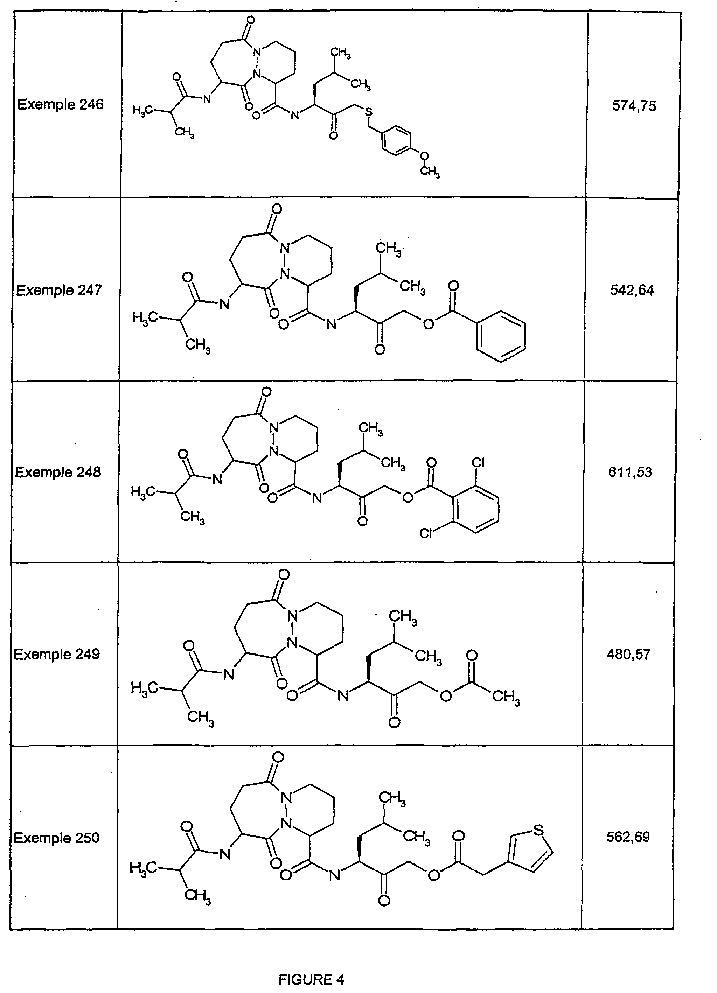

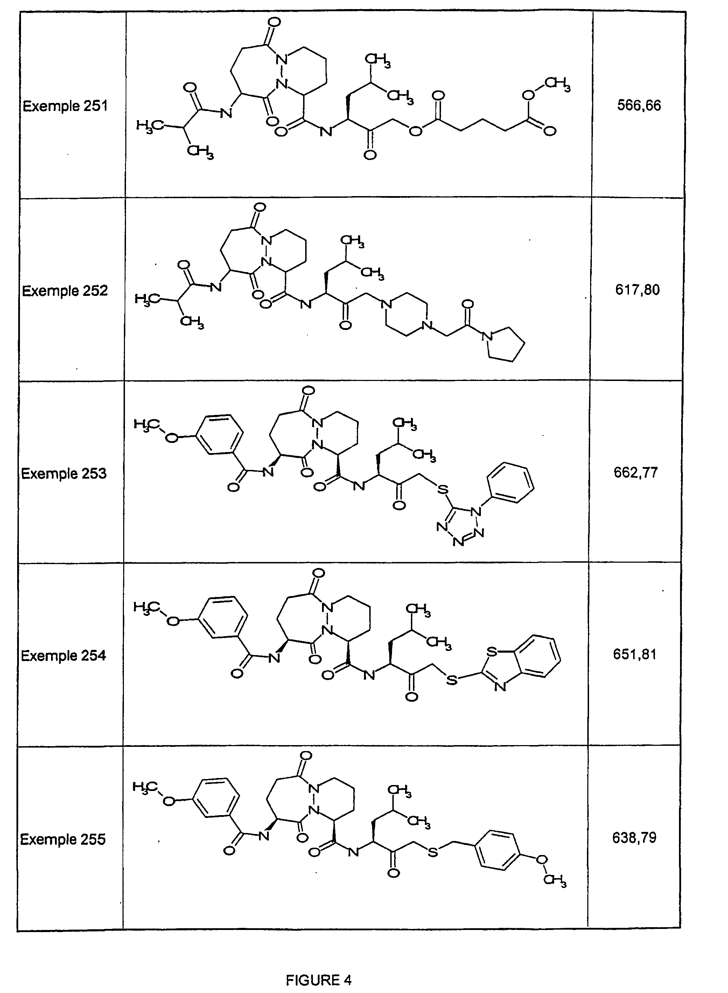

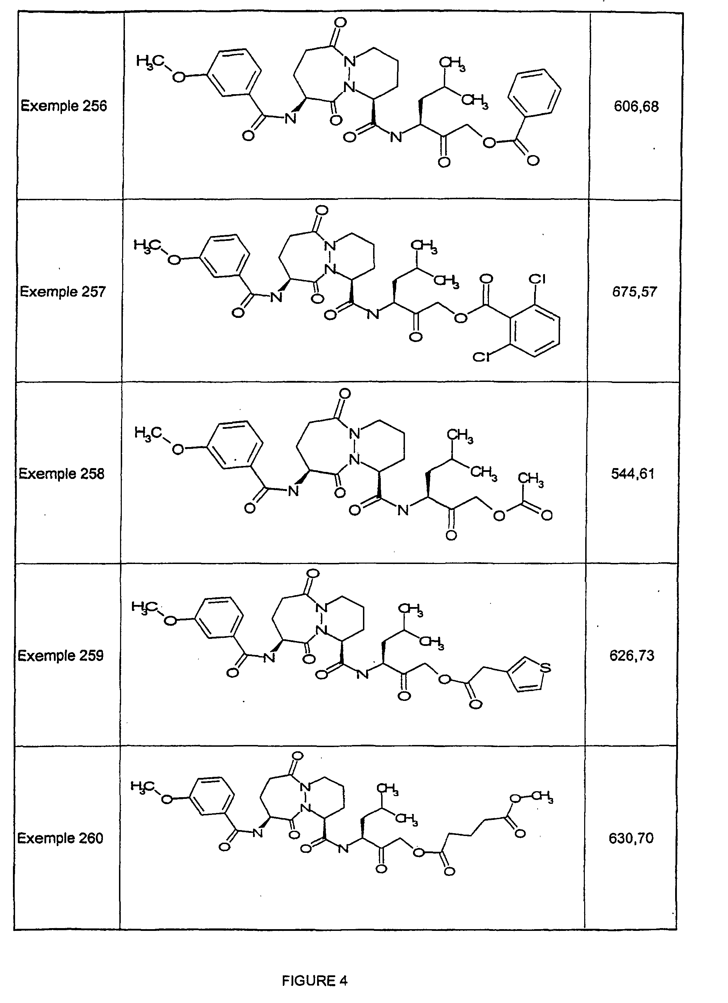

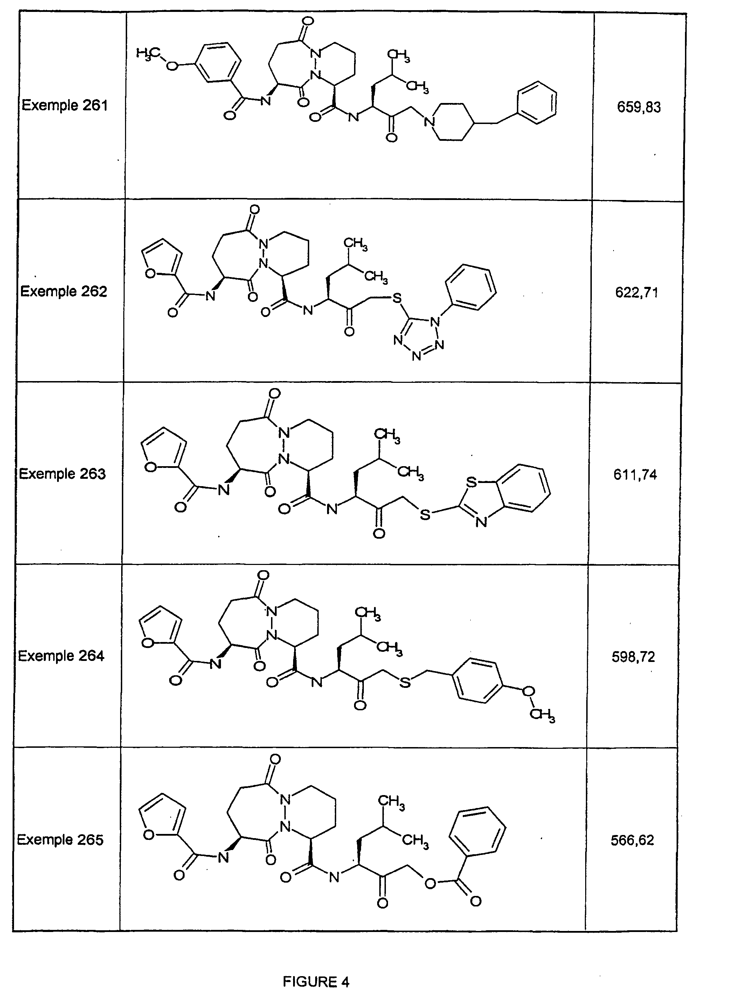

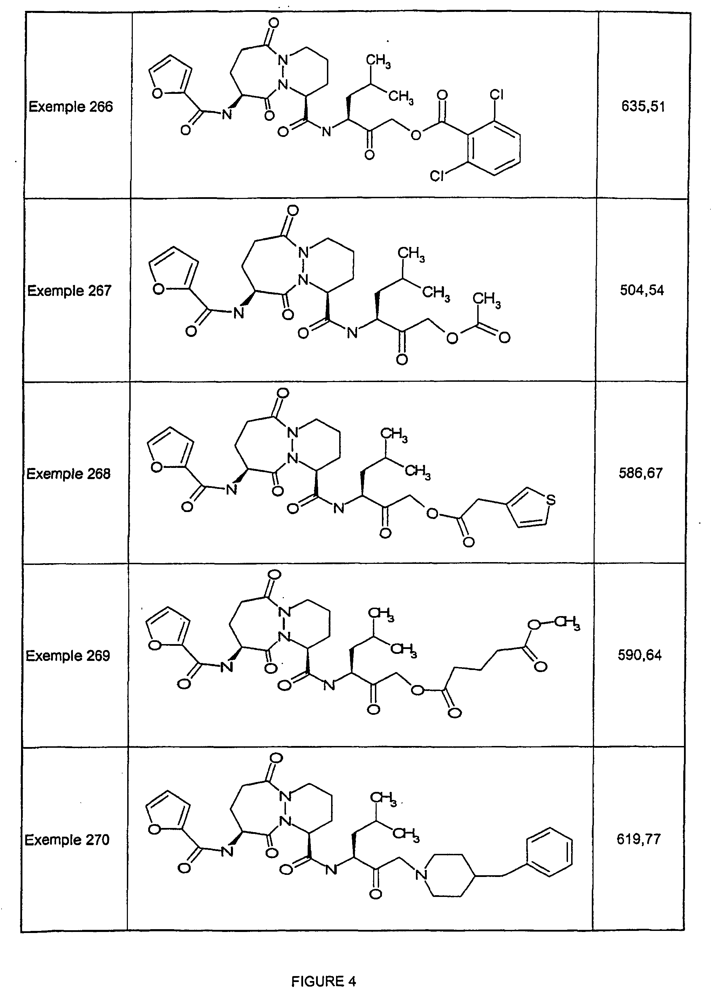

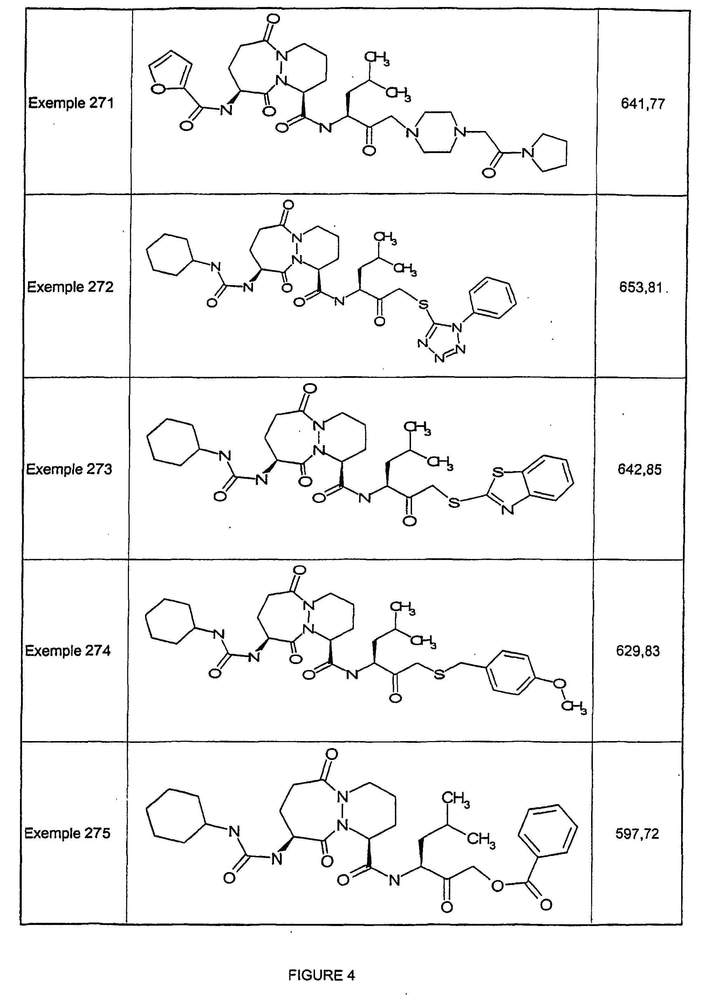

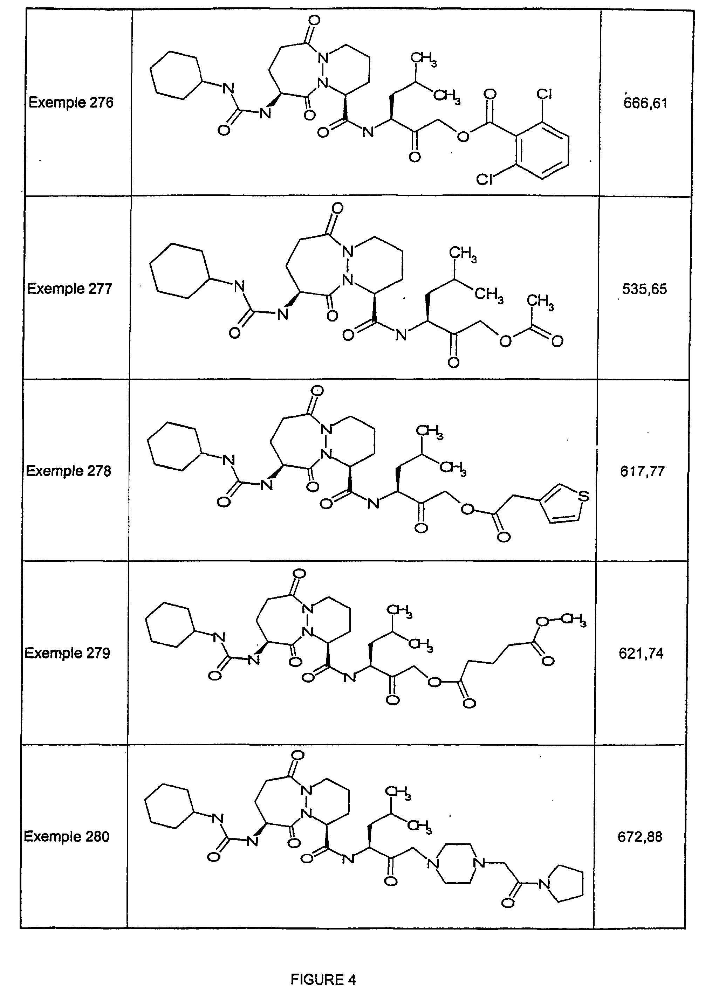

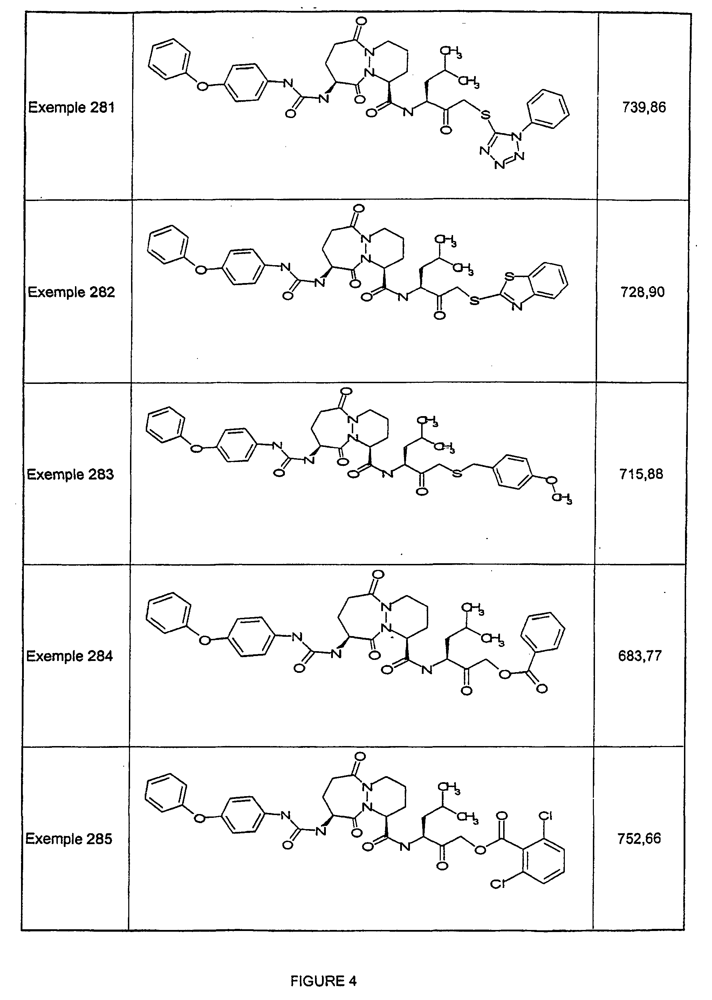

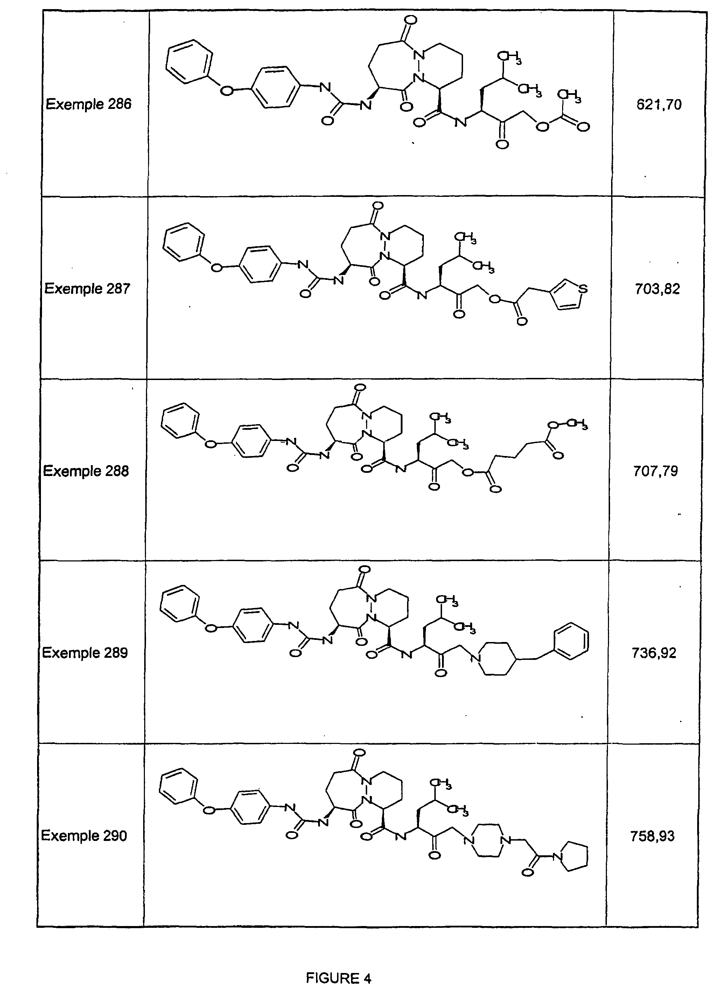

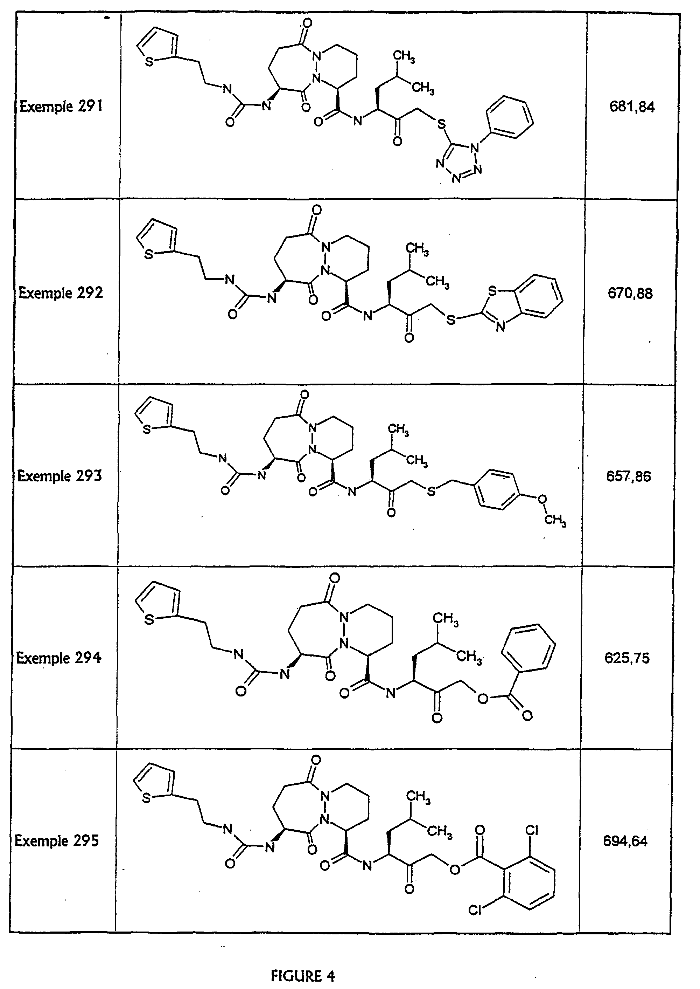

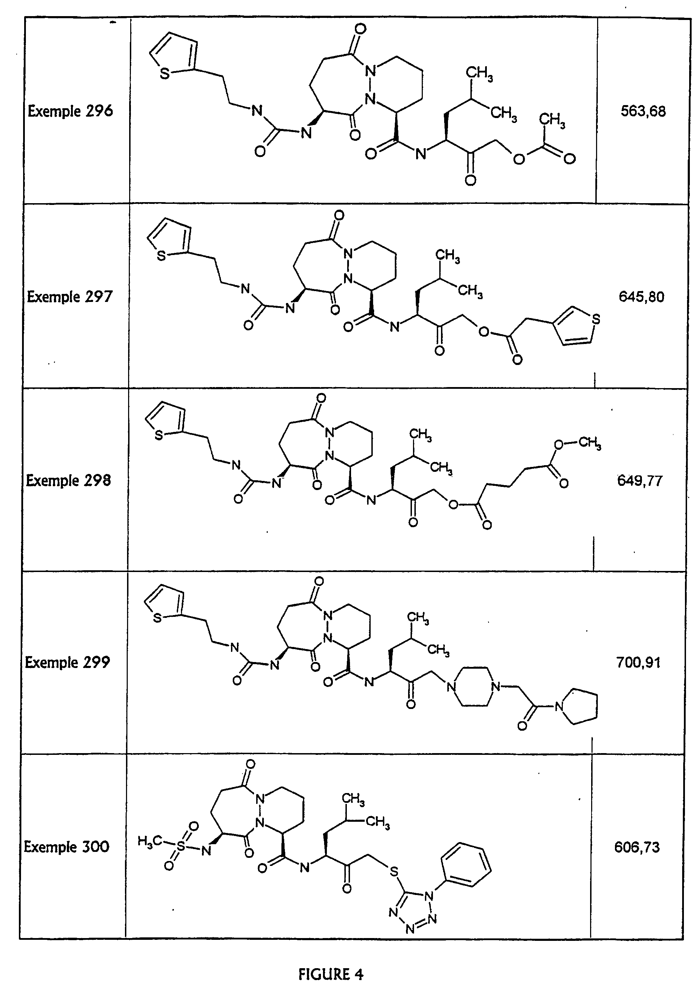

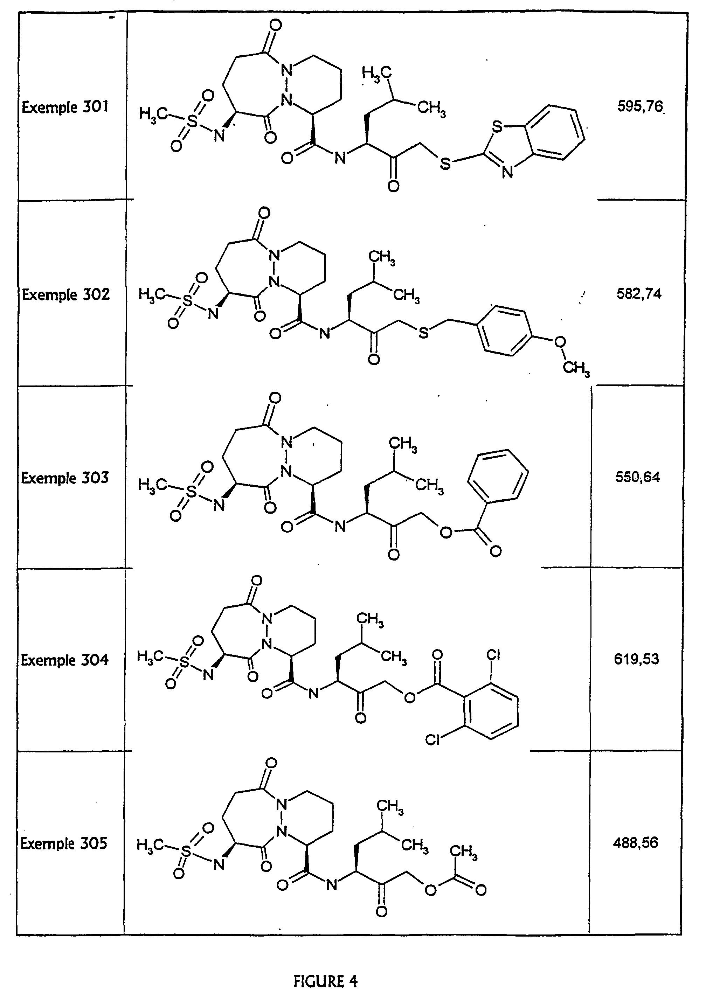

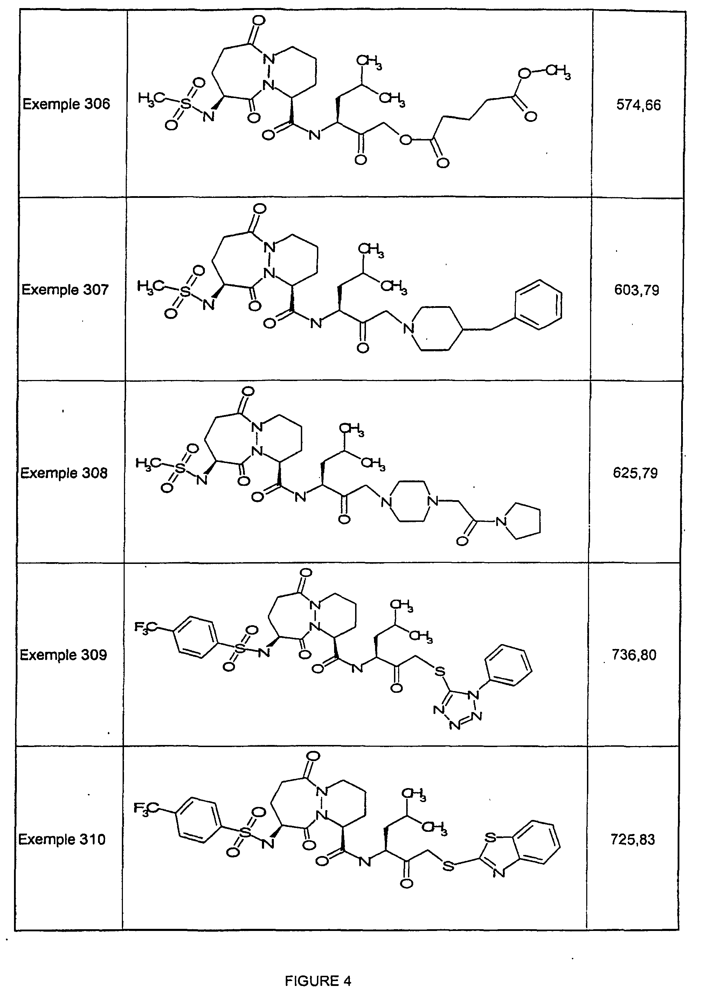

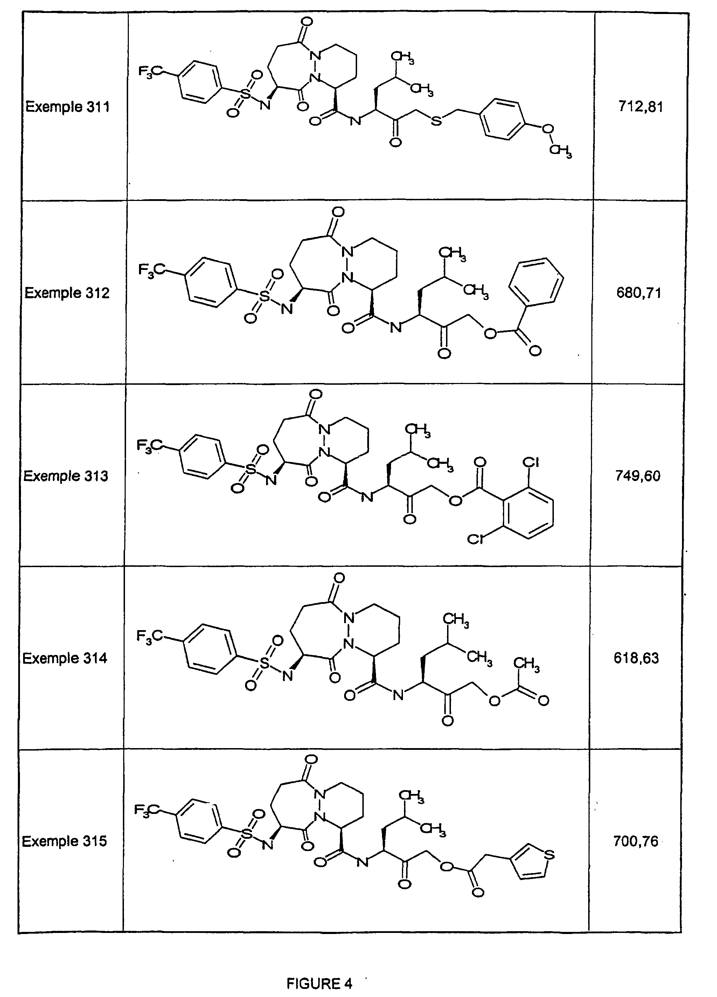

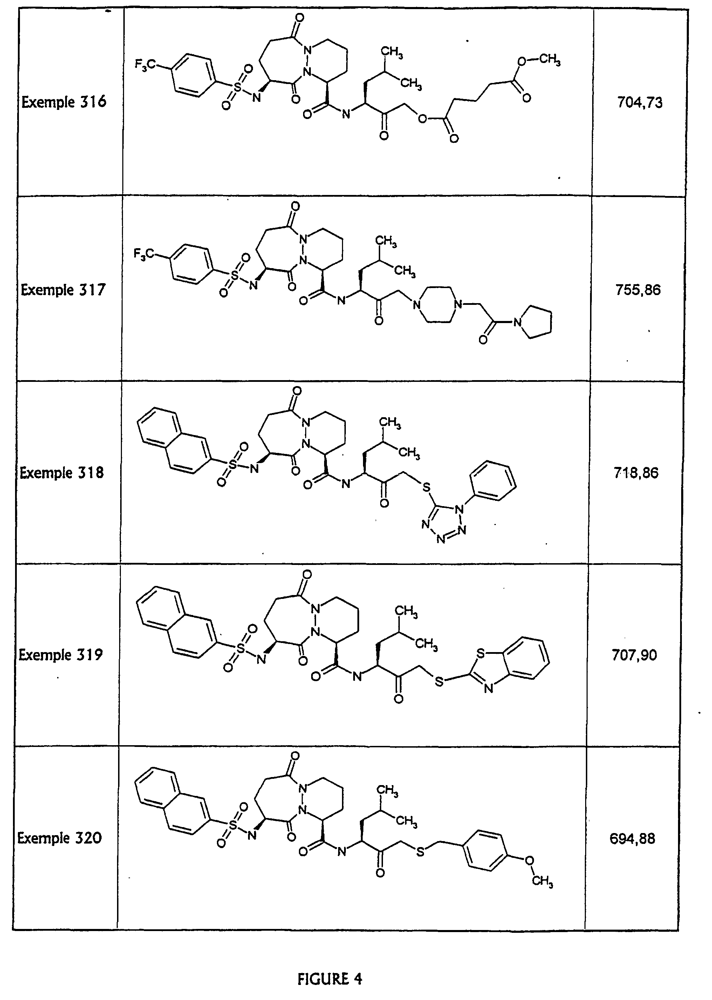

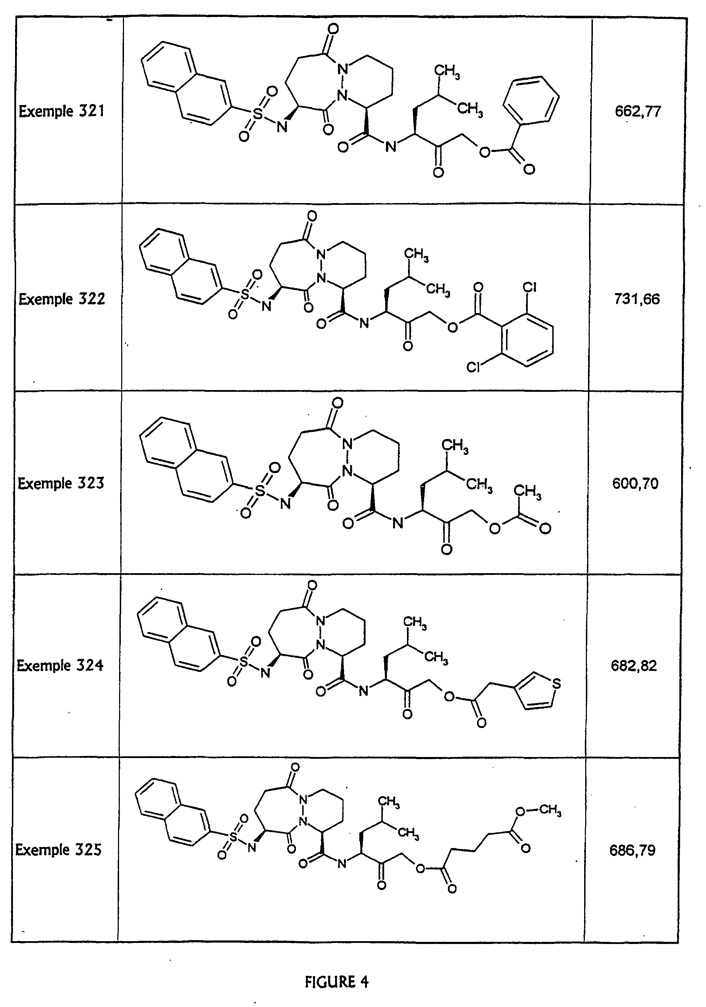

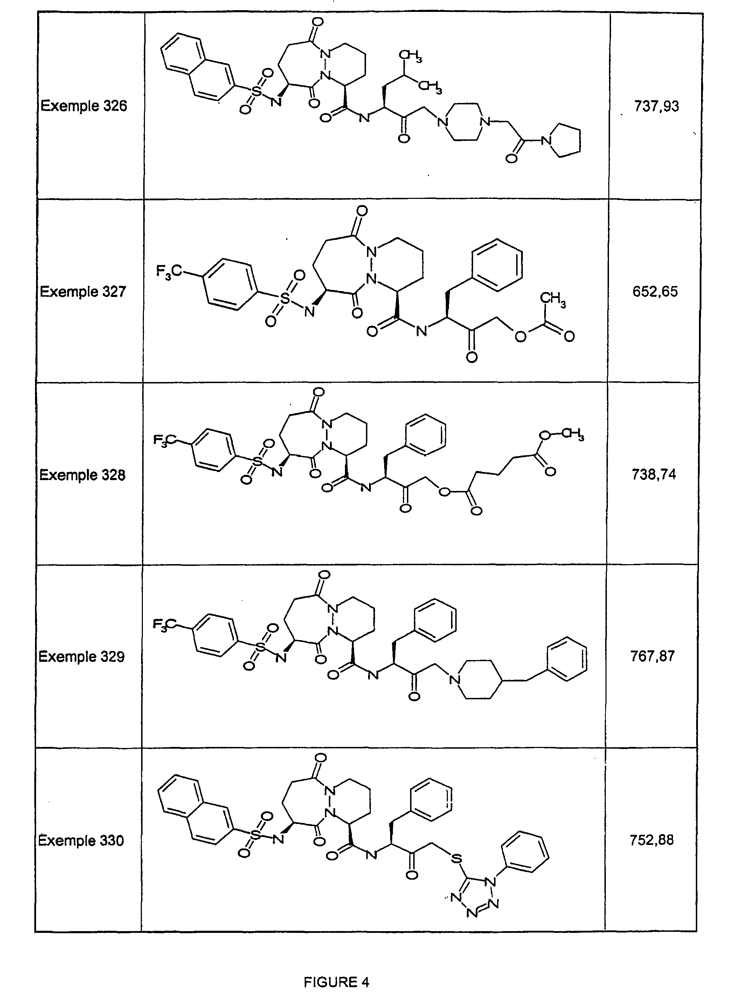

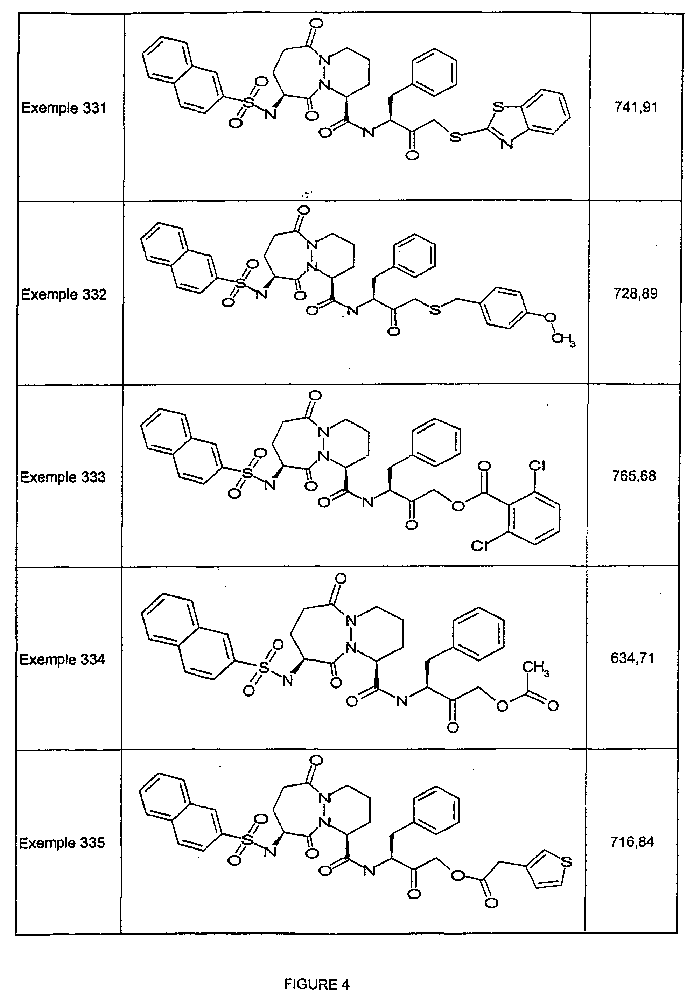

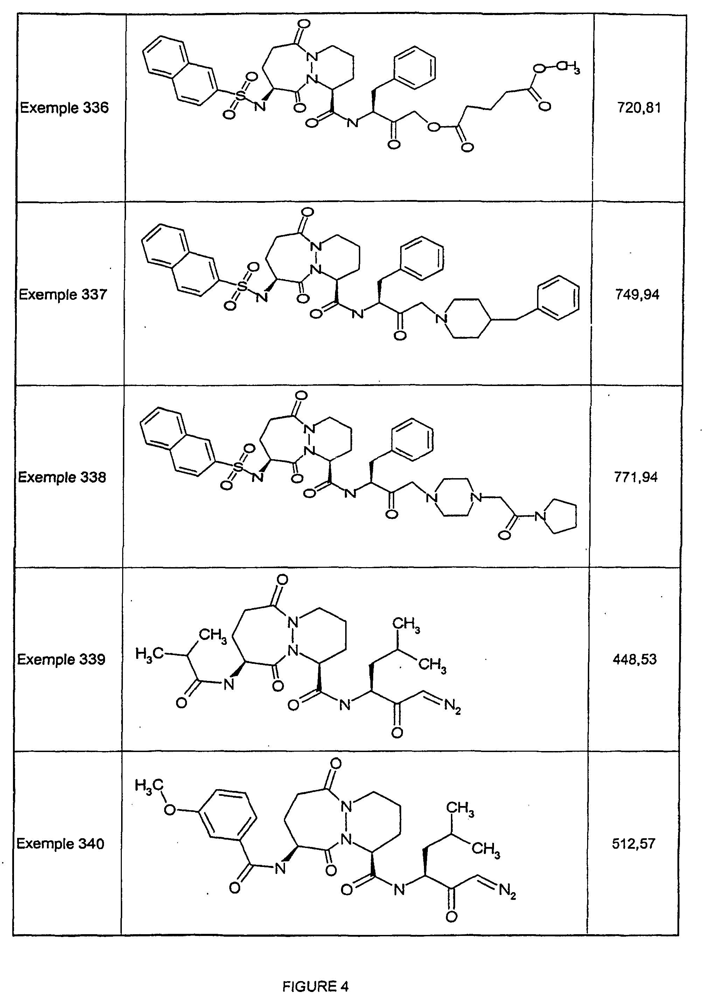

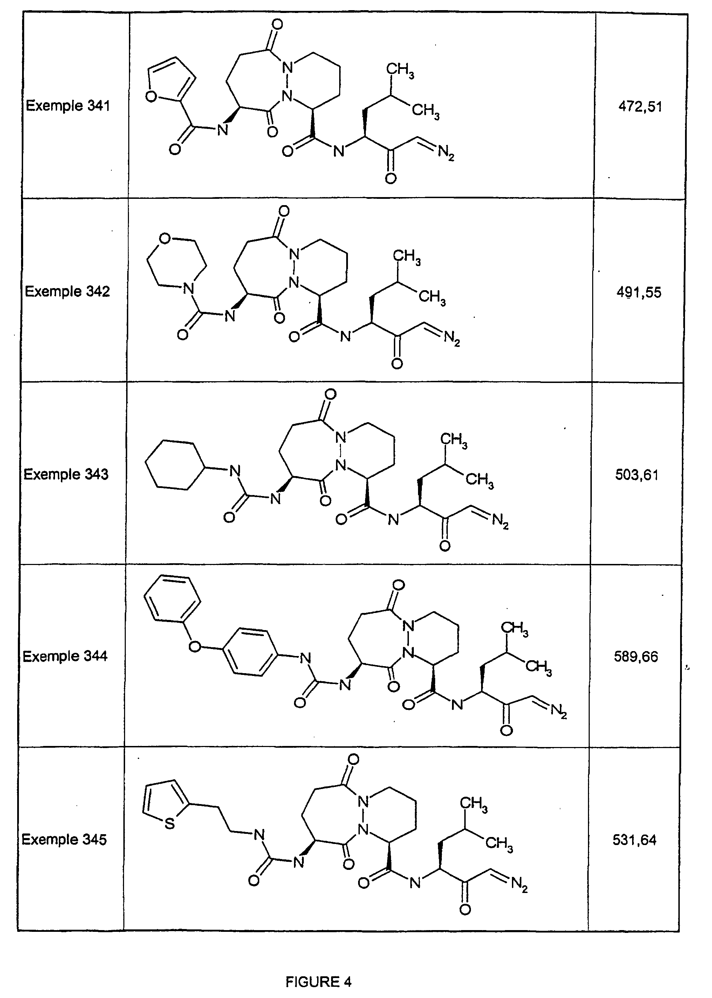

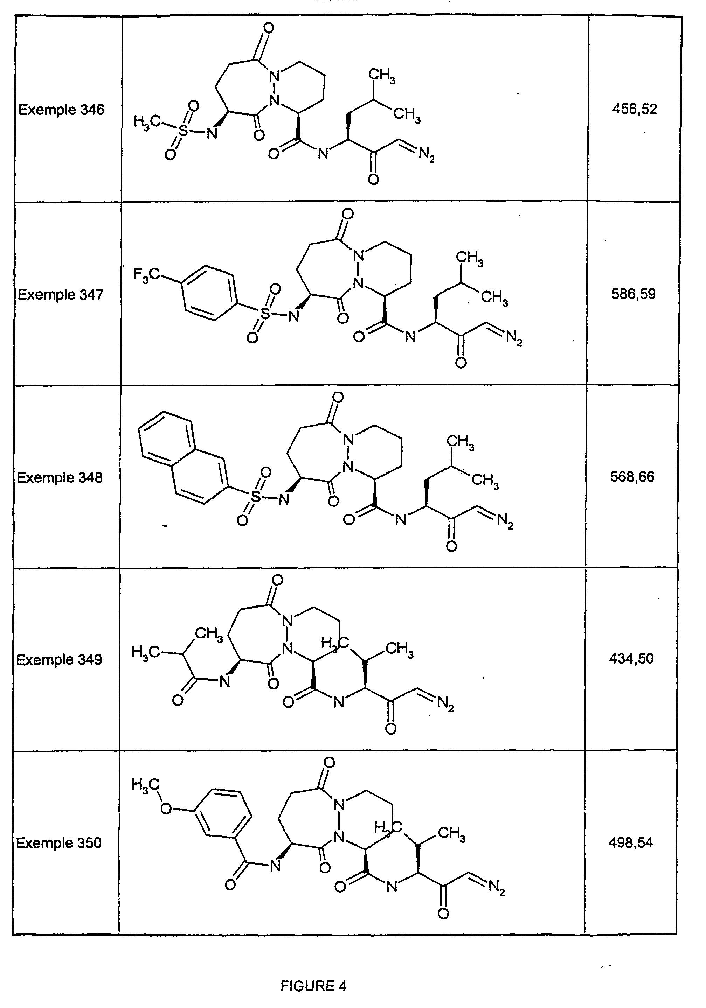

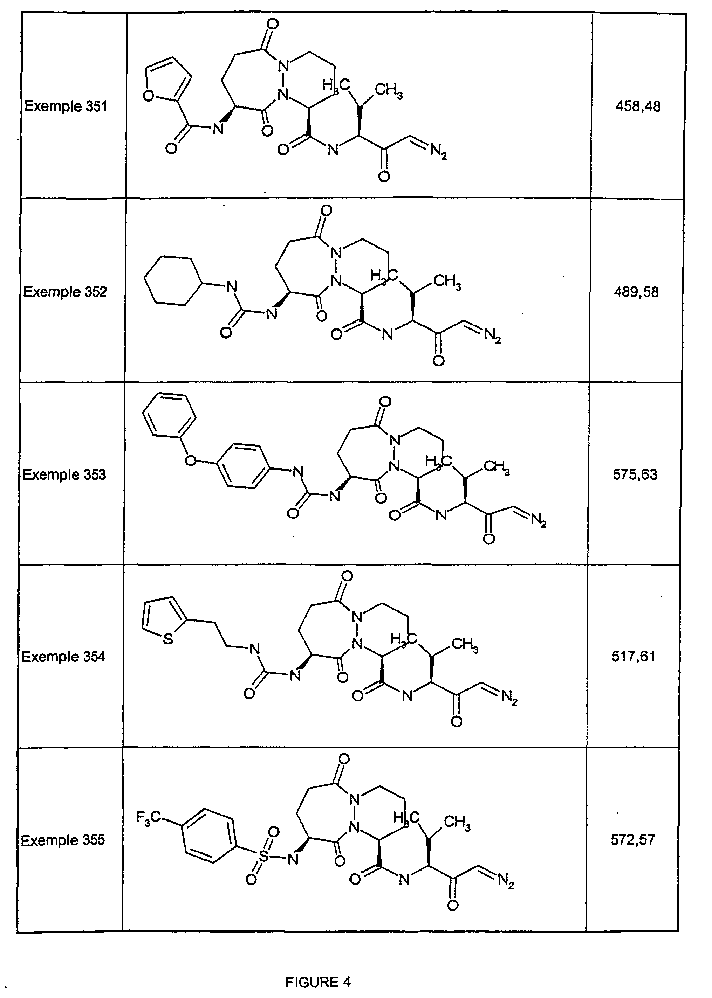

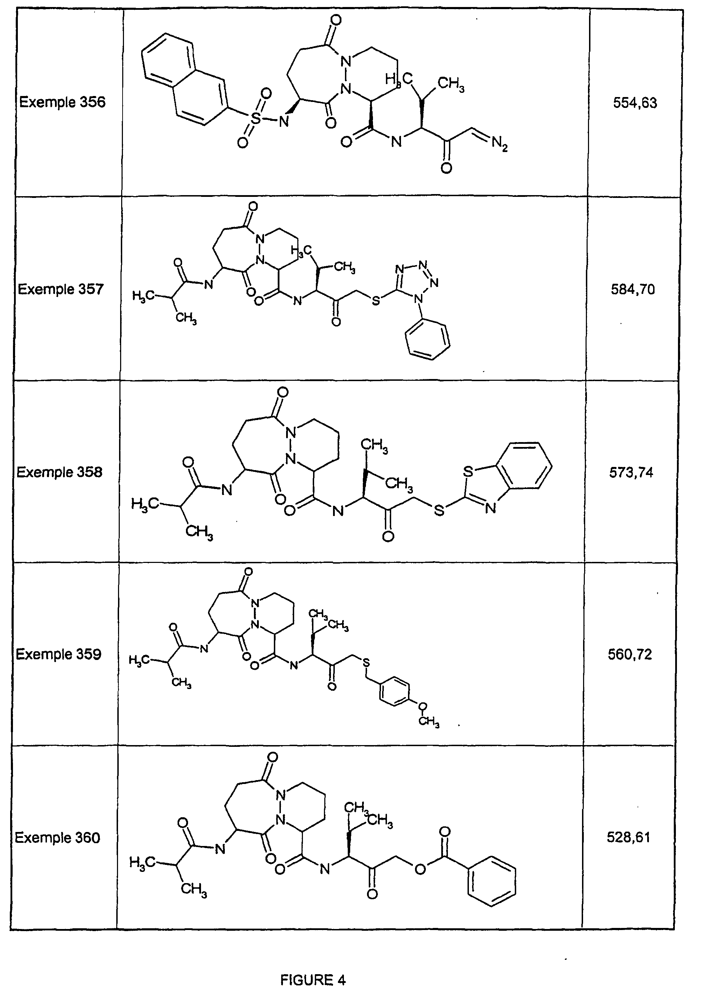

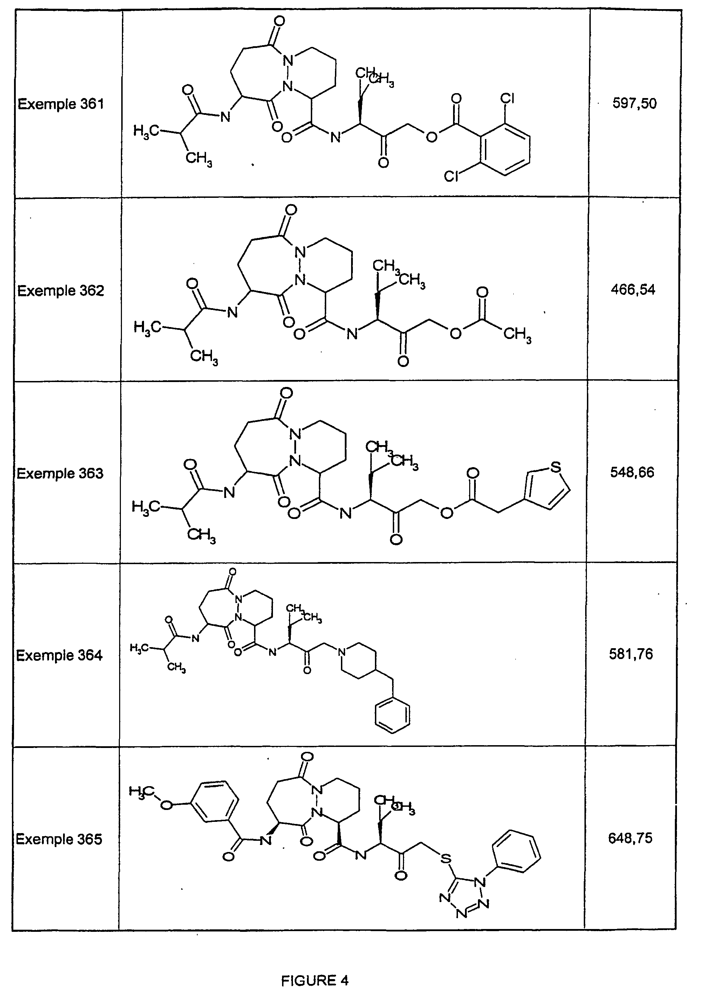

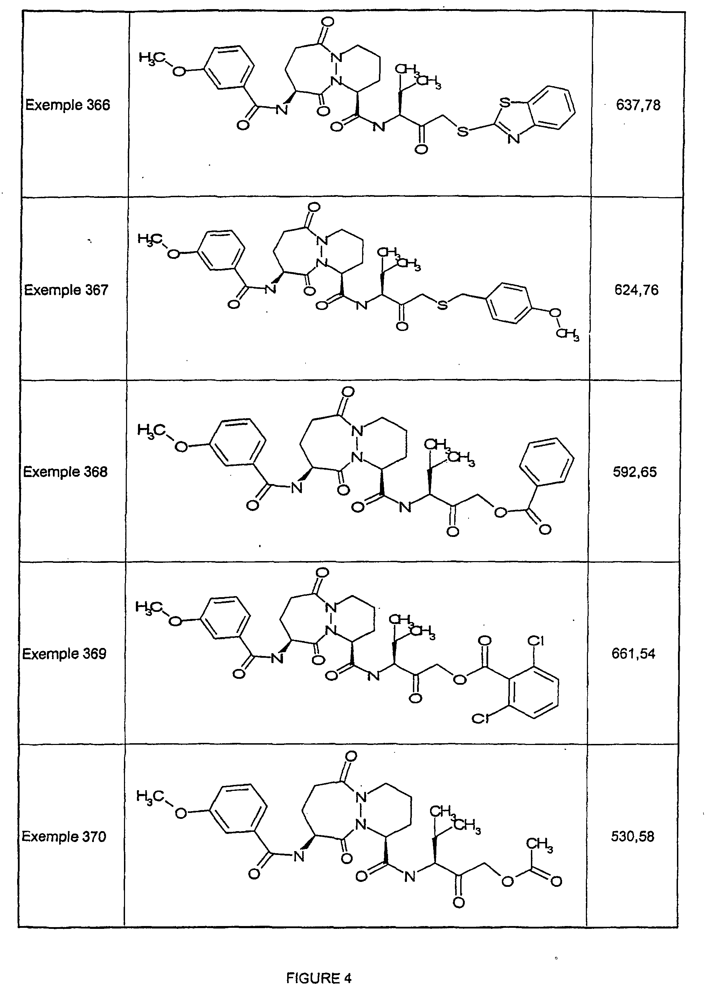

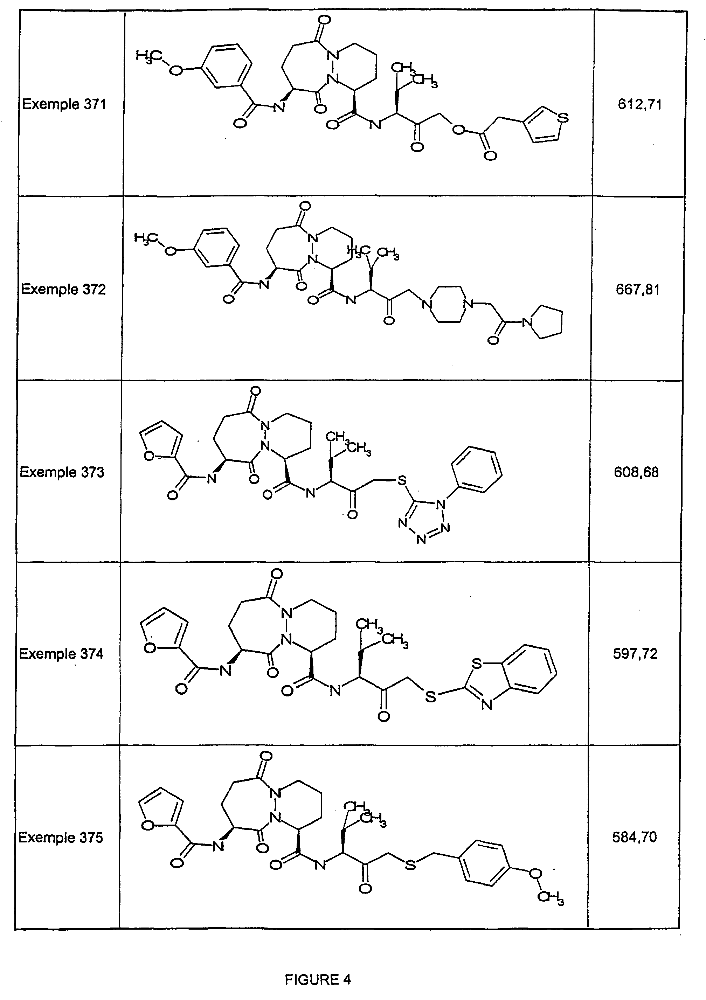

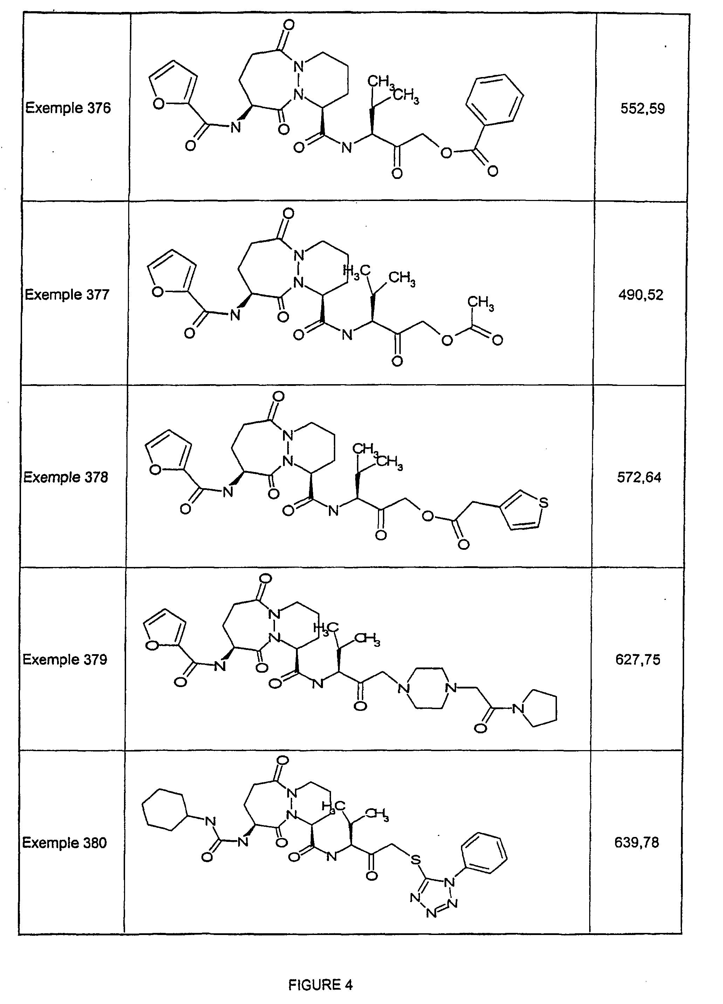

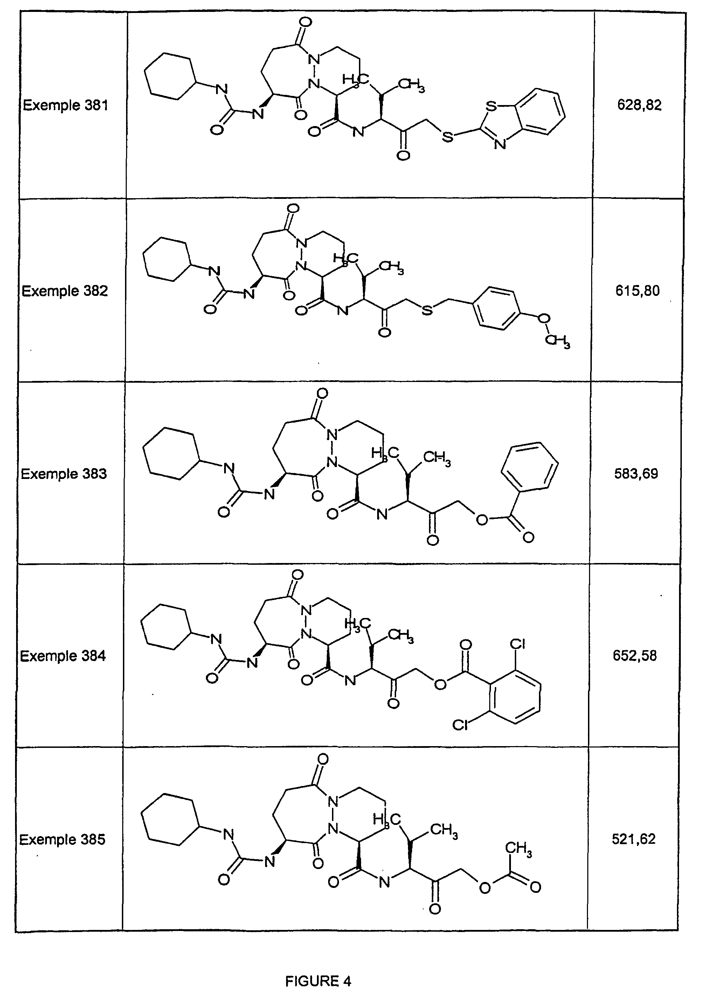

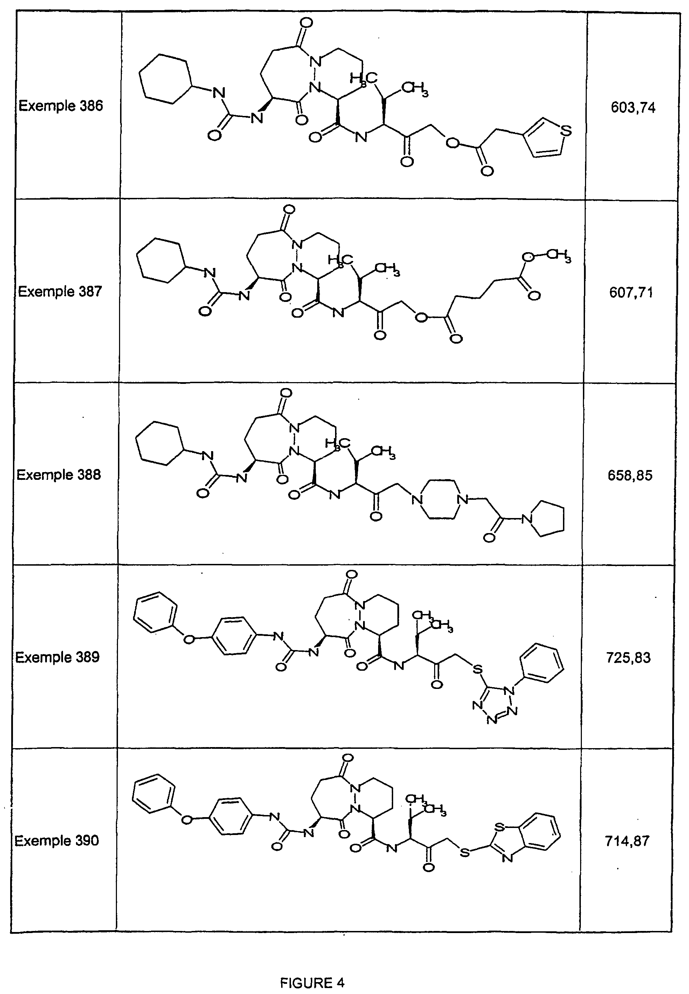

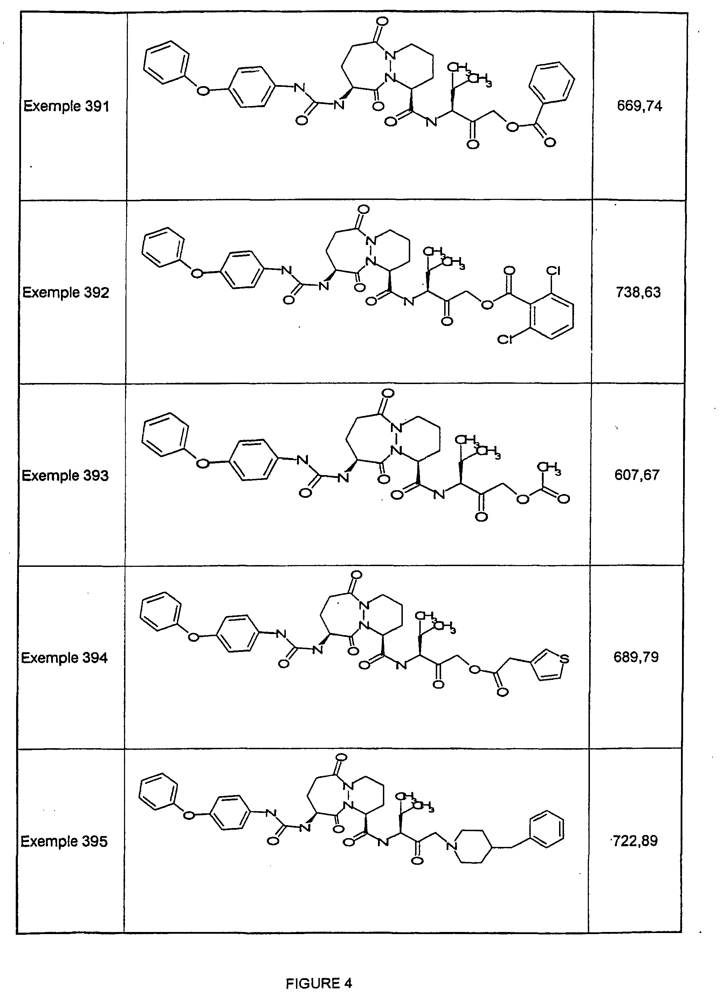

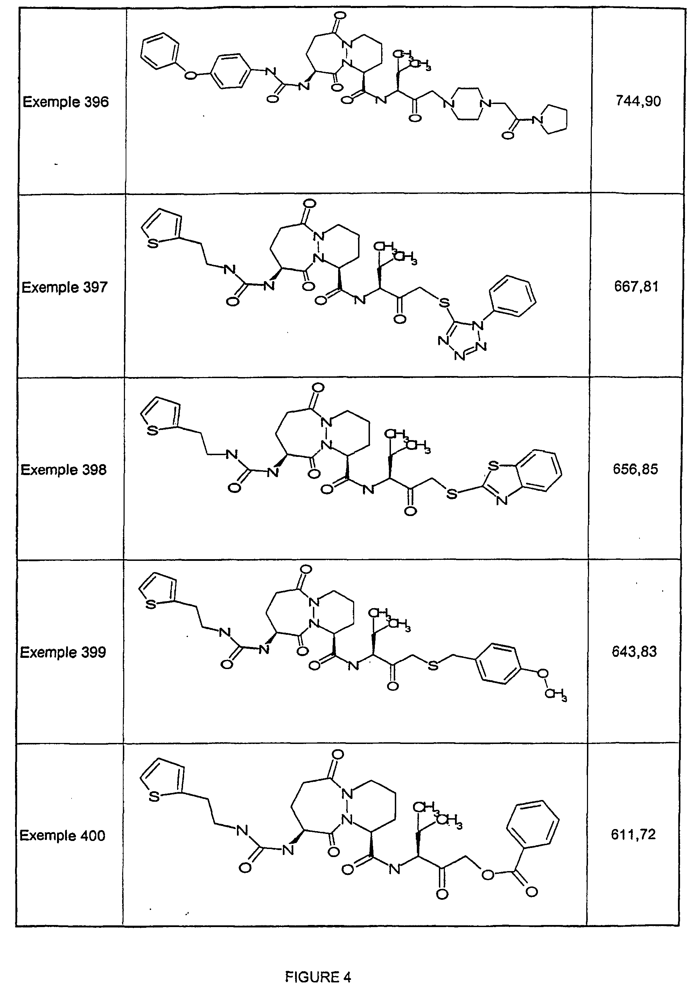

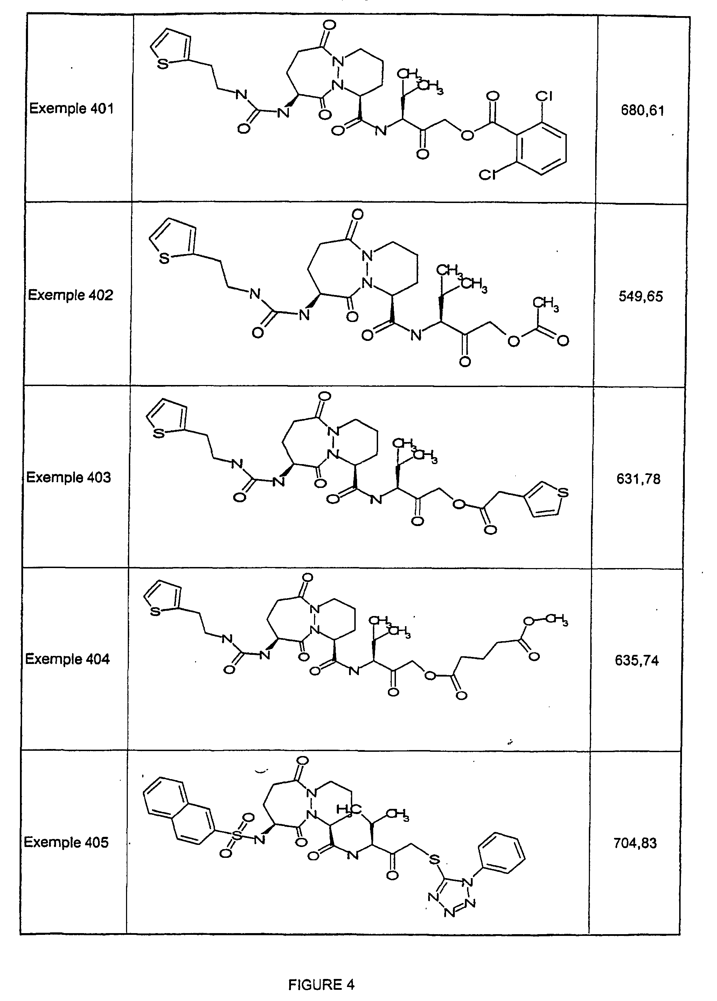

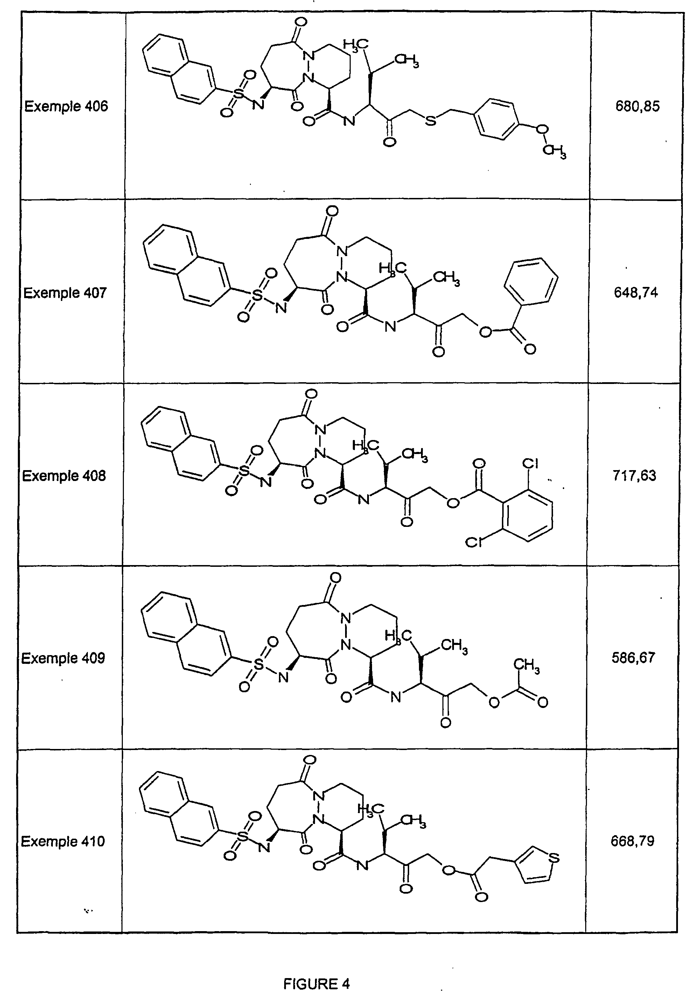

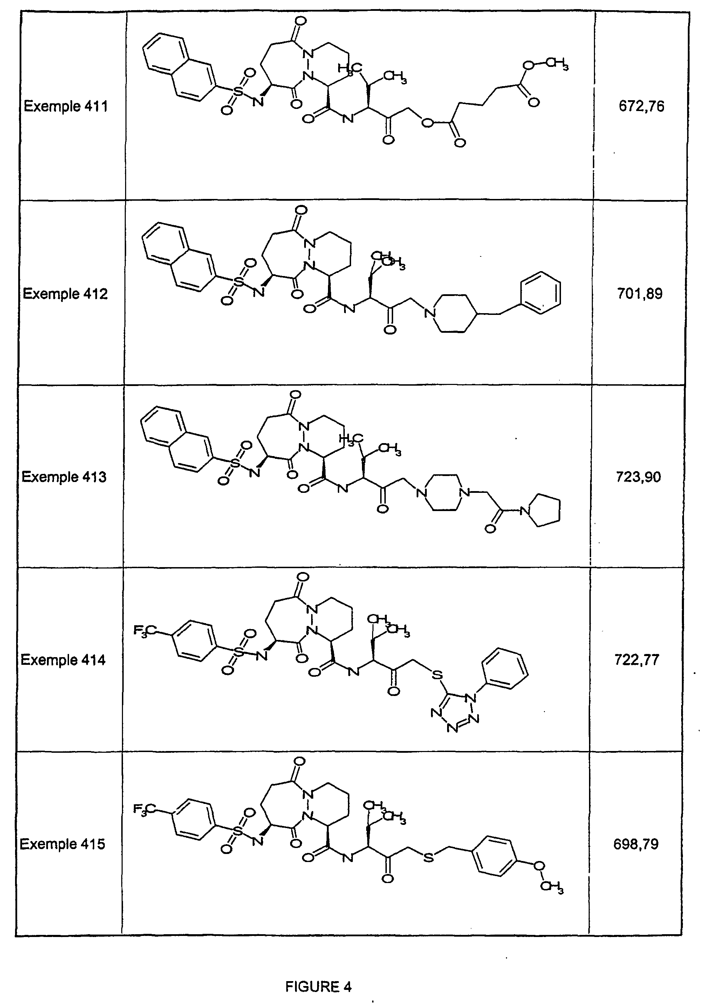

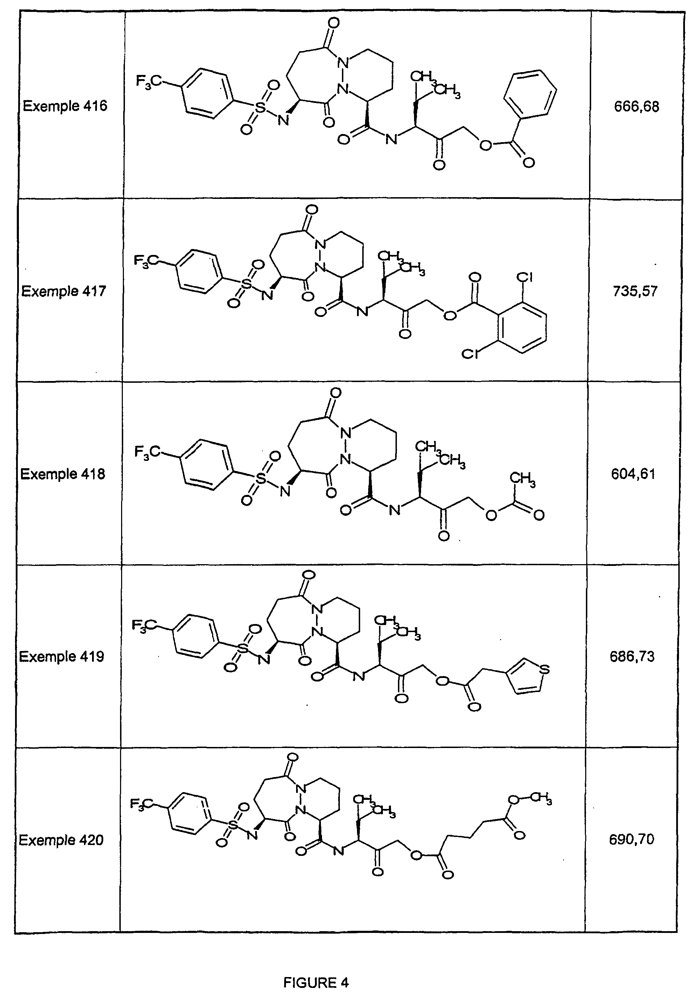

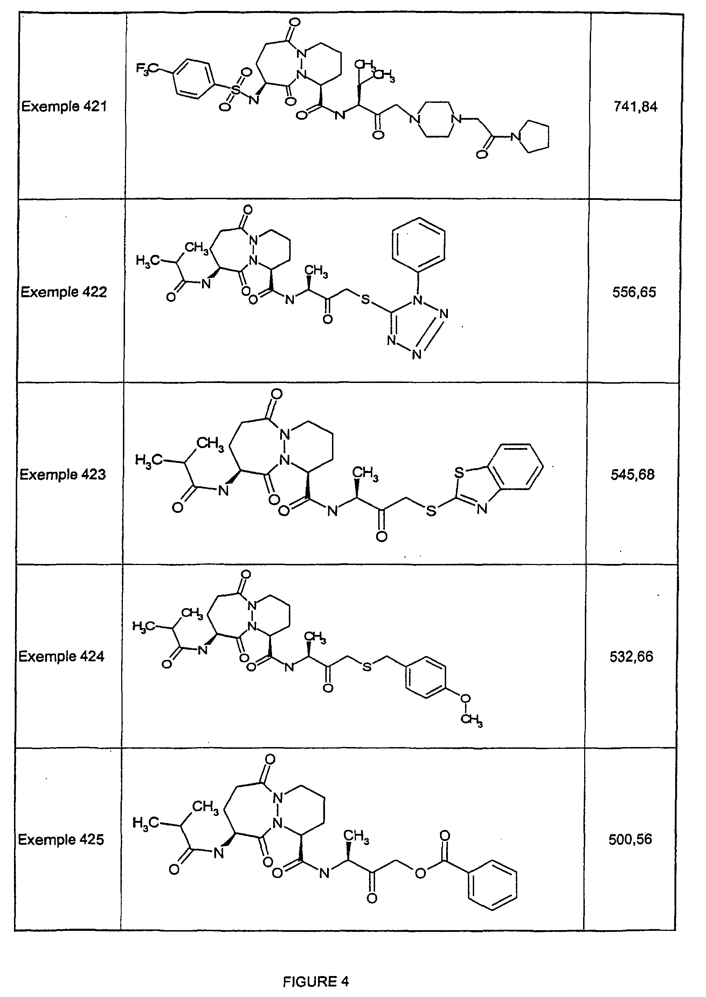

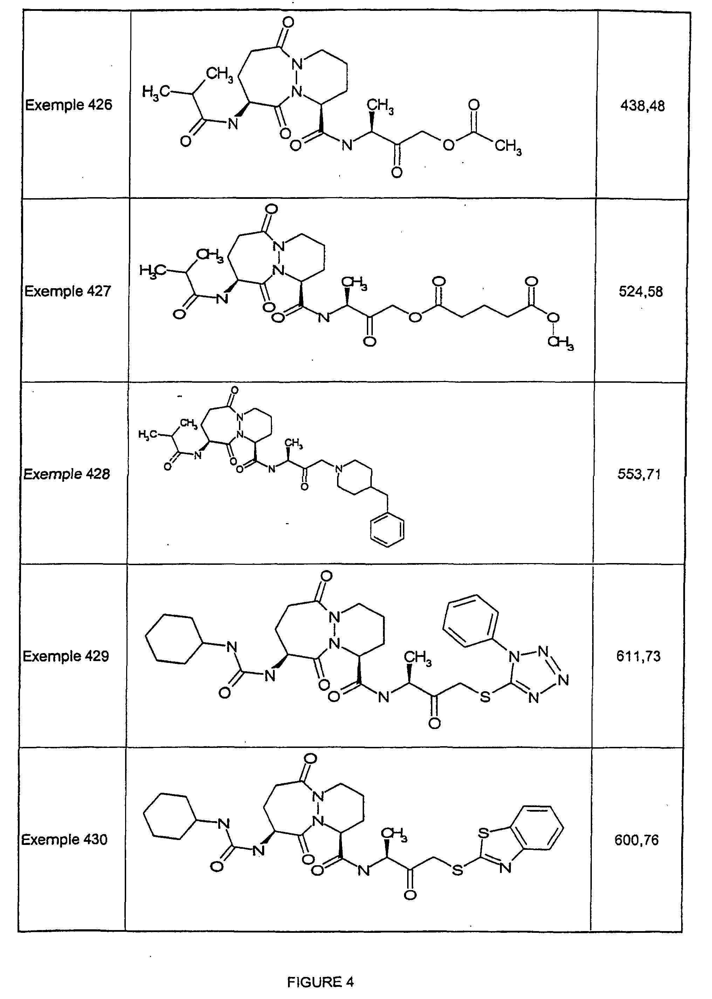

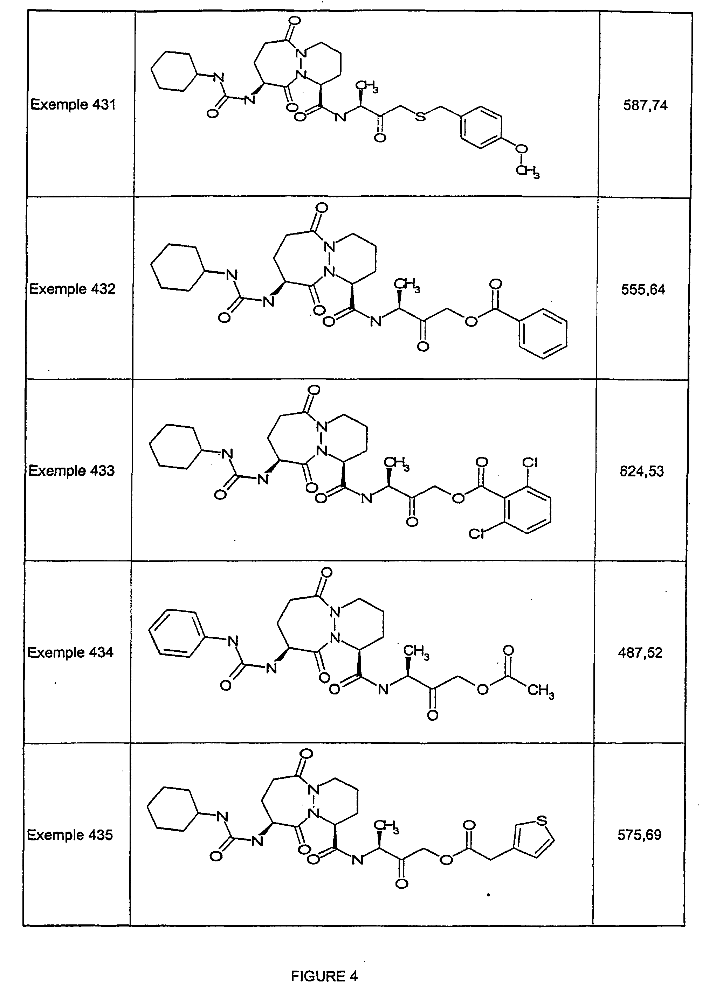

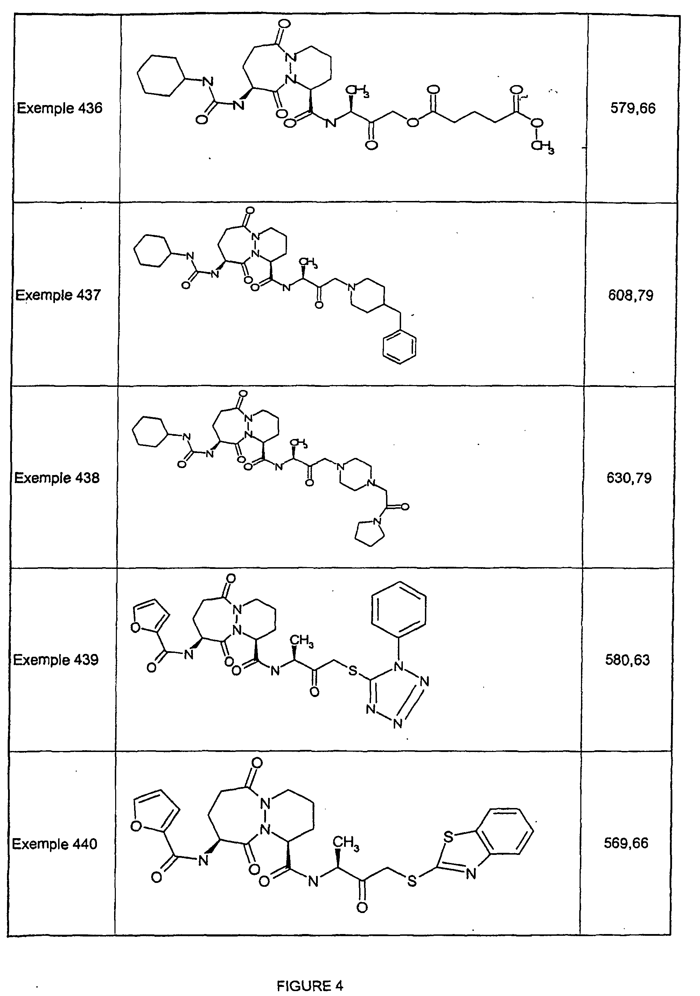

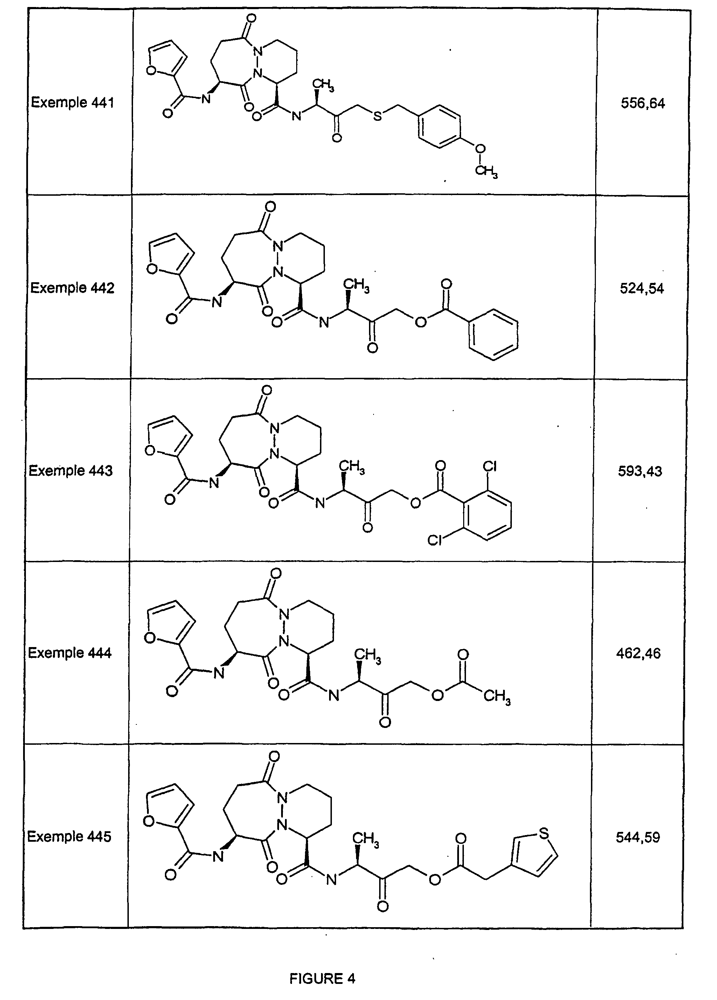

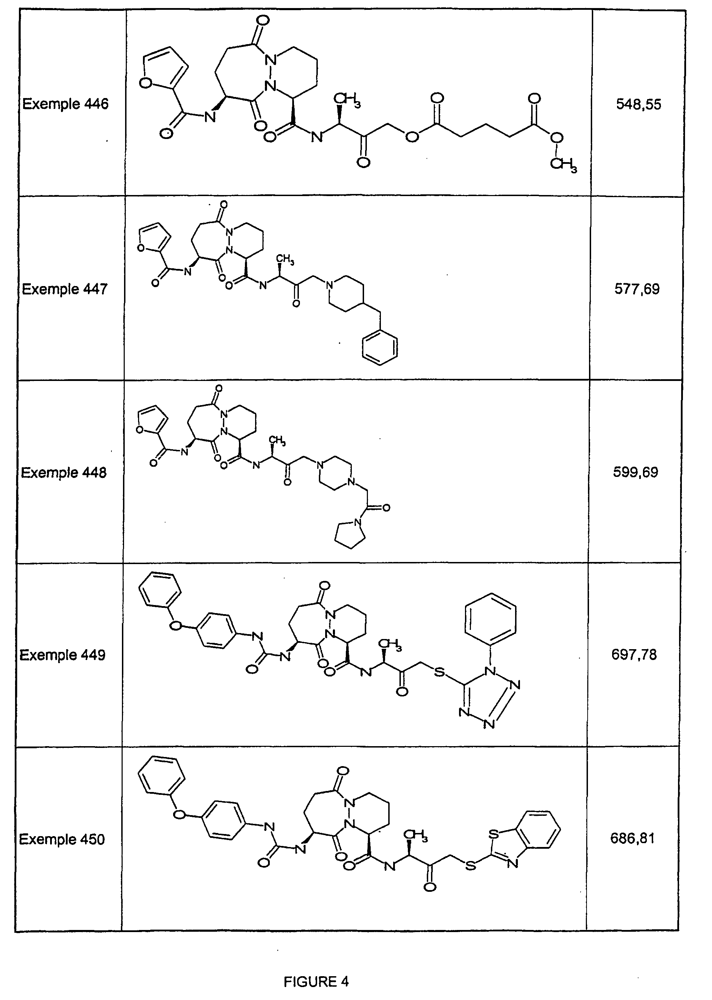

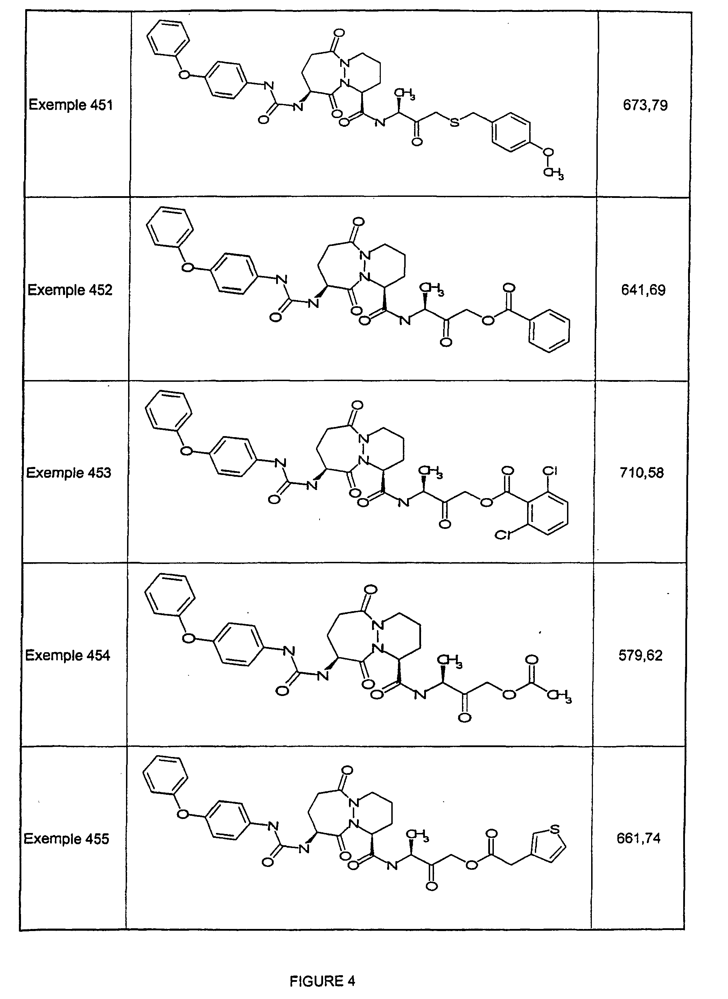

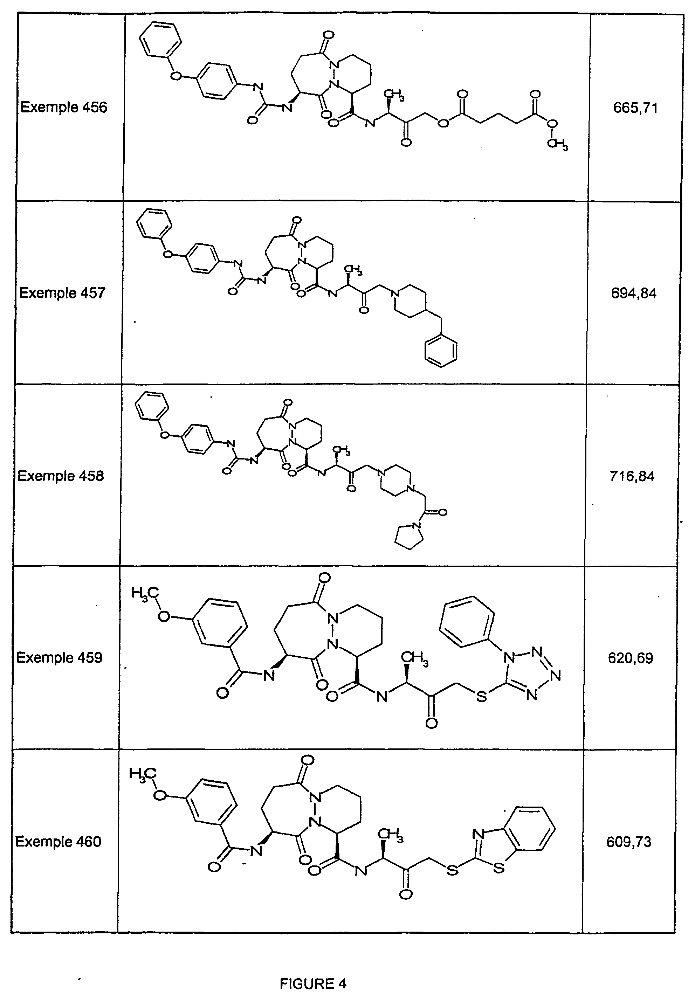

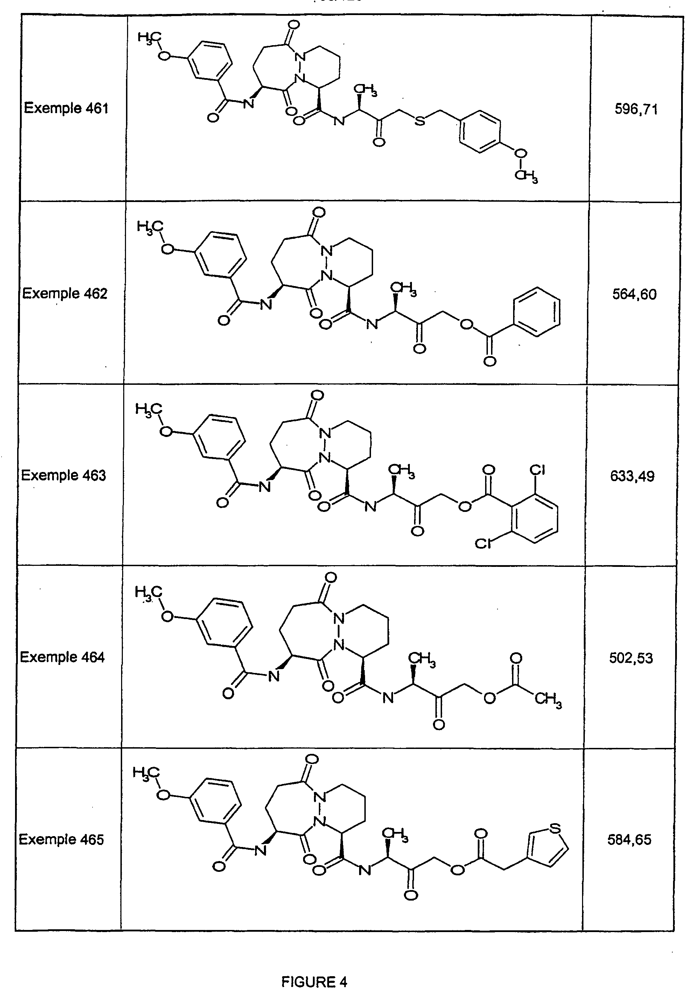

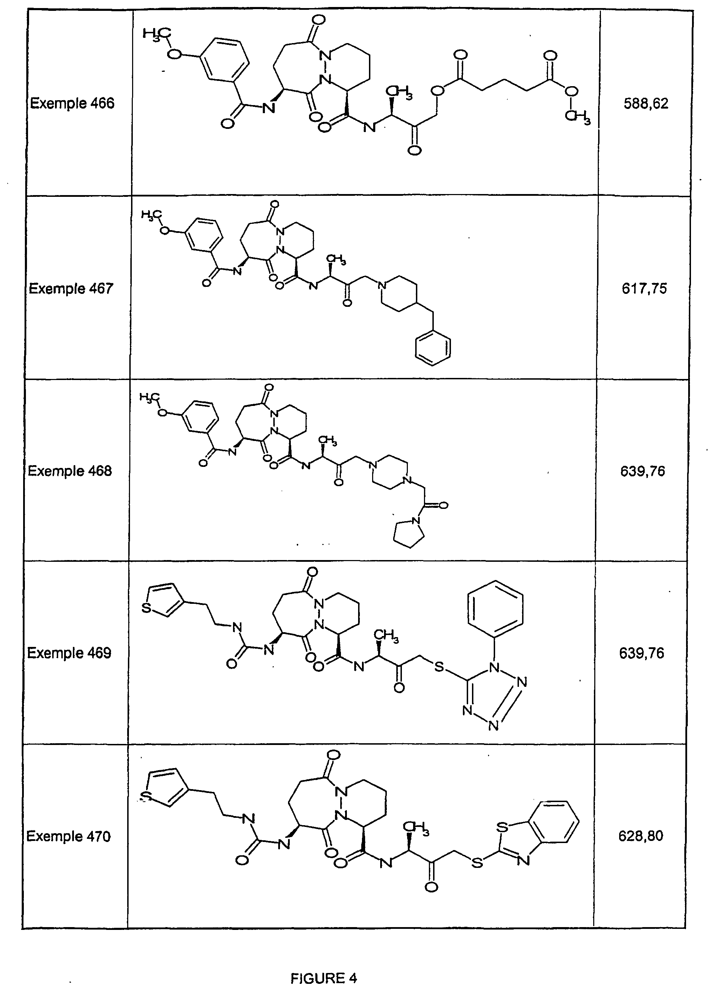

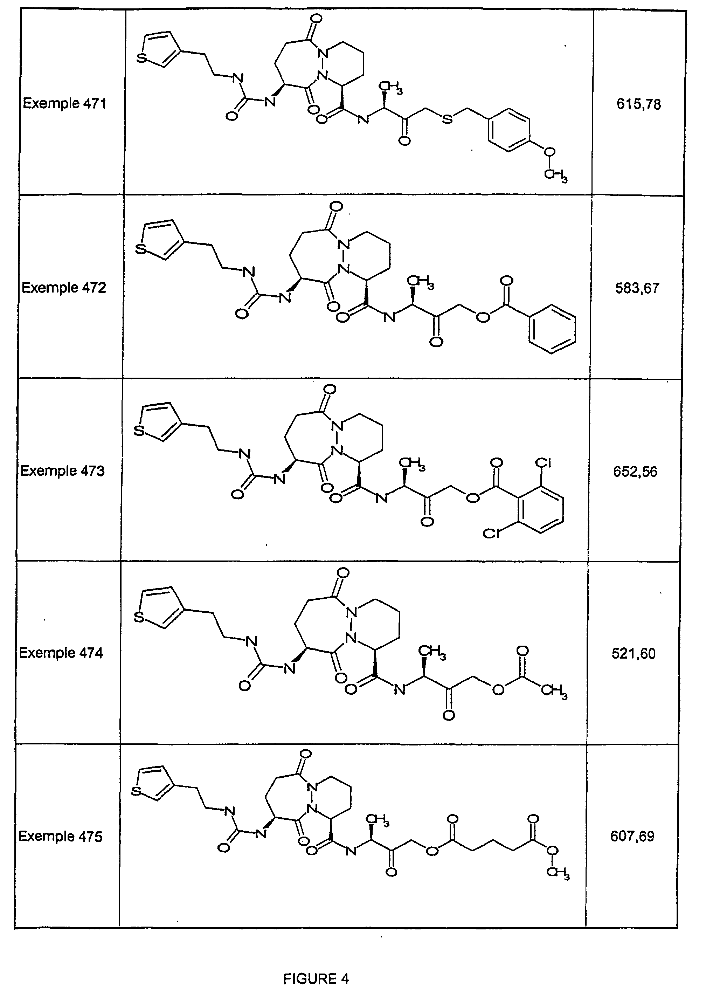

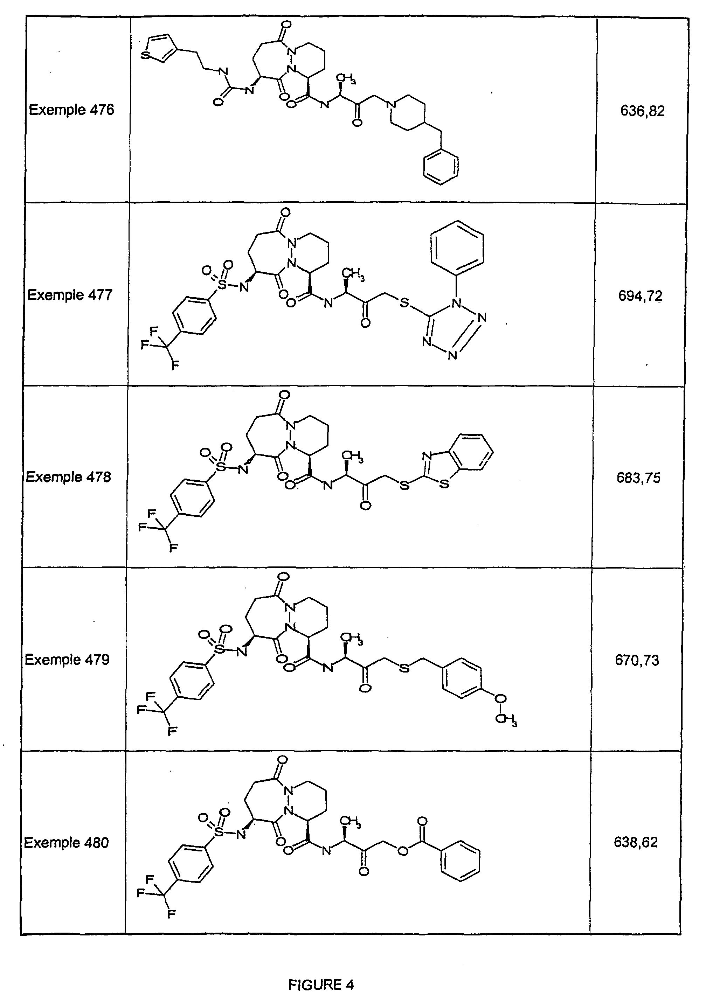

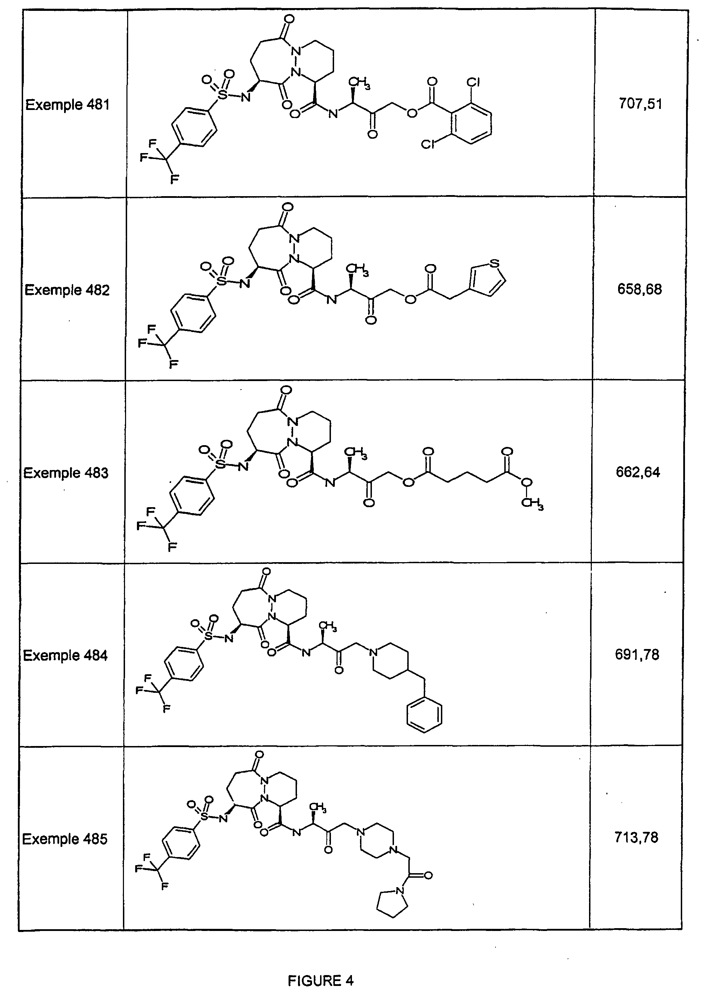

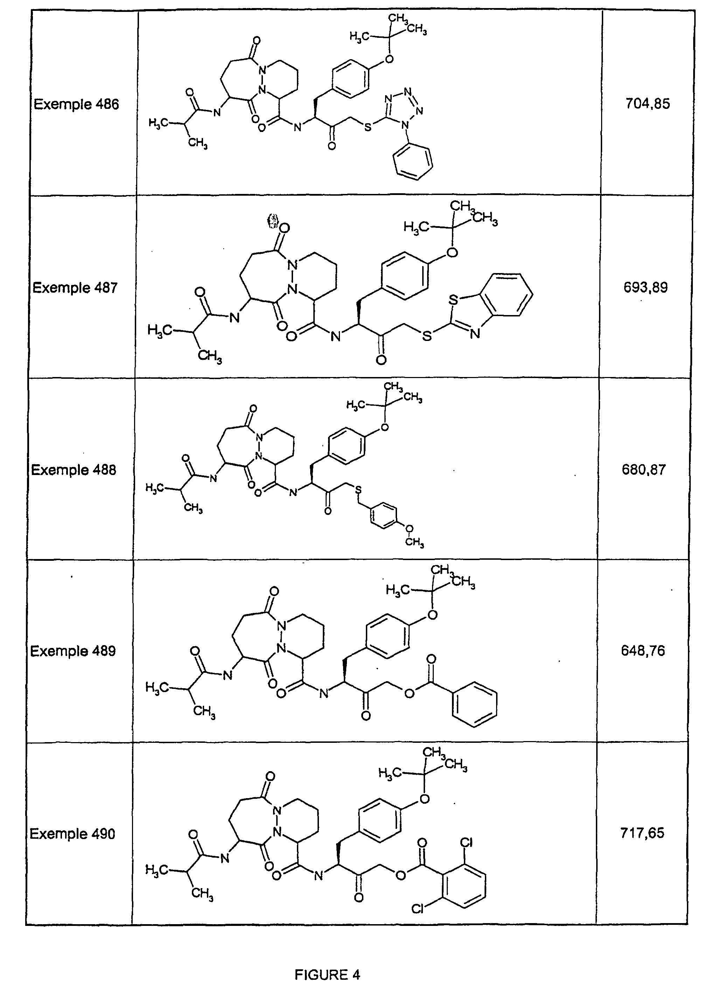

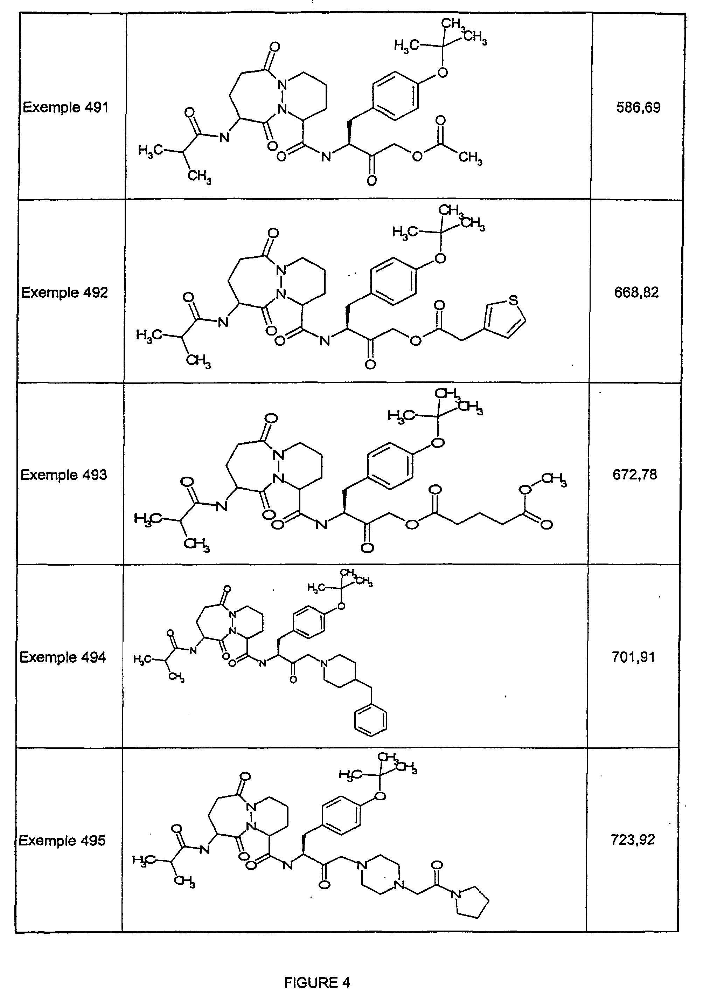

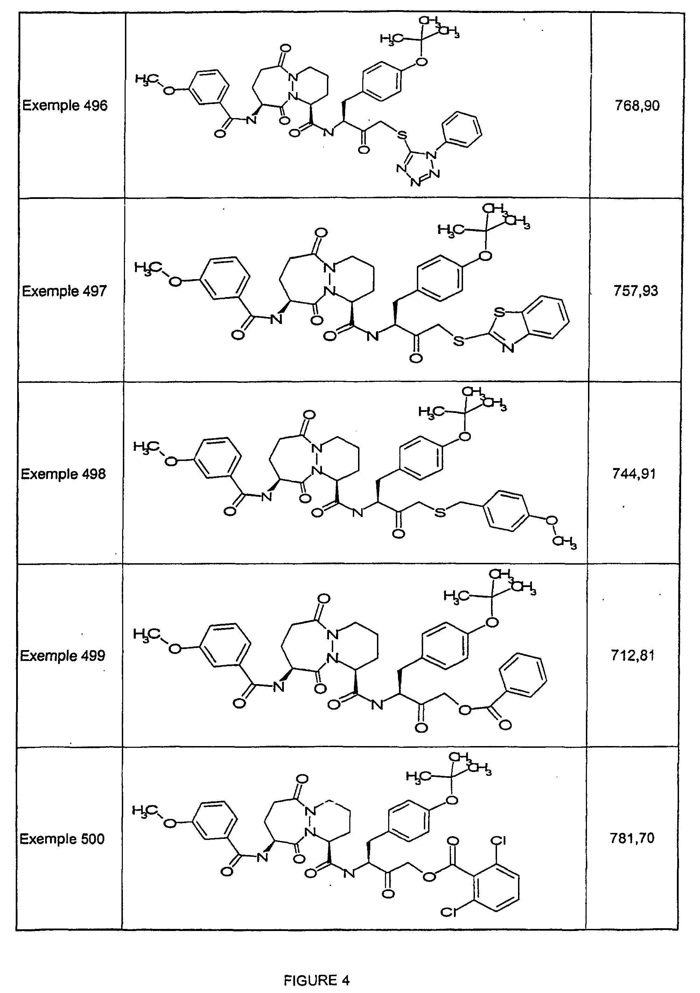

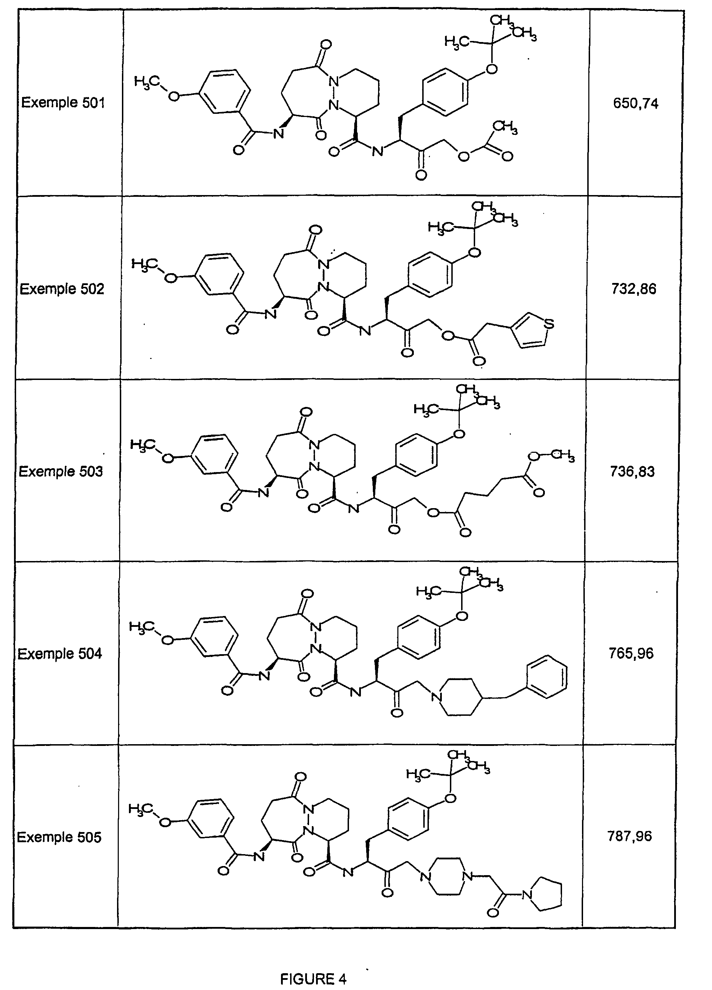

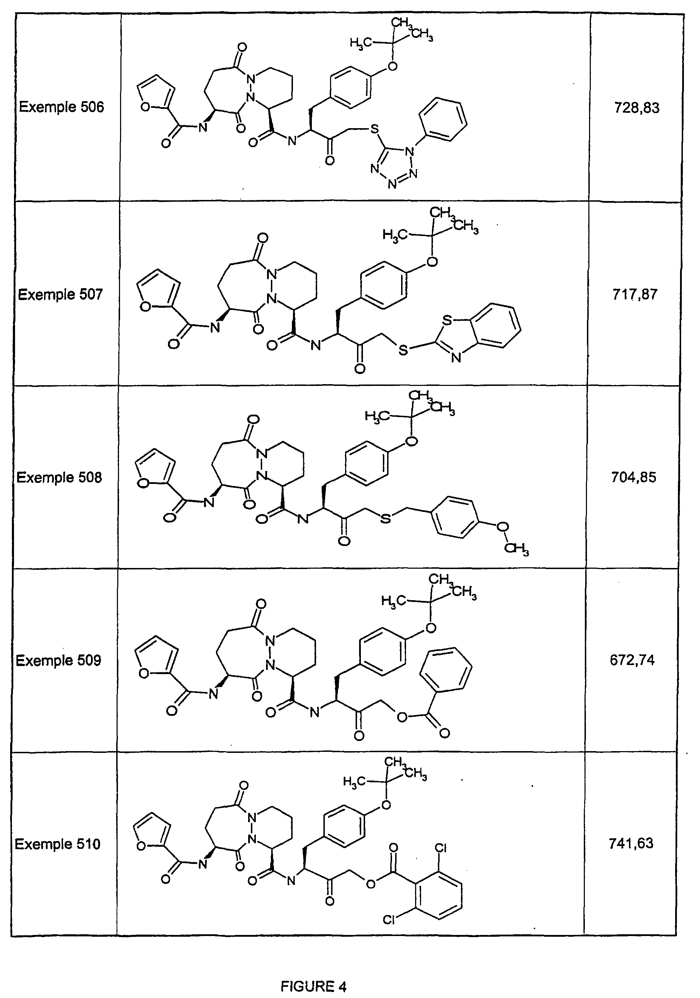

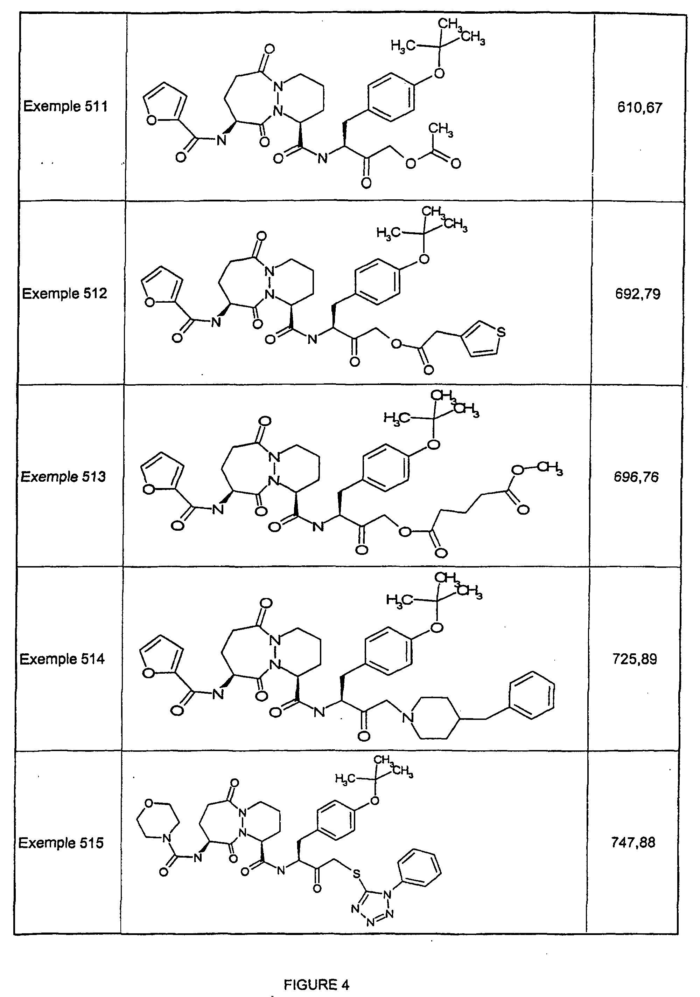

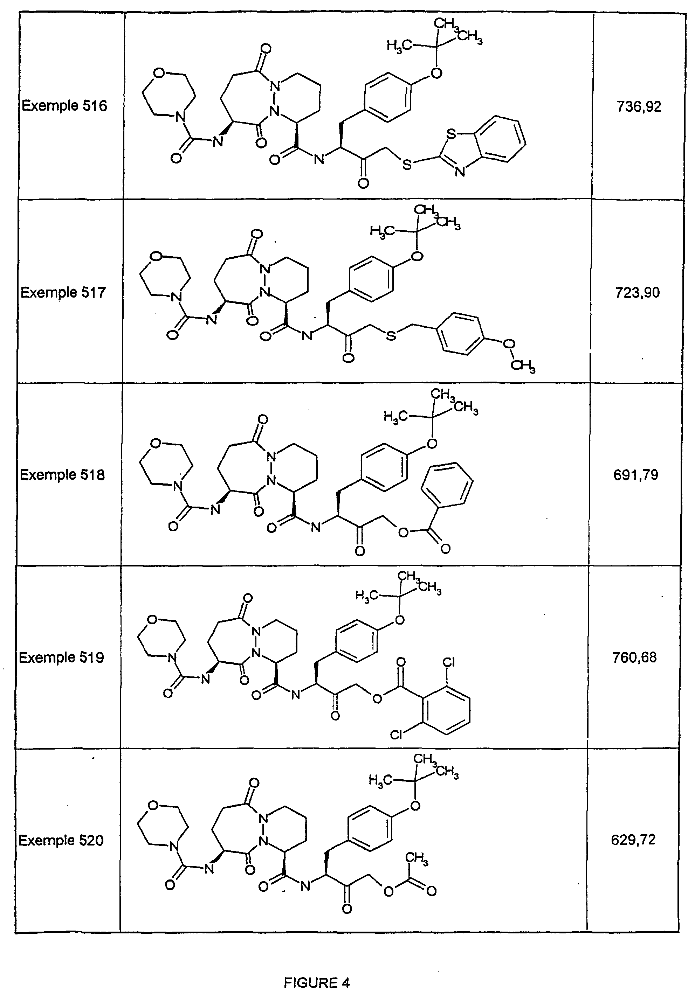

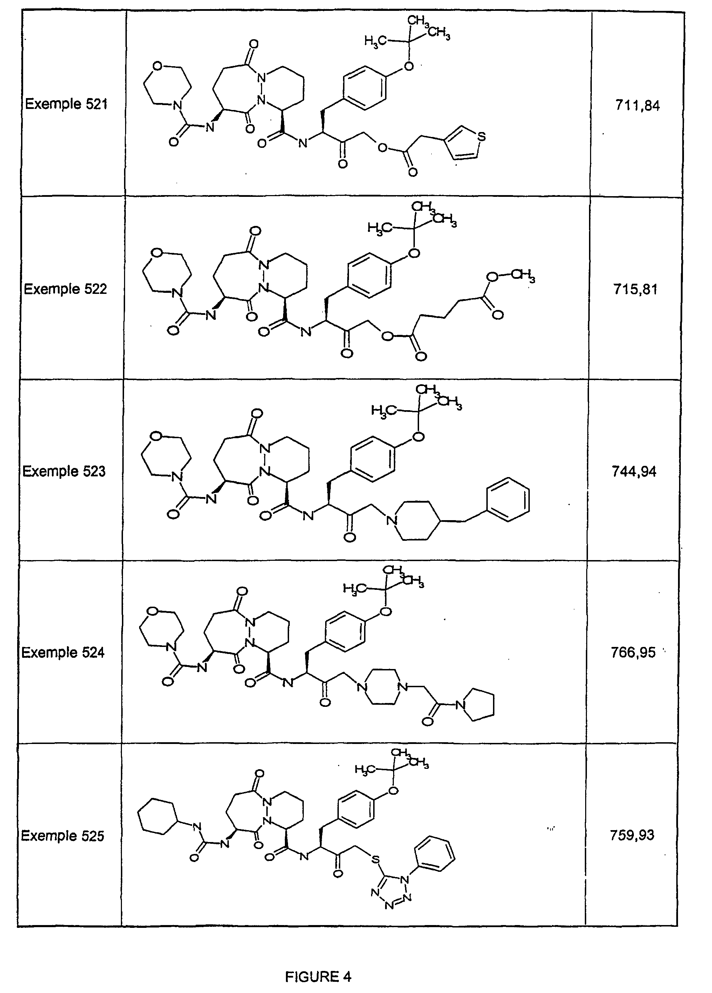

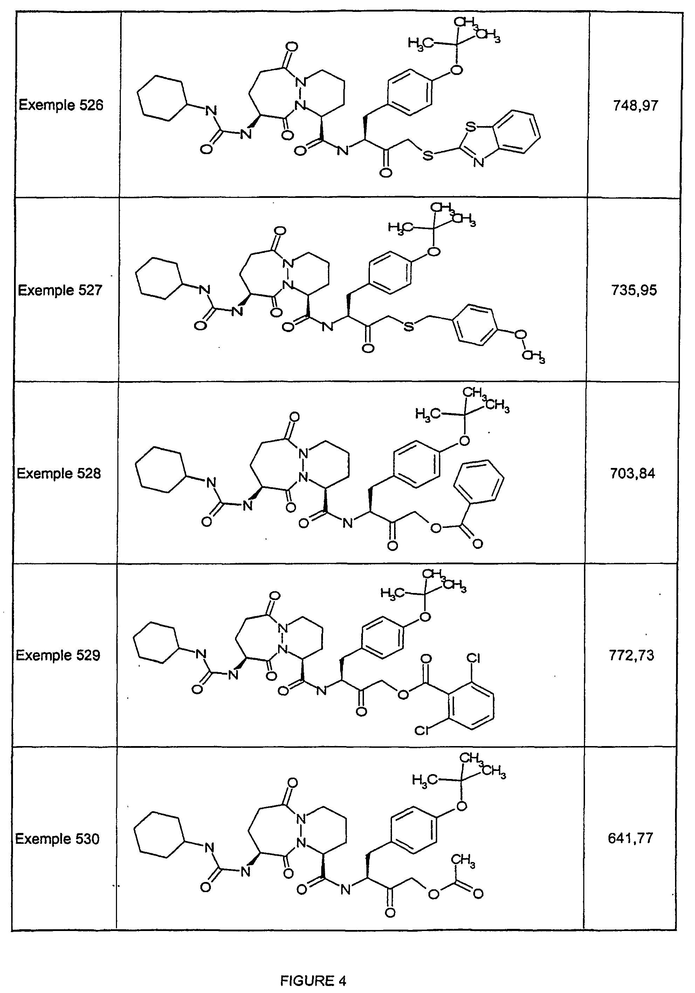

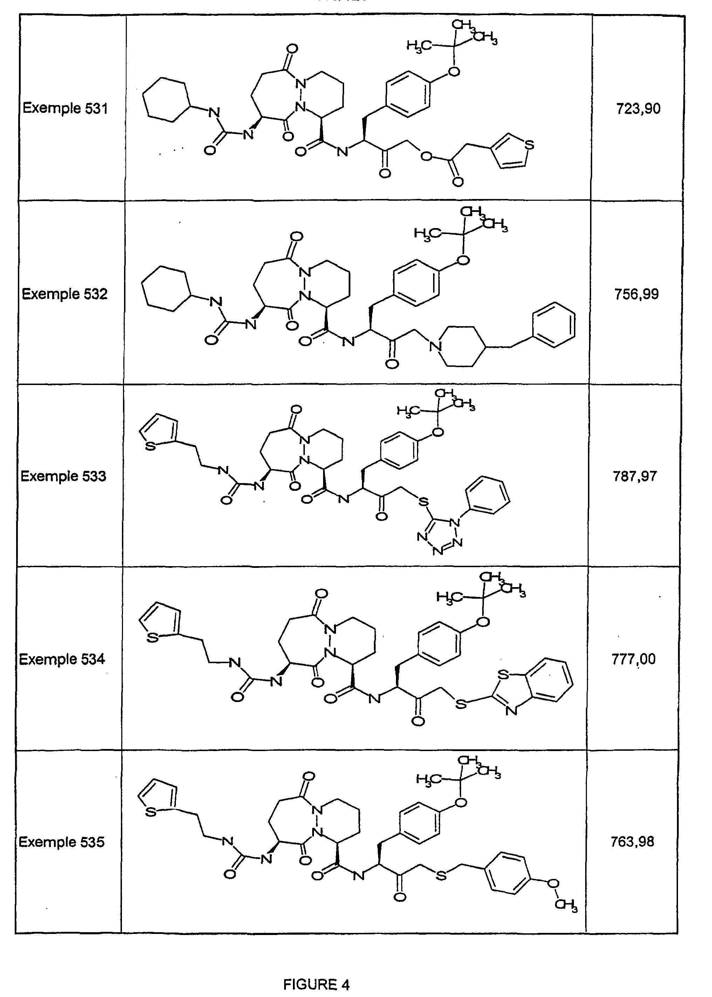

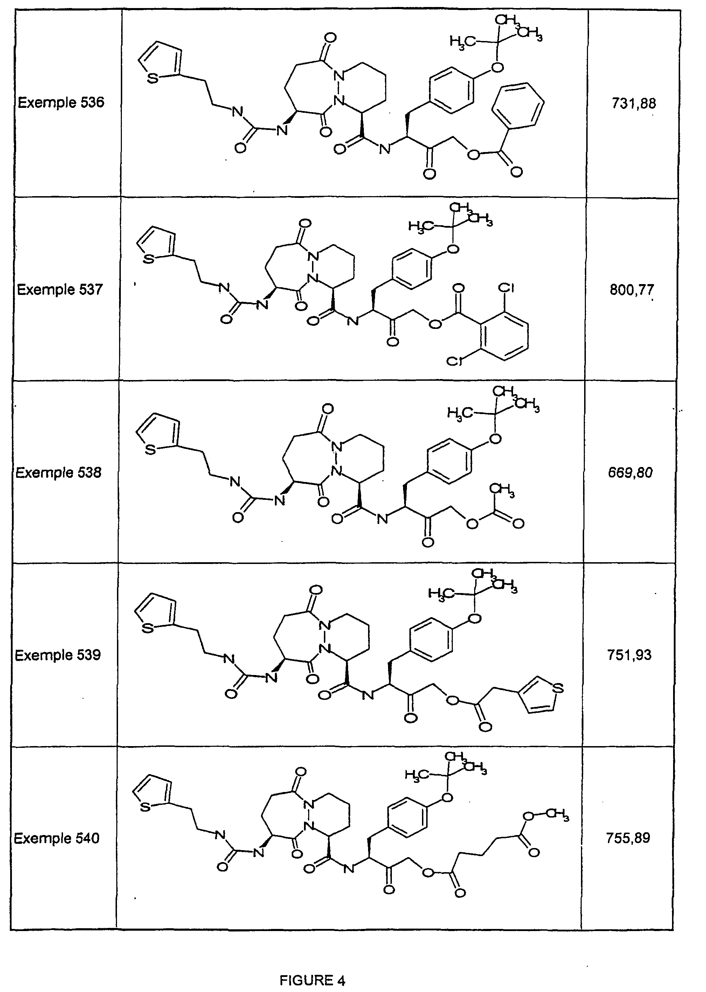

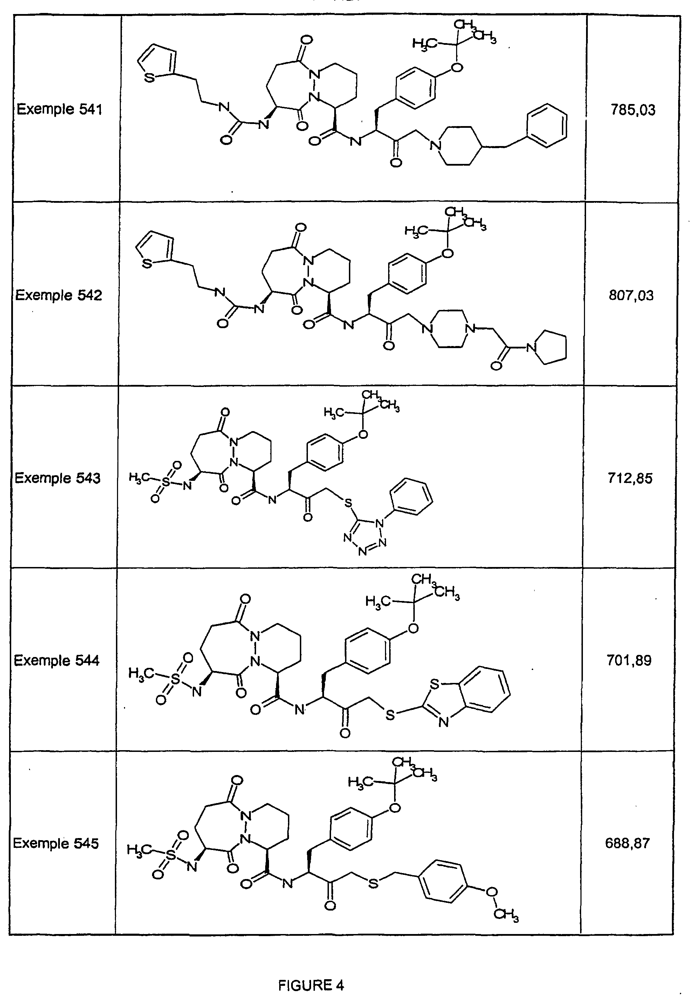

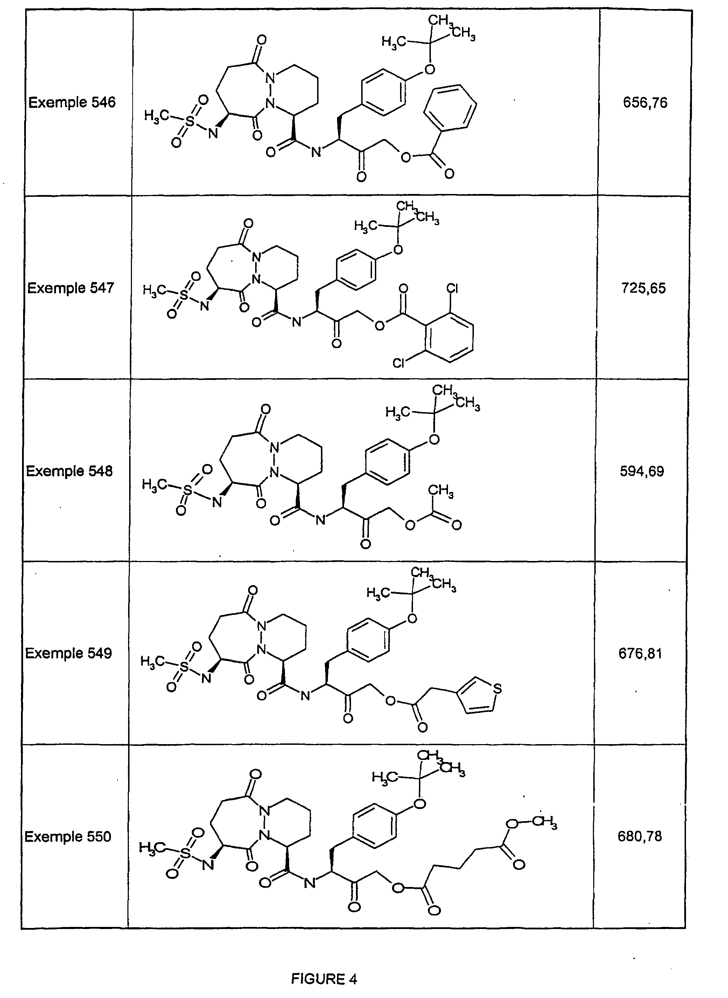

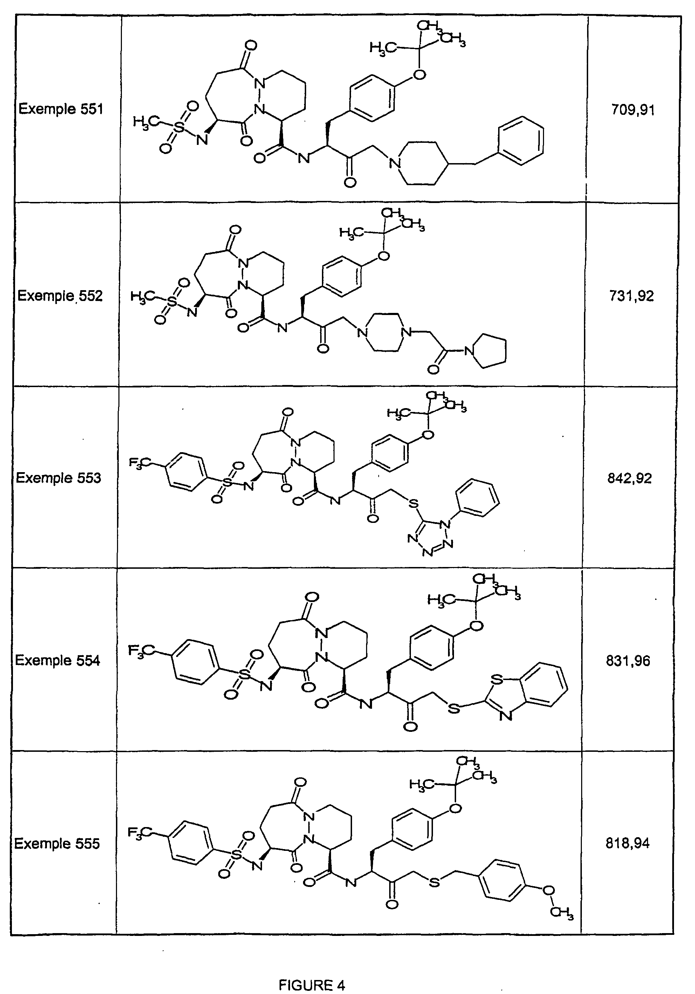

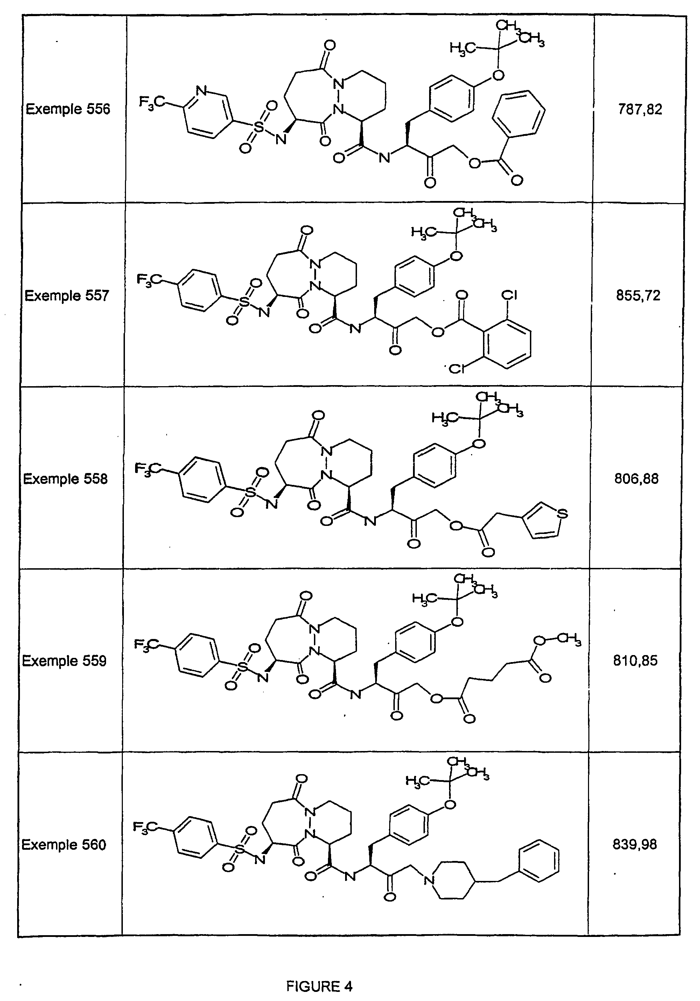

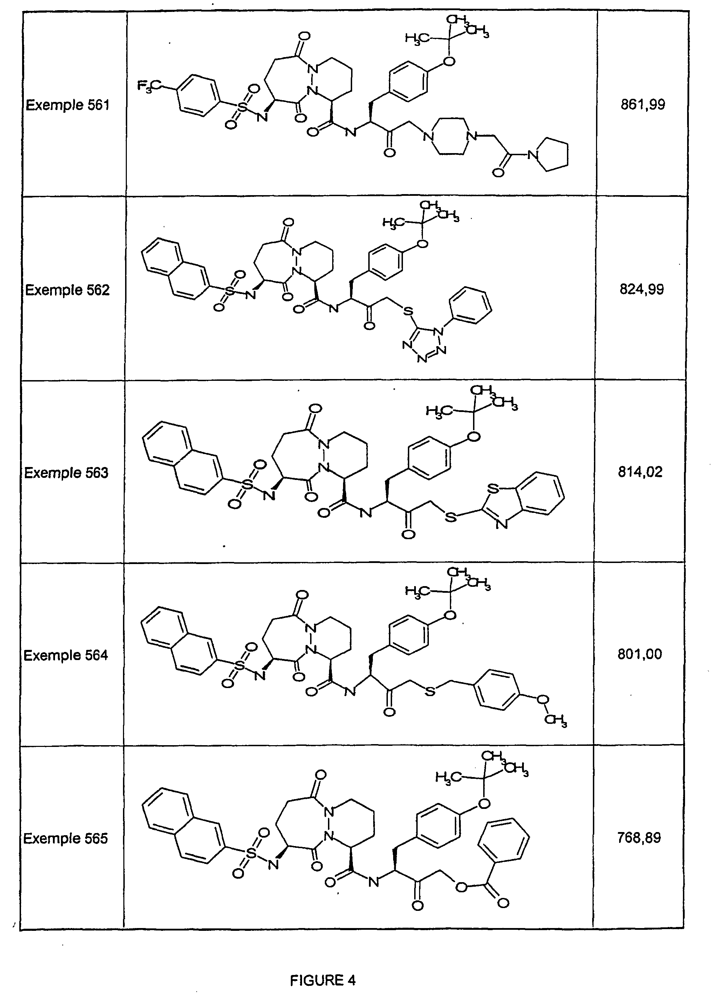

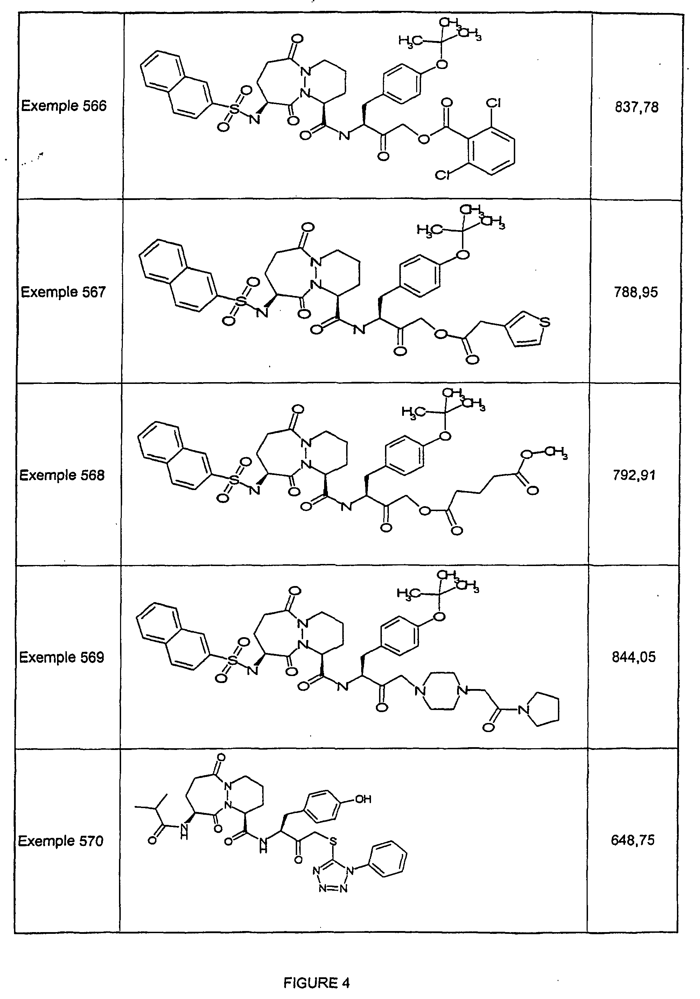

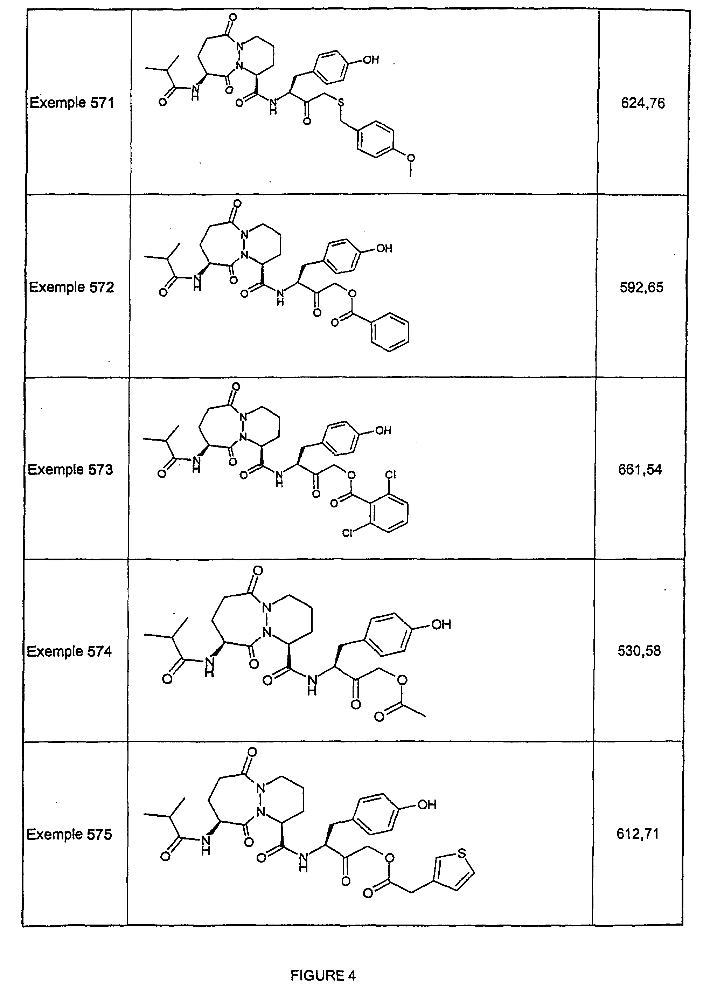

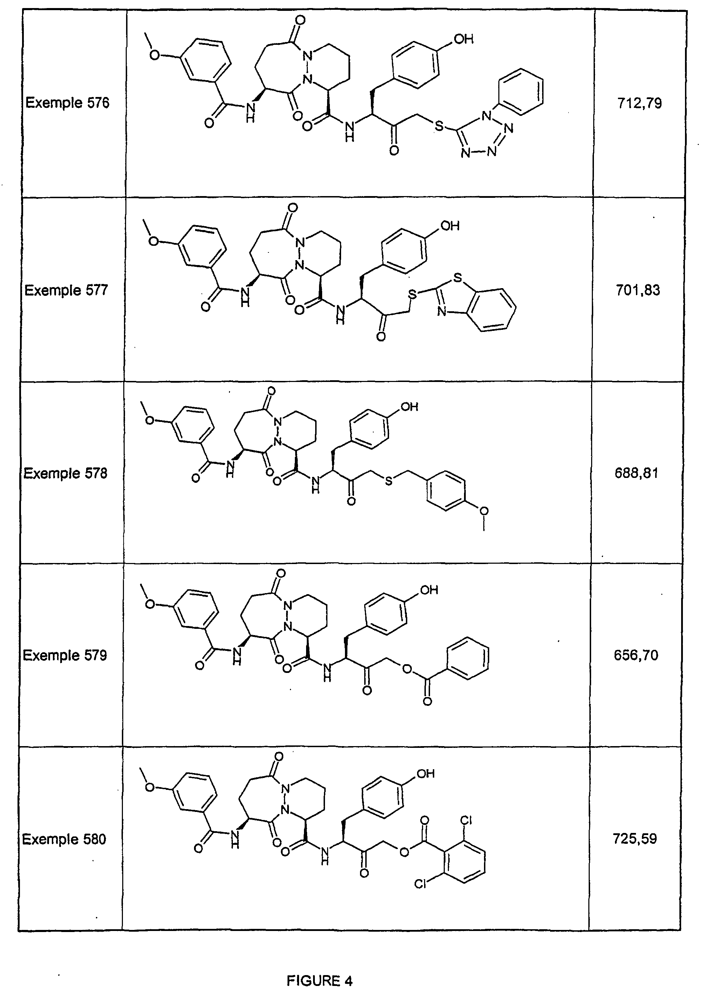

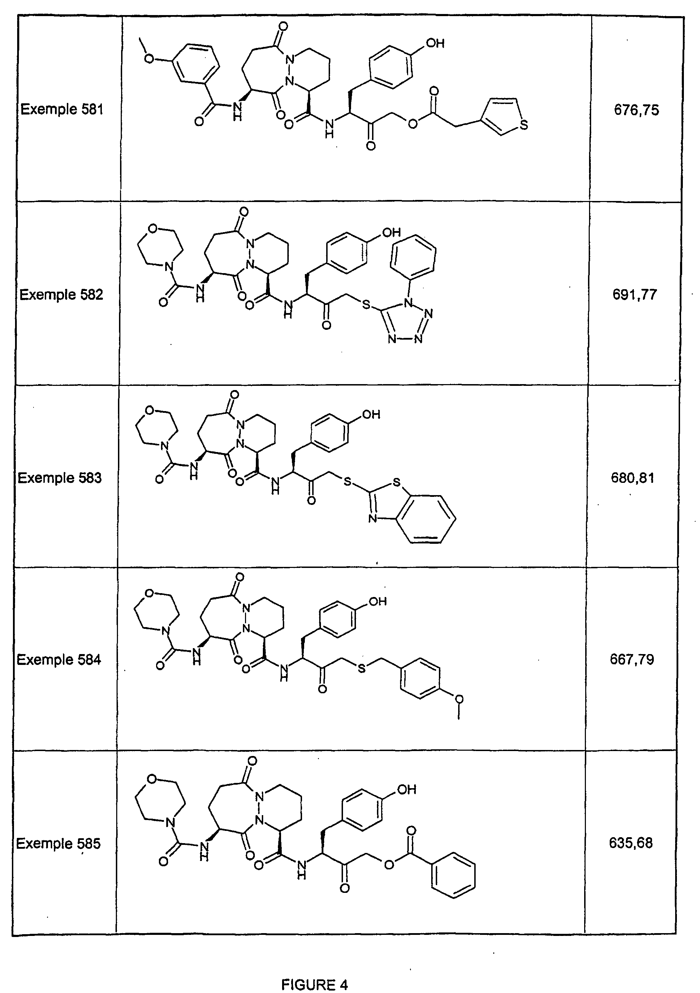

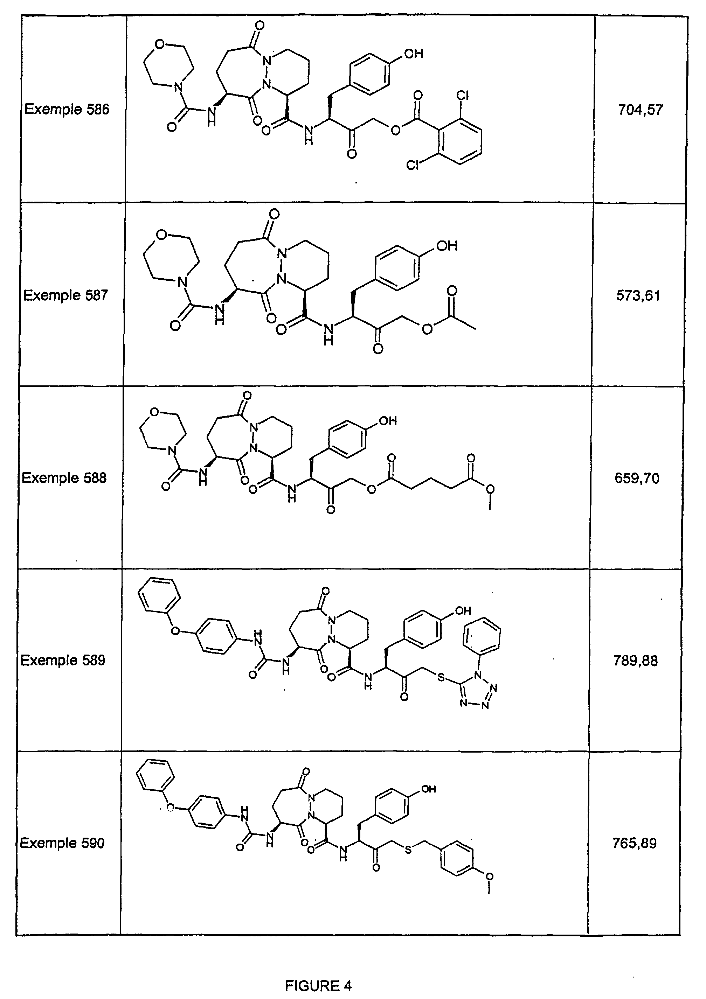

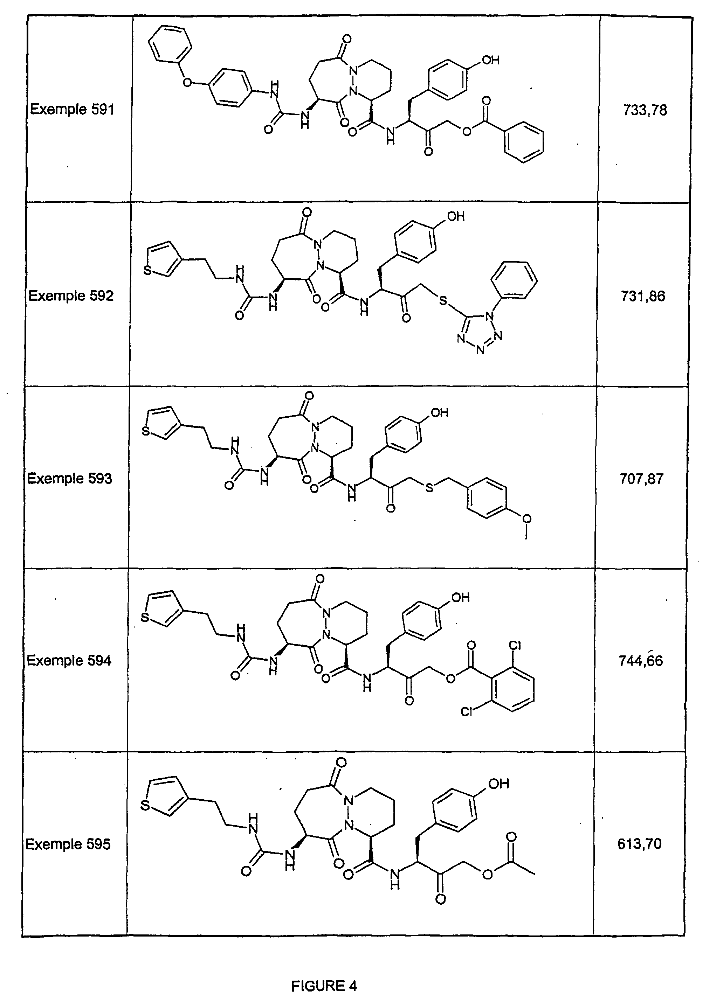

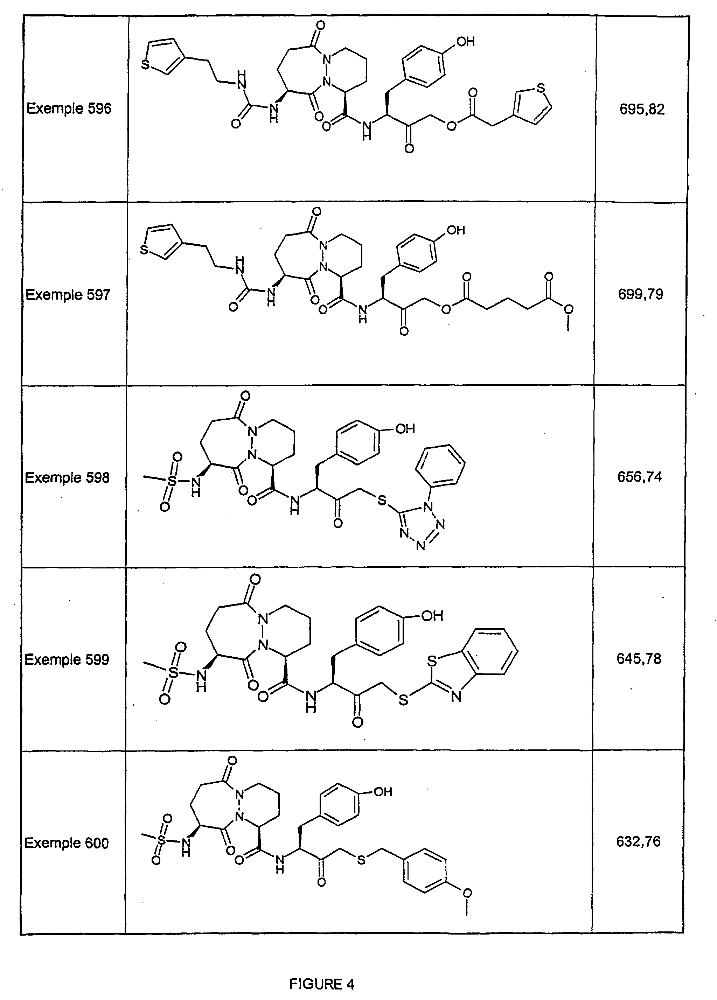

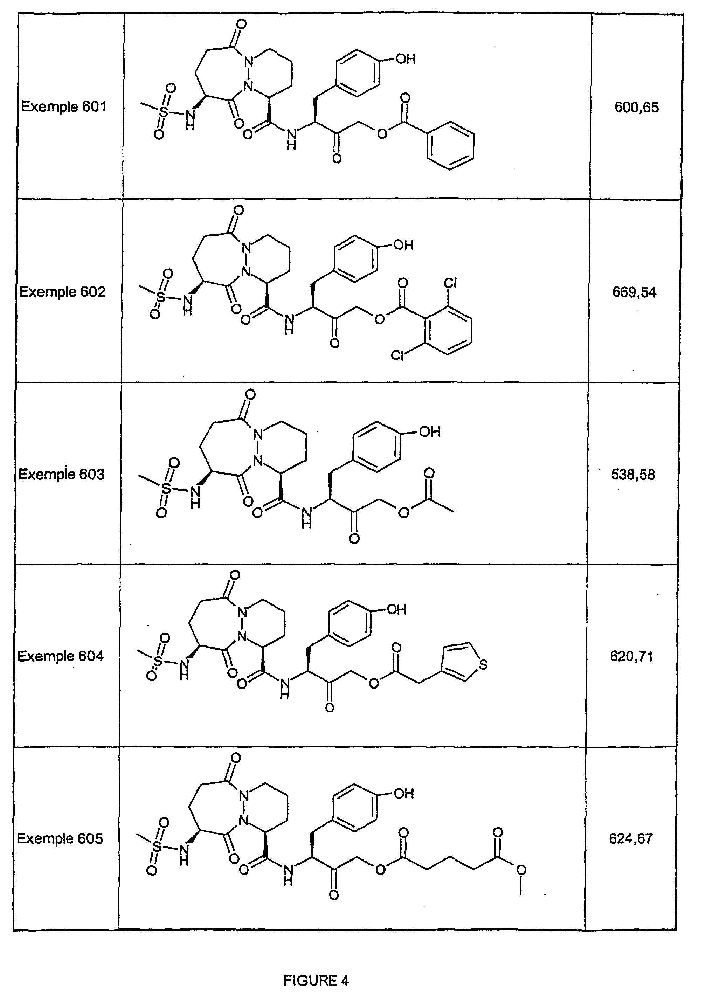

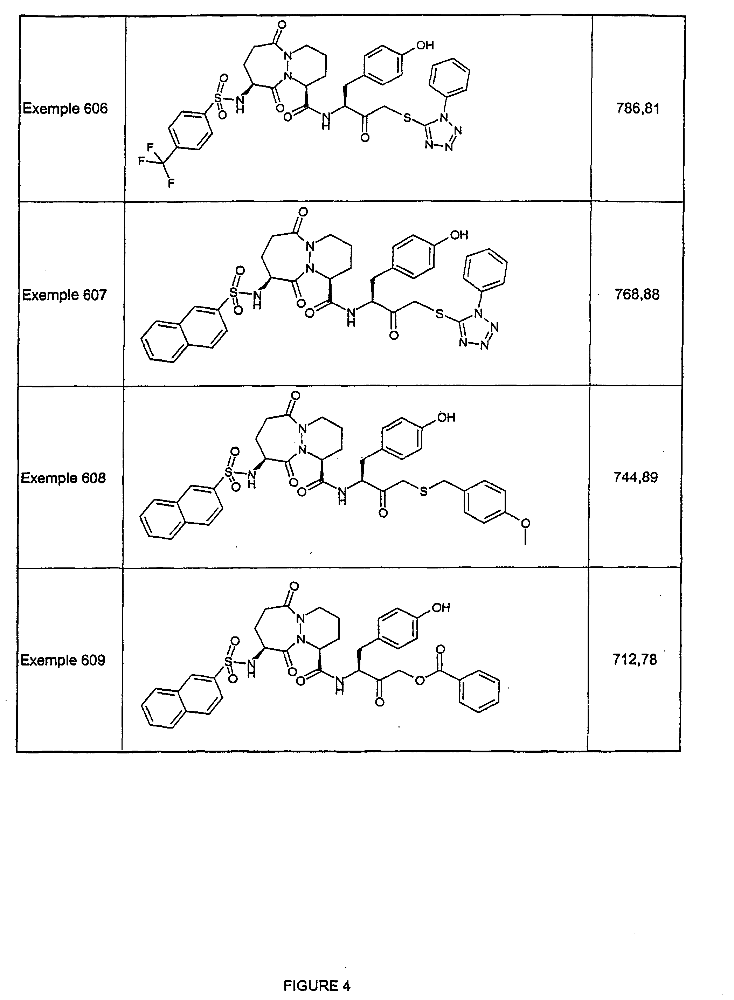

On peut noter que de telles réactions de transformation de substituants en d'autres substituants peuvent également être effectuées sur les produits de départ ainsi que sur les intermédiaires tels que définis ci-dessus avant de poursuivre la synthèse selon les réactions indiquées dans le procédé décrit ci-dessus.
Le procédé décrit ci-dessus, que représente la figure 1, est le procédé utilisé pour obtenir tous les produits de formule (I) de la présente demande et notamment ceux décrits aux exemples 1 à 3 dans la partie expérimentale de la présente demande ainsi que ceux représentés dans les tableaux de la figure 4 décrite ci-après.

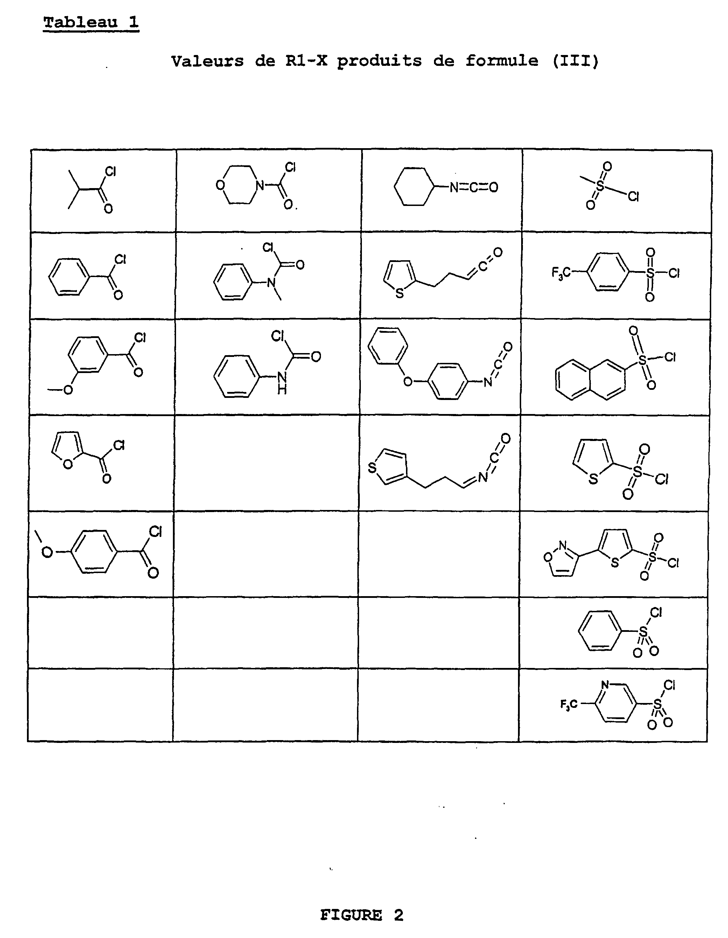

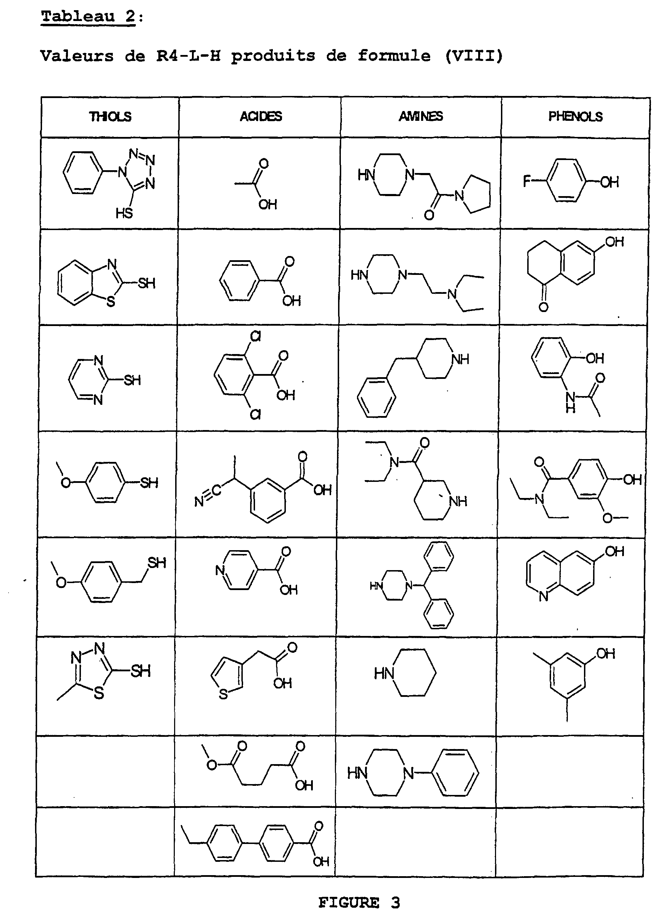

Un tel procédé représenté à la figure 1 est constitué des 4 étapes suivantes :
L'étape 1 permet d'obtenir le produit de formule (VI) à partir de l'acide aminé de formule (VIa)
L'étape 2 permet d'obtenir le produit de formule (V) à partir du produit de départ de formule (II) par réaction avec le produit de formule (III) soit R1-X choisi notamment parmi les valeurs indiquées au tableau 1 de la figure 2.
L'étape 3 permet d'obtenir le produit de formule (Ix) par couplage peptidique du produit de formule (VI) obtenu à l'étape 1 avec le produit de formule (V) obtenu à l'étape 2. L'étape 4 permet d'obtenir le produit de formule (Iy) à partir du produit de formule (Ix) obtenu à l'étape 3 par réaction avec le composé de formule (VIII) soit H-L-R4' choisi notamment parmi les valeurs indiquées au tableau 2 représenté à la figure 3 : cette étape 4 peut être réalisée selon deux méthodes différentes l'une dite 'KF/DMF' (fluorure de potassium dans le diméthylformamide) et l'autre dite 'Amine supportée'.

On entend par 'amine supportée' une amine greffée sur un support polystyrène que l'on peut trouver dans le commerce telle que par exemple la résine diméthylaminométhylpolystyrène, diéthylaminométhyl ou autre amine tertiaire supportée commerciale.

Dans des conditions préférentielles de mise en oeuvre de l'invention, le procédé de la présente invention tel que représenté à la figure 1 décrite ci-après et tel que défini ci-dessus en 4 étapes, peut-être réalisé de la façon suivante.

### Etape 1 : préparation de l'acide aminé diazoté de formule (VI) :

Cette étape se décompose en 3 stades a1), b1) et c1)

### a1) obtention du produit (VIb) à partir d'un acide aminé (VIa)

Addition de fmoc sur la fonction amine N-terminale de l'acide aminé de départ : les conditions de cette réaction sont décrites notamment dans l'article référencé ci-dessus:
'Mueller,A. ;Vogt,C. ; Sewald,N. ; Synthesis (1998),(6),837-841'. Concernant les Fmoc-amino acids, on peut citer également le document : 'Methods Enzymol. (1997), 289 (Solid-phase peptide synthesis), 44-67.

De telles conditions réactionnelles peuvent être appliquées à tout acide aminé dit naturel ou dit non naturel.

### b1)obtention du produit fmoc diazoté de formule (VIc) à partir du produit de formule (VIb) obtenu ci-dessus en a) en deux réactions bi) et bii) décrites ci-après :

### bi) préparation de l'anhydride mixte

A une suspension du produit de formule (VIb) obtenu au stade a) ci-dessus, dans le dichlorométhane (3 ml/mmole) sont additionnés successivement sous azote et à -10°C 1.05 équivalent de 4-Méthyl morpholine suivi goutte à goutte de 1.03 équivalent de Chloroformate d'isobutyle. La réaction est maintenue 1 heure à -10°C et le mélange filtré rapidement.

### bii) Diazotation

Le filtrat est refroidi à -10°C, puis sous azote , 2 équivalents de diazométhane en solution dans le dichlorométhane (0.3M) sont additionnés goutte à goutte. Le milieu réactionnel est ramené progressivement à TA puis laissé sous agitation 1 heure. La solution est versée dans une solution saturée de bicarbonate de sodium (10 ml/mmole) . La phase organique est lavée à l'eau (10 ml/mmole), sèchée sur sulfate de magnésium et concentrée sous vide. Le produit brut obtenu est chromatographié sur silice avec un mélange CH2Cl2/AcOEt 95/5.

### c1)obtention de l'acide aminé diazoté de formule (VI) à partir du produit de formule (VIc) obtenu ci-dessus en b) par déprotection des fmoc-amino diazocétones

Une solution du produit Fmoc-amino diazocétone de formule (VIc) obtenu au stade b2) ci-dessus dans le dichlorométhane (3.5ml /mmole) est traitée à 0°C sous azote par 2 équivalents de Diazabicyclo-undecene (DBU) (2.1 équi.). La réaction est laissée ½ heure , puis la solution directement déposée sur une colonne de silice. L'amine est rapidement éluée par une solution de CH2Cl2 /MeOH 90 /10. L'amine libre brute obtenue est directement mis en réaction pour le couplage peptidique que constitue l'étape 3.

### Etape 2: préparation du scaffold bicyclique de formule (V) :

Cette étape se décompose en 2 stades a2) et b2)

### a2) Fonctionalisation de l'amine soit obtention du produit de formule (IV) à partir du produit de formule (II) par réaction avec un produit de formule (III),

Le produit de formule (II) est l'ester méthylique de l'acide 9(S)-amino-6,10-dioxo-1,2,3,4,7,8,9,10-octahydro-6H-pyridazino[1,2-a][1,2]diazepine-1(S)-carboxylique. Pour obtenir le produit (IV) à partir du produit de formule (II) ,on fait réagir le produit de formule (II) avec un produit R1-X de formule (III) tel que défini ci-dessus choisi par exemple dans le tableau 1 décrit à la figure 2 et on procède comme suit :

A une solution de l'amine de formule (II) dans le dichlorométhane (6 ml/ mmole) est additionnée successivement 1 équivalent de chlorure d'acide, de chlorure de sulfonyle, de chlorure de carbamoyle ou d'isocyanate, suivi de 2 équivalents de diisopropyléthylamine (DIEA) à 0°C et sous azote. Le milieu réactionnel est versé dans HCl 1N (3 ml /mmole). La solution organique est lavée successivement par une solution saturée de NaCl (3ml/ mmole), puis une solution saturée de bicarbonate de sodium (3ml/ mmole). Après séchage sur sulfate de magnésium la solution est concentrée sous vide . Le produit brut obtenu est directement utilisé pour l'étape suivante de saponification de la fonction ester

### b2)Obtention du produit de formule (V) à partir du produit de formule (IV) par saponification de la fonction ester

L'ester méthylique obtenu au stade a) ci-dessus est traitée par une solution méthanolique de lithine 2M (2 équi.). Après une nuit de réaction à TA, le mélange est concentré au demi au rotavapor, puis le même volume d'eau est ajouté. Le pH de la solution est amené à 1 par addition d'acide concentré . Le mélange est extrait par l'acétate d'éthyle (2X5ml /mmole). La phase organique est lavée par une solution saturée de chlorure de sodium.. Après séchage sur sulfate de magnésium , la solution est concentrée à sec. Le produit obtenu est purifié si besoin sur silice en utilisant un mélange CH2Cl2 /MeOH /AcOH, 95/5/0.5 comme éluant.

### Etape 3 : Couplage peptidique

Le couplage peptidique est opéré par réaction du produit de formule (VI) obtenu à l'étape 1 et du produit de formule (V) obtenu à l'étape 2 pour obtenir un produit de formule (Ix)

A une solution de l'amine libre et de l'acide de formule (V) dans le CH2Cl2 (3.5ml/mmole) sont additionnés successivement, sous azote et à 0°C, 1.1 équivalent de TBTU (2-(1H-Benzotriazole-lyl)1,1,3,3-tétramethyluronium tétrafluoroborate) et 2 équivalent de DIEA. La réaction est laissée une nuit à TA . La solution est versée dans une solution saturée de NaHCO3 (20 ml /mmole) et extraite par l'acétate d'éthyle (2X10ml/mmole). Les phases organiques rassemblées sont lavées à l'eau (20ml/mmole), puis une solution saturée de KHSO3, sèchée sur MgSO4 et concentrée sous vide.

### Etape 4 : obtention d'un produit de formule (I) soit (Iy) à partir d'un autre produit de formule (I) soit un produit de formule (Ix) obtenue à l'étape 3 ci-dessus

Cette étape se décompose en 2 stades a4) et b4).

### a4) bromation de la diazocétone du produit de formule (Ix)

Une solution de diazocétone dans CH2Cl2 (6ml/mmole) est traitée à 0°C sous azote par une solution de HBr (37%)/acide acétique 30/70 (0.6ml/mmole). En fin de réaction le mélange est versé dans une solution saturée de NaHCO3 (50 ml/mmole). Le mélange est extrait par l'acétate d'éthyle (2X30ml/mmole). La solution organique est lavée à l'eau (230ml/mmole), sèchée sur MgSO4 et concentrée à sec. Si besoin le brut est purifié sur silice en utilisant CH2Cl2/MeOH 95/5 comme éluant.

### b4) Substitution par deux méthodes possibles b4i) ou b4ii)

### b4i) Méthode au KF

A une solution de bromocétone dans le DMF (6ml /mmole) est additionné 2 équivalents de fluorure de potassium (KF) suivi de 1.2 équivalent de nucléophile de formule (VIII) soit R4'-L-H qui peut être notamment sous forme d'acide, de thiol ou d'hydroxyle comme indiqué au tableau 2 de la figure 3 décrite ci-après. La réaction est laissée une nuit sous agitation puis le mélange est versé dans une solution saturée de NaHCO3 (100ml/mmole) et extrait par l'acétate d'éthyle (2X50ml /mmole). Les phases organiques rassemblées sont lavées à l'eau, sèchées sur MgSO4 et concentrées à sec. Si nécessaire une chromatographie sur silice est effectuée en utilisant CH2Cl2/MeOH comme éluant.

### b4ii) Méthode utilisant la résine diméthylaminométhylpolystyrène.

A une solution de bromocétone (1 équivalent) et de nucléophile de formule (VIII) (1.1 équivalent) dans le DMF (15ml/mmole) est additionné 2 équivalent de résine diméthylaminométhylpolystyrène (Fluka 3-4 mmole de base /g). La réaction est laissée une nuit sous agitation douce. Le mélange réactionel est filtré puis évaporé sous vide. Si nécessaire les produits obtenus sont purifiés sur silice en utilisant un mélange Méthanol/CH2Cl2 comme éluant.

A titre d'exemple, on décrit ci-après dans la partie expérimentale de la présente demande la préparation des produits des exemples 1, 2 et 3 de la présente demande .

On peut noter que l'étape 1 décrite ci-après pour l'exemple 1 est celle de tous les produits de formule (I) de la présente demande pour lesquels R2 représente un reste de l'acide aminé Leucine et donc pour lesquels l'acide aminé de départ de formule (VIa) est la leucine (Leu) : la même méthode peut être appliquée dans le cas où l'acide aminé est différent soit notamment lorsque l'acide aminé de formule (VIa) de départ représente GLU-alloc, GLY, ALA, VAL, PHE ou TYR(tBu). On peut trouver de tels acides aminés et également le composé GLU-alloc dans le commerce.

On trouve notamment dans le commerce les acides aminés AA sous forme de composé fmoc-AA de formule (VIb).

Lorsque l'acide aminé de départ de formule (VIa) est la tyrosine TYR, le procédé comporte une étape de plus comme indiqué à la préparation de l'exemple 3 ci-après.

La réaction du produit de formule (II) avec le produit de formule (III) pour donner un produit de formule (IV) peut être réalisée notamment en présence de DIEA ou encore dans un solvant tel que CH2-Cl2 ou encore le (DMF).

Dans le produit de formule (III), le radical R1' représente les valeurs indiquées ci-dessus pour R1 sous une forme protégée si nécessaire et X représente un atome d'halogène tel que chlore ou brome.

Le produit de formule (IV) ainsi obtenu est soumis, pour donner le produit de formule (V), à une réaction de saponification dans les conditions connues de l'homme du métier telle que notamment en présence de LiOH dans un alcool tel que le méthanol ou encore l'éthanol, le dioxane ou le diméthoxyéthane, en présence de soude ou de potasse.

Le produit de formule (VIb) est obtenu par fixation du radical fmoc sur la fonction amine terminale de l'acide aminé de formule (VIa) : un tel acide aminé de formule (VIa) peut être tout acide aminé dit naturel ou non naturel comme indiqué ci-dessus.

Le produit de formule (VIb) est soumis en deux stades comme indiqué ci-dessus à une réaction de diazotation pour donner le produit de formule (VIc).

Le produit de formule (VIc) ainsi obtenu est alors libéré du radical fmoc pour donner le produit de formule (VI).

La réaction de couplage du produit de formule (V) avec le produit de formule (VI) pour donner le produit de formule (Ix) est réalisée par exemple dans TBTU/DIEA dans un solvant tel que le DMF ou encore le dichlorométhane.

Les produits de formule (Ix) représentent des produits de formule (I) dans lesquelles les fonctions réactives sont éventuellement protégées et dans lesquelles R3 représente le radical CH=N2.

Afin d'obtenir des produits de formule (I) dans lesquelles R3 représente le radical -CH2-L-R4, les produits de formule (Ix) sont soumis d'abord à une bromation dans les conditions telles que définies ci-dessus pour donner les produits de formule (VII). Les produits de formule (VII) pour donner les produits de formule (Iy) telle que définie ci-dessus sont alors soumis à l'action de produits de formule (VIII) H-L-R4' : une telle réaction peut être réalisée notamment selon deux modes réactionnels différents comme indiqué ci-dessus.
Selon les valeurs de R'1, R2', R4' et R7', les produits de formules (Ix) et (Iy) constituent ou non des produits de formule (I) et peuvent donner des produits de formule (I), ou être transformés en d'autres produits de formule (I) en étant soumis à une ou plusieurs des réactions a) à k) indiquées ci-dessus.

Ainsi les diverses fonctions réactives que peuvent porter certains composés des réactions définies ci-dessus peuvent, si nécessaire, être protégées : il s'agit par exemple des radicaux hydroxyle, acyle, carboxy libres ou encore amino et monoalkylamino qui peuvent être protégés par les groupements protecteurs appropriés.
La liste suivante, non exhaustive, d'exemples de protection de fonctions réactives peut être citée :
- les groupements hydroxyle peuvent être protégés par exemple par les radicaux alkyle tels que tert-butyle, triméthylsilyle, tert-butyldiméthylsilyle, méthoxyméthyle, tétrahydropyrannyle, benzyle ou acétyle,
- les groupements amino peuvent être protégés par exemple par les radicaux acétyle, trityle, benzyle, tert-butoxycarbonyle, benzyloxycarbonyle, phtalimido ou d'autres radicaux connus dans la chimie des peptides,
- les groupements acyles tel que le groupement formyle peuvent être protégés par exemple sous forme de cétals ou de thiocétals cycliques ou non cycliques tels que le diméthyl ou diéthylcétal ou l'éthylène dioxycétal, ou le diéthylthiocétal ou l'éthylènedithiocétal,
- les fonctions acide des produits décrits ci-dessus peuvent être, si désiré, amidifiées par une amine primaire ou secondaire par exemple dans du chlorure de méthylène en présence, par exemple, de chlorhydrate de 1-éthyl-3-(diméthylaminopropyl) carbodiimide à la température ambiante :

- les fonctions acide peuvent être protégées par exemple sous forme d'esters formés avec les esters facilement clivables tels que les esters benzyliques ou ter butyliques ou des esters connus dans la chimie des peptides.

Les réactions auxquelles les produits de formules (Ix) et(Iy) telles que définies ci-dessus peuvent être soumis, si désiré ou si nécessaire, peuvent être réalisées, par exemple, comme indiqué ci-après.
a) Les produits décrits ci-dessus peuvent, si désiré, faire l'objet, sur les éventuelles fonctions carboxy, de réactions d'estérification qui peuvent être réalisées selon les méthodes usuelles connues de l'homme du métier.
b) Les éventuelles transformations de fonctions ester en fonction acide des produits décrits ci-dessus peuvent être, si désiré, réalisées dans les conditions usuelles connues de l'homme du métier notamment par hydrolyse acide ou alcaline par exemple par de la soude ou de la potasse en milieu alcoolique tel que, par exemple, dans du méthanol ou encore par de l'acide chlorhydrique ou sulfurique.
c) Les éventuels groupements alkylthio des produits décrits ci-dessus peuvent être, si désiré, transformés en les fonctions sulfoxyde ou sulfone correspondantes dans les conditions usuelles connues de l'homme du métier telles que par exemple par les peracides comme par exemple l'acide peracétique ou l'acide métachloroperbenzoïque ou encore par l'ozone, l'oxone, le périodate de sodium dans un solvant tel que par exemple le chlorure de méthylène ou le dioxanne à la température ambiante.
   L'obtention de la fonction sulfoxyde peut être favorisée par un mélange équimolaire du produit renfermant un groupement alkylthio et du réactif tel que notamment un peracide.
   L'obtention de la fonction sulfone peut être favorisée par un mélange du produit renfermant un groupement alkylthio avec un excès du réactif tel que notamment un peracide.
d) La réaction de transformation d'une fonction,cétone en oxime peut être réalisée dans les conditions usuelles connues de l'homme de métier, telle que notamment une action en présence d'une hydroxylamine éventuellement O-substituée dans un alcool tel que par exemple l'éthanol, à température ambiante ou en chauffant.
e) Les éventuelles fonctions carboxy libre ou estérifié des produits décrits ci-dessus peuvent être, si désiré, réduites en fonction alcool par les méthodes connues de l'homme de métier : les éventuelles fonctions carboxy estérifié peuvent être, si désiré, réduites en fonction alcool par les méthodes connues de l'homme du métier et notamment par de l'hydrure de lithium et d'aluminium dans un solvant tel que par exemple le tétrahydrofuranne ou encore le dioxane ou l'éther éthylique. Les éventuelles fonctions carboxy libre des produits décrits ci-dessus peuvent être, si désiré, réduites en fonction alcool notamment par de l'hydrure de bore.
f) Les éventuelles fonctions alcoxy telles que notamment méthoxy des produits décrits ci-dessus peuvent être, si désiré, transformées en fonction hydroxyle dans les conditions usuelles connues de l'homme du métier par exemple par du tribromure de bore dans un solvant tel que par exemple le chlorure de méthylène, par du bromhydrate ou chlorhydrate de pyridine ou encore par de l'acide bromhydrique ou chlorhydrique dans de l'eau ou de l'acide trifluoro acétique au reflux.
g) Les éventuelles fonctions alcool des produits décrits ci-dessus peuvent être, si désiré, transformées en fonction aldéhyde ou acide par oxydation dans les conditions usuelles connues de l'homme du métier telles que par exemple par action de l'oxyde de manganèse pour obtenir les aldéhydes ou du réactif de Jones pour accéder aux acides.
h) Les éventuelles fonctions nitrile des produits décrits ci-dessus peuvent être, si désiré, transformées en tétrazolyle dans les conditions usuelles connues de l'homme du métier telles que par exemple par cycloaddition d'un azidure métallique tel que par exemple l'azidure de sodium ou un azidure de trialkylétain sur la fonction nitrile ainsi qu'il est indiqué dans la méthode décrite dans l'article référencé comme suit :
J. Organometallic Chemistry., 33, 337 (1971) KOZIMA S.& coll. On peut noter que la réaction de transformation d'un carbamate en urée et notamment d'un sulfonylcarbamate en sulfonylurée, peut être réalisée par exemple au reflux d'un solvant comme par exemple le toluène en présence de l'amine adéquate.
   Il est entendu que les réactions décrites ci-dessus peuvent être effectuées comme indiqué ou encore, le cas échéant, selon d'autres méthodes usuelles connues de l'homme. du métier.
i) L'élimination de groupements protecteurs tels que par exemple ceux indiqués ci-dessus peut être effectuée dans les conditions usuelles connues de l'homme de métier notamment par une hydrolyse acide effectuée avec un acide tel que l'acide chlorhydrique, benzène sulfonique ou para-toluène sulfonique, formique ou trifluoroacétique ou encore par une hydrogénation catalytique.
   Le groupement phtalimido peut être éliminé par l'hydrazine.
   On trouvera une liste de différents groupements protecteurs utilisables par exemple dans le brevet BF 2 499 995.
j) Les produits décrits ci-dessus peuvent, si désiré, faire l'objet de réactions de salification par exemple par un acide minéral ou organique ou par une base minérale ou organique selon les méthodes usuelles connues de l'homme du métier. La réaction de salification peut être par exemple réalisée en présence d'acide chlorhydrique par exemple ou encore d'acide tartrique, citrique ou méthane sulfonique, dans un alcool tel que par exemple l'éthanol ou le méthanol.
k) Les éventuelles formes optiquement actives des produits décrits ci-dessus peuvent être préparées par dédoublement des racémiques selon les méthodes usuelles connues de l'homme du métier.

Des illustrations de telles réactions définies ci-dessus sont données dans la préparation des exemples décrits ci-après.

Les produits de formule (I) tels que définis ci-dessus ainsi que leurs sels d'addition avec les acides présentent d'intéressantes propriétés pharmacologiques.
Les produits de la présente invention peuvent ainsi être doués de propriétés inhibitrices d'une ou plusieurs enzymes métaboliques telles que définies ci-dessus notamment de kinases ou de protéases.
Certains produits de formule (I) de la présente invention tels que définis ci-dessus, peuvent donc notamment posséder des propriétés inhibitrices de certaines protéines kinases ou de protéases.

Plusieurs inhibiteurs de kinases ont été décrits comme la butyrolactone, le flavopiridol et la 2(2-hydroxyéthylamino)-6-benzylamino-9-méthylpurine appelée olomoucine.

Plusieurs inhibiteurs de protéases ont été également décrits, notamment dans 'Journal of Medicinal Chemistry, vol. 43 - n° 3 (D. Leung, G. Abbenante et D.P. Fairlie). Selon une liste non exhaustive, on peut citer par exemple comme inhibiteurs de thrombine : l'argatroban, le napsagatran, l'inogatran, l'efegatran, le CVS-1123 ainsi que le melagatran.

On peut également citer comme puissants inhibiteurs du facteur Xa le DX-9065a, le YM-60828 et le ZK 807191 ou encore le FX 2212.

On peut citer comme inhibiteurs d'élastase le ICI-200800, le MR 889, le L-658,758, le MDL 101,146, le ZD 8321 ou encore différents dérivés hétérocycliques tels que des pénicillines, des pénèmes, des β-lactames, des isocoumarines, des benzisothiazolones, des alkylazetidinones.

Comme inhibiteurs de tryptases, on peut citer l'APC-366, comme inhibiteurs de "complément convertases" on peut citer le sepimostat mésylate (FUT-187) ou le nafamostat mésylate (FUT 175).

On peut également citer comme puissant inhibiteur de la cathepsine K, le Cbz-Leu-Leu-Leu-CHO, le 1,3-bis(acylamino)-2-propanone ou des dérivés du 1,5-diacylcarbohydrazide ; comme inhibiteurs de caspases, on peut citer des dérivés 5-aminopyrimidine-6-one, des dérivés phenyl kétométhyl éther ou amino méthylène cétone ; comme inhibiteurs de calpaïnes, on peut citer des aldéhydes peptidiques tels que Cbz-Val-Phe-CHO et calpeptine.

Les niveaux, la régulation et l'activité d'un certain nombre de protéines kinases ou protéases jouent un rôle dans plusieurs pathologies humaines. L'activité d'une protéine kinase ou protéase peut notamment être associée à des récepteurs possédant des domaines transmembranaires ou à des protéines intracellulaires.

Certaines kinases ou protéases peuvent jouer un rôle dans l'initiation, le développement et l'achèvement des évènements du cycle cellulaire et ainsi, des molécules inhibitrices de telles kinases ou protéases sont susceptibles de limiter des proliférations cellulaires non désirées telles que celles observées dans les cancers, psoriasis, croissance de champignons, de parasites (animaux, protistes): de telles molécules inhibitrices de ces kinases ou protéases sont ainsi également susceptibles d'intervenir dans la régulation de maladies neurodégénératives telles que la maladie d'Alzheimer.
Certains produits de formule (I) de la présente invention peuvent ainsi être doués de propriétés antimitotiques. Certains produits de formule (I) telle que définie ci-dessus peuvent comme inhibiteurs de kinase ou protéase avoir notamment la propriété d'inhiber la résorption osseuse médiée par les ostéoclastes. Ils peuvent donc être utiles pour le traitement thérapeutique ou prophylactique de maladies qui sont causées au moins en partie par une augmentation non désirée de la résorption osseuse, par exemple l'ostéoporose. Certains produits de formule (I) de la présente invention peuvent ainsi par exemple inhiber l'adhésion des ostéoclastes sur la surface de l'os et ainsi la résorption osseuse par les ostéoclastes.

Les maladies de l'os dont le traitement ou la prévention nécessitent l'emploi des composés de formule (I) ou de leurs prodrugs, sont notamment l'ostéoporose, l'hypercalcémie, l'ostéopénie, par exemple causée par les métastases osseuses, les désordres dentaires par exemple les parodontites, l'hyperparathyroïdisme, les érosions périarticulaires dans l'arthrite rhumatoïde, la maladie de Paget, l'ostéopénie induite par l'immobilisation. En outre les composés de formule (I) peuvent être utilisés pour soulager, empêcher ou traiter les désordres de l'os qui sont causés par les traitements, par les glucocorticoides, les thérapies liées à la prise de stéroides ou de corticostéroïdes ou par les déficiences d'hormones sexuelles mâles ou femelles. Tous ces désordres sont caractérisés par une perte osseuse, qui est basée par un défaut d'équilibre entre la formation osseuse et la destruction osseuse et qui peut être influencé favorablement par l'inhibition de la résorption osseuse par les ostéoclastes.

Certains produits de formule (I) de la présente invention peuvent posséder en plus de leurs propriétés inhibitrices spécifiques de kinases ou protéases, des effets cellulaires intéressants tels que des propriétés antiprolifératives et notamment des effets sur l'apoptose. On sait par des travaux décrits dans la littérature tel que dans WO 97/20842, que des rapports existent entre le cycle cellulaire et l'apoptose. Parmi les voies conduisant à l'apoptose, certaines sont dépendantes de kinases ou de protéases.

Les produits de la présente invention sont notamment utiles pour la thérapie de tumeurs.
Les produits de l'invention peuvent également ainsi augmenter les effets thérapeutiques d'agents anti-tumoraux couramment utilisés.
Les produits de formule (I) de la présente invention possèdent donc tout particulièrement des propriétés antimitotiques et anti-neurodégénératives.

Certains produits de la présente invention peuvent être inhibiteurs d'effets vasoconstricteurs et hypertenseurs et ainsi produire un effet anti-ischémique, ou encore s'opposer à des effets stimulants au niveau de certains types cellulaires notamment les cellules musculaires lisses, les fibroblastes, les cellules neuronales et les cellules osseuses.

Les produits selon la présente invention peuvent ainsi être utilisés dans le traitement de maladies telles que les maladies prolifératives, le cancer, la resténose, l'inflammation; les allergies, les maladies cardiovasculaires ou certaines maladies infectieuses.

Les produits de la présente invention peuvent également être utilisés dans le traitement de certains désordres gastro-intestinaux, gynécologiques et en particulier pour un effet relaxant au niveau de l'utérus.

Les produits de formule (I) de la présente demande peuvent ainsi posséder d'intéressantes propriétés pharmacologiques justifiant leur application en thérapeutique.

L'invention a ainsi particulièrement pour objet à titre de médicaments, les produits de formule (I)telle que définie ci-dessus, lesdits produits de formule (I) étant sous toutes les formes isomères possibles racémiques, énantiomères et diastéréoisomères, ainsi que les sels d'addition avec les acides minéraux et organiques ou avec les bases minérales et organiques pharmaceutiquement acceptables desdits produits de formule (I).

L'invention a ainsi plus particulièrement pour objet à titre de médicaments, les produits tels que définis par la formule (Ia) ou (Ib), lesdits produits de formule (Ia) ou (Ib) étant sous toutes les formes isomères possibles racémiques, énantiomères et diastéréo-isomères, ainsi que les sels d'addition avec les acides minéraux et organiques ou avec les bases minérales et organiques pharmaceutiquement acceptables desdits produits de formule (Ia) ou (Ib).

L'invention a tout particulièrement pour objet, à titre de médicaments, les produits décrits ci-après dans les exemples et notamment les produits de formule (I) telle que définie ci-dessus, répondant aux formules suivantes :
- le 3-[ 9(S)- benzoylamino -6,10-dioxo-1,2,3,4,7,8,9,10-octahydro -6H-pyridazino[1,2-a][1,2]diazepine-1(S)-carboxamide]-5-méthyl-1-benzoyloxy-hexane-2-one
- le 3-[ 9(S)- (2-furanoyl)amino -6,10-dioxo-1,2,3,4,7,8,9,10- octahydro -6H-pyridazino[1,2-a][1,2]diazepine-1(S)-carboxamide]-5-méthyl-1-(2,6-dichlorobenzoyloxy-hexane-2-one
- le 3(S)-[ 9(S)- (2-furanoyl)amino -6,10-dioxo-1,2,3,4,7,8,9,10- octahydro -6H-pyridazino[1,2-a][1,2]diazepine-1(S)-carboxamide]-4-(4-hydroxyphényl)-1-(2,6-dichlorobenzoyloxy)-butane-2-one ainsi que les produits de formule (I) telle que définie ci-dessus répondant aux formules suivantes :

- (4S) 6-diazo-4-[[[(1S,9S)-9-[(2-methyl-1-oxo-propyl)amino]-octahydro-6,10-dioxo-6H-pyridazino[1,2-a][1,2]diazepine-1-yl]carbonyl]amino]-5-oxo-Hexanoate de (2-propenyle)
- (4S) 6-diazo-4-[[[(1S,9S)-9-[(3-methoxybenzoyl)amino]-octahydro-6,10-dioxo-6H-pyridazino[1,2-a][1,2]diazepine-1-yl]carbonyl]amino]-5-oxo-Hexanoate de (2-propenyle)
- (4S) 6-diazo-4-[[[(1S,9S)-9-[[(2-furanyl)carbonyl] amino]-octahydro-6,10-dioxo-6H-pyridazino[1,2-a] [1,2]diazepine-1-yl]carbonyl]amino]-5-oxo-Hexanoate de(2-propenyle)
- (4S) 6-diazo-4-[[[(1S,9S)-9-[[(cyclohexylamino) carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-diazo-4-[[[(1S,9S)-9-[[[(4-phenoxyphenyl)amino] carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-diazo-4-[[[(1S,9S)-9-[[[[2-(p-2-yl)-ethyl]amino]carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-diazo-4-[[[(1S,9S)-9-[(methylsulfonyl)amino]-octahydro-6,10-dioxo-6H-pyridazino[1,2-a][1,2]diazepine-1-yl]carbonyl]amino]-5-oxo-Hexanoate de (2-propenyle)
- (4S) 6-diazo-4-[[[(1S,9S)-9-[[[4-(trifluoromethyl) phenyl]sulfonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-diazo-4-[[[(1S,9S)-9-[[(2-naphthalenyl) sulfonyl]amino]-octahydro-6,10-dioxo-6H-pyridazino[1,2-a] [1,2]diazepine-1-yl]carbonyl]amino]-5-oxo-Hexanoate de(2-propenyle)
- (4S) 6-diazo-4-[[[(1S,9S)-9-[[[5-(isoxazol-3-yl)-2-thienyl]sulfonyl]amino]-octahydro-6,10-dioxo-6H-pyridazino[1,2-a][1,2]diazepine-1-yl]carbonyl]amino]-5-oxo-Hexanoate de (2-propenyle)
- 9-[(9S)-[[(4-phenoxyphenyl)amino]carbonyl]amino]-N-[(2S)-4-diazo-1-[4-[(1,1-dimethyl)ethoxy]phenyl]-3-oxo-2-butyl]-6H-(1S)-pyridazino[1,2-a][1,2]diazepine-1-carboxamide
- (4S) 6-[(2,6-dichlorobenzoyl)oxy] -4-[[[(1S,9S)-9-[[(2-furanyl)carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[(2,6-dichlorobenzoyl)oxy] -4-[[[(1S,9S)-9-[[[4-(trifluoromethyl)phenyl]sulfonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[4-[[2-oxo-2-(1-pyrrolidinyl)ethyl]piperazin-1-yl]-4-[[[(1S,9S)-9-[[[4-(trifluoromethyl)phenyl] sulfonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[4-[[2-oxo-2-(1-pyrrolidinyl)ethyl]piperazin-1-yl]-4-[[[(1S,9S)-9-[[(2-naphthalenyl)sulfonyl]amino]-octahydro-6,10-dioxo-6H-pyridazino[1,2-a][1,2] diazepine-1-yl]carbonyl]amino]-5-oxo-Hexanoate de (2-propenyle)
- (4S) 6-[(2,6-dichlorobenzoyl)oxy] -4-[[[(1S,9S)-9-[[(cyclohexylamino)carbonyl]amino]-octahydro-6,10-dioxo-6H-pyridazino[1,2-a] [1,2]diazepine-1-yl]carbonyl]amino]-5-oxo-Hexanoate de (2-propenyle)
- (4S) 6-[(1-phenyl-1H-tetrazol-5-yl)thio]-4-[[[(1S,9S)-9-[[[(4-phenoxyphenyl)amino]carbonyl]amino]-octahydro-6,10-dioxo-6H-pyridazino[1,2-a][1, 2] diazepine-1-yl]carbonyl]amino]-5-oxo-Hexanoate de (2-propenyle)
- (4S) 6-[(benzothiazol-2-yl)thio]-4-[[[(1S,9S)-9-[[[(4-phenoxyphenyl)amino]carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[[(4-methoxyphenyl)methyl]thio]-4-[[[(1S,9S)-9-[[[(4-phenoxyphenyl)amino]carbonyl]amino]-octahydro-6,10-dioxo-6H-pyridazino[1,2-a][1,2]diazepine-1-yl] carbonyl)amino]-5-oxo-Hexanoate de (2-propenyle)
- (4S) 6-[[(4-pyridinyl)carbonyl]oxy]-4-[[[(1S,9S)-9-[[[(4-phenoxyphenyl)amino]carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[(2,6-dichlorobenzoyl)oxy]-4-[[[(1S,9S)-9-[[[(4-phenoxyphenyl)amino]carbonyl]amino]-octahydro-6,10-dioxo-6H-pyridazino[1,2-a][1,2]diazepine-1-yl] carbonyl]amino]-5-oxo-Hexanoate de (2-propenyle)
- ((4S) 6-acetyloxy-4-[[[(1S,9S)-9-[[[(4-phenoxyphenyl)amino]carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[1-oxo-2-(thien-3-yl)ethoxy]-4-[[[(1S,9S)-9-[[[(4-phenoxyphenyl)amino]carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[(1-phenyl-1H-tetrazol-5-yl)thio]-4-[[[(1S,9S)-9-[[[[2-(thien-2-yl)-ethyl]amino]carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[(2,6-dichlorobenzoyl)oxy]-4-[[[(1S,9S)-9-[[[[2-(thien-2-yl)-ethyl]amino]carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[(2,6-dichlorobenzoyl)oxy]-4-[[[(1S,9S)-9-[(methylsulfonyl)amino]-octahydro-6,10-dioxo-6H-pyridazino[1,2-a][1,2]diazepine-1-yl]carbonyl]amino]-5-oxo-Hexanoate de (2-propenyle)
- 9-[(9S)[[(4-phenoxyphenyl)amino]carbonyl]amino]-N-((2S)-4-diazo-1-phenyl-3-oxo-2-butyl)- octahydro-6,10-dioxo -6H-(1S)-pyridazino[1,2-a][1,2]diazepine-1-carboxamide
- 9-[(9S)-[[[2-(thien-2-yl)-ethyl]amino]carbonyl]amino]-N-((2S)-4-diazo-1-phenyl-3-oxo-2-butyl)- octahydro-6,10-dioxo-6H-(1S)-pyridazino[1,2-a][1,2]diazepine-1-carboxamide
- 9-[(9S)-[(2-naphthalenyl)sulfonyl]amino ]-N-((2S)-4-diazo-1-phenyl-3-oxo-2-butyl)- octahydro-6,10-dioxo-6H-(1S)-pyridazino[1,2-a][1,2]diazepine-1-carboxamide
- 9-[(9S)-[[5-(isoxazol-3-yl)-2-thienyl]sulfonyl]amino]-N-((2S)-4-diazo-1-phenyl-3-oxo-2-butyl)- octahydro-6,10-dioxo-6H-(1S)-pyridazino[1,2-a][1,2]diazepine-1-carboxamide
- 9-[(9S)-[[(4-phenoxyphenyl)amino]carbonyl]amino]-N-((2S)-4-diazo-3-oxo-2-butyl)- octahydro-6,10-dioxo -6H-(1S)-pyridazino[1,2-a][1,2]diazepine-1-carboxamide
- 9-[(9S)-[[[3-(thien-2-yl)-ethyl]amino]carbonyl]amino]-N-((2S)-4-diazo-3-oxo-2-butyl)- octahydro-6,10-dioxo -6H-(1S)-pyridazino[1,2-a][1,2]diazepine-1-carboxamide
- 9-[(9S)-(3-methoxybenzoyl)amino]-N-((2S)-4-[(2,6-dichlorobenzoyl)oxy] -1-phenyl-3-oxo-2-butyl]- octahydro-6,10-dioxo-6H-(1S)-pyridazino[1,2-a][1,2] diazepine-1-carboxamide
- 9-[(9S)-[(2-furanyl)carbonyl]amino]-N-[(2S)-4-[(2,6-dichlorobenzoyl)oxy] -1-phenyl-3-oxo-2-butyl]- octahydro-6,10-dioxo-6H-(1S)-pyridazino[1,2-a][1,2] diazepine-1-carboxamide
- 9-[(9S)-[(cyclohexylamino)carbonyl]amino]-N-[(2S)-4-[(2,6-dichlorobenzoyl)oxy] -1-phenyl-3-oxo-2-butyl]- octahydro-6,10-dioxo-6H-(1S)-pyridazino[1,2-a][1,2] diazepine-1-carboxamide
- 9-[(9S)-[[(4-phenoxyphenyl)amino]carbonyl]amino]-N-[(2S)-1-phenyl-4-[(1-phenyl-1H-tetrazol-5-yl)thio]-3-oxo-2-butyl]-octahydro-6,10-dioxo-6H-(1S)-pyridazino[1,2-a][1,2]diazepine-1-carboxamide
- 9-[(9S)-[[(4-phenoxyphenyl)amino]carbonyl]amino]-N-[(2S)-1-phenyl-4-[1-oxo-2-(3-thienyl)ethoxy]-3-oxo-2-butyl]-octahydro-6,10-dioxo-6H-(1S)-pyridazino[1,2-a][1,2]diazepine-1-carboxamide
- Pentanedioate de (methyle)[2-oxo-4-phenyl-3[(3S)-[[(4-phenoxyphenyl)amino]carbonyl]amino]- octahydro-6,10-dioxo-6H-(1S)-pyridazino[1,2-a][1,2]diazepin-1-yl]carbonyl] amino] butyle]
- 9-[(9S)-[[(4-phenoxyphenyl)amino]carbonyl]amino]-N-[(2S)-4-[(4-phenylmethyl)-1-piperidinyl ]-1-phenyl-3-oxo-2-butyl]-octahydro-6,10-dioxo-6H-(1S)-pyridazino[1,2-a][1,2]diazepine-1-carboxamide
- 9-[(9S)-[[[2-(thien-2-yl)-ethyl]amino]carbonyl]amino]-N-[(2S)-1-phenyl-4-[(1-phenyl-1H-tetrazol-5-yl)thio]-3-oxo-2-butyl]- octahydro-6,10-dioxo-6H-(1S)-pyridazino [1,2-a][1,2]diazepine-1-carboxamide
- 9-[(9S)-[[[2-(thien-2-yl)-ethyl]amino]carbonyl]amino]-N-[(2S)-1-phenyl-4-[(2-benzothiazolyl)thio]-3-oxo-2-butyl]-octahydro-6,10-dioxo-6H-(1S)-pyridazino[1,2-a][1,2]diazepine-1-carboxamide
- 9-[(9S)-[[[2-(thien-2-yl)-ethyl]amino]carbonyl]amino]-N-[(2S)-1-phenyl-4-[(-2,6-dichlorobenzoyl)oxy] -3-oxo-2-butyl]-octahydro-6,10-dioxo-6H-(1S)-pyridazino[1,2-a][1,2]diazepine-1-carboxamide
- 9-[(9S)-[[[2-(thien-2-yl)-ethyl]amino]carbonyl]amino]-N-[(2S)-1-phenyl-4-[4-[2-(1-pyrrolidinyl)-2-oxo-ethyl]piperazin-1-yl]] -3-oxo-2-butyl]- octahydro-6,10-dioxo-6H-(1S)-pyridazino[1,2-a][1,2]diazepine-1-carboxamide
- 9-[(9S)-(methylsulfonyl)amino]-N-[(2S)-1-phenyl-4-[(-2,6-dichlorobenzoyl)oxy] -3-oxo-2-butyl]- octahydro-6,10-dioxo-6H-(1S)-pyridazino[1,2-a][1,2]diazepine-1-carboxamide
- 9-[(9S)-[[[4-(trifluoromethyl)phenyl]sulfonyl]amino]-N-[(2S)-1-phenyl-4-[(1-phenyl-1H-tetrazol-5-yl)thio]-3-oxo-2-butyl]- octahydro-6,10-dioxo-6H-(1S)-pyridazino [1,2-a][1,2]diazepine-1-carboxamide
- 9-[(9S)-[[[4-(trifluoromethyl)phenyl]sulfonyl]amino]-N-[(2S)-1-phenyl-4-(2-benzothiazolyl)thio]-3-oxo-2-butyl]-octahydro-6,10-dioxo-6H-(1S)-pyridazino[1,2-a][1,2]diazepine-1-carboxamide
- 9-[(9S)-(methylsulfonyl)amino]-N-[(3S)-5-methyl-1-[(2,6-dichlorobenzoyl)oxy] -2-oxo-3-hexyl]- octahydro-6,10-dioxo-6H-(1S)-pyridazino[1,2-a][1,2]diazepine-1-carboxamide
- 9-[(9S)-[[[4-(trifluoromethyl)phenyl]sulfonyl]amino]]-N-[(3S)-5-methyl-1-[(2,6-dichlorobenzoyl)oxy] -2-oxo-3-hexyl]-octahydro-6,10-dioxo-6H-(1S)-pyridazino[1,2-a][1,2]diazepine-1-carboxamide
- 9-[(9S)-[(2-naphthalenyl)sulfonyl]amino]-N-[(3S)-5-methyl-1-[(2,5-dichlorobenzoyl)oxy] -2-oxo-3-hexyl]- octahydro-6,10-dioxo-6H-(1S)-pyridazino[1,2-a][1,2] diazepine-1-carboxamide
- 9-[(9S)-(2-methyl-1-oxo-propyl)amino]-N-((3S)-1-diazo-5-methyl-2-oxo-3-hexyl)- octahydro-6,10-dioxo -6H-(1S)-pyridazino[1,2-a][1,2]diazepine-1-carboxamide
- 9-[(9S)- (3-methoxybenzoyl)amino]-N-((3S)-1-diazo-5-methyl-2-oxo-3-hexyl)- octahydro-6,10-dioxo -6H-(1S)-pyridazino[1,2-a][1,2]diazepine-1-carboxamide
- 9-[(9S)-[(2-furanyl)carbonyl]amino]-N-((3S)-1-diazo-5-methyl-2-oxo-3-hexyl)- octahydro-6,10-dioxo -6H-(1S)-pyridazino[1,2-a][1,2]diazepine-1-carboxamide
- 9-[(9S)-[(cyclohexylamino)carbonyl]amino]-N-((3S)-1-diazo-5-methyl-2-oxo-3-hexyl)- octahydro-6,10-dioxo -6H-(1S)-pyridazino[1,2-a][1,2]diazepine-1-carboxamide
- 9-[(9S)-[[(4-phenoxyphenyl)amino]carbonyl]amino]-N-((3S)-1-diazo-5-methyl-2-oxo-3-hexyl)- octahydro-6,10-dioxo -6H-(1S)-pyridazino[1,2-a][1,2]diazepine-1-carboxamide
- 9-[(9S)-[[[2-(thien-2-yl)-ethyl]amino]carbonyl]amino]-N-((3S)-1-diazo-5-methyl-2-oxo-3-hexyl)- octahydro-6,10-dioxo-6H-(1S)-pyridazino[1,2-a][1,2]diazepine-1-carboxamide
- 9-[(9S)-(methylsulfonyl)amino]-N-((3S)-1-diazo-5-methyl-2-oxo-3-hexyl)- octahydro-6,10-dioxo -6H-(1S)-pyridazino[1,2-a][1,2]diazepine-1-carboxamide
- 9-[(9S)-[[4-(trifluoromethyl)phenyl]sulfonyl]amino]-N-((3S)-1-diazo-5-methyl-2-oxo-3-hexyl)- octahydro-6,10-dioxo-6H-(1S)-pyridazino[1,2-a][1,2]diazepine-1-carboxamide
- 9-[(9S)-[(2-naphthalenyl)sulfonyl]amino]-N-((3S)-1-diazo-5-methyl-2-oxo-3-hexyl)- octahydro-6,10-dioxo -6H-(1S)-pyridazino[1,2-a][1,2]diazepine-1-carboxamide
- 9-[(9S)- (3-methoxybenzoyl)amino]-N-[(3S)-1-[(benzothiazol-2-yl)thio]-4-methyl-2-oxo-3-pentyl]- octahydro-6,10-dioxo-6H-(1S)-pyridazino[1,2-a][1,2] diazepine-1-carboxamide
- 9-[(9S)-[(2-furanyl)carbonyl]amino]-N-[(3S)-1-[(benzothiazol-2-yl)thio]-4-methyl-2-oxo-3-pentyl]-octahydro-6,10-dioxo -6H-(1S)-pyridazino[1,2-a][1,2] diazepine-1-carboxamide
- 9-[(9S)-[(cyclohexylamino)carbonyl]amino]-N-[(3S)-1-[(benzothiazol-2-yl)thio]-4-methyl-2-oxo-3-pentyl]-octahydro-6,10-dioxo -6H-(1S)-pyridazino[1,2-a][1,2] diazepine-1-carboxamide
- 9-[(9S)-[[(4-phenoxyphenyl)amino]carbonyl]amino]-N-[(3S)-1-[(1-phenyl-1H-tetrazol-5-yl)thio]-4-methyl-2-oxo-3-pentyl]-octahydro-6,10-dioxo -6H-(1S)-pyridazino [1,2-a][1,2]diazepine-1-carboxamide
- 9-[(9S)-[[(4-phenoxyphenyl)amino]carbonyl]amino]-N-[3S)-1-[(benzothiazol-2-yl)thio]-4-methyl-2-oxo-3-pentyl]-octahydro-6,10-dioxo -6H-(1S)-pyridazino[1,2-a][1,2]diazepine-1-carboxamide
- 9-[(9S)-[[[2-(thien-2-yl)-ethyl]amino]carbonyl]amino]-N-[(3S)-1-[(1-phenyl-1H-tetrazol-5-yl)thio]-4-methyl-2-oxo-3-pentyl]- octahydro-6,10-dioxo -6H-(1S)-pyridazino [1,2-a][1,2]diazepine-1-carboxamide
- 9-[(9S)-[[[2-(thien-2-yl)-ethyl]amino]carbonyl]amino]-N-[(3S)- 1-[(benzothiazol-2-yl)thio]-4-methyl-2-oxo-3-pentyl]-octahydro-6,10-dioxo -6H-(1S)-pyridazino[1,2-a][1,2]diazepine-1-carboxamide
- 9-[(9S)-[(cyclohexylamino)carbonyl]amino]-N-[(2S)-4-[(2,6-dichlorobenzoyl)oxy]-3-oxo-2-butyl]- octahydro-6,10-dioxo-6H-(1S)-pyridazino[1,2-a][1,2]diazepine-1-carboxamide
- 9-[(9S)-[(2-furanyl)carbonyl]amino]-N-[(2S)-4-[(2,6-dichlorobenzoyl)oxy]-3-oxo-2-butyl]- octahydro-6,10-dioxo-6H-(1S)-pyridazino[1,2-a][1,2]diazepine-1-carboxamide.

Les produits de formule (I) de la présente invention peuvent donc notamment être utilisés sous forme de médicaments pour inhiber un mécanisme non désiré provoquant une pathologie sous l'effet d'une ou plusieurs protéase(s) ou kinase(s) : la méthode peut ainsi consister en l'administration au patient dont le traitement requiert l'inhibition de l'effet de telle protéase ou kinase, d'une quantité inhibitrice d'un tel médicament selon la présente invention.

Les médicaments objet de l'invention peuvent ainsi être utilisés dans le traitement et la prévention d'affections cardiovasculaires associées à une altération de la vasomotricité ou de la volémie, de l'infarctus du myocarde et ses conséquences, l'insuffisance cardiaque, l'insuffisance rénale, l'angine de poitrine, l'hyperaldostéronisme, hypertension artérielle et ses conséquences , le traitement et la prévention des complications en particulier les hypertrophies cardiaques et vasculaires ainsi que le développement de fibroses au niveau des organes cibles, prévention et traitement de l'hypertension, de désordres métaboliques, endocrinologiques et neurologiques, des chocs endotoxiques, des hémorragies 'subarachnoid', de l'arythmie, de l'asthme, de l'insuffisance rénale aiguë ('acute renal failure'), de 'preeclampsia' et de diabètes.

Les médicaments objet de l'invention peuvent ainsi être utilisés dans le traitement de spasmes vasculaires, dans le traitement des suites d'une hémorragie cérébrale, dans le traitement des spasmes coronariens, des spasmes vasculaires périphériques. Ces médicaments peuvent notamment être utilisés dans le traitement de l'insuffisance cardiaque congestive, dans la prévention des resténoses post-angioplastie, la prévention des fibroses cardiaques et vasculaires, dans le traitement de l'athérosclérose et de certaines formes d'hypertension comme l'hypertension pulmonaire ainsi que dans le traitement de l'asthme.

Ces médicaments objet de l'invention pourraient également être utilisés pour le traitement du glaucome et de différents types de spasmes viscéraux, ainsi qu'à titre de substances protectrices neuronales ou encore dans la prévention des resténoses post-angioplastie. Notamment les médicaments objet de l'invention peuvent être utilisés pour leurs effets anti-hypertrophique et anti-fibrotique aux niveaux cardiaque et vasculaire. Tout particulièrement, ils peuvent être utilisés pour le traitement et la prévention des désordres cardiovasculaires associés au diabète.

Les médicaments objet de la présente invention peuvent également trouver une application dans le traitement de l'ostéoporose et en tant que protecteurs neuronaux.
Les médicaments objet de l'invention peuvent également être utilisés dans le traitement des troubles de la mémoire et des fonctions cognitives ainsi que de l'anxiété.

Les médicaments, objet de l'invention, peuvent également trouver, comme antimitotiques, leur emploi dans la chimiothérapie des cancers, ou encore dans le traitement de psoriasis, de parasitoses telles que celles dues à des protistes ou à des champignons ou encore dans le traitement de la maladie d'Alzheimer ou dans le traitement de l'apoptose neuronale.

L'invention a tout particulièrement pour objet les compositions pharmaceutiques contenant à titre de principe actif l'un au moins des médicaments tels que définis ci-dessus.

L'invention a tout particulièrement pour objet les compo-sitions pharmaceutiques telles que définies ci-dessus caractérisées en ce qu'elles sont utilisées comme médicaments tels que définis ci-dessus.

Les compositions pharmaceutiques de la présente invention telles que définies ci-dessus peuvent être administrées par voie buccale, par voie parentérale ou par voie locale en application topique sur la peau et les muqueuses ou par injection par voie intraveineuse ou intramusculaire.

Ces compositions peuvent être solides ou liquides et se présenter sous toutes les formes pharmaceutiques couramment utilisées en médecine humaine comme, par exemple, les comprimés simples ou dragéifiés, les pilules, les tablettes, les gélules, les gouttes, les granulés, les préparations injectables, les pommades, les crèmes ou les gels ; elles sont préparées selon les méthodes usuelles. Le principe actif peut y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

La posologie usuelle, variable selon le produit utilisé, le sujet traité et l'affection en cause, peut être, par exemple, de 0,05 -à 5 g par jour chez l'adulte, ou de préférence de 0,1 à 2 g par jour.

L'invention a notamment pour objet les compositions pharmaceutiques telles que définies ci-dessus caractérisées en ce qu'elles sont utilisées comme médicament antimitotique, en particulier pour la chimiothérapie de cancers ou encore pour le traitement de psoriasis, de parasitoses telles que celles dues à des champignons ou à des protistes ou de la maladie d'Alzheimer.

L'invention a également tout particulièrement pour objet les compositions pharmaceutiques telles que définies ci-dessus caractérisées en ce qu'elles sont utilisées comme médicaments antineurodégénératifs notamment anti-apoptose neuronale.

L'invention a particulièrement pour objet l'utilisation des produits de formule (1) telle que définie ci-dessus pour la préparation de compositions pharmaceutiques destinées au traitement ou à la prévention d'affections nécessitant une inhibition d'une ou plusieurs enzymes métaboliques telles que définies ci-dessus.

L'invention a ainsi particulièrement pour objet l'utilisation des produits de formule (I) telle que définie ci-dessus pour la préparation de compositions pharmaceutiques destinées au traitement de maladies résultant d'anomalies dans les niveaux, la régulation ou l'activité d'un certain nombre d'enzymes métaboliques. De telles anomalies jouent un rôle dans certaines pathologies humaines qui intéressent particulièrement la présente invention.

La présente invention concerne donc notamment l'utilisation des produits de formule (I) de la présente invention comme inhibiteurs de protéases ou kinases. La présente invention concerne ainsi l'inhibition de certaines de telles enzymes protéases ou kinases et revendique l'emploi de produits de la présente invention comme inhibiteurs dans les cas où l'inhibition de telle protéase ou kinase est indiquée.

L'invention a ainsi tout particulièrement pour objet l'utilisation des produits de formule (I) telle que définie ci-dessus ou de sels pharmaceutiquement acceptables desdits produits de formule (I) pour la préparation de médicaments destinés à la prévention ou au traitement de médicaments destinés à la prévention ou au traitement de maladies dans lesquelles des enzymes métaboliques telles que des protéases ou des kinases sont impliquées.

L'invention a plus particulièrement pour objet l'utilisation des produits de formule (I) telle que définie ci-dessus ou de sels pharmaceutiquement acceptables desdits produits de formule (I) caractérisée en ce que.les maladies à prévenir ou à traiter sont choisis dans le groupe de maladies suivant : maladies cardiovasculaires, cancers , maladies du système nerveux central, maladies inflammatoires, maladies infectieuses ou encore maladies de l'os.

L'invention a tout particulièrement pour objet l'utilisation des produits de formule (I) telle que définie ci-dessus dessus ou de sels pharmaceutiquement acceptables desdits produits de formule (I) caractérisée en ce que.les maladies à prévenir ou à traiter sont des maladies du système nerveux central ou des maladies de l'os telles que notamment l'ostéoporose.

La présente invention a ainsi pour objet l'utilisation des produits de formule (I) telle que définie ci-dessus ou de sels pharmaceutiquement acceptables desdits produits de formule (I) pour la préparation de médicaments destinés notamment à la prévention et au traitement de maladies du métabolisme osseux.

L'invention a ainsi tout particulièrement pour objet l'utilisation des produits de formule (I) telle que définie ci-dessus pour la préparation de compositions pharmaceutiques destinées au traitement de l'hypertension artérielle, de l'insuffisance cardiaque, des resténoses post-angioplastie,. de spasmes vasculaires, des suites d'une hémorragie cérébrale et des insuffisances rénales.

L'invention a ainsi tout particulièrement pour objet l'utilisation des produits de formule (1) telle que définie ci-dessus pour la préparation de compositions pharmaceutiques destinées au traitement de l'infarctus du myocarde, à la prévention des resténoses post-angioplastie et à la prévention des fibroses cardiaques et vasculaires.

L'invention a ainsi tout particulièrement pour objet l'utilisation des produits de formule (1) telle que définie ci-dessus pour la préparation de compositions pharmaceutiques destinées au traitement de l'insuffisance rénale.

L'invention a ainsi tout particulièrement pour objet l'utilisation des produits de formule (1) telle que définie ci-dessus pour la préparation de compositions pharmaceutiques destinées au traitement et à la prévention des désordres cardiovasculaires associés au diabète.

L'invention a notamment pour objet l'utilisation des produits de formule (I) telle que définie ci-dessus pour la préparation de médicaments destinés à la chimiothérapie de cancers, au traitement de psoriasis, de parasitoses telles que celles dues à des champignons ou à des protistes, au traitement de la maladie d'Alzheimer ou au traitement d'affections neurodégénératives notamment l'apoptose neuronale.

Les produits de départ de formule (VIa) qui sont donc des acides aminés naturels ou non naturels peuvent être trouvés dans le commerce ou préparés selon les méthodes usuelles connues de l'homme du métier.

On peut citer notamment les produits de formule (VIa) suivants : Glu, Gly, Ala, Val, Phe ou Tyr, ces acides aminés étant éventuellement sous une forme protégée par exemple par un groupe alloc ou terbutyle.

Les produits de formule (VIb) peuvent être préparés à partir d'acides aminés dits naturels ou non naturels de formule (VIa), comme indiqué ci-dessus, par fixation du radical Fmoc sur la fonction amine N-terminale de l'acide aminé concerné : une telle réaction est notamment décrite dans l'article dont la référence suit :
'Mueller,A. ;Vogt,C. ; Sewald,N. ; Synthesis (1998),(6),837-841'.

On trouve également dans le commerce un grand nombre de produits de formule (VIb) qui sont donc des acides aminés sous forme protégée fmoc et qui peuvent alors constituer des produits de départ pour le procédé de la présente demande décrit à la figure 1.

On peut citer notamment les produits de formule (VIb) suivants : Fmoc-Leu, Fmoc-L-Glu(Alloc); Fmoc-Gly; Fmoc-Ala; Fmoc-Val; Fmoc-Phe ou encore Fmoc-Tyr(OtBu)

Le produit de formule (II) est l'ester méthylique de l'acide 9(S)-amino-6,10-dioxo-1,2,3,4,7,8,9,10-octahydro-6H-pyridazino[1,2-a][1,2]diazepine-1(S)-carboxylique. Un tel produit de départ de formule (II) peut être préparé selon les méthodes usuelles connues de l'homme du métier ou encore comme indiqué dans la demande de brevet EP 94095 ou dans les publications suivantes:
- Attwood M. and al, J. Chem. Soc., Perkin trans, 1, (1986), (6), 1011-19
- Thomas, W et al, J. Chem. Soc., Perkin trans, 2, (1986), (5), 747-55

Le produit de départ de formule (III) soit R1X dans lequel X représente un atome d'halogène peut notamment être choisi dans le tableau 1 décrit à la figure 2 de la présente invention : dans un tel tableau, X représente un atome de chlore mais peut tout aussi bien représenter un atome de brome. Un tel produit de formule (III) peut être trouvé dans le commerce ou peut être préparé selon les méthodes usuelles connues de l'homme du métier.

On peut citer par exemple les produits de formule (III) suivants : le chlorure de benzoyle, le chlorure de l'acide isopropanoïque, le cyclohexylisocyanate, le chlorure de méthanesulfonyle ou encore le chlorure de benzène sulfonyle. Le produit de départ de formule (VIII) soit R4'-L-H dans lequel R4' et L ont les significations indiquées ci-dessus peut notamment être choisi dans le tableau 2 décrit à la figure 3 de la présente invention Un tel produit de formule (VIII) peut être trouvé dans le commerce ou peut être préparé selon les méthodes usuelles connues de l'homme du métier.

On peut citer notamment les produits de formule (VIII) suivants : mercaptobenzimidazole, acide benzoïque, 1-phénylpipérazine, 4-fluorophénol, pipéridine.

La partie expérimentale ci-après donne des exemples de tels produits de départ.

La présente invention a enfin pour objet à titre de produits industriels nouveaux, les composés de formule (VII) telle que définie dans le procédé de la présente invention. Les tableaux de la figure 4 représentés ci-après aux pages 69 à 182 représentent des produits qui ont été obtenus par le procédé décrit ci-dessus selon la présente invention : ces produits constituent des exemples de produits de formule (I) de la présente invention et sont caractérisés dans les tableaux ci-dessous par leurs masses moléculaires représentées par MM sur ces tableaux. De telles valeurs MM indiqués dans ces tableaux ont été déterminés comme suit. Les produits représentés dans les tableaux de la figure 4 ont été préparés et caractérisés par leurs spectres HPLC-SM soit par chromatographie sous haute pression couplée à un spectrographe de masse : de telles chromatographies ont été effectuées en utilisant une colonne de type ODS-AQ et un mélange éluant acétonitrile/eau en proportion variable en fonction des composés. L'électrospray en mode positif a été utilisé pour la détermination des masses moléculaires. La masse de chaque produit a été déterminée par la masse de l'ion moléculaire H+ ou sous forme d'adduit du sodium ou de l'acétonitrile.

On peut noter que l'acide aminé de départ pour les produits des exemples 1 et 2 est la Leucine : en fait le produit de formule (VIb) correspondant soit Fmoc-Leu est commercialisé et constitue le point de départ de la synthèse du produit de l'exemple 1.

Pour les autres exemples de la présente demande indiqués sur les tableaux de la figure 4 pour lesquels l'acide aminé concerné est également la Leu, on procède à l'étape 1 de la synthèse de ces produits de la même façon que pour l'exemple 1 décrit ci-après dans la partie expérimentale.

Pour les autres exemples de la présente demande indiqués sur les tableaux de la figure 4 pour lesquels l'acide aminé concerné est un acide aminé autre que la Leu, on procède à l'étape 1 de la synthèse de ces produits également de la même façon que pour l'exemple 1 décrit ci-après dans la partie expérimentale en utilisant à la place de Fmoc-Leu, le composé de formule (VIb) correspondant à l'acide aminé concerné soit. les Fmoc acides aminés suivants : Fmoc-L-Glu(Alloc); Fmoc-Gly; Fmoc-Ala; Fmoc-Val; Fmoc-Phe et Fmoc-Tyr(OtBu) : on obtient ainsi respectivement des produits finals dans lesquels le reste d'acide aminé est un reste de Glu ou Glu(Alloc), Gly, Ala, Val, Phe ou Tyr(OtBu).

Pour les autres exemples de la présente demande indiqués sur les tableaux de la figure 4 pour lesquels l'acide aminé concerné est la tyrosine, on procède comme indiqué ci-après à la préparation de l'exemple 3, en utilisant le produit de formule (VI) Fmoc-Tyr(OtBu) pour obtenir le produit final de formule (I) correspondant dans lequel le radical hydroxyle est sous forme terbutoxy : on libère ensuite sur le produit de formule (I) ainsi obtenu, la fonction hydroxyle afin d'obtenir un nouveau produit de formule (I) dans lequel le reste d'acide aminé est alors un reste de tyrosine. La déprotection de tels dérivés de formule (I) où R2 représente (tBuO)PhCH2 peut être réalisée par exemple par la méthode classique à l'acide trifluoroacétique en mélange 50 /50 avec du dichlorométhane. Le schéma d'une telle réaction est indiqué ci-après.

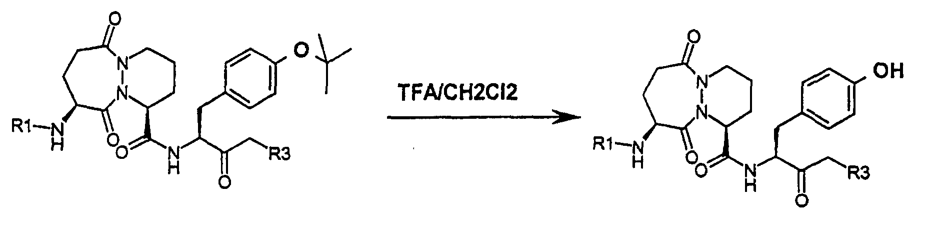

Les exemples donnés dans les tableaux de la figure 4 décrite ci-après illustrent le procédé décrit ci-dessus mais ne limitent en aucun cas la présente invention.

Les exemples décrits à la partie expérimentale de la présente demande qui sont donc les exemples 1 à 3 des tableaux de la figure 4, donnent des exemples de réalisation de la présente invention qui illustrent l'invention sans toutefois la limiter.

### Partie expérimentale

### Exemple 1 : 3-[ 9(S)- benzoylamino -6,10-dioxo-1,2,3,4,7,8,9,10- octahydro -6H-pyridazino[1,2-a][1,2]diazepine-1(S)-carboxamide]-5-méthyl-1-benzoyloxy-hexane-2-one

### Etape 1 : N-Fmoc-3-amino-1-diazo-5-méthyl-hexane-2-one

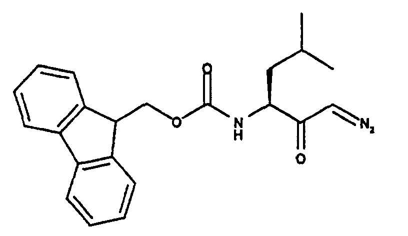

A une suspension de 3.52g de Fmoc-Leucine (11.3 mmoles) dans 35 ml de dichlorométhane sont additionnés successivement sous azote et à -10°C , 1.3 ml de 4-méthyl-morpholine (11.9 mmoles; 1.05 équ.) et goutte à goutte, 1.5 ml de de chloroformate d'isobutyle (11.6 mmoles; 1.03 équ.). Le mélange réactionnel devient limpide, puis un précipité de chlohydrate de 4-méthyl-morpholine apparaît. Après 1 heure d'agitation à -10°C, le précipité est filtré.

La solution résultante est refroidie à -10°C, puis, sous azote, 75ml (19.1 mmoles, 2équ.) de solution 0.3M de diazométhane dans CH2Cl2 sont additionnés.
La température est ramené progressivement à TA . Après 1 heure de réaction à TA le mélange est versé dans 100 ml de solution saturée de bicarbonate de sodium. La phase organique est lavée à l'eau (100 ml), sèchée sur sulfate de magnésium, puis concentrée à sec. Le brut est directement chromatographié sur 500g de silice en utilisant CH2Cl2/AcOEt 95/5. On isole 2.6g (rdt= 65%) d'un solide jaunâtre).
MS : MH⁺378
RMN H¹ (CDCl3) (250MHz) 0.95 (6H;d ;2CH3), 1.40 à 1.85(3H ;2m ; 1CH et 1CH2) 4.18(1H,t,CH Fmoc),4.4 à 4.55(2H ;m ;1CH et 1CHN₂), 5.28(1H,dl,NHCO), 4.42(2H ;dl ;CH2 Fmoc), 5.12(1H ;sl ; CHN2), 6.90 à 7.45(9H ;m ;H aromatiques), 7.55(2H ;tl ;H aromatiques), 7.77(2H ;dl ;H aromatiques)

### Etape 2 :

### a) ester méthylique de l'acide 9(S)- benzoylamino -6,10-dioxo-1,2,3,4,7,8,9,10- octahydro -6H-pyridazino[1,2-a][1,2]diazepine-1(S)-carboxylique

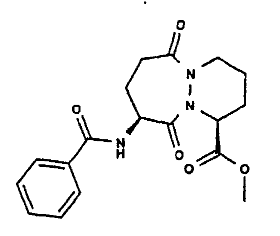

A une solution de 500 mg d'amine (1) (1,96 mmoles) dans 12 ml de CH2Cl2, est,additionnée, à 0°C sous azote, successivement 0,68 ml de diisopropylethylamine (2 mmoles ; 2 équi.) suivie de 0,26 ml de chlorure de benzoyl (2,16 mmoles, 1.1 équi.). La température est ramenée à TA. Après 1 heure d'agitation, 12 ml d'HCl 1N sont additionnées. Le mélange est décanté et la phase organique successivement lavée avec 12 ml de solution saturée de NaCl et 12 ml de solution saturée de bicarbonate de sodium. La solution organique est sèchée sur sulfate de magnésium puis concentrée à sec. On isole 0,424 mg (rdt =54%) de benzoylaminopyrazépate de méthyl.
MS : [MH]⁻ 358 ; MH⁺ 360 ; MNa⁺ 382

### b) l'acide 9(S)- benzoylamino -6,10-dioxo-1,2,3,4,7,8,9,10-octahydro -6H-pyridazino[1,2-a][1,2]diazepine-1(S)-carboxylique

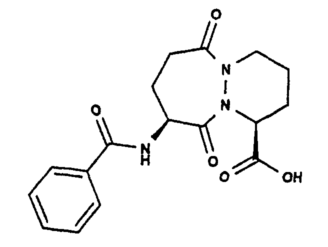

A une solution de 424 mg (1,18 mmole) de 9(S)-benzoylamino-octahydro-6,10-dioxo-6H-pyridainzo[1,2-a][1,2]diazepine-1(S)-carboxylique methyl ester dans 10 ml de méthanol est aditionné 99 mg d'hydoxyde de lithium (2,36 mmoles ; 2équi.) à 0°C. La température est remontée à TA puis la réaction maintenue 1 nuit. La solution est concentrée au rotavapor jusqu'à 5 ml. 5 ml d'eau sont additionnés et le pH de la solution est ajustée à 1 par addition de quelques gouttes d'HCl concentré. Le mélange est extrait par 2X5ml d'acétate d'éthyle. La phase organique est lavée par 5 ml d'une solution saturée de NaCl, sèchée sur MgSO4 et concentrée à sec. On obtient 305 mg d'acide 9(S)-benzoylamino-octahydro-6,10-dioxo-6H-pyridazino[1,2-a] [1,2]diazepine-1(S)-carboxylique (rdt = 75%).
MS : [MH]⁻ 344, MH⁺346, MNa⁺ 368
RMN-H¹ (CDCl3) (300MHz) : 1,76(2H ;m ;CH 2) , 1,96 et 2,35(2H ;2m ;CH2), 1,97 et 2,84(2H ;2m ;CH2), 2,41 et3,48(2H ;2m ;CH2), 4,96 et 4.68(2H ;m et dl ;CH2), 5,17(1H ;m ;CHN), 5,47(1H ;dd ;CHCO2H), 7,26(1H ;d ;CONH), 7,38 à 7,53(3H ;m ;H aromatiques), 7,81(2H ;dl ;2H aromatiques)

### Etape 3 : 3-[ 9(S)- benzoylamino -6,10-dioxo-1,2,3,4,7,8,9,10- octahydro -6H-pyridazino[1,2-a] [1,2]diazepine-1(S)-carboxamide]-5-méthyl-1-diazo-hexane-2-one

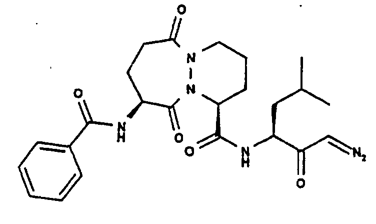

378mg(1 mmole) de N-Fmoc-3-amino-1-diazo-5-méthyl-hexane-2-one en solution dans 3.5ml de CH2Cl2 est traitée par 0.31ml de DBU(2.1mmole ; 2.1 équi.) à 0°C sous azote. Après une heure de réaction la solution est déposée sur une colonne de silice (40g) et éluée rapidement par une solution de CH2Cl2/méthanol 90/10. L'huile jaune obtenue (198mg, rdt=100%) est solubilisée dans 3.5 ml de CH2Cl2 puis 0,35 g (1mmole) d'acide obtenu à l'étape 2 b) et ajoute . 625mg de TBTU(1.1mmole ; 1.1 équi.) suivi de 0.35ml de DIEA(2mmoles ; 2équi) sont successivement additionnés sous azote à 0°C . La réaction est maintenue 1 nuit à TA puis le milieu réactionnel versé dans 20ml de solution saturée de NaHCO3 et extrait par 2X10ml d'acétate d'éthyle. Les phases organiques rassemblées sont lavées à l'eau (20ml) puis avec 20ml de solution saturée de KHSO3. La solution est sèchée sur MgSO4 et concentrée à sec. On isole 244mg d'une mousse (rdt=50%).
MS : [M+Na]⁺ 505 ; MH⁺-N2 455 ; MH⁺483
RMN-H¹ (CDCl3) (300MHz) : 0.95(6H ;dd ;2CH3), 1.56(2H ;m ;CH2), 1.69(1H ;m ;CH), 4.53(2H ;m ;CH2 ; 6.40(1H;dl;NH), 1.66(2H ;m ;CH2), 1.92(2H ;m ;CH2), 1.90 et 2.90(2H ;2m,CH2) ; 2.39 et3.24(2H ;1dd et 1dt ; CH2), 3.01 et 4.66(2H ;1dt et 1m ;CH2), 4.66(1H ;td ;CH), 5.17(1H ;m ;CH) , 7.02(1H,d ;NH), 5.40(1H ;sl ;CHN₂ ), 7.46(2H ;dd ;2H aromatiques méta), 7.51(1H ;m ; H aromatique en para), 7.81(2H ;dd ;H aromatiques en ortho).

### Etape 4: 3-[ 9(S)- benzoylamino -6,10-dioxo-1,2,3,4,7,8,9,10- octahydro -6H-pyridazino[1,2-a][1,2]diazepine-1(S)-carboxamide]-5-méthyl-1-benzoyloxy-hexane-2-one

### a) 3-[ 9(S)- benzoylamino -6,10-dioxo-1,2,3,4,7,8,9,10-octahydro -6H-pyridazino[1,2-a] [1,2]diazepine-1(S)-carboxamide]-5-méthyl-1-bromo-hexane-2-one

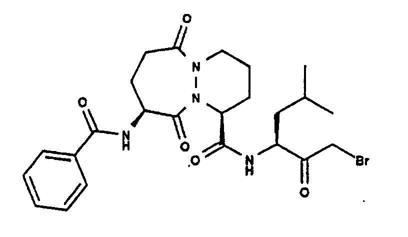

219mg (0.38mmole) en solution dans 2ml de CH2Cl2 sont traitées à 0°C sous azote avec 0.2ml de solution d'HBr(37%)/AcOH 30/70. Après 1/2heure de réaction à O°C le mélange est versé dans 20ml de solution saturée de NaHCO3. Le mélange est extrait par 2X10ml d'AcOEt. Les phases organiques sont lavées à l'eau (20ml), séchées sur MgSO4 et concentrées sous vide. On isole 203mg(rdt=94%) de bromocétone sous forme d'une mousse jaunâtre.
MS : M+H⁺ 535
RMN H¹ (CDCl3) (300MHz) : 0.97(6H ;d ;2CH3),1.67(1H ;m ;CH), 1.51-1.69(2H,2m ;2H), 1.66(2H ;m ;CH2), 1.94(2H ;m ;CH2), 1.88 à1.98 et 2.12 à 2.90(2H ;2m ;CH2), 2.40 et 3.16 à 3.47(2H ;dd et m ;CH2), 3.01-4.61(2H ;2m ;CH2), 4.57(1H ;t ;CH), 5.16(1H ;m ;CH) ; 7.00(1H ;d ;NH), 4.05(2H ;AB ;CH2), 7.47(2H ;td ;2H aromatique en méta), 7.53(1H ;m ; H aromatique en para), 7.82(2H ;dd ;2H aromatique en para).

### b): 3-[ 9(S)- benzoylamino -6,10-dioxo-1,2,3,4,7,8,9,10-octahydro -6H-pyridazino[1,2-a][1,2]diazepine-1(S)-carboxamide]-5-méthyl-1-benzoyloxy-hexane-2-one

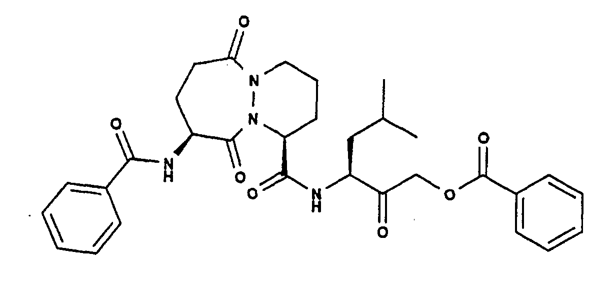

A une solution de 50mg de bromocétone (0.093mmole) obtenue précédemment dans 0.6ml de DMF est additionné 11mg de KF (0.186 mmole ; 2équi.) suivi de 13.7mg d'acide benzoique. La réaction est laissée une nuit sous agitation à TA. Le mélange réactionnel est versé dans 10ml de solution saturée de NaHCO3 et extrait par 2X5ml d'acétate d'éthyle. Les phases organiques rassemblées sont lavées à l'eau (10ml), séchées sur MgSO4 et concentrées sous vide. On obtient 42mg de résine jaune pâle (rdt=78%)
SM : MH⁺577 ; MNa⁺599
RMN-H¹ : 0.97(6H ;d ;2CH3), 1.71(1H ;m ;CH), 1.52-1.75(2H ;2m ;CH2), 4.82(1H ;m ;CHNH), 6.27(1H ;d ;CHNH), 2.40-3.19(2H ;2m ;CH2), 3.02-4.60(2H ;2m;CH2),1.50 à 2.20(2H ;m ;CH2), 5.03(2H ;AB J=17Hz ;COCH2O), 5.17(2H ;m ;2CH), 7.00(1H ;d ;CONHCH), 7.40 à 7.65(6H ;m ; 6H aromatiques), 7.81 et 8.09(4H ;dd ;2X2H aromatiques en ortho du CO)

### Exemple 2: 3-[ 9(S)- (2-furanoyl)amino -6,10-dioxo-1,2,3,4,7,8,9,10- octahydro -6H-pyridazino[1,2-a][1,2]diazepine-1(S)-carboxamide]-5-méthyl-1-(2,6-dichlorobenzoyloxy-hexane-2-one

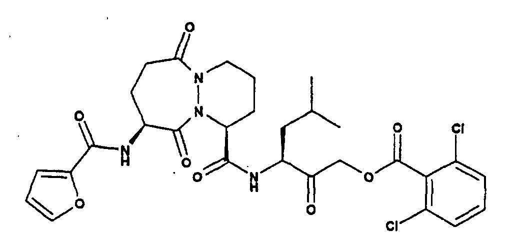

On procède à partir du produit obtenu au stade b) de l'étape 3 de l'exemple 1 comme suit :
A 11.6 mg(0.022 mmole) de 3(S)-[ 9(S)-(2-furanoyl)amino-6,10-dioxo-1,2,3,4,7,8,9,10- octahydro -6H-pyridazino[1,2-a][1,2]diazepine-1(S)-carboxamide]-5-méthyl-1-bromo-hexane-2-one en solution dans 0.35 ml de DMF est additionné 11mg de résine diméthylaminométhylpolystyrène (0.044mmole ; 2équi.) La réaction est laissée une nuit sous agitation. Le mélange est filtré sur verre fritté et la résine lavé 2 fois avec 2ml de CH2Cl2. Après évaporation intensive sous vide on isole 9 mg de produit attendu (rdt=82%)
LC-MS(pureté >80%) MH⁺ 636

### Exemple 3: 3(S)-[ 9(S)- (2-furanoyl)amino -6,10-dioxo-1,2,3,4,7,8,9,10- octahydro -6H-pyridazino[1,2-a][1,2]diazepine-1(S)-carboxamide]-4-(4-hydroxyphényl)-1-(2,6-dichlorobenzoyloxy)-butane-2-one

### Stade 1 : 3(S)-[ 9(S)-(2-furanoyl)amino -6,10-dioxo-1,2,3,4,7,8,9,10- octahydro -6H-pyridazino[1,2-a][1,2]diazepine-1(S)-carboxamide]-4-(4-terbutyloxyphényl) 1-(2,6-dichlorobenzoyloxy)-butane-2-one

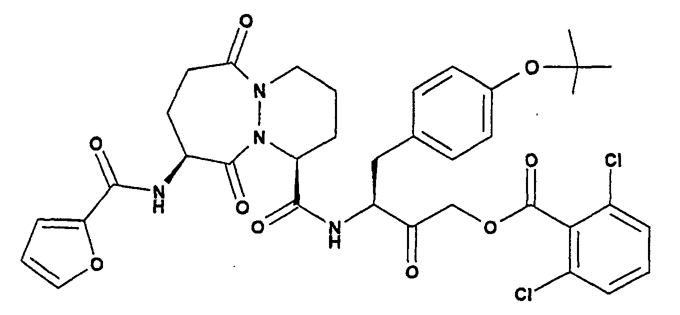

On procède comme à l'étape 1 de l'exemple 1 en utilisant à la place du composé de formule (VI): la Leu-fmoc, un autre composé de formule (VI) : la fmoc-TYR(OtBu) en même quantité.

Puis on opère dans les mêmes conditions réactionnelles que pour les étapes 2 à 4 de l'exemple 1 et obtient ainsi le produit attendu.

### Stade 2 : 3(S)-[ 9(S)- (2-furanoyl)amino -6,10-dioxo-1,2,3,4,7,8,9,10- octahydro -6H-pyridazino[1,2-a] [1,2]diazepine-1(S)-carboxamide]-4-(4-hydroxyphényl)-1-(2,6-dichlorobenzoyloxy)-butane-2-one

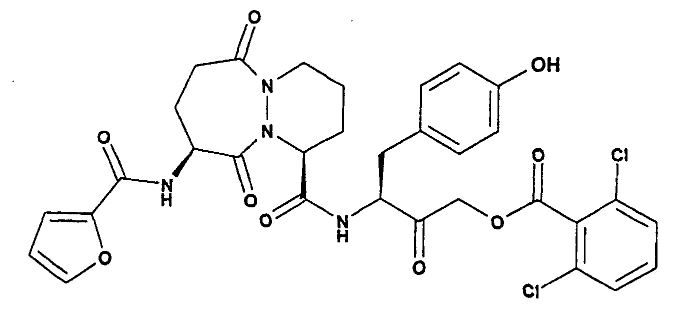

16mg (0.022 mmole) du produit obtenu ci-dessus au stade 1 en solution dans 0.25 ml de CH2Cl2 sont traités par 0.25ml d'acide trifluoroacétique La réaction est laissée une heure sous agitation à TA. Le solvant est évaporé à sec. On isole 14.4 mg de produit attendu (rdt=95%)
LC-MS (pureté >80%) MH⁺ 686

En opérant comme indiqué ci-dessus, on a préparé les produits des exemples 4 à 609 dont les structures sont indiquées dans les tableaux de la figure 4.

### Exemple 610 : composition pharmaceutique

On a préparé des comprimés répondant à la formule suivante :

| | |
|---|---|
| Produit de l'exemple 1 | O,2 g |
| Excipient pour un comprimé terminé à | lg |
| (détail de l'excipient : lactose, talc, amidon, stéarate de magnésium). | |

### Etude pharmacologique

### Etude de l'inhibition de Cathepsine K :

Les produits à tester (10 mM) sont dilués à 1 mM en DMSO et repartis dans des plaques 96 puits polystyrène Nunc à raison de 2 µl par puits. La colonne 12 de la plaque est réservée pour les contrôles et reçoit donc 1 µl de DMSO (sans produits) par puits. Les plaques sont conservées à -80°C et décongelées le jour de l'expérience.

Les produits sont dilués à 50 µM par addition de 38 µl de tampon réaction : acétate de sodium 100 mM, EDTA 5 mM, DTT 1 mM pH 5,5. L'addition ainsi que tous les pipetages suivant sont réalisés par un pipeteur 96 cônes CybiWell. Après mélange des solutions, chaque produit est transféré dans 2 puits (duplicates) d'une plaque 384 puits noire Greiner à raison de 10 µl par puits. On peut donc tester 2 plaques 96 dans une plaque 384.

Une solution de substrat à 50 µM, Z-Val-arg-AMC (Calbiochem), est préparée dans le tampon réaction . Le substrat, est ensuite distribué dans tous les puits de la plaque 384 (20 µl par puits).

Une solution de Cathepsine K à 12,5 ng/ml est préparée dans le tampon réaction et distribuée dans tous les puits de la plaque 384 (20 µl par puits) exceptés les 16 puits servant de contrôles 100 % d'inhibition (colonne 23 et 24, lignes I à P) qui recevront 20 µl de tampon sans enzyme. Les contrôles 100 % d'inhibition sont réalisés dans les colonnes 23 et 24, lignes A à H qui ne contiennent pas de produits.

Les plaques sont ensuite incubées 2H à température ambiante, puis lues sur Fluoroskan (Labsystems) : excitation 390 nm ; émission 460 nm

Les concentrations finales de chacun des réactifs sont : Produits 10 µM, Substrat 20 µM, enzyme 5 ng/ml.

Les % d'inhibition pour chacun des produits sont calculés en utilisant les points à 0 et 100 % d'inhibition de chaque plaque comme références. Les produits présentant une inhibition significative sont ensuite retestés sur une gamme de concentration allant de 50 à 0,5µM pour déterminer une IC50.

### Etude de l'inhibition de Cathepsine B:

Protocole identique à celui de Cathepsine K exceptés :
- substrat Z-arg-arg-AMC (Calbiochem), solution à 50 µM, concentration finale 20 µM
- Cathepsine B (Calbiochem), solution à 40 ng/ml, concentration finale 16 ng/ml
- Incubation 1 H à température ambiante

### Etude de l'inhibition de Papain:

Protocole identique à celui de Cathepsine K exceptés :
- Papain (Sigma), solution à 50 ng/ml, concentration finale 20 ng/ml
- Incubation 30 min à température ambiante

### Résultats

Les Ic50 trouvées pour les produits des exemples sont données dans le tableau I ci-après, en micromoles

**TABLEAU I**

| N° Exemple | IC₅₀ µM Cathepsin K | IC₅₀ µM Cathepsin B | IC₅₀ µM Papain |
|---|---|---|---|
| 38 | 5 | 20 (30%) | 20(25%) |
| 39 | 3 | 20 | 20 (30%) |
| 40 | 1.8 | 20 (40%) | 20 (30%) |
| 42 | 4 | 20(38%) | 20 (25%) |
| 43 | 1.5 | 15 | 20(25%) |
| 44 | 2 | 20 (40%) | 20 |
| 45 | 0.9 | 20(35%) | O |
| 46 | 6 | 20(30%) | 20(30%) |
| 47 | 5 | 20(40%) | 20 (40%) |
| 48 | 3 | 7 | 20 (20%) |
| 54 | 0 | 20 (30%) | 15 |
| 91 | 6 | <0.2 | 0 |
| 101 | 0 | 6 | 20(20%) |
| 107 | 20 | 0 | 0 |
| 117 | 7 | 20 (25%) | 0 |
| 122 | 18 | 0.7 | 20(45%) |
| 128 | 20(30%) | 20(40%) | 6 |
| 129 | 0 | 20(30%) | 4 |
| 130 | 20 (35%) | 20 (30%) | 6 |
| 131 | 7 | 0 | 9 |
| 132 | 20(40%) | 2 | 6 |
| 133 | 20(25%) | O | 20 |
| 134 | 20 (25%) | 20(30%) | 7 |
| 135 | 20(35%) | 0 | 18%) |
| 136 | 20 | 20(25%) | 20(20%) |
| 138 | 29(20%) | 0 | 8 |
| 141 | 9 | 0.7 | 20 |
| 150 | 6 | 0.6 | 0 |
| 161 | 0 | 9 | 19 |
| 162 | 20(30%) | 20(20%) | 20 |
| 165 | 20(40%) | 20(30%) | 20 |
| 166 | 20(20%) | 20 (20%) | 2 |
| 171 | 8 | 20(30%) | 20 (20%) |
| 172 | 20 | 20(20%) | 20(10%) |
| 185 | 0 | 7 | 0 |
| 192 | 20(15%) | 18 | 0 |
| 206 | 0 | 20 | 20 |
| 211 | 20 (20%) | 20 (30%) | 6 |
| 216 | 0 | 20 (40%) | 6 |
| 217 | 20(30%) | 0 | 7 |
| 218 | 20(25%) | 20 | 20 |
| 220 | 20 (20%) | 20 (30%) | 6 |
| 221 | 20(25%) | 20(30%) | 7 |
| 224 | 20(20%) | 9 | 8 |
| 229 | 20(40%) | 18 | 20(15%) |
| 234 | 20(30%) | 18 | 20(15%) |
| 240 | 0 | 0 | 20 |
| 241 | 0 | 20 (20%) | 7 |
| 304 | 12 | 1.5 | 0 |
| 313 | 0 | 5 | 0 |
| 322 | 20(20%) | 6 | 20(20%) |
| 339 | 10 | 0 | 0 |
| 340 | 7 | 20(0%) | 0 |
| 341 | 8 | 20(25%) | 0 |
| 343 | 14 | 20(20%) | 0 |
| 344 | 5 | 0 | 20(40%) |
| 345 | 5 | 20(25%) | 0 |
| 346 | 6 | 0 | 0 |
| 347 | 12 | 20(25%) | 0 |
| 348 | 10 | 20(35%) | 20(20%) |
| 366 | 0 | 0 | 17 |
| 374 | 20(20%) | 0 | 7 |
| 382 | 20 (20%) | 0 | 9 |
| 389 | 20(20%) | 20(30%) | 6 |
| 390 | 20(20%) | 19 | 6 |
| 397 | 20 (20%) | 0 | 18 |
| 398 | 0 | 20 (20%) | 9 |
| 433 | 20 (20%) | 4 | 0 |
| 443 | 0 | 5 | 0 |
| 450 | 20(25%) | 20 (20%) | 18 |
| 453 | 20(30%) | 9 | 20 (40%) |
| 463 | 0 | 1.5 | 0 |
| 473 | 20(25%) | 1.5 | 0 |
| 481 | 0 | 6 | 0 |
| 526 | 0 | 0 | 18 |
| 534 | 20 (20%) | 0 | 7 |
| 562 | 20(20%) | 20(20%) | 19 |
| 563 | 0 | 20(20%) | 18 |

## Revendications

1. Produits de formule (I) : 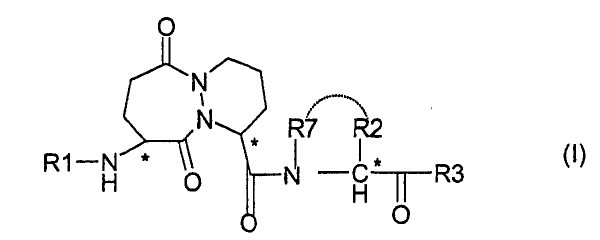 dans laquelle :
R₁ représente le radical -C(O)-R5, -SO2-R5 ou -C(O)-NR6R5 dans lesquels R6 représente un atome d'hydrogène ou un radical alkyle linéaire ou ramifié renfermant au plus 4 atomes de carbone et R5 représente un radical alkyle linéaire ou ramifié renfermant au plus 6 atomes de carbone, cycloalkyle renfermant au plus 6 atomes de carbone, phényle, naphtyle ou un radical hétérocyclique monocyclique à 5 ou 6 chaînons saturé ou insaturé contenant un ou plusieurs hétéroatomes identiques ou différents choisis parmi O, N ou S,
les radicaux alkyle, phényle et hétérocyclique tels que définis ci-dessus pour R1 , étant éventuellement substitués par un ou plusieurs radicaux choisis parmi les atomes d'halogène et les radicaux alkyle ou alcoxy linéaires ou ramifiés renfermant au plus 4 atomes de carbone, hydroxyle, acyle renfermant au plus 7 atomes de carbone, trifluorométhyle, cyano, thiényle, phényle, phénoxy et isoxazolyle ;
R2 et R7 sont tels que
soit R7 représente un atome d'hydrogène et
R2 est tel que le groupement 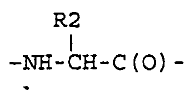 dans les produits de formule (I) représente le reste d'un acide aminé dit naturel ou dit non naturel de structure 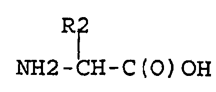 à l'exception de l'acide aspartique ,
soit R2 et R7 forment un cycle ensemble avec les atomes d'azote et de carbone auxquels ils sont liés de telle manière que le groupement ainsi formé dans les produits de formule (I) : 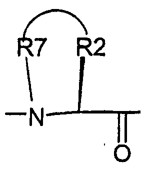 représente le reste d'un acide aminé dit naturel ou dit non naturel de structure : 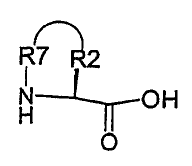
R3 représente le radical -CH=N2 ou le radical -CH2-L-R4 dans lequel L représente une simple liaison ou un radical divalent choisi parmi -O-, -O-C(O)-, -NH- et -S-(CH2)n-, avec n représente un entier de 0 à 6,
et R4 représente un radical alkyle linéaire ou ramifié renfermant au plus 6 atomes de carbone ou un radical carbocyclique ou hétérocyclique monocyclique ou bicyclique renfermant de 5 à 10 chaînons, saturé ou insaturé, contenant un ou plusieurs hétéroatomes identiques ou différents choisis parmi O, N, NH ou S et pouvant contenir un chaînon -C(O), les radicaux alkyle, carbocyclique et hétérocyclique tels que définis ci-dessus pour R4, étant éventuellement substitués par un ou plusieurs radicaux choisis parmi les atomes d'halogène ; les radicaux hydroxyle; carboxy libre, salifié ou estérifié ; -C(O)-NH2 , -C(O)-NH(alkyl), - C(O)-N(alkyl)(alkyl), -NH-C(O)-(alkyl), -N(alkyl)-C(O)-(alkyl) ; thiényle; phényle ; alkylphényle ; alkyle et alcoxy eux-mêmes éventuellement substitués par un ou plusieurs radicaux choisis parmi les radicaux acyl renfermant au plus 7 atomes de carbone, cyano, -NH2, -NH(alkyl), -N(alkyl)(alkyl) et phényle, étant entendu que dans les radicaux ci-dessus, les radicaux alkyle et alcoxy sont linéaires ou ramifiés et renferment au plus 4 atomes de carbone,
le signe * indiquant les atomes de carbone qui peuvent être sous configuration S ou R,
lesdits produits de formule (I) étant sous toutes les formes isomères possibles racémiques, énantiomères et diastéréo-isomères, ainsi que les sels d'addition avec les acides miné- raux et organiques ou avec les bases minérales et organiques desdits produits de formule (I).

2. Produits de formule (1) telle que définie à la revendication 1 dans laquelle R1 et R3 ont les significations indiquées à la revendication 1 et R2 représente le reste d'un acide aminé dit naturel à l'exception de l'acide aspartique, lesdits produits de formule (I) étant sous toutes les formes isomères possibles racémiques, énantiomères et diastéréoisomères, ainsi que les sels d'addition avec les acides minéraux et organiques ou avec les bases minérales et organiques desdits produits de formule (I).

3. Produits de formule (I) telle que définie à l'une quelconque des revendications 1 et 2 dans laquelle R1 et R3 ont les significations indiquées à la revendication 1 ou 2 et R2 et R7 sont tels que :
soit R7 représente un atome d'hydrogène et R2 représente un atome d'hydrogène ou un radical alkyle linéaire ou ramifié renfermant au plus 6 atomes de carbone éventuellement substitué par un ou plusieurs radicaux choisis parmi les radicaux amino ; acylamino; NH=C(NH2)-NH- ; mercapto ; alkylthio linéaire ou ramifié renfermant au plus 4 atomes de carbone ; hydroxyle ; alcoxy linéaire ou ramifié renfermant au plus 4 atomes de carbone ; imidazolyle ; indolyle ; phényle lui-même éventuellement substitué par un ou plusieurs radicaux choisis parmi les atomes d'halogène, le radical hydroxyle, un radical alcoxy linéaire ou ramifié renfermant au plus 4 atomes de carbone et le radical phénoxy lui-même éventuellement substitué par un ou plusieurs radicaux choisis parmi les atomes d'iode et le radical hydroxyle ; et le radical -C(O)-O-R8 dans lequel R8 représente un atome d'hydrogène ou un radical alkyle ou alkényle linéaire ou ramifié renfermant au plus 6 atomes de carbone,
soit R2 et R7 forment ensemble avec les atomes d'azote et de carbone auxquels ils sont liés un cycle comprenant 5 à 7 chaînons éventuellement substitué par un ou plusieurs radicaux hydroxyle ou alcoxy linéaire ou ramifié renfermant au plus 4 atomes de carbone,
lesdits produits de formule (I) étant sous toutes les formes isomères possibles racémiques, énantiomères et diastéréo-isomères, ainsi que les sels d'addition avec les acides miné- raux et organiques ou avec les bases minérales et organiques desdits produits de formule (I).

4. Produits de formule (I) telle que définie à l'une quelconque des revendications 1 à 3 dans laquelle R1 et R3 ont les significations indiquées à l'une quelconque des revendications 1 à 3,
R2 et R7 sont tels que :
soit R7 représente un atome d'hydrogène et R2 a la signification indiquée à la revendication 3
soit R2 et R7 forment ensemble avec les atomes d'azote et de carbone un cycle pyrrolidinyle éventuellement substitué par un ou plusieurs radicaux hydroxyle ou alcoxy linéaire ou ramifié renfermant au plus 4 atomes de carbone,
lesdits produits de formule (I) étant sous toutes les formes isomères possibles racémiques, énantiomères et diastéréo-isomères, ainsi que les sels d'addition avec les acides miné- raux et organiques ou avec les bases minérales et organiques desdits produits de formule (I).

5. Produits de formule (I) telle que définie à l'une quelconque des revendications 1 à 3 répondant à la formule (Ia) : 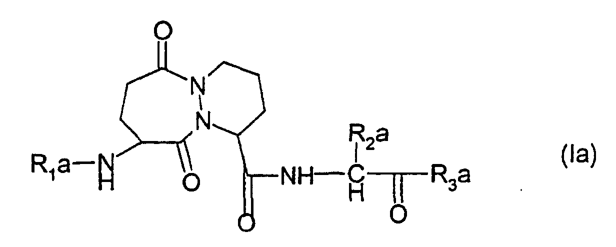 dans laquelle
R₁ₐ représente le radical -C(O)-R5a, -SO2-R5a ou -C(O)-NR6aR5a dans lesquels R6a représente un atome d'hydrogène ou un radical alkyle linéaire ou ramifié renfermant au plus 4 atomes de carbone et R5a représente un radical choisi parmi les radicaux alkyle linéaire ou ramifié renfermant au plus 6 atomes de carbone ; cycloalkyle renfermant au plus 6 atomes de carbone ; phényle ; naphtyle ; furyle ; morpholinyle ; thiényle ; pyridyle,
les radicaux alkyle, phényle, thiényle et pyridyle étant éventuellement substitués par un ou plusieurs radicaux choisis parmi les atomes d'halogène et les radicaux alkyle ou alcoxy linéaires ou ramifiés renfermant au plus 4 atomes de carbone, hydroxyle, acyle renfermant au plus 7 atomes de carbone, phénoxy, trifluorométhyle, cyano, thiényle, phényle et isoxazolyle ;
R2a a la signification indiquée à l'une quelconque des revendications 1 à 4 lorsque R7 représente un atome d'hydrogène,
R3a représente le radical -CH=N2 ou le radical -CH2-La-R4a dans lequel La représente une simple liaison ou un radical divalent choisi parmi -O-, -O-C(O)- et -S-(CH2)na-, avec na représente un entier de 0 à 3,
et R4a représente un radical choisi parmi les radicaux; alkyle linéaire ou ramifié renfermant au plus 6 atomes de carbone ; phényle ; tétrazolyle ; pipérazinyle ; pipéridyle ; pyridyle ; benzothiazolyle ; quinolyle ; thiadiazolyle ; pyrimidinyle ; tétralone ;
les radicaux alkyle, phényle, tétrazolyle, pipérazinyle et pipéridyle tels que définis ci-dessus pour R4a, étant éventuellement substitués par un ou plusieurs radicaux choisis parmi les atomes d'halogène ; les radicaux hydroxyle; carboxy libre, salifié ou estérifié ; -C(O)-NH2 ; -C(O)-NH(alkyl) ; -C(O)-N(alkyl)(alkyl) ; -NH-C(O)-(alkyl) ; -N(alkyl)-C(O)-(alkyl) ; thiényle; phényle ; alkylphényle ; alkyle et alcoxy eux-mêmes éventuellement substitués par un ou plusieurs radicaux choisis parmi les radicaux acyl renfermant au plus 7 atomes de carbone, cyano, -NH2, -NH(alkyl), -N(alkyl)(alkyl) et phényle,
étant entendu que dans les radicaux ci-dessus, les radicaux alkyle et alcoxy sont linéaires ou ramifiés et renferment au plus 4 atomes de carbone,
lesdits produits de formule (Ia) étant sous toutes les formes isomères possibles racémiques, énantiomères et diastéréoisomères, ainsi que les sels d'addition avec les acides minéraux et organiques ou avec les bases minérales et organiques desdits produits de formule (Ia).

6. Produits de formule (I) telle que définie à l'une quelconque des revendications 1 à 5 répondant à la formule (Ib) : 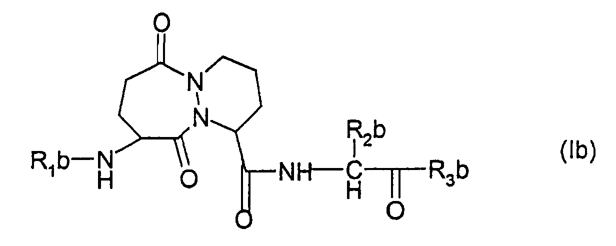 dans laquelle :
R_{1b} représente le radical -C(O)-R5b, -SO2-R5b ou -C(O)-NR6bR5b
dans lesquels R6b représente un atome d'hydrogène ou un radical méthyle et R5b représente un radical choisi parmi les radicaux alkyle linéaire ou ramifié renfermant au plus 4 atomes de carbone éventuellement substitué par un radical thiényle ; cyclohexyle ; phényle éventuellement substitué par un radical alcoxy linéaire ou ramifié renfermant au plus 4 atomes de carbone, un radical phénoxy ou trifluorométhyle ; naphtyle ; furyle ; morpholinyle ; thiényle éventuellement substitué par un radical isoxazolyle ; pyridyle éventuellement substitué par un radical trifluorométhyle R2b représente un atome d'hydrogène ou un radical alkyle linéaire ou ramifié renfermant au plus 6 atomes de carbone éventuellement substitué soit par le radical phényle lui-même éventuellement substitué par les radicaux hydroxyle ou alcoxy linéaire ou ramifié renfermant au plus 4 atomes de carbone, soit par le radical -C(O)-O-R8b dans lequel R8b représente un radical alkyle ou alkényle linéaire ou ramifié renfermant au plus 6 atomes de carbone,
R3b représente le radical -CH=N2 ou le radical -CH2-Lb-R4b dans lequel Lb représente une simple liaison ou un radical divalent choisi parmi -O-, -O-C(O)- et -S-(CH2)nb-, avec nb représente l'entier 0 ou 1,
et R4b représente un radical choisi parmi les radicaux pyridyle ; benzothiazolyle ; quinolyle ; thiadiazolyle ; pyrimidinyle ; tétralone ; alkyle linéaire ou ramifié renfermant au plus 4 atomes de carbone éventuellement substitué par un radical carboxy libre, salifié ou estérifié ou thiényle ; phényle éventuellement substitué par un ou plusieurs radicaux choisis parmi les atomes d'halogène, les radicaux carboxy libre, salifié ou estérifié, les radicaux alkyle et alcoxy linéaires ou ramifiés renfermant au plus 4 atomes de carbone, cyanoalkyle, alkylphényle, -NH-C(O)-CH3, -C(O)-N(alkyl)(alkyl) ; tétrazolyle éventuellement substitué par un radical phényle ; pipérazinyle éventuellement substitué sur le second atome d'azote par un radical phényle ou alkyle linéaire ou ramifié renfermant au plus 4 atomes de carbone lui-même éventuellement substitué par un radical acyl (pyrrolidinylcarbonyl), un ou deux radicaux phényle ou un radical -NH2, -NH(alkyl) ou -N(alkyl)(alkyl) ; pipéridyle éventuellement substitué par un radical benzyle où -C(O)- N(alkyl)(alkyl) , étant entendu que dans les radicaux ci-dessus, les radicaux alkyle et alcoxy sont linéaires ou ramifiés et renferment au plus 4 atomes de carbone,
lesdits produits de formule (Ib) étant sous toutes les formes isomères possibles racémiques, énantiomères et diastéréo-isomères, ainsi que les sels d'addition avec les acides miné- raux et organiques ou avec les bases minérales et organiques desdits produits de formule (Ib).

7. Produits de formule (I) telle que définie à l'une quelconque des revendications 1 à 6 dans laquelle R1 représente un radical choisi parmi les radicaux suivants : 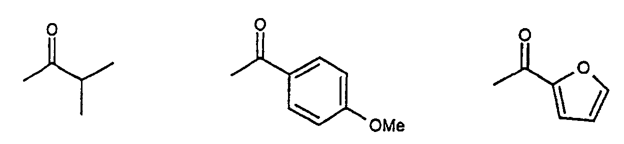 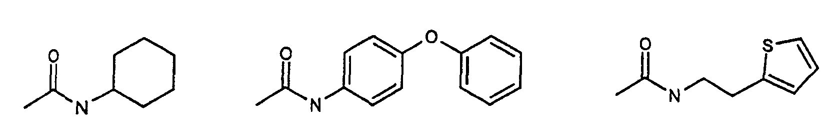 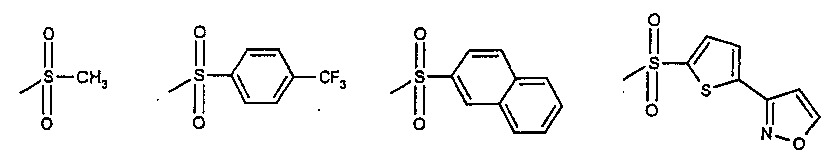 et R2, R3 et R7 ont les valeurs indiquées à la revendication 1.

8. Produits de formule (I) telle que définie à l'une quelconque des revendications 1 à 7 dans laquelle R7 représente un atome d'hydrogène et R2 représente un radical choisi parmi les radicaux suivants : 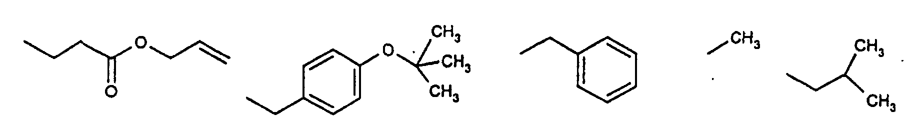 et R1 et R3 ont les valeurs indiquées à la revendication 1.

9. Produits de formule (I) telle que définie à l'une quelconque des revencideations 1 à 8 dans laquelle R3 représente un radical choisi parmi les radicaux suivants : 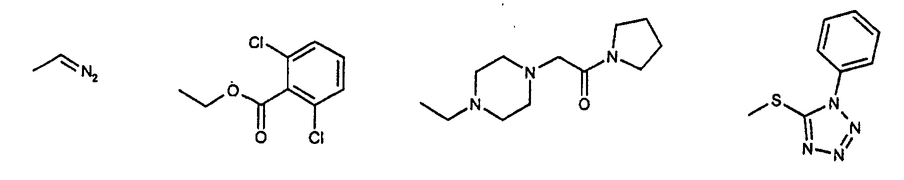 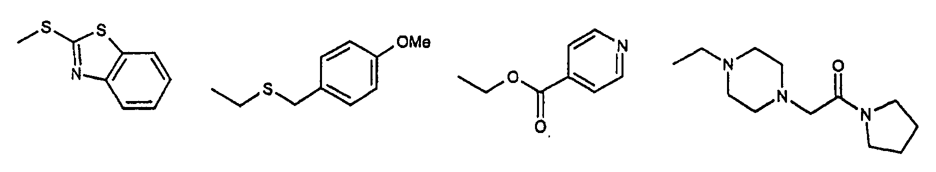 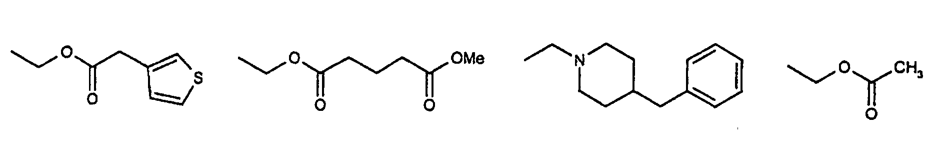 et R1, R2 et R7 ont les valeurs indiquées à la revendication 1.

10. Produits de formule (I) dont les noms suivent :
- le 3-[ 9(S)- benzoylamino -6,10-dioxo-1,2,3,4,7,8,9,10-octahydro -6H-pyridazino[1,2-a][1,2]diazepine-1(S)-carboxamide]-5-méthyl-1-benzoyloxy-hexane-2-one
- le 3-[ 9(S)- (2-furanoyl)amino -6,10-dioxo-1,2,3,4,7,8,9,10- octahydro -6H-pyridazino[1,2-a][1,2]diazepine-1(S)-carboxamide]-5-méthyl-1-(2,6-dichlorobenzoyloxy-hexane-2-one
- le 3(S)-[9(S)-(2-furanoyl) amino -6,10-dioxo-1,2,3,4,7,8,9,10- octahydro -6H-pyridazino[1,2-a][1,2]diazepine-1(S)-carboxamide]-4-(4-hydroxyphényl)-1-(2,6-dichlorobenzoyloxy)-butane-2-one

11. Produits de formule (I) dont les noms suivent :
- (4S) 6-diazo-4-[[[(1S,9S)-9-[(2-methyl-1-oxo-propyl)amino]-octahydro-6,10-dioxo-6H-pyridazino[1,2-a][1,2]diazepine-1-yl]carbonyl]amino]-5-oxo-Hexanoate de (2-propenyle)
- (4S) 6-diazo-4-[[[(1S,9S)-9-[(3-methoxybenzoyl)amino]-octahydro-6,10-dioxo-6H-pyridazino[1,2-a][1,2]diazepine-1-yl]carbonyl]amino]-5-oxo-Hexanoate de (2-propenyle)
- (4S) 6-diazo-4-[[[(1S,9S)-9-[[(2-furanyl)carbonyl] amino]-octahydro-6,10-dioxo-6H-pyridazino[1,2-a] [1,2]diazepine-1-yl]carbonyl]amino]-5-oxo-Hexanoate de(2-propenyle)
- (4S) 6-diazo-4-[[[(1S,9S)-9-[[(cyclohexylamino) carbonyl]amino]-octahydro-6,10-dioxo-6H-pyridazino[1,2-a] [1,2]diazepine-1-yl]carbonyl]amino]-5-oxo-Hexanoate de (2-propenyle)
- (4S) 6-diazo-4-[[[(1S,9S)-9-[[[(4-phenoxyphenyl)amino] carbonyl]amino]-octahydro-6,10-dioxo-6H-pyridazino[1,2-a] [1,2] diazepine-1-yl]carbonyl]amino]-5-oxo-Hexanoate de (2-propenyle)
- (4S) 6-diazo-4-[[[(1S,9S)-9-[[[[2-(p-2-yl)-ethyl]amino]carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-diazo-4-[[[(1S,9S)-9-[(methylsulfonyl)amino]-octahydro-6,10-dioxo-6H-pyridazino[1,2-a][1,2]diazepine-1-yl]carbonyl]amino]-5-oxo-Hexanoate de (2-propenyle)
- (4S) 6-diazo-4-[[[(1S,9S)-9-[[[4-(trifluoromethyl) phenyl]sulfonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-diazo-4-[[[(1S,9S)-9-[[(2-naphthalenyl) sulfonyl]amino]-octahydro-6,10-dioxo-6H-pyridazino[1,2-a] [1,2]diazepine-1-yl]carbonyl]amino]-5-oxo-Hexanoate de (2-propenyle)
- (4S) 6-diazo-4-[[[(1S,9S)-9-[[[5-(isoxazol-3-yl)-2-thienyl]sulfonyl]amino]-octahydro-6,10-dioxo-6H-pyridazino[1,2-a][1,2]diazepine-1-yl]carbonyl]amino]-5-oxo-Hexanoate de (2-propenyle)
- 9-[(9S)-[[(4-phenoxyphenyl)amino]carbonyl]amino]-N-[(2S)-4-diazo-1-[4-[(1,1-dimethyl)ethoxy]phenyl]-3-oxo-2-butyl]-6H-(1S)-pyridazino[1,2-a][1,2]diazepine-1-carboxamide
- (4S) 6-[(2,6-dichlorobenzoyl)oxy] -4-[[[(1S,9S)-9-[[(2-furanyl)carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[(2,6-dichlorobenzoyl)oxy] -4-[[[(1S,9S)-9-[[[4-(trifluoromethyl)phenyl]sulfonyl]amino]-octahydro-6,10-dioxo-6H-pyridazino[1,2-a] [1,2]diazepine-1-yl]carbonyl]amino]-5-oxo-Hexanoate de (2-propenyle)
- (4S) 6-[4-[[2-oxo-2-(1-pyrrolidinyl)ethyl]piperazin-1-yl]-4-[[[(1S,9S)-9-[[[4-(trifluoromethyl)phenyl] sulfonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[4-[[2-oxo-2-(1-pyrrolidinyl)ethyl]piperazin-1-yl]-4-[[[(1S,9S)-9-[[(2-naphthalenyl)sulfonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[(2,6-dichlorobenzoyl)oxy] -4-[[[(1S,9S)-9-[[(cyclohexylamino)carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[(1-phenyl-1H-tetrazol-5-yl)thio]-4-[[[(1S,9S)-9-[[[(4-phenoxyphenyl)amino]carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[(benzothiazol-2-yl)thio]-4-[[[(1S,9S)-9-[[[(4-phenoxyphenyl)amino]carbonyl]amino]-octahydro-6,10-dioxo-6H-pyridazino[1,2-a] [1,2]diazepine-1-yl]carbonyl]amino]-5-oxo-Hexanoate de (2-propenyle)
- (4S) 6-[[(4-methoxyphenyl)methyl]thio]-4-[[[(1S,9S)-9-[[[(4-phenoxyphenyl)amino]carbonyl]amino]-octahydro-6,10-dioxo-6H-pyridazino[1,2-a][1,2]diazepine-1-yl] carbonyl]amino]-5-oxo-Hexanoate de (2-propenyle)
- (4S) 6-[[(4-pyridinyl)carbonyl]oxy]-4-[[[(1S,9S)-9-[[[(4-phenoxyphenyl)amino]carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[(2,6-dichlorobenzoyl)oxy]-4-[[[(1S,9S)-9-[[[(4-phenoxyphenyl)amino]carbonyl]amino]-octahydro-6,10-dioxo-6H-pyridazino[1,2-a][1,2]diazepine-1-yl] carbonyl]amino]-5-oxo-Hexanoate de (2-propenyle)
- ((4S) 6-acetyloxy-4-[[[(1S,9S)-9-[[[(4-phenoxyphenyl)amino]carbonyl]amino]-octahydro-6,10-dioxo-6H-pyridazino[1,2-a][1,2]diazepine-1-yl]carbonyl]amino]-5-oxo-Hexanoate de (2-propenyle)
- (4S) 6-[1-oxo-2-(thien-3-yl)ethoxy]-4-[[[(1S,9S)-9-[[[(4-phenoxyphenyl)amino]carbonyl]amino]-octahydro-6,10-dioxo-6H-pyridazino[1,2-a] [1,2]diazepine-1-yl]carbonyl]amino]-5-oxo-Hexanoate de (2-propenyle)
- (4S) 6-[(1-phenyl-1H-tetrazol-5-yl)thio]-4-[[[(1S,9S)-9-[[[[2-(thien-2-yl)-ethyl]amino]carbonyl]amino]-octahydro-6, 10-dioxo-6H-pyridazino [1,2-a] [1,2]diazepine-1-yl]carbonyl]amino]-5-oxo-Hexanoate de (2-propenyle)
- (4S) 6-[(2,6-dichlorobenzoyl)oxy]-4-[[[(1S,9S)-9-[[[[2-(thien-2-yl)-ethyl]amino]carbonyl]amino]-octahydro-6,10-dioxo-6H-pyridazino[1,2-a] [1,2]diazepine-1-yl]carbonyl]amino]-5-oxo-Hexanoate de (2-propenyle)
- (4S) 6-[(2,6-dichlorobenzoyl)oxy]-4-[[[(1S,9S)-9-[(methylsulfonyl)amino]-octahydro-6,10-dioxo-6H-pyridazino[1,2-a][1,2]diazepine-1-yl]carbonyl]amino]-5-oxo-Hexanoate de (2-propenyle)
- 9-[(9S) [[(4-phenoxyphenyl)amino]carbonyl]amino]-N-((2S)-4-diazo-1-phenyl-3-oxo-2-butyl)- octahydro-6,10-dioxo -6H-(1S)-pyridazino[1,2-a][1,2]diazepine-1-carboxamide
- 9-[(9S)-[[[2-(thien-2-yl)-ethyl]amino]carbonyl]amino]-N-((2S)-4-diazo-1-phenyl-3-oxo-2-butyl)- octahydro-6,10-dioxo-6H-(1S)-pyridazino[1,2-a][1,2]diazepine-1-carboxamide
- 9-[(9S)-[(2-naphthalenyl)sulfonyl]amino ]-N-((2S)-4-diazo-1-phenyl-3-oxo-2-butyl)- octahydro-6,10-dioxo-6H-(1S)-pyridazino[1,2-a][1,2]diazepine-1-carboxamide
- 9-[(9S)-[[5-(isoxazol-3-yl)-2-thienyl]sulfonyl]amino]-N-((2S)-4-diazo-1-phenyl-3-oxo-2-butyl)- octahydro-6,10-dioxo-6H-(1S)-pyridazino[1,2-a][1,2]diazepine-1-carboxamide
- 9-[(9S)-[[(4-phenoxyphenyl)amino]carbonyl]amino]-N-((2S)-4-diazo-3-oxo-2-butyl)- octahydro-6,10-dioxo -6H-(1S)-pyridazino[1,2-a][1,2]diazepine-1-carboxamide
- 9-[(9S)-[[[3-(thien-2-yl)-ethyl]amino]carbonyl]amino]-N-((2S)-4-diazo-3-oxo-2-butyl)- octahydro-6,10-dioxo -6H-(1S)-pyridazino[1,2-a][1,2]diazepine-1-carboxamide
- 9-[(9S)-(3-methoxybenzoyl)amino]-N-((2S)-4-[(2,6-dichlorobenzoyl)oxy] -1-phenyl-3-oxo-2-butyl]- octahydro-6,10-dioxo-6H-(1S)-pyridazino[1,2-a][1,2] diazepine-1-carboxamide
- 9-[(9S)-[(2-furanyl)carbonyl]aminol-N-[(2S)-4-[(2,6-dichlorobenzoyl)oxy] -1-phenyl-3-oxo-2-butyl]- octahydro-6,10-dioxo-6H-(1S)-pyridazino[1,2-a][1,2] diazepine-1-carboxamide
- 9-[(9S)-[(cyclohexylamino)carbonyl]amino]-N-[(2S)-4-[(2,6-dichlorobenzoyl)oxy] -1-phenyl-3-oxo-2-butyl]- octahydro-6,10-dioxo-6H-(1S)-pyridazino[1,2-a][1,2] diazepine-1-carboxamide
- 9-[(9S)-[[(4-phenoxyphenyl)amino]carbonyl]amino]-N-[(2S)-1-phenyl-4-[(1-phenyl-1H-tetrazol-5-yl)thio]-3-oxo-2-butyl]-octahydro-6,10-dioxo-6H-(1S)-pyridazino[1,2-a][1,2]diazepine-1-carboxamide
- 9-[(9S)-[[(4-phenoxyphenyl)amino]carbonyl]amino]-N-[(2S)-1-phenyl-4-[1-oxo-2-(3-thienyl)ethoxy]-3-oxo-2-butyl]-octahydro-6,10-dioxo-6H-(1S)-pyridazino[1,2-a][1,2]diazepine-1-carboxamide
- Pentanedioate de (methyle) [2-oxo-4-phenyl-3[(3S)-[[(4-phenoxyphenyl)amino]carbonyl]amino]- octahydro-6,10-dioxo-6H-(1S)-pyridazino[1,2-a][1,2]diazepin-1-yl]carbonyl]amino]butyle]
- 9-[(9S)-[[(4-phenoxyphenyl)amino]carbonyl]amino]-N-[(2S)-4-[(4-phenylmethyl)-1-piperidinyl ]-1-phenyl-3-oxo-2-butyl]-octahydro-6,10-dioxo-6H-(1S)-pyridazino[1,2-a][1,2]diazepine-1-carboxamide
- 9-[(9S)-[[[2-(thien-2-yl)-ethyl]amino]carbonyl]amino]-N-[(2S)-1-phenyl-4-[(1-phenyl-1H-tetrazol-5-yl)thio]-3-oxo-2-butyl]- octahydro-6,10-dioxo-6H-(1S)-pyridazino [1,2-a][1,2]diazepine-1-carboxamide
- 9-[(9S)-[[[2-(thien-2-yl)-ethyl]amino]carbonyl]amino]-N-[(2S)-1-phenyl-4-[(2-benzothiazolyl)thio]-3-oxo-2-butyl]-octahydro-6,10-dioxo-6H-(1S)-pyridazino[1,2-a][1,2]diazepine-1-carboxamide
- 9-[(9S)-[[[2-(thien-2-yl)-ethyl]amino]carbonyl]amino]-N-[(2S)-1-phenyl-4-[(-2,6-dichlorobenzoyl)oxy] -3-oxo-2-butyl]-octahydro-6,10-dioxo-6H-(1S)-pyridazino[1,2-a][1,2]diazepine-1-carboxamide
- 9-[(9S)-[[[2-(thien-2-yl)-ethyl]amino]carbonyl]amino]-N-[(2S)-1-phenyl-4-[4-[2-(1-pyrrolidinyl)-2-oxo-ethyl]piperazin-1-yl]] -3-oxo-2-butyl]- octahydro-6,10-dioxo-6H-(1S)-pyridazino[1,2-a][1,2]diazepine-1-carboxamide
- 9-[(9S)-(methylsulfonyl)amino]-N-[(2S)-1-phenyl-4-[(-2,6-dichlorobenzoyl)oxy] -3-oxo-2-butyl]- octahydro-6,10-dioxo-6H-(1S)-pyridazino[1,2-a][1,2]diazepine-1-carboxamide
- 9-[(9S)-[[[4-(trifluoromethyl)phenyl]sulfonyl]amino]-N-[(2S)-1-phenyl-4-[(1-phenyl-1H-tetrazol-5-yl)thio]-3-oxo-2-butyl]- octahydro-6,10-dioxo-6H-(1S)-pyridazino [1,2-a][1,2]diazepine-1-carboxamide
- 9-[(9S)-[[[4-(trifluoromethyl)phenyl]sulfonyl]amino]-N-[(2S)-1-phenyl-4-(2-benzothiazolyl)thio]-3-oxo-2-butyl]-octahydro-6,10-dioxo-6H-(1S)-pyridazino[1,2-a][1,2]diazepine-1-carboxamide
- 9-[(9S)-(methylsulfonyl)amino]-N-[(3S)-5-methyl-1-[(2,6-dichlorobenzoyl)oxy] -2-oxo-3-hexyl]- octahydro-6,10-dioxo-6H-(1S)-pyridazino[1,2-a][1,2]diazepine-1-carboxamide
- 9-[(9S)-[[[4-(trifluoromethyl)phenyl]sulfonyl]amino]]-N-[(3S)-5-methyl-1-[(2,6-dichlorobenzoyl)oxy] -2-oxo-3-hexyl]-octahydro-6,10-dioxo-6H-(1S)-pyridazino[1,2-a][1,2]diazepine-1-carboxamide
- 9-[(9S)-[(2-naphthalenyl)sulfonyl]amino ]-N-[(3S)-5-methyl-1-[(2,6-dichlorobenzoyl)oxy] -2-oxo-3-hexyl]- octahydro-6,10-dioxo-6H-(1S)-pyridazino[1,2-a][1,2] diazepine-1-carboxamide
- 9-[(9S)-(2-methyl-1-oxo-propyl)amino]-N-((3S)-1-diazo-5-methyl-2-oxo-3-hexyl)- octahydro-6,10-dioxo -6H-(1S)-pyridazino[1,2-a][1,2]diazepine-1-carboxamide
- 9-[(9S)- (3-methoxybenzoyl)amino]-N-((3S)-1-diazo-5-methyl-2-oxo-3-hexyl)- octahydro-6,10-dioxo -6H-(1S)-pyridazino[1,2-a] [1,2]diazepine-1-carboxamide
- 9-[(9S)-[(2-furanyl)carbonyl]amino]-N-((3S)-1-diazo-5-methyl-2-oxo-3-hexyl)- octahydro-6,10-dioxo -6H-(1S)-pyridazino[1,2-a][1,2]diazepine-1-carboxamide
- 9-[(9S)-[(cyclohexylamino)carbonyl]amino]-N-((3S)-1-diazo-5-methyl-2-oxo-3-hexyl)- octahydro-6,10-dioxo -6H-(1S)-pyridazino[1,2-a][1,2]diazepine-1-carboxamide
- 9-[(9S)-[[(4-phenoxyphenyl)amino]carbonyl]amino]-N-((3S)-1-diazo-5-methyl-2-oxo-3-hexyl)- octahydro-6,10-dioxo -6H-(1S)-pyridazino[1,2-a][1,2]diazepine-1-carboxamide
- 9-[(9S)-[[[2-(thien-2-yl)-ethyl]amino]carbonyl]amino]-N-((3S)-1-diazo-5-methyl-2-oxo-3-hexyl)- octahydro-6,10-dioxo-6H-(1S)-pyridazino[1,2-a][1,2]diazepine-1-carboxamide
- 9-[(9S)-(methylsulfonyl)amino]-N-((3S)-1-diazo-5-methyl-2-oxo-3-hexyl)- octahydro-6,10-dioxo -6H-(1S)-pyridazino[1,2-a][1,2]diazepine-1-carboxamide
- 9-[(9S)-[[4-(trifluoromethyl)phenyl]sulfonyl]amino]-N-((3S)-1-diazo-5-methyl-2-oxo-3-hexyl)- octahydro-6,10-dioxo-6H-(1S)-pyridazino[1,2-a][1,2]diazepine-1-carboxamide
- 9-[(9S)-[(2-naphthalenyl)sulfonyl]amino]-N-((3S)-1-diazo-5-methyl-2-oxo-3-hexyl)- octahydro-6,10-dioxo -6H-(1S)-pyridazino[1,2-a][1,2]diazepine-1-carboxamide
- 9-[(9S)- (3-methoxybenzoyl)amino]-N-[(3S)-1-[(benzothiazol-2-yl)thio]-4-methyl-2-oxo-3-pentyl]-octahydro-6,10-dioxo-6H-(1S)-pyridazino[1,2-a][1,2] diazepine-1-carboxamide
- 9- [(9S) - [(2-furanyl)carbonyl]amino]-N-[(3S)-1-[(benzothiazol-2-yl)thio]-4-methyl-2-oxo-3-pentyl]-octahydro-6,10-dioxo -6H-(1S)-pyridazino[1,2-a][1,2] diazepine-1-carboxamide
- 9-[(9S)-[(cyclohexylamino)carbonyl]amino]-N-[(3S)-1-[(benzothiazol-2-yl)thio]-4-methyl-2-oxo-3-pentyl]-octahydro-6,10-dioxo -6H-(1S)-pyridazino[1,2-a][1,2] diazepine-1-carboxamide
- 9-[(9S)-[[(4-phenoxyphenyl)amino]carbonyl]amino]-N-[(3S)-1-[(1-phenyl-1H-tetrazol-5-yl)thio]-4-methyl-2-oxo-3-pentyl]-octahydro-6,10-dioxo -6H-(1S)-pyridazino [1,2-a][1,2]diazepine-1-carboxamide
- 9-[(9S)-[[(4-phenoxyphenyl)amino]carbonyl]amino]-N-[3S)-1-[(benzothiazol-2-yl)thio]-4-methyl-2-oxo-3-pentyl]-octahydro-6,10-dioxo -6H-(1S)-pyridazino[1,2-a][1,2]diazepine-1-carboxamide
- 9-[(9S)-[[[2-(thien-2-yl)-ethyl]amino]carbonyl]amino]-N-[(3S)-1-[(1-phenyl-1H-tetrazol-5-yl)thio]-4-methyl-2-oxo-3-pentyl]- octahydro-6,10-dioxo -6H-(1S)-pyridazino [1,2-a][1,2]diazepine-1-carboxamide
- 9- [(9S)-[[[2- (thien-2-yl) -ethyl]amino]carbonyl]amino]-N-[(3S)- 1-[(benzothiazol-2-yl)thiol-4-methyl-2-oxo-3-pentyl]-octahydro-6,10-dioxo -6H-(1S)-pyridazino[1,2-a] [1,2]diazepine-1-carboxamide
- 9-[(9S)-[(cyclohexylamino)carbonyl]amino]-N-[(2S)-4-[(2,6-dichlorobenzoyl)oxy]-3-oxo-2-butyl]- octahydro-6,10-dioxo-6H-(1S)-pyridazino[1,2-a][1,2]diazepine-1-carboxamide
- 9-[(9S)-[(2-furanyl)carbonyl]amino]-N-[(2S)-4-[(2,6-dichlorobenzoyl)oxy]-3-oxo-2-butyl]- octahydro-6,10-dioxo-6H-(1S)-pyridazino[1,2-a][1,2]diazepine-1-carboxamide
- 9-[(9S)-[[(4-phenoxyphenyl)amino]carbonyl]amino]-N-[(2S)-4-[(benzothiazol-2-yl)thio]- 3-oxo-2-butyl]- octahydro-6,10-dioxo -6H-(1S)-pyridazino[1,2-a][1,2] diazepine-1-carboxamide
- 9-[(9S)-[[(4-phenoxyphenyl)amino]carbonyl]amino]-N-[(2S)-4-[(2,6-dichlorobenzoyl)oxy]-3-oxo-2-butyl]- octahydro-6,10-dioxo -6H-(1S)-pyridazino[1,2-a][1,2] diazepine-1-carboxamide
- 9-[(9S)- (3-methoxybenzoyl)amino]-N-[(2S)-4-[(2,6-dichlorobenzoyl)oxy]-3-oxo-2-butyl]- octahydro-6,10-dioxo-6H-(1S)-pyridazino[1,2-a][1,2]diazepine-1-carboxamide
- 9-[(9S)-[[[2-(thien-2-yl)-ethyl]amino]carbonyl]amino]-N-[(2S)-4-[(2,6-dichlorobenzoyl)oxy]-3-oxo-2-butyl]- octahydro-6,10-dioxo -6H-(1S)-pyridazino[1,2-a][1,2] diazepine-1-carboxamide
- 9-[(9S)-[[4-(trifluoromethyl)phenyl]sulfonyl]amino]-N-[(2S)-4-[(2,6-dichlorobenzoyl)oxy]-3-oxo-2-butyl]- octahydro-6,10-dioxo -6H-(1S)-pyridazino[1,2-a][1,2] diazepine-1-carboxamide
- 9- [(9S)-[(cyclohexylamino)carbonyl]amino]-N-[(2S)-4-[(benzothiazol-2-yl)thio]-1-[4-[(1,1-dimethyl) ethoxy]phenyl]-3-oxo-2-butyl]-6H-(1S)-pyridazino[1,2-a][1,2]diazepine-1-carboxamide

12. Procédé de préparation des produits de formule (I), telle que définie à la revendication 1, **caractérisé en ce que** l'on soumet le composé de formule (II) : 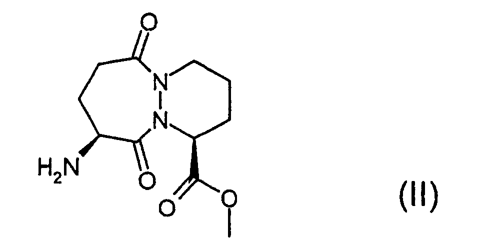 à une réaction avec un composé de formule (III) :
R1'-X (III)
dans laquelle X représente un atome d'halogène et R1' a la signification indiquée à la revendication 1 pour R1, dans laquelle les éventuelles fonctions réactives sont éventuellement protégées par des groupements protecteurs, pour obtenir le produit de formule (IV) : 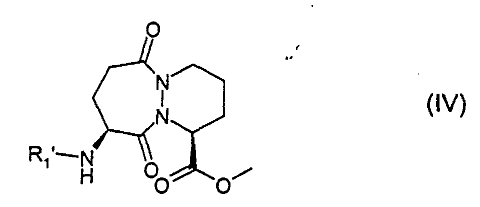 dans laquelle R1' a la signification indiquée ci-dessus, produit de formule (IV) que l'on soumet à une réaction de saponification pour obtenir le produit de formule (V) : 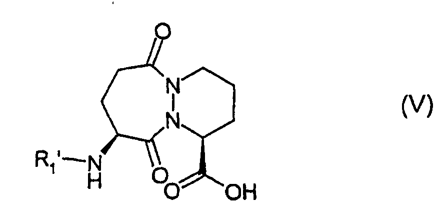 dans laquelle R1' a la signification indiquée ci-dessus, produit de formule (V) que l'on soumet à une réaction avec un produit de formule (VI) : 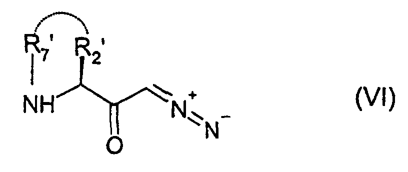 dans laquelle R2' et R7' ont les significations indiquées à la revendication 1 respectivement pour R2 et R7, dans lesquelles les éventuelles fonctions réactives sont éventuellement protégées par des groupements protecteurs, pour obtenir un produit de formule (Ix) : 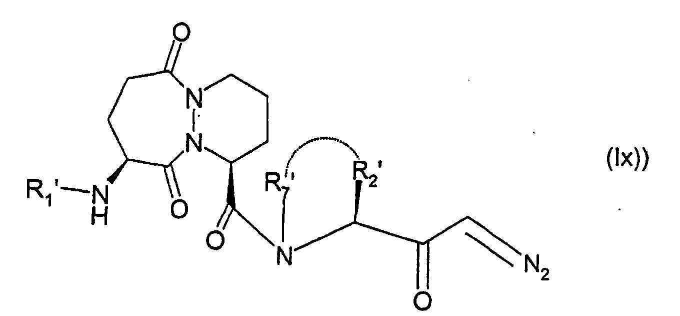 dans laquelle R1', R2' et R7' ont les significations indiquées ci-dessus,
produit de formule (Ix) que l'on peut soumettre à une réaction de bromation pour obtenir un produit de formule (VII): 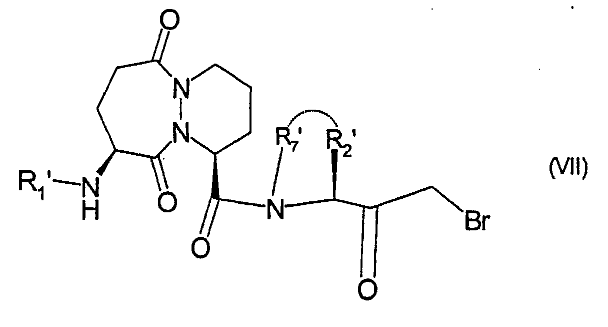 dans laquelle R1', R2' et R7' ont les significations indiquées ci-dessus,
produit de formule (VII) que l'on soumet à une réaction avec un produit de formule (VIII) :
H'-L-R4' (VIII)
dans laquelle L a la signification indiquée à la revendication 1 et R4' a la signification indiquée à la revendication 1 pour R4, dans laquelle les éventuelles fonctions réactives sont éventuellement protégées par des groupements protecteurs,
pour obtenir un produit de formule (Iy) : 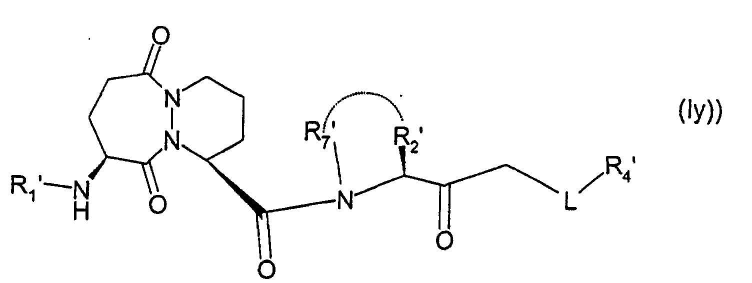 dans laquelle R1', R2', R4' et R7' ont les significations indiquées ci-dessus,
produits de formules (Ix) et (Iy) qui peuvent être des produits de formule (I) et que, pour obtenir des ou d'autres produits de formule (I), l'on peut soumettre, si désiré et si nécessaire, à l'une ou plusieurs des réactions de transformations suivantes, dans un ordre quelconque :
a) une réaction d'estérification de fonction acide,
b) une réaction de saponification de fonction ester en fonction acide,
c) une réaction d'oxydation de groupement alkylthio en sulfoxyde ou sulfone correspondant,
d) une réaction de transformation de fonction cétone en fonction oxime,
e) une réaction de réduction de la fonction carboxy libre ou estérifié en fonction alcool,
f) une réaction de transformation de fonction alcoxy en fonction hydroxyle, ou encore de fonction hydroxyle en fonction alcoxy,
g) une réaction d'oxydation de fonction alcool en fonction aldéhyde, acide ou cétone,
h) une réaction de transformation de radical nitrile en tétrazolyle,
i) une réaction d'élimination des groupements protecteurs que peuvent porter les fonctions réactives protégées,
j) une réaction de salification par un acide minéral ou organique ou par une base pour obtenir le sel correspondant,
k) une réaction de dédoublement des formes racémiques en produits dédoublés, lesdits produits de formule (I) ainsi obtenus étant sous toutes les formes isomères possibles racémiques, énantiomères et diastéréoisomères.

13. A titre de médicaments, les produits de formule (I) telle que définie aux revendications 1 à 4, ainsi que les sels d'addition avec les acides minéraux ou organiques ou avec les bases minérales ou organiques pharmaceutiquement acceptables desdits produits de formule (I).

14. A titre de médicaments, les produits de formules (Ia) et (Ib) telles que définies à la revendication 5 ou 6, ainsi que les sels d'addition avec les acides minéraux ou organiques ou avec les bases minérales ou organiques pharmaceutiquement acceptables desdits produits de formules (Ia) et (Ib).

15. A titre de médicaments les produits de formule (I) telle que définie à l'une quelconque des revendications 7 à 9 ainsi que les sels d'addition avec les acides minéraux ou organiques ou avec les bases minérales ou organiques pharmaceutiquement acceptables desdits produits de formule (I).

16. A titre de médicaments, les produits de formule (I) telle que définie à la revendication 10 ou 11, ainsi que les sels d'addition avec les acides minéraux ou organiques ou avec les bases minérales ou organiques pharmaceutiquement acceptables desdits produits de formule (I).

17. Les compositions pharmaceutiques contenant à titre de principe actif, l'un au moins des médicaments tels que définis aux revendications 13 à 16.

18. Utilisation des produits selon l'une des revendications 1 à 11 ou de sels pharmaceutiquement acceptables desdits produits pour la préparation de médicaments destinés à la prévention ou au traitement de maladies dans lesquelles des enzymes métaboliques telles que des protéases ou des kinases sont impliquées.

19. Utilisation selon la revendication 18 pour la préparation de médicaments destinés à la prévention ou au traitement de maladies dans lesquelles sont impliquées la cathepsine K, la cathepsine B ou la papaine.

20. Utilisation selon la revendication 18 **caractérisée en ce que** les maladies à prévenir ou à traiter sont choisies dans le groupe de maladies suivant : maladies cardiovasculaires, cancers, maladies du système nerveux central, maladies inflammatoires, maladies infectieuses ou encore maladies de l'os.

21. Utilisation selon la revendication 18 **caractérisée en ce que** les maladies à prévenir ou à traiter sont des maladies du système nerveux central ou des maladies de l'os telles que notamment l'ostéoporose.

## Patentansprüche

1. Produkte der Formel (I): 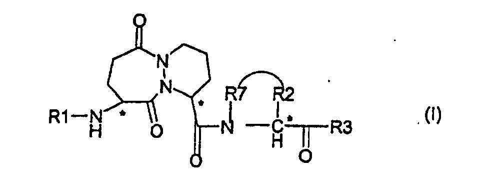 worin:
R₁ den Rest ―C(O)-R5, -SO2-R5 oder ―C(O)-NR6R5 bedeutet, worin R6 ein Wasserstoffatom oder einen linearen oder verzweigten Alkylrest mit höchstens 4 Kohlenstoffatomen wiedergibt und R5 einen linearen oder verzweigten Alkylrest mit höchstens 6 Kohlenstoffatomen, Cycloalkyl mit höchstens 6 Kohlenstoffatomen, Phenyl, Naphthyl oder einen gesättigten oder ungesättigten monocyclischen heterocyclischen Rest mit 5 oder 6 Gliedern, der ein oder mehrere identische oder verschiedene Heteroatome aufweist, ausgewählt unter O, N oder S, bedeutet,
wobei die vorstehend für R1 definierten Alkyl-, Phenyl- und heterocyclischen Reste gegebenenfalls substituiert sind durch einen oder mehrere Reste, ausgewählt unter den Halogenatomen und den linearen oder verzweigten Alkyl- oder Alkoxyresten mit höchstens 4 Kohlenstoffatomen, Hydroxyl, Acyl mit höchstens 7 Kohlenstoffatomen, Trifluormethyl, Cyano, Thienyl, Phenyl, Phenoxy und Isoxazolyl;
R2 und R7 derart sind, dass
entweder R7 ein Wasserstoffatom bedeutet und
R2 derart ist, dass die Gruppe 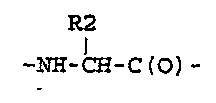 in den Produkten der Formel (I) den Rest einer sogenannten natürlichen oder sogenannten nicht-natürlichen Aminosäure der Struktur 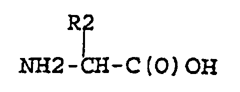 mit Ausnahme von Asparaginsäure wiedergibt,
oder R2 und R7 gemeinsam mit dem Stickstoffatom und dem Kohlenstoffatom, an das sie gebunden sind, einen Ring bilden derart, dass die in den Produkten der Formel (I) so gebildete Gruppe: 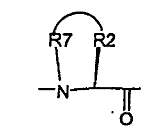 den Rest einer sogenannten natürlichen oder sogenannten nicht-natürlichen Aminosäure der Struktur: 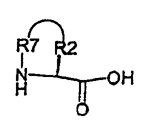 wiedergibt,
R3 den Rest -CH=N2 oder den Rest -CH2-L-R4 bedeutet, worin L eine einfache Bindung oder einen zweiwertigen Rest, ausgewählt unter -O-, -O-C(O)-, -NH- und -S-(CH2)nwiedergibt, wobei n eine ganze Zahl von 0 bis 6 bedeutet,
und R4 einen linearen oder verzweigten Alkylrest mit höchstens 6 Kohlenstoffatomen oder einen gesättigten oder ungesättigten, monocyclischen oder bicyclischen carbocyclischen oder heterocyclischen Rest mit 5 bis 10 Gliedern bedeutet, der ein oder mehrere identische oder verschiedene Heteroatome, ausgewählt unter O, N, NH oder S, enthält, und der ein Glied -C(O) enthalten kann, wobei die für R4 vorstehend definierten Alkyl-, carbocyclischen und heterocyclischen Reste gegebenenfalls substituiert sind durch einen oder mehrere Reste, ausgewählt unter dem Halogenatom; den Resten Hydroxyl; freies, in ein Salz überführtes oder verestertes Carboxy; -C(O)-NH2, -C(O)-NH-(Alkyl), -C(O)-N-(Alkyl)-(alkyl), -NH-C(O)-(Alkyl), -N-(Alkyl)-C(O)-(alkyl); Thienyl; Phenyl; Alkylphenyl, wobei Alkyl und Alkoxy ihrerseits gegebenenfalls substituiert sind durch einen oder mehrere Reste, ausgewählt unter den Acylresten mit höchstens 7 Kohlenstoffatomen, Cyano, -NH2, -NH-(Alkyl), -N-(Alkyl)-(alkyl) und Phenyl, mit der Maßgabe, dass in den vorstehenden Resten die Alkyl- und Alkoxyreste linear oder verzweigt sind und höchstens 4 Kohlenstoffatome enthalten,
wobei das Symbol * die Kohlenstoffatome anzeigt, die in der S- oder R-Konfiguration vorliegen können,
wobei die Produkte der Formel (I) in ihren sämtlichen möglichen isomeren racemischen, enantiomeren und diastereomeren Formen vorliegen können, ebenso wie die Additionssalze mit mineralischen und organischen Säuren oder mit mineralischen und organischen Basen der Produkte der Formel (I).

2. Produkte der Formel (I)', wie in Anspruch 1 definiert, worin R1 und R3 die in Anspruch 1 angegebenen Bedeutungen besitzen und R2 den Rest einer sogenannten natürlichen Aminosäure mit Ausnahme der Asparaginsäure wiedergibt, wobei die Produkte der Formel (I) in ihren sämtlichen möglichen isomeren racemischen, enantiomeren und diastereomeren Formen vorliegen können, ebenso wie die Additionssalze mit mineralischen und organischen Säuren oder mit mineralischen und organischen Basen der Produkte der Formel (I).

3. Produkte der Formel (I), wie in einem der Ansprüche 1 und 2 definiert, worin R1 und R3 die in Anspruch 1 oder 2 angegebenen Definitionen besitzen, und R2 und R7 derart sind, dass:
entweder R7 ein Wasserstoffatom bedeutet und R2 ein Wasserstoffatom oder einen linearen oder verzweigten Alkylrest mit höchstens 6 Kohlenstoffatomen, der gegebenenfalls substituiert ist durch einen oder mehrere Reste, ausgewählt unter den Resten Amino; Acylamino; NH=C(NH2)-NH-; Mercapto; lineares oder verzweigtes Alkylthio mit höchstens 4 Kohlenstoffatomen; Hydroxyl; lineares oder verzweigtes Alkoxy mit höchstens 4 Kohlenstoffatomen; Imidazolyl; Indolyl; Phenyl, das seinerseits gegebenenfalls substituiert ist durch einen oder mehrere Reste, ausgewählt unter den Halogenatomen, dem Hydroxylrest, einem linearen oder verzweigten Alkoxyrest mit höchstens 4 Kohlenstoffatomen und dem Phenoxyrest, der seinerseits gegebenenfalls substituiert ist durch einen oder mehrere Reste, ausgewählt unter den Iodatomen und dem Hydroxylrest, und dem Rest -C(O)-O-R8, worin R8 ein Wasserstoffatom oder einen linearen oder verzweigten Alkyl- oder Alkenylrest mit höchstens 6 Kohlenstoffatomen bedeutet, wiedergibt,
oder R2 und R7 gemeinsam mit den Stickstoff- und Kohlenstoffatomen, an die sie gebunden sind, einen Ring mit 5 bis 7 Gliedern bilden, der gegebenenfalls substituiert ist durch einen oder mehrere Hydroxylreste oder lineare oder verzweigte Alkoxyreste mit höchstens 4 Kohlenstoffatomen,
wobei die Produkte der Formel (I) in sämtlichen ihrer möglichen isomeren racemischen, enantiomeren und diastereomeren Formen vorliegen können, ebenso wie die Additionssalze mit mineralischen und organischen Säuren oder mit mineralischen und organischen Basen der Produkte der Formel (I).

4. Produkte der Formel (I), wie in einem der Ansprüche 1 bis 3 definiert, worin R1 und R3 die in einem der Ansprüche 1 bis 3 angegebenen Bedeutungen besitzen,
R2 und R7 derart sind, dass
entweder R7 ein Wasserstoffatom bedeutet und R2 die in Anspruch 3 angegebene Bedeutung besitzt,
oder R2 und R7 gemeinsam mit den Stickstoff- und Kohlenstoffatomen einen Pyrrolidinylring bilden, der gegebenenfalls durch einen oder mehrere Hydroxyl- oder lineare oder verzweigte Alkoxyreste mit höchstens 4 Kohlenstoffatomen substituiert ist,
wobei die Produkte der Formel (I) in sämtlichen ihrer möglichen isomeren racemischen, enantiomeren und diastereomeren Formen vorliegen, ebenso wie die Additionssalze mit mineralischen und organischen Säuren oder mit mineralischen und organischen Basen der Produkte der Formel (I).

5. Produkte der Formel (I), wie in einem der Ansprüche 1 bis 3 definiert, entsprechend der Formel (Ia): 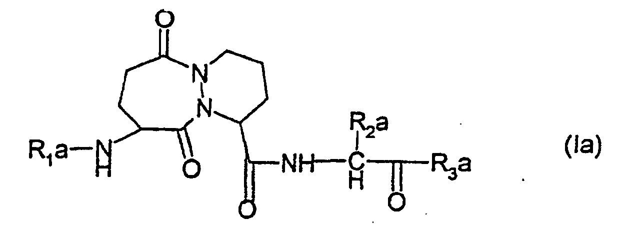 worin:
R₁ₐ den Rest -C(O)-R5a, -SO2-R5a oder -C(O)-NR6aR5a wiedergibt, worin R6a ein Wasserstoffatom oder einen linearen oder verzweigten Alkylrest mit höchstens 4 Kohlenstoffatomen bedeutet und R5a einen Rest wiedergibt, ausgewählt unter den linearen oder verzweigten Alkylresten mit höchstens 6 Kohlenstoffatomen; Cycloalkyl mit höchstens 6 Kohlenstoffatomen; Phenyl; Naphthyl; Furyl; Morpholinyl; Thienyl; Pyridyl,
wobei die Alkyl-, Phenyl-, Thienyl- und Pyridylreste gegebenenfalls substituiert sind durch einen oder mehrere Reste, ausgewählt unter den Halogenatomen und den linearen oder verzweigten Alkyl- oder Alkoxyresten mit höchstens 4 Kohlenstoffatomen, Hydroxyl, Acyl mit höchstens 7 Kohlenstoffatomen, Phenoxy, Trifluormethyl, Cyano, Thienyl, Phenyl und Isoxazolyl,
R2a die in einem der Ansprüche 1 bis 4 angegebene Bedeutung besitzt, wenn R7 ein Wasserstoffatom bedeutet,
R3a den Rest -CH=N2 oder den Rest -CH2-La-R4a wiedergibt, worin La eine einfache Bindung oder einen zweiwertigen Rest, ausgewählt unter -O-, -O-C(O)- und -S-(CH2)nawiedergibt, wobei na eine ganze Zahl von 0 bis 3 darstellt,
und R4a einen Rest bedeutet, ausgewählt unter den linearen oder verzweigten Alkylresten mit höchstens 6 Kohlenstoffatomen; Phenyl; Tetrazolyl; Piperazinyl; Piperidyl; Pyridyl; Benzothiazolyl; Chinolyl; Thiadiazolyl; Pyrimidinyl; Tetralon;
wobei die Alkyl-, Phenyl-, Tetrazolyl-, Piperazinyl- und Piperidylreste, wie sie für R4a definiert sind, gegebenenfalls substituiert sind durch einen oder mehrere Reste, ausgewählt unter den Halogenatomen; den Resten Hydroxyl; freies, in ein Salz überführtes oder verestertes Carboxy; -C(O)-NH2; -C(O)-NH-(Alkyl); -C(O)-N-(Alkyl)-(alkyl); -NH-C(O)-(Alkyl); -N-(Alkyl)-C(O)-(alkyl); Thienyl; Phenyl; Alkylphenyl; wobei Alkyl und Alkoxy ihrerseits gegebenenfalls substituiert sind durch einen oder mehrere Reste, ausgewählt unter den Acylresten mit höchstens 7 Kohlenstoffatomen, Cyano, -NH2, -NH-(Alkyl), -N-(Alkyl)-(alkyl) und Phenyl,
mit der Maßgabe, dass in den vorstehenden Resten die Alkyl- und Alkoxyreste linear oder verzweigt sind und höchstens 4 Kohlenstoffatome besitzen,
wobei die Produkte der Formel (Ia) in sämtlichen ihrer möglichen isomeren racemischen, enantiomeren und diasteromeren Formen vorliegen können, ebenso wie die Additionssalze mit mineralischen und organischen Säuren oder mit mineralischen und organischen Basen der Produkte der Formel (Ia).

6. Produkte der Formel (I), wie in einem der Ansprüche 1 bis 5 definiert, mit der Formel (Ib): 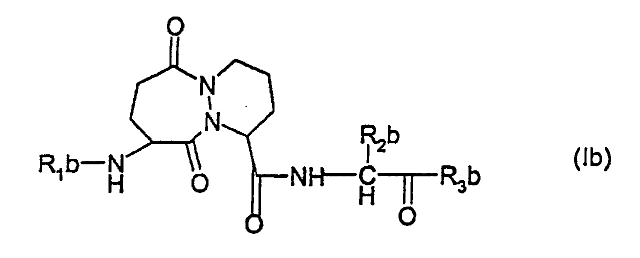 worin:
R_{1b} den Rest -C(O)-R5b, -SO2-R5b oder -C(O)-NR6bR5b wiedergibt,
worin R6b ein Wasserstoffatom oder einen Methylrest bedeutet und R5b einen Rest bedeutet, ausgewählt unter den linearen oder verzweigten Alkylresten mit höchstens 4 Kohlenstoffatomen, gegebenenfalls substituiert durch einen Thienylrest; Cyclohexyl; Phenyl, gegebenenfalls substituiert durch einen linearen oder verzweigten Alkoxyrest mit höchstens 4 Kohlenstoffatomen, einen Phenoxy- oder Trifluormethylrest; Naphthyl; Furyl; Morpholinyl; Thienyl, gegebenenfalls substituiert durch einen Isoxazolylrest; Pyridyl, gegebenenfalls substituiert durch einen Trifluormethylrest,
R2b ein Wasserstoffatom oder einen linearen oder verzweigten Alkylrest mit höchstens 6 Kohlenstoffatomen bedeutet, der gegebenenfalls substituiert ist entweder durch den Phenylrest, der seinerseits gegebenenfalls substituiert ist durch Hydroxyl oder lineare oder verzweigte Alkoxyreste mit höchstens 4 Kohlenstoffatomen, oder durch den Rest -C(O)-O-R8b, worin R8b einen linearen oder verzweigten Alkyl- oder Alkenylrest mit höchstens 6 Kohlenstoffatomen bedeutet,
R3b den Rest -CH=N2 oder den Rest -CH2-Lb-R4b wiedergibt, worin Lb eine Einfachbindung oder einen zweiwertigen Rest bedeutet, ausgewählt unter -O-, -O-C(O)- und -S-(CH2)nb-, wobei nb die ganze Zahl 0 oder 1 wiedergibt,
und R4b einen Rest bedeutet, ausgewählt unter den Resten Pyridyl; Benzothiazolyl; Chinolyl; Thiadiazolyl; Pyrimidinyl; Tetralon; lineares oder verzweigtes Alkyl mit höchstens 4 Kohlenstoffatomen, gegebenenfalls substituiert durch einen freien, in ein Salz überführten oder veresterten Carboxyrest oder Thienyl; Phenyl, gegebenenfalls substituiert durch einen oder mehrere Reste, ausgewählt unter den Halogenatomen, den freien, in ein Salz überführten oder veresterten Carboxyresten, den linearen oder verzweigten Alkyl- und Alkoxyresten mit höchstens 4 Kohlenstoffatomen, Cyanoalkyl, Alkylphenyl, -NH-C(O)-CH3, -C(O)-N-(Alkyl)-(alkyl); Tetrazolyl, das gegebenenfalls durch einen Phenylrest substituiert ist; Piperazinyl, das gegebenenfalls an dem zweiten Stickstoffatom substituiert ist durch einen Phenyl- oder linearen oder verzweigten Alkylrest mit höchstens 4 Kohlenstoffatomen, der seinerseits gegebenenfalls substituiert ist durch einen Rest Acyl (Pyrrolidinylcarbonyl), ein oder zwei Phenylreste oder einen Rest -NH2, -NH-(Alkyl) oder -N-(Alkyl)-(alkyl); Piperidyl, das gegebenenfalls substituiert ist durch einen Benzylrest oder
-C(O)-N-(Alkyl)-(alkyl), mit der Maßgabe, dass in den vorstehenden Resten die Alkylund Alkoxyreste linear oder verzweigt sind und höchstens 4 Kohlenstoffatome aufweisen,
wobei die Produkte der Formel (Ib) in sämtlichen ihrer möglichen isomeren racemischen, enantiomeren und diasteromeren Formen vorliegen können, ebenso wie die Additionssalze mit mineralischen und organischen Säuren oder mit mineralischen und organischen Basen der Produkte der Formel (Ib).

7. Produkte der Formel (I), wie in einem der Ansprüche 1 bis 6 definiert, worin R1 einen Rest bedeutet, ausgewählt unter den folgenden Resten: 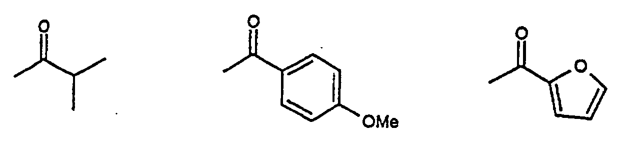 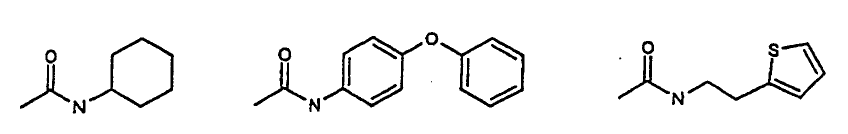 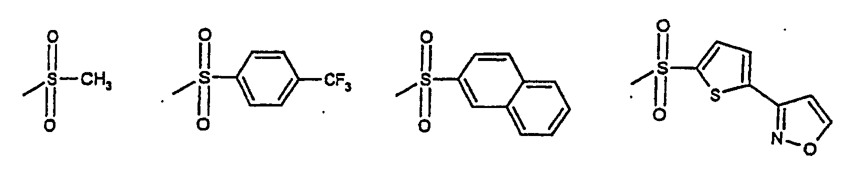 und R2, R3 und R7 die in Anspruch 1 angegebenen Bedeutungen besitzen.

8. Produkte der Formel (I), wie in einem der Ansprüche 1 bis 7 definiert, worin R7 ein Wasserstoffatom bedeutet und R2 einen Rest wiedergibt, ausgewählt unter den folgenden Resten: 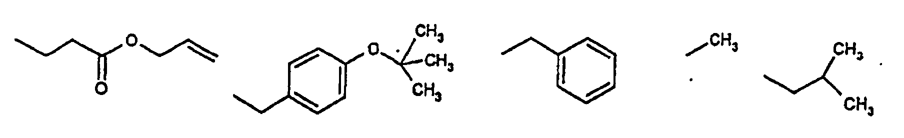 und R1 und R3 die in Anspruch 1 angegebenen Bedeutungen besitzen.

9. Produkte der Formel (I), wie in einem der Ansprüche 1 bis 8 definiert, worin R3 einen Rest bedeutet, ausgewählt unter den folgenden Resten: 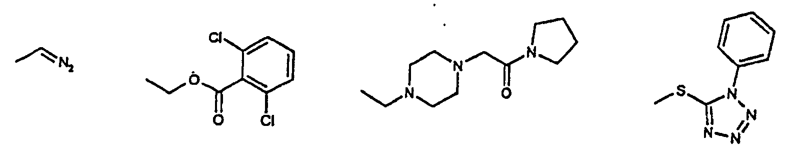 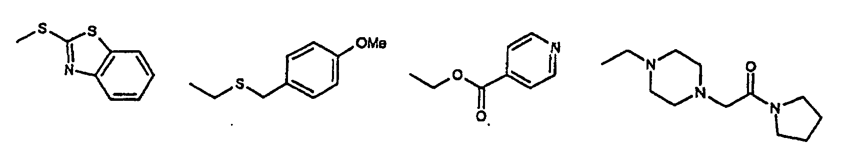 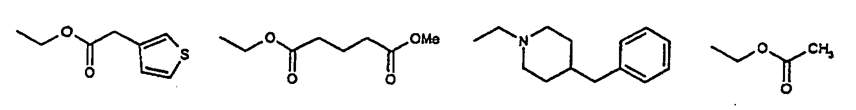 und R1, R2 und R7 die in Anspruch 1 angegebenen Bedeutungen besitzen.

10. Produkte der Formel (I) mit den folgenden Bezeichnungen:
- 3-[9(S)-Benzoylamino-6,10-dioxo-1,2,3,4,7,8,9,10-octahydro-6H-pyridazino-[1,2-a]-[1,2]-diazepin-1(S)-carboxamid]-5-methyl-1-benzoyloxy-hexan-2-on,
- 3-[9(S)-(2-Furanoyl)-amino-6,10-dioxo-1,2,3,4,7,8,9,10-octahydro-6H-pyridazino-[1,2-a]-[1,2]-diazepin-1(S)-carboxamid]-5-methyl-1-(2,6-dichlorbenzoyloxy)-hexan-2-on
- 3(S)-[9(S)-(2-Furanoyl)-amino-6,10-dioxo-1,2,3,4,7,8,9,10-octahydro-6H-pyridazino-[1,2-a]-[1,2]-diazepin-1(S)-carboxamid-4-(4-hydroxyphenyl)-1-(2,6-dichlorbenzoyloxy)-butan-2-on.

11. Produkte der Formel (I) mit den folgenden Bezeichnungen:
- (4S)-6-Diazo-4-[[[(1S,9S)-9-[(2-methyl-1-oxo-propyl)-amino]-octahydro-6,10-dioxo-6H-pyridazino-[1,2-a]-[1,2]-diazepin-1-yl]-carbonyl]-amino]-5-oxo-(2-propenyl)-hexanoat,
- (4S)-Diazo-4-[[[(1S,9S)-9-[(3-methoxybenzoyl)-amino]-octahydro-6,10-dioxo-6H-pyridazino-[1,2-a)-[ 1,2]-diazepin-1-yl]-carbonyl]-amino]-5-oxo-(2-propenyl)-hexanoat,
- (4S)-6-Diazo-4-[[[(1S,9S)-9-[[(2-furanyl)-carbonyl]-amino]-octahydro-6,10-dioxo-6H-pyridazino-[1,2-a]-[1,2]-diazepin-1-yl]-carbonyl]-amino]-5-oxo-(2-propenyl)-hexanoat,
- (4S)-6-Diazo-4-[[[(1S,9S)-9-[[(cyclohexylamino)-carbonyl]-amino]-octahydro-6,10-dioxo-6H-pyridazino-[1,2-a]-[1,2]-diazepin-1-yl]-carbonyl]-amino]-5-oxo-(2-propenyl)-hexanoat,
- (4S)-6-Diazo-4-[[[(1S,9S)-9-[[[(4-phenoxyphenyl)-amino]-carbonyl]-amino]-octahydro-6,10-dioxo-6H-pyridazino-[1,2-a)-[1,2]-diazepin-1-yl]-carbonyl]-amino]-5-oxo-(2-propenyl)-hexanoat,
- (4S)-6-Diazo-4-[[[(1S,9S)-9-[[[[2-(p-2-yl)-ethyl]-amino]-carbonyl]-amino]-octahydro-6,10-dioxo-6H-pyridazino-[1,2-a]-[1,2]-diazepin-1-yl]-carbonyl]-amino]-5-oxo-(2-propenyl)-hexanoat,
- (4S)-6-Diazo-4-[[[(1S,9S)-9-[(methylsulfonyl)-amino]-octahydro-6,10-dioxo-6H-pyridazino-[ 1,2-a]-[1,2]-diazepin-1-yl]-carbonyl]-amino]-5-oxo-(2-propenyl)-hexanoat,
- (4S)-6-Diazo-4-[[[(1S,9S)-9-[[[4-trifluormethyl)-phenyl]-sulfonyl]-amino]-octahydro-6,10-dioxo-6H-pyridazino-[1,2-a]-[1,2]-diazepin-1-yl]-carbonyl]-amino]-5-oxo-(2-propenyl)-hexanoat,
- (4S)-6-Diazo-4-[[[(1S,9S)-9-[[(2-naphthalenyl)-sulfonyl]-amino]-octahydro-6,10-dioxo-6H-pyridazino-[1,2-a]-[1,2]-diazepin-1-yl]-carbonyl]-amino]-5-oxo-(2-propenyl)-hexanoat,
- (4S)-6-Diazo-4-[[[(1S,9S)-9-[[(5-(isoxazol-3-yl)-2-thienyl)-sulfonyl]-amino]-octahydro-6,10-dioxo-6H-pyridazino-[1,2-a]-[1,2]-diazepin-1-yl]-carbonyl]-amino]-5-oxo-(2-propenyl)-hexanoat,
- 9-[(9S)-[[(4-Phenoxyphenyl)-amino]-carbonyl]-amino]-N-[(2S)-4-diazo-1-[4-[(1,1,dimethyl)-ethoxy]-phenyl]-3-oxo-2-butyl]-6H-(1S)-pyridazino-[1,2-a]-[1,2]-diazepin-1-carboxamid
- (4S)-6-[(2,6-Dichlorbenzoyl)-oxy]-4-[[[(1S,9S)-9-[[(2-furanyl)-carbonyl]-amino]-octahydro-6,10-dioxo-6H-pyridazino-[1,2-a]-[1,2]-diazepin-1-yl]-carbonyl]-amino]-5-oxo(2-propenyl)-hexanoat,
- (4S)-6-[(2,6-Dichlorbenzoyl)-oxy]-4-[[[(1S,9S)-9-[[[4-(trifluormethyl)-phenyl]-sulfonyl]-amino]-octahydro-6,10-dioxo-6H-pyridazino-[1,2-a]-[1,2]-diazepin-1-yl]-carbonyl]-amino]-5-oxo-(2-propenyl)-hexanoat,
- (4S)-6-[4-[[2-Oxo-2-[1-pyrrolidinyl)-ethyl]-piperazin-1-yl]-4-[[[(1S,9S)-9-[[[4-(trifluormethyl)-phenyl]-sulfonyl]-amino)-octahydro-6,10-dioxo-6H-pyridazino-[1,2-a]-[1,2]-diazepin-1-yl]-carbonyl]-amino)-5-oxo-(2-propenyl)-hexanoat,
- (4S)-6-[4-[[2-Oxo-2-(1-pyrrolidinyl)-ethyl]-piperazin-1-yl]-4-[[[(1S,9S)-9-[[(2-naphthalinyl)-sulfonyl]-amino]-octahydro-6,10-dioxo-6H-pyridazino-[1,2-a]-[1,2]-diazepin-1-yl]-carbonyl]-amino]-5-oxo-(2-propenyl)-hexanoat,
- (4S)-6-[(2,6-Dichlorbenzoyl)-oxy]-4-[[[(1S,9S)-9-[[(cyclohexylamino)-carbonyl]-amino]-octahydro-6,10-dioxo-6H-pyridazino-[1,2-a]-[1,2]-diazepin-1-yl]-carbonyl]-amino]-5-oxo-(2-propenyl)-hexanoat,
- (4S)-6-[(1-Phenyl-1H-tetrazol-5-yl)-thio]-4-[[[(1S,9S)-9-[[[(4-phenoxyphenyl)-amino]-carbonyl]-amino]-octahydro-6,10-dioxo-6H-pyridazino-[1,2-a]-[1,2]-diazepin-1-yl]-carbonyl]-amino]-5-oxo-(2-propenyl)-hexanoat,
- (4S)-6-[(Benzothiazol-2-yl)-thio]-4-[[[(1S,9S)-9-[[[(4-phenoxyphenyl)-amino]-carbonyl]-amino]-octahydro-6,10-dioxo-6H-pyridazino-[1,2-a]-[ 1,2]-diazepin-1-yl]-carbonyl]-amino]-5-oxo-(2-propenyl)-hexanoat,
- (4S)-6-[[(4-Methoxyphenyl)-methyl]-thio]-4-[[[(1S,9S)-9-[[[(4-phenoxyphenyl)-amino]-carbonyl]-amino]-octahydro-6,10-dioxo-6H-pyridazino-[1,2-a]-[1,2]-diazepin-1-yl]-carbonyl]-amino]-5-oxo-(2-propenyl)-hexanoat,
- (4S)-6-[[(4-Pyridinyl)-carbonyl]-oxy]-4-[[[(1S,9S)-9-[[[(4-phenoxyphenyl)-amino]-carbonyl]-amino]-octahydro-6,10-dioxo-6H-pyridazino-[1,2-a]-[1,2]-diazepin-1-yl]-carbonyl]-amino]-5-oxo-(2-propenyl)-hexanoat,
- (4S)-6-[(2,6-Dichlorbenzoyl)-oxy]-4-[[[(1S,9S)-9-[[[(4-phenoxyphenyl)-amino]-carbonyl]-amino]-octahydro-6,10-dioxo-6H-pyridazino-[1,2-a]-[1,2]-diazepin-1-yl]-carbonyl]-amino]-5-oxo-(2-propenyl)-hexanoat,
- ((4S)-6-Acetyloxy-4-[[[(1S,9S)-9-[[[(4-phenoxyphenyl)-amino]-carbonyl]-amino]-octahydro-6,10-dioxo-6H-pyridazino-[1,2-a]-[1,2]-diazepin-1-yl]-carbonyl]-amino]-5-oxo-(2-propenyl)-hexanoat,
- (4S)-6-[1-Oxo-2-(thien-3-yl)-ethoxy]-4-[[[(1S,9S)-9-[[[(4-phenoxyphenyl)-amino]-carbonyl]-amino]-octahydro-6,10-dioxo-6H-pyridazino-[1,2-a]- [1,2]-diazepin-1-yl]-carbonyl]-amino]-5-oxo-(2-propenyl)-hexanoat,
- (4S)-6-[(1-Phenyl-1H-tetrazol-5-yl)-thio]-4-[[[(1S,9S)-9-[[[[2-(thien-2-yl)-ethyl]-amino]-carbonyl]-amino]-octahydro-6,10-dioxo-6H-pyridazino-[1,2-a]-[1,2]-diazepin-1-yl]-carbonyl]-amino]-5-oxo-(2-propenyl)-hexanoat,
- (4S)-6-[(2,6-Dichlorbenzoyl)-oxy]-4-[[[(1S,9S)-9-[[[[2-(thien-2-yl)-ethyl]-amino]-carbonyl]-amino]-octahydro-6,10-dioxo-6H-pyridazino-[1,2-a]-[1,2]-diazepin-1-yl]-carbonyl]-amino]-5-oxo-(2-propenyl)-hexanoat,
- (4S)-6-[(2,6-Dichlorbenzoyl)-oxy]-4-[[[(1S,9S)-9-[(methylsulfonyl)-amino]-octahydro-6,10-dioxo-6H-pyridazino-[1,2-a]-[1,2]-diazepin-1-yl]-carbonyl]-amino]-5-oxo-(2-propenyl)-hexanoat,
- 9-[(9S)-[[(4-Phenoxyphenyl)-amino]-carbonyl]-amino]-N-((2S)-4-diazo-1-phenyl-3-oxo-2-butyl)-octahydro-6,10-dioxo-6H-(1S)-pyridazino-[1,2-a]-[1,2]-diazepin-1-carboxamid,
- 9-[(9S)-[[[2-(thien-2-yl)-ethyl]-amino]-carbonyl]-amino]-N-((2S)-4-diazo-1-phenyl-3-oxo-2-butyl)-octahydro-6,10-dioxo-6H-(1S)-pyridazino-[1,2-a]-[1,2]-diazepin-1-carboxamid,
- 9-[(9S)-[(2-Naphthalinyl)-sulfonyl]-amino]-N-((2S)-4-diazo-1-phenyl-3-oxo-2-butyl)-octahydro-6,10-dioxo-6H-(1S)-pyridazino-[1,2-a]-[1,2]-diazepin-1-carboxamid,
- 9-[(9S)-[[5-(Isoxazol-3-yl)-2-thienyl]-sulfonyl]-amino]-N-((2S)-4-diazo-1-phenyl-3-oxo-2-butyl)-octahydro-6,10-dioxo-6H-(1S)-pyridazino-[1,2-a]-[1,2]-diazepin-1-carboxamid,
- 9-[(9S)-[[(4-Phenoxyphenyl)-amino]-carbonyl]-amino]-N-((2S)-4-diazo-3-oxo-2-butyl)-octahydro-6,10-dioxo-6H-(1S)-pyridazino-[1,2-a]-[1,2]-diazepin-1-carboxamid,
- 9-[(9S)-[[[3-(Thien-2-yl)-ethyl]-amino]-carbonyl]-amino]-N-((2S)-4-diazo-3-oxo-2-butyl)-octahydro-6,10-dioxo-6H-(1S)-pyridazino-[1,2-a]-[1,2]-diazepin-1-carboxamid,
- 9-[(9S)-(3-Methoxybenzoyl)-amino]-N-((2S)-4-[(2,6-dichlorbenzoyl)-oxy]-1-phenyl-3-oxo-2-butyl]-octahydro-6,10-dioxo-6H-(1S)-pyridazino-[1,2-a]-[1,2]-diazepin-1-carboxamid,
- 9-[(9S)-[(2-Furanyl)-carbonyl]-amino]-N-[(2S)-4-[(2,6-dichlorbenzoyl)-oxy]-1-phenyl-3-oxo-2-butyl]-octahydro-6,10-dioxo-6H-(1S)-pyridazino-[1,2-a]-[1,2]-diazepin-1-carboxamid,
- 9-[(9S)-[(Cyclohexylamino)-carbonyl]-amino]-N-[(2S)-4-[(2,6-dichlorbenzoyl)-oxy]-1-phenyl-3-oxo-2-butyl]-octahydro-6,10-dioxo-6H-(1S)-pyridazino-[1,2-a]-[1,2]-diazepin-1-carboxamid,
- 9-[(9S)-[[(4-Phenoxyphenyl)-amino]-carbonyl]-amino]-N-[(2S)-1-phenyl-4-[(1-phenyl-1H-tetrazol-5-yl)-thio]-3-oxo-2-butyl]-octahydro-6,10-dioxo-6H-(1S)-pyridazino-[1,2-a]-[1,2]-diazepin-1-carboxamid,
- 9-[(9S)-[[(4-Phenoxyphenyl)-amino]-carbonyl]-amino]-N-[(2S)-1-phenyl-4-[1-oxo-2-(3-thienyl)-ethoxy]-3-oxo-2-butyl]-octahydro-6,10-dioxo-6H-(1S)-pyridazino-[1 ,2-a]-[1,2]-diazepin-1-carboxamid,
- (Methyl)-[2-oxo-4-Phenyl-3-[(3S)-[[(4-phenoxyphenyl)-amino]-carbonyl]-amino]-octahydro-6,10-dioxo-6H-(1S)-pyridazino-[1,2-a]-[1,2]-diazepin-1-yl]-carbonyl]-amino]-butyl]-pentandioat,
- 9-[(9S)-[[(4-Phenoxyphenyl)-amino]-carbonyl]-amino]-N-[(2S)-4-[(4-phenylmethyl)-1-piperidinyl]-1-phenyl-3-oxo-2-butyl]-octahydro-6,10-dioxo-6H-(1S)-pyridazino-[1,2-a]-[1,2]-diazepin-1-carboxamid,
- 9-[(9S)-[[[2-(Thien-2-yl)-ethyl]-amino]-carbonyl]-amino]-N-[(2S)-1-phenyl-4-[(1-phenyl-1H-tetrazol-5-yl)-thio]-3-oxo-2-butyl]-octahydro-6,10-dioxo-6H-(1S)-pyridazino-[ 1,2-a]-[1,2]-diazepin-1-carboxamid,
- 9-[(9S)-[[[2-(Thien-2-yl)-ethyl]-amino]-carbonyl]-amino]-N-[(2S)-1-phenyl-4-[(2-benzothiazolyl)-thio]-3-oxo-2-butyl]-octahydro-6, 10-dioxo-6H-(1S)-pyridazino-[1,2-a]-[1,2]-diazepin-1-carboxamid,
- 9-[(9S)-[[[2-(Thien-2-yl)-ethyl]-amino]-carbonyl]-amino]-N-[(2S)-1-phenyl-4-[(-2,6-dichlorbenzoyl)-oxy]-3-oxo-2-butyl]-octahydro-6,10-dioxo-6H-(1S)-pyridazino-[1,2-a]-[1,2]-diazepin-1-carboxamid,
- 9-[(9S)-[[[2-(Thien-2-yl)-ethyl]-amino]-carbonyl]-amino]-N-[(2S)-1-phenyl-4-[4-[2-(1-pyrrolidinyl)-2-oxo-ethyl]-piperazin-1-yl]]-3-oxo-2-butyl]-octahydro-6,10-dioxo-6H-(1S)-pyridazino-[1,2-a]-[1,2]-diazepin-1-carboxamid,
- 9-[(9S)-(Methylsulfonyl)-amino]-N-[(2S)-1-phenyl-4-[(-2,6-dichlorbenzoyl)-oxy]-3-oxo-2-butyl]-octahydro-6,10-dioxo-6H-(1S)-pyridazino-[1,2-a]-[1,2]-diazepin-1-carboxamid,
- 9-[(9S)-[[[4-(Trifluormethyl)-phenyl]-sulfonyl]-amino]-N-[(2S)-1-phenyl-4-[(1-phenyl-1H-tetrazol-5-yl)-thio]-3-oxo-2-butyl]-octahydro-6,10-dioxo-6H-(1S)-pyridazino-[1,2-a]-[1,2]-diazepin-1-carboxamid,
- 9-[(9S)-[[[4-(Trifluormethyl)-phenyl]-sulfonyl]-amino]-N-[(2S)-1-phenyl-4-(2-benzothiazolyl)-thio]-3-oxo-2-butyl]-octahydro-6,10-dioxo-6H-(1S)-pyridazino-[1,2-a]-[1,2]-diazepin-1-carboxamid,
- 9-[(9S)-(Methylsulfonyl)-amino]-N-[(3S)-5-methyl-1-[(2,6-dichlorbenzoyl)-oxy]-2-oxo-3-hexyl]-octahydro-6,10-dioxo-6H-(1S)-pyridazino-[1,2-a]-[1,2]-diazepin-1-carboxamid,
- 9-[(9S)-[[[4-(Trifluormethyl)-phenyl]-sulfonyl]-amino]]-N-[(3S)-5-methyl-1-[(2,6-dichlorbenzoyl)-oxy]-2-oxo-3-hexyl]-octahydro-6,10-dioxo-6H-(1S)-pyridazino-[1,2-a]-[1,2]-diazepin-1-carboxamid,
- 9-[(9S)-[(2-Naphthalinyl)-sulfonyl]-amino]-N-[(3S)-5-methyl-1-[(2,6-dichlorbenzoyl)-oxy]-2-oxo-3-hexyl]-octahydro-6,10-dioxo-6H-(1S)-pyridazino-[1,2-a]-[1,2]-diazepin-1-carboxamid,
- 9-[(9S)-(2-Methyl-1-oxo-propyl)-amino]-N-((3S)-1-diazo-5-methyl-2-oxo-3-hexyl)-octahydro-6,10-dioxo-6H-(1S)-pyridazino-[1,2-a]-[1,2]-diazepin-1-carboxamid,
- 9-[(9S)-(3-Methoxybenzoyl)-amino]-N-((3S)-1-diazo-5-methyl-2-oxo-3-hexyl)-octahydro-6,10-dioxo-6H-(1S)-pyridazino-[1,2-a]-[1,2]-diazepin-1-carboxamid,
- 9-[(9S)-[(2-Furanyl)-carbonyl]-amino]-N-((3S)-1-diazo-5-methyl-2-oxo-3-hexyl)-octahydro-6,10-dioxo-6H-(1S)-pyridazino-[1,2-a]-[1,2]-diazepin-1-carboxamid,
- 9-[(9S)-[(Cyclohexylamino)-carbonyl]-amino]-N-((3S)-1-diazo-5-methyl-2-oxo-3-hexyl)-octahydro-6,10-dioxo-6H-(1S)-pyridazino-[1,2-a]-[1,2]-diazepin-1-carboxamid,
- 9-[(9S)-[[(4-Phenoxyphenyl)-amino]-carbonyl]-amino)-N-((3S)-1-diazo-5-methyl-2-oxo-3-hexyl)-octahydro-6,10-dioxo-6H-(1S)-pyridazino-[1,2-a]-[1,2]-diazepin-1-carboxamid,
- 9-[(9S)-[[[2-(Thien-2-yl)-ethyl]-amino]-carbonyl]-amino]-N-((3S)-1-diazo-5-methyl-2-oxo-3-hexyl)-octahydro-6,10-dioxo-6H-(1S)-pyridazino-[1,2-a]-[1,2]-diazepin-1-carboxamid,
- 9-[(9S)-(Methylsulfonyl)-amino]-N-((3S)-1-diazo-5-methyl-2-oxo-3-hexyl)-octahydro-6,10-dioxo-6H-(1S)-pyridazino-[1,2-a]-[1,2]-diazepin-1-carboxamid,
- 9-[(9S)-[[4-(Trifluormethyl)-phenyl]-sulfonyl]-amino]-N-((3S)-1-diazo-5-methyl-2-oxo-3-hexyl)-octahydro-6,10-dioxo-6H-(1S)-pyridazino-[1,2-a]-[1,2]-diazepin-1-carboxamid,
- 9-[(9S)-[(2-Naphthalinyl)-sulfonyl]-amino]-N-((3S)-1-diazo-5-methyl-2-oxo-3-hexyl)-octahydro-6,10-dioxo-6H-(1S)-pyridazino-[1-2-a]-[1,2]-diazepin-1-carboxamid,
- 9-[(9S)-(3-Methoxybenzoyl)-amino]-N-[(3S)-1-[(benzothiazol-2-yl)-thio]-4-methyl-2-oxo-3-pentyl]-octahydro-6,10-dioxo-6H-(1S)-pyridazino-[1,2-a]-[1,2]-diazepin-1-carboxamid,
- 9-[(9S)-[(2-Furanyl)-carbonyl]-amino]-N-[(3S)-1-[(benzothiazol-2-yl)-thio]-4-methyl-2-oxo-3-pentyl]-octahydro-6,10-dioxo-6H-(1S)-pyridazino-[1,2-a]-[1,2]-diazepin-1-carboxamid,
- 9-[(9S)-[(Cyclohexylamino)-carbonyl]-amino]-N-((3S)-1-[(benzothiazol-2-yl)-thio]-4-methyl-2-oxo-3-pentyl]-octahydro-6,10-dioxo-6H-(1S)-pyridazino-[1,2-a]-[1,2]-diazepin-1-carboxamid,
- 9-[(9S)-[[(4-Phenoxyphenyl)-amino]-carbonyl]-amino]-N-[(3S)-1-[(1-phenyl-1H-tetrazol-5-yl)-thio]-4-methyl-2-oxo-3-pentyl]-octahydro-6,10-dioxo-6H-(1S)-pyridazino-[1,2-a]-[1,2]-diazepin-1-carboxamid,
- 9-[(9S)-[[(4-Phenoxyphenyl)-amino]-carbonyl]-amino]-N-[3S)-1-[(benzothiazol-2-yl)-thio]-4-methyl-2-oxo-3-pentyl]-octahydro-6,10-dioxo-6H-(1S)-pyridazino-[1,2-a]-[1,2]-diazepin-1-carboxamid,
- 9-[(9S)-[[[2-(Thien-2-yl)-ethyl]-amino]-carbonyl]-amino]-N-[(3S)-1-[(1-phenyl-1H-tetrazol-5-yl)-thio]-4-methyl-2-oxo-3-pentyl]-octahydro-6,10-dioxo-6H-(1S)-pyridazino-[1,2-a]-[1,2]-diazepin-1-carboxamid,
- 9-[(9S)-[[[2-(Thien-2-yl)-ethyl]-amino]-carbonyl]-amino]-N-[(3S)-1-[(benzothiazol-2-yl)-thio]-4-methyl-2-oxo-3-pentyl]-octahydro-6,10-dioxo-6H-(1S)-pyridazino-[1,2-a]-[1,2]-diazepin-1-carboxamid,
- 9-[(9S)-[(Cyclohexylamino)-carbonyl]-amino]-N-[(2S)-4-[(2,6-dichlorbenzoyl)-oxy]-3-oxo-2-butyl]-octahydro-6,10-dioxo-6H-(1S)-pyridazino-[1,2-a]-[1,2]-diazepin-1-carboxamid,
- 9-[(9S)-[(2-Furanyl)-carbonyl]-amino]-N-[(2S)-4-[(2,6-dichlorbenzoyl)-oxy]-3-oxo-2-butyl]-octahydro-6,10-dioxo-6H-(1S)-pyridazino-[1,2-a]-[1,2]-diazepin-1-carboxamid,
- 9-[(9S)-[[(4-Phenoxyphenyl)-amino]-carbonyl]-amino]-N-[(2S)-4-[(benzothiazol-2-yl)-thio]-3-oxo-2-butyl]-octahydro-6,10-dioxo-6H-(1S)-pyridazino-[1,2-a]-[ 1,2]-diazepin-1-carboxamid,
- 9-[(9S)-[[(4-Phenoxyphenyl)-amino]-carbonyl]-amino]-N-[(2S)-4-[(2,6-dichlorbenzoyl)-oxy]-3-oxo-2-butyl]-octahydro-6,10-dioxo-6H-(1S)-pyridazino-[1,2-a]-[1,2]-diazepin-1-carboxamid,
- 9-[(9S)-(3-Methoxybenzoyl)-amino]-N-[(2S)-4-[(2,6-dichlorbenzoyl)-oxy]-3-oxo-2-butyl]-octahydro-6,10-dioxo-6H-(1S)-pyridazino-[1,2-a]-[1,2]-diazepin-1-carboxamid,
- 9-[(9S)-[[[2-(Thien-2-yl)-ethyl]-amino]-carbonyl]-amino]-N-[(2S)-4-[(2,6-dichlorbenzoyl)-oxy]-3-oxo-2-butyl]-octahydro-6,10-dioxo-6H-(1S)-pyridazino-[1,2-a]-[1,2]-diazepin-1-carboxamid,
- 9-[(9S)-[(4-(Trifluormethyl)-phenyl]-sulfonyl]-amino]-N-[(2S)-4-[(2,6-dichlorbenzoyl)-oxy]-3-oxo-2-butyl]-octahydro-6,10-dioxo-6H-(1S)-pyridazino-[1,2-a]-[1,2]-diazepin-1-carboxamid,
- 9-[(9S)-[(Cyclohexylamino)-carbonyl]-amino]-N-[(2S)-4-[(benzothiazol-2-yl)-thio]-1-[4-[(1,1-dimethyl)-ethoxy]-phenyl]-3-oxo-2-butyl]-6H-(1S)-pyridazino-[1,2-a]-[1,2]-diazepin-1-carboxamid.

12. Verfahren zur Herstellung von Produkten der Formel (I), wie in Anspruch 1 definiert, **dadurch gekennzeichnet, dass** man die Verbindung der Formel (II) 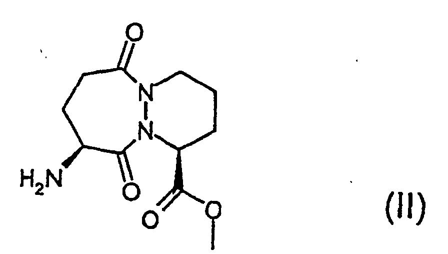 einer Umsetzung mit einer Verbindung der Formel (III)
R1'-X (III)
unterzieht, worin X ein Halogenatom bedeutet und R1' die in Anspruch 1 für R1 angegebene Bedeutung besitzt, wobei etwaige reaktive Funktionen gegebenenfalls durch Schutzgruppen geschützt werden, um zu dem Produkt der Formel (IV) 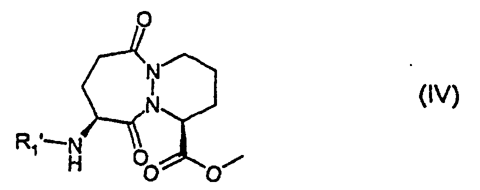 zu gelangen, worin R1' die vorstehend angegebene Bedeutung besitzt, welches Produkt der Formel (IV) man einer Verseifungsreaktion unterzieht, um zu dem Produkt der Formel (V) 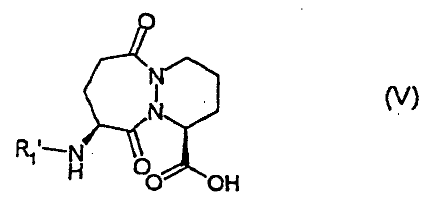 zu gelangen, worin R1' die vorstehend angegebene Bedeutung besitzt, welches Produkt der Formel (V) man einer Umsetzung mit einem Produkt der Formel (VI) 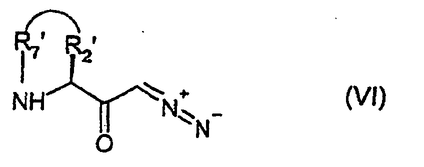 unterzieht, worin R2' und R7' die jeweils in Anspruch 1 für R2 und R7 angegebenen Bedeutungen besitzen, und worin die etwaigen reaktiven Funktionen gegebenenfalls durch Schutzgruppen geschützt werden, um zu einem Produkt der Formel (Ix) 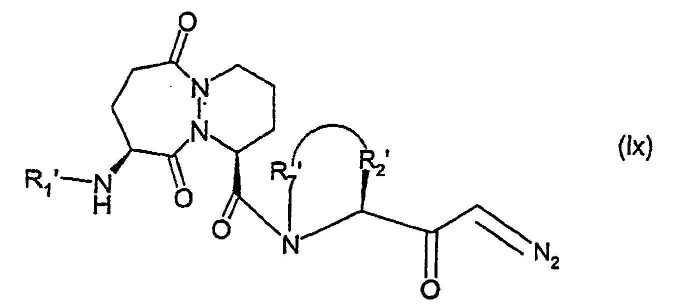 zu gelangen, worin R1', R2' und R7' die vorstehend angegebenen Bedeutungen besitzen, welches Produkt der Formel (Ix) man einer Bromierungsreaktion unterziehen kann, um zu einem Produkt der Formel (VII) 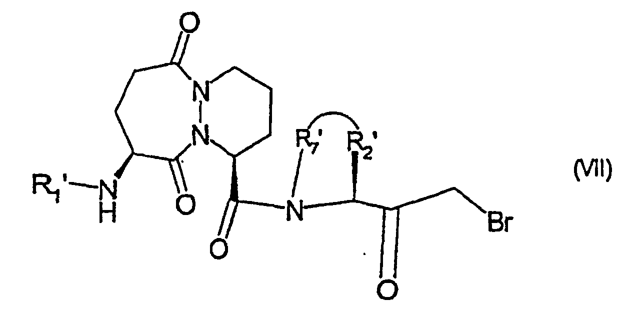 zu gelangen, worin R1', R2' und R7' die vorstehend angegebenen Bedeutungen besitzen, welches Produkt der Formel (VII) man einer Umsetzung mit einem Produkt der Formel (VIII)
H'-L-R4' (VIII)
unterzieht, worin L die in Anspruch 1 angegebene Bedeutung besitzt und R4' die in Anspruch 1 für R4 angegebene Bedeutung besitzt, und worin die etwaigen reaktiven Funktionen gegebenenfalls durch Schutzgruppen geschützt werden,
um zu einem Produkt der Formel (Iy) 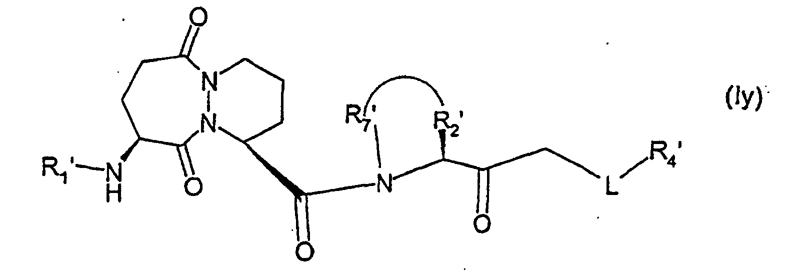 zu gelangen, worin R1', R2', R4' und R7' die vorstehend angegebenen Bedeutungen besitzen,
wobei die Produkte der Formeln (Ix) und (Iy) Produkte der Formeln (I) sein können, und man diese, um zu den Produkten der Formel (I) oder zu anderen Produkten der Formel (I) zu gelangen, gewünschtenfalls und erforderlichenfalls einer oder mehreren der nachstehenden Umwandlungen in beliebiger Reihenfolge unterziehen kann:
a) einer Veresterungsreaktion der Säurefunktion,
b) einer Verseifungsreaktion der Esterfunktion in eine Säurefunktion,
c) einer Oxidationsreaktion der Alkylthiogruppe in die entsprechende Sulfoxid- oder Sulfongruppe,
d) einer Umwandlungsreaktion der Ketonfunktion in eine Oximfunktion,
e) einer Reduktionsreaktion der freien oder der veresterten Carboxyfunktion in eine Alkoholfunktion,
f) einer Umwandlungsreaktion der Alkoxyfunktion in eine Hydroxylfunktion oder auch der Hydroxylfunktion in eine Alkoxyfunktion,
g) einer Oxidationsreaktion der Alkoholfunktion in eine Aldehyd-, Säure- oder Ketonfunktion,
h) einer Umwandlungsreaktion des Nitrilrestes in Tetrazolyl,
i) einer Eliminierungsreaktion der Schutzgruppen, die die geschützten reaktiven Funktionen tragen können,
j) einer Verseifungsreaktion durch eine mineralische oder organische Säure oder durch eine Base zur Erzielung des entsprechenden Salzes,
k) einer Spaltungsreaktion der racemischen Formen in die gespaltenen Produkte, wobei die so erhaltenen Produkte der Formel (I) in sämtlichen ihrer möglichen isomeren racemischen, enantiomeren und diastereomeren Formen vorliegen können.

13. Als Arzneimittel die Produkte der Formel (I), wie in den Ansprüchen 1 bis 4 definiert, sowie die Additionssalze mit pharmazeutisch verträglichen mineralischen oder organischen Säuren oder mineralischen oder organischen Basen der Produkte der Formel (I).

14. Als Arzneimittel die Produkte der Formeln (Ia) und (Ib), wie in den Ansprüchen 5 oder 6 definiert, sowie die Additionssalze mit pharmazeutisch verträglichen mineralischen oder organischen Säuren oder mineralischen oder organischen Basen der Produkte der Formeln (Ia) und (Ib).

15. Als Arzneimittel die Produkte der Formel (I), wie in einem der Ansprüche 7 bis 9 definiert, sowie die Additionssalze mit pharmazeutisch verträglichen mineralischen oder organischen Säuren oder mineralischen oder organischen Basen der Produkte der Formel (I).

16. Als Arzneimittel die Produkte der Formel (I), wie in Anspruch 10 oder 11 definiert, sowie die Additionssalze mit pharmazeutisch verträglichen mineralischen oder organischen Säuren oder mineralischen oder organischen Basen der Produkte der Formel (I).

17. Pharmazeutische Zusammensetzungen enthaltend als Wirkstoff zumindest eines der Arzneimittel, wie in den Ansprüchen 13 bis 16 definiert.

18. Verwendung der Produkte gemäß einem der Ansprüche 1 bis 11 oder der pharmazeutisch verträglichen Salze der Produkte zur Herstellung von Arzneimitteln für die Prävention oder Behandlung von Erkrankungen, bei denen die metabolischen Enzyme, wie Proteasen oder Kinasen, eine Rolle spielen.

19. Verwendung gemäß Anspruch 18 zur Herstellung der Arzneimittel für die Prävention oder Behandlung von Erkrankungen, bei denen Cathepsin-K, Cathepsin-B oder Papain eine Rolle spielen.

20. Verwendung gemäß Anspruch 18, **dadurch gekennzeichnet, dass** die zu verhindernden oder zu behandelnden Erkrankungen ausgewählt sind aus der folgenden Gruppe von Erkrankungen: kardiovaskuläre Erkrankungen, Krebs, Erkrankungen des zentralen Nervensystems, inflammatorische Erkrankungen, infektiöse Erkrankungen oder auch Knochenerkrankungen.

21. Verwendung gemäß Anspruch 18, **dadurch gekennzeichnet, dass** die zu verhindernden oder zu behandelnden Erkrankungen Erkrankungen des zentralen Nervensystems oder Erkrankungen der Knochen, wie insbesondere Osteoporose, sind.

## Claims

1. Products of formula (I): 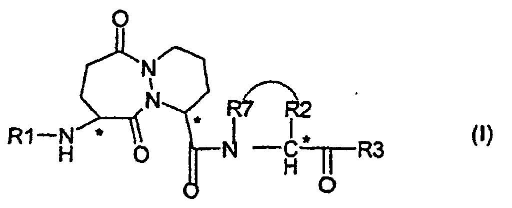 in which:
R1 represents the -C(O)-R5, -SO₂-R5 or -C(O)-NR6R5 radical in which R6 represents a hydrogen atom or a linear or branched alkyl radical including at most 4 carbon atoms and R5 represents a linear or branched alkyl radical including at most 6 carbon atoms, a cycloalkyl radical including at most 6 carbon atoms, a phenyl radical, a naphthyl radical or a saturated or unsaturated, 5- or 6-membered, monocyclic heterocyclic radical comprising one or more identical or different heteroatoms chosen from O, N or S,
the alkyl, phenyl and heterocyclic radicals as defined above for R1 optionally being substituted by one or more radicals chosen from halogen atoms and linear or branched alkyl or alkoxy radicals including at most 4 carbon atoms, the hydroxyl radical, acyl radicals including at most 7 carbon atoms, the trifluoromethyl radical, the cyano radical, the thienyl radical, the phenyl radical, the phenoxy radical and the isoxazolyl radical;
R2 and R7 are such that
either R7 represents a hydrogen atom and
R2 is such that the 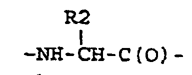 group in the products of formula (I) represents the residue of a "natural" or "unnatural" amino acid with the structure 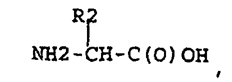 with the exception of aspartic acid,
or R2 and R7 form a ring, together with the nitrogen and carbon atoms to which they are bonded, so that the group thus formed in the products of formula (I) : 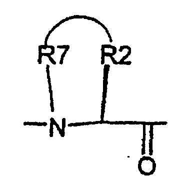 represents the residue of a "natural" or "unnatural" amino acid with the structure: 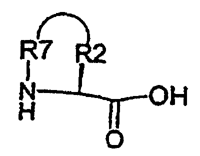
R3 represents the -CH=N₂ radical or the -CH₂-L-R4 radical in which L represents a single bond or a divalent radical chosen from -O-, -O-C(O)-, -NH- and -S-(CH₂)ₙ-, with n representing an integer from 0 to 6, and R4 represents a linear or branched alkyl radical including at most 6 carbon atoms or a saturated or unsaturated, 5- to 10-membered, monocyclic or bicyclic, carbocyclic or heterocyclic radical which comprises one or more identical or different heteroatoms chosen from O, N, NH or S and which can comprise a -C(O) ring member, the alkyl, carbocyclic and heterocyclic radicals as defined above for R4 optionally being substituted by one or more radicals chosen from halogen atoms or hydroxyl, free, salified or esterified carboxyl, -C(O)-NH₂, -C(O)-NH(alkyl), - C(O)-N(alkyl)(alkyl), -NH-C(O)-(alkyl), - N(alkyl)-C(O)-(alkyl), thienyl, phenyl, alkylphenyl, alkyl and alkoxy radicals, themselves optionally substituted by one or more radicals chosen from acyl radicals including at most 7 carbon atoms, the cyano radical, the -NH₂ radical, -NH(alkyl) radicals, -N(alkyl)(alkyl) radicals and the phenyl radical, it being understood that, in the above radicals, the alkyl and alkoxy radicals are linear or branched and include at most 4 carbon atoms,
the symbol * indicating the carbon atoms which can be in the S or R configuration,
the said products of formula (I) being in all the possible isomeric forms, racemic, enantiomeric and diastereoisomeric, and the addition salts with inorganic and organic acids or with inorganic and organic bases of the said products of formula (I).

2. Products of formula (I) as defined in Claim 1 in which R1 and R3 have the meanings indicated in Claim 1 and R2 represents the residue of a "natural" amino acid, with the exception of aspartic acid, the said products of formula (I) being in all the possible isomeric forms, racemic, enantiomeric and diastereoisomeric, and the addition salts with inorganic and organic acids or with inorganic and organic bases of the said products of formula (I).

3. Products of formula (I) as defined in either one of Claims 1 and 2 in which R1 and R3 have the meanings indicated in Claim 1 or 2 and R2 and R7 are such that:
either R7 represents a hydrogen atom and R2 represents a hydrogen atom or a linear or branched alkyl radical including at most 6 carbon atoms which is optionally substituted by one or more radicals chosen from the following radicals: amino; acylamino; NH=C(NH₂)-NH-; mercapto; linear or branched alkylthio including at most 4 carbon atoms; hydroxyl; linear or branched alkoxy including at most 4 carbon atoms; imidazolyl; indolyl; phenyl, itself optionally substituted by one or more radicals chosen from halogen atoms, the hydroxyl radical, a linear or branched alkoxy radical including at most 4 carbon atoms, and the phenoxy radical, itself optionally substituted by one or more radicals chosen from iodine atoms and the hydroxyl radical; and the -C(O)-O-R8 radical in which R8 represents a hydrogen atom or a linear or branched alkyl or alkenyl radical including at most 6 carbon atoms,
or R2 and R7 form, together with the nitrogen and carbon atoms to which they are bonded, a 5- to 7-membered ring optionally substituted by one or more hydroxyl radicals or linear or branched alkoxy radicals including at most 4 carbon atoms,
the said products of formula (I) being in all the possible isomeric forms, racemic, enantiomeric and diastereoisomeric, and the addition salts with inorganic and organic acids or with inorganic and organic bases of the said products of formula (I).

4. Products of formula (I) as defined in any one of Claims 1 to 3 in which R1 and R3 have the meanings indicated in any one of Claims 1 to 3,
R2 and R7 are such that:
either R7 represents a hydrogen atom and R2 has the meaning indicated in Claim 3,
or R2 and R7 form, together with the nitrogen and carbon atoms to which they are bonded, a pyrrolidinyl ring optionally substituted by one or more hydroxyl radicals or linear or branched alkoxy radicals including at most 4 carbon atoms,
the said products of formula (I) being in all the possible isomeric forms, racemic, enantiomeric and diastereoisomeric, and the addition salts with inorganic and organic acids or with inorganic and organic bases of the said products of formula (I).

5. Products of formula (I) as defined in any one of Claims 1 to 3 corresponding to the formula (Ia): 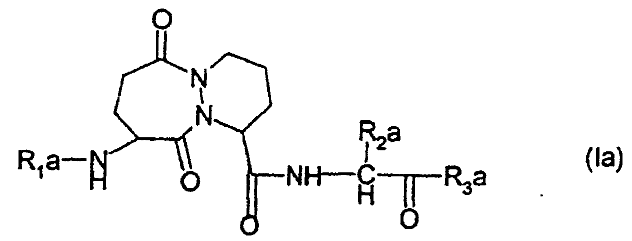 in which
R1a represents the -C(O)-R5a, -SO₂-R5a or -C(O)-NR6aR5a radical in which R6a represents a hydrogen atom or a linear or branched alkyl radical including at most 4 carbon atoms and R5a represents a radical chosen from linear or branched alkyl radicals including at most 6 carbon atoms, cycloalkyl radicals including at most 6 carbon atoms, the phenyl radical, the naphthyl radical, the furyl radical, the morpholinyl radical, the thienyl radical or the pyridyl radical,
the alkyl, phenyl, thienyl and pyridyl radicals optionally being substituted by one or more radicals chosen from halogen atoms and linear or branched alkyl or alkoxy radicals including at most 4 carbon atoms, the hydroxyl radical, acyl radicals including at most 7 carbon atoms, the phenoxy radical, the trifluoromethyl radical, the cyano radical, the thienyl radical, the phenyl radical and the isoxazolyl radical;
R2a has the meaning indicated in any one of Claims 1 to 4 when R7 represents a hydrogen atom,
R3a represents the -CH=N₂ radical or the -CH₂-La-R4a radical in which La represents a single bond or a divalent radical chosen from -O-, -O-C(O)- and -S-(CH₂)ₙₐ-, with na representing an integer from 0 to 3,
and R4a represents a radical chosen from the following radicals: linear or branched alkyl including at most 6 carbon atoms; phenyl; tetrazolyl; piperazinyl; piperidyl; pyridyl; benzothiazolyl; quinolyl; thiadiazolyl; pyrimidinyl; or tetralonyl;
the alkyl, phenyl, tetrazolyl, piperazinyl and piperidyl radicals as defined above for R4a optionally being substituted by one or more radicals chosen from halogen atoms or the following radicals: hydroxyl, free, salified or esterified carboxyl, -C(O)-NH₂, - C(O)-NH(alkyl), -C(O)-N(alkyl)(alkyl), - NH-C(O)-(alkyl), -N(alkyl)-C(O)-(alkyl), thienyl, phenyl, alkylphenyl, alkyl and alkoxy radicals, themselves optionally substituted by one or more radicals chosen from acyl radicals including at most 7 carbon atoms, the cyano radical, the -NH₂ radical, -NH(alkyl) radicals, -N(alkyl)(alkyl) radicals and the phenyl radical,
it being understood that, in the above radicals, the alkyl and alkoxy radicals are linear or branched and include at most 4 carbon atoms,
the said products of formula (Ia) being in all the possible isomeric forms, racemic, enantiomeric and diastereoisomeric, and the addition salts with inorganic and organic acids or with inorganic and organic bases of the said products of formula (Ia).

6. Products of formula (I) as defined in any one of Claims 1 to 5, corresponding to the formula (Ib): 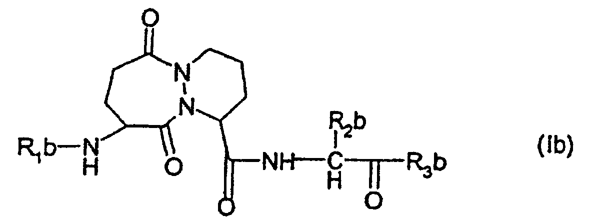 in which:
R1b represents the -C(O)-R5b, -SO₂-R5b or -C(O)-NR6bR5b radical,
in which R6b represents a hydrogen atom or a methyl radical and R5b represents a radical chosen from the following radicals: linear or branched alkyl including at most 4 carbon atoms which is optionally substituted by a thienyl radical; cyclohexyl; phenyl optionally substituted by a linear or branched alkoxy radical including at most 4 carbon atoms, a phenoxy radical or a trifluoromethyl radical; naphthyl; furyl; morpholinyl; thienyl optionally substituted by an isoxazolyl radical; or pyridyl optionally substituted by a trifluoromethyl radical,
R2b represents a hydrogen atom or a linear or branched alkyl radical including at most 6 carbon atoms which is optionally substituted either by the phenyl radical, itself optionally substituted by hydroxyl radicals or linear or branched alkoxy radicals including at most 4 carbon atoms, or by the -C(O)-O-R8b radical in which R8b represents a linear or branched alkyl or alkenyl radical including at most 6 carbon atoms,
R3b represents the -CH=N₂ radical or the -CH₂-Lb-R4b radical in which Lb represents a single bond or a divalent radical chosen from -O-, -O-C(O)- and -S-(CH₂)_{nb}-, with nb representing the integer 0 or 1,
and R4b represents a radical chosen from the following radicals: pyridyl; benzothiazolyl; quinolyl; thiadiazolyl; pyrimidinyl; tetralonyl; linear or branched alkyl including at most 4 carbon atoms which is optionally substituted by a free, salified or esterified carboxyl radical or a thienyl radical; phenyl optionally substituted by one or more radicals chosen from halogen atoms, free, salified or esterified carboxyl radicals, linear or branched alkyl and alkoxy radicals including at most 4 carbon atoms, cyanoalkyl radicals, alkylphenyl radicals, the -NH-C(O)-CH₃ radical or -C(O)-N(alkyl)(alkyl) radicals; tetrazolyl optionally substituted by a phenyl radical; piperazinyl optionally substituted on the second nitrogen atom by a phenyl radical or a linear or branched alkyl radical including at most 4 carbon atoms, which is itself optionally substituted by an acyl (pyrrolidinylcarbonyl) radical, one or two phenyl radicals or an -NH₂, -NH(alkyl) or -N(alkyl) (alkyl) radical; or piperidyl optionally substituted by a benzyl or -C(O)-N(alkyl)(alkyl) radical, it being understood that, in the above radicals, the alkyl and alkoxy radicals are linear or branched and include at most 4 carbon atoms,
the said products of formula (Ib) being in all the possible isomeric forms, racemic, enantiomeric and diastereoisomeric, and the addition salts with inorganic and organic acids or with inorganic and organic bases of the said products of formula (Ib).

7. Products of formula (I) as defined in any one of Claims 1 to 6, in which R1 represents a radical chosen from the following radicals: 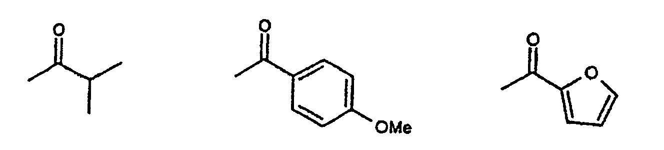 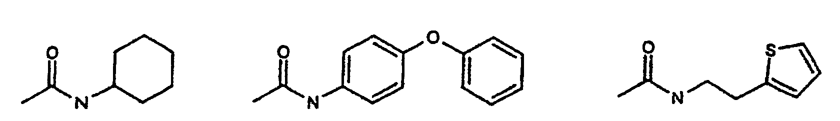 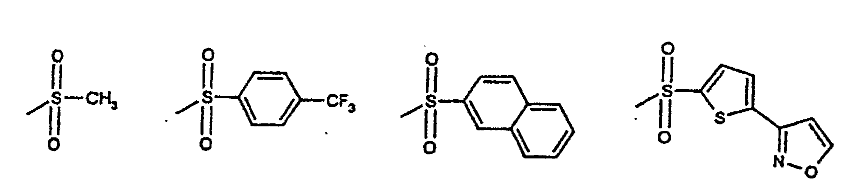 and R2, R3 and R7 have the values indicated in Claim 1.

8. Products of formula (I) as defined in any one of Claims 1 to 7, in which R7 represents a hydrogen atom and R2 represents a radical chosen from the following radicals: 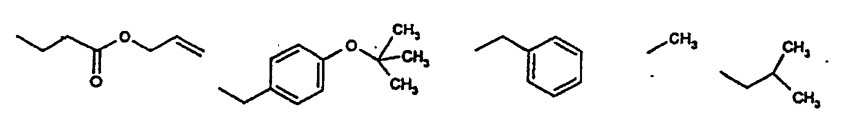 and R1 and R3 have the values indicated in Claim 1.

9. Products of formula (I) as defined in any one of Claims 1 to 8, in which R3 represents a radical chosen from the following radicals: 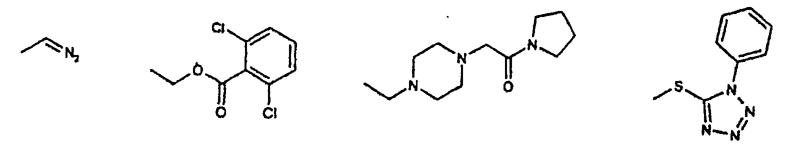 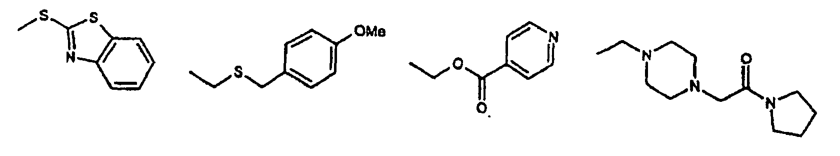 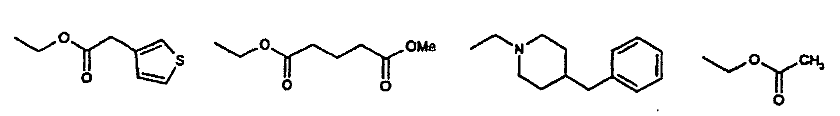 and R1, R2 and R7 have the values indicated in Claim 1.

10. Products of formula (I) with the following names:
- 3-[9(S)-benzoylamino-6,10-dioxo-1,2,3,4,7,8,9,10-octahydro-6H-pyridazino[1,2-a][1,2]diazepine-1(S)-carboxamido]-5-methyl-1-(benzoyloxy)hexan-2-one,
- 3-[9(S)-(2-furanoyl)amino-6,10-dioxo-1,2,3,4,7,8,9,10-octahydro-6H-pyridazino[1,2-a][1,2]-diazepine-1(S)-carboxamido]-5-methyl-1-(2,6-dichlorobenzoyloxy)hexan-2-one,
- 3(S)-[9(S)-(2-furanoyl)amino-6,10-dioxo-1,2,3,4,7,8,9,10-octahydro-6H-pyridazino[1,2-a][1,2]-diazepine-1(S)-carboxamido]-4-(4-hydroxyphenyl)-1-(2,6-dichlorobenzyloxy)butan-2-one.

11. Products of formula (I) with the following names:
- 2-propenyl (4S)-6-diazo-4-[[[(1S,9S)-9-[(2-methyl-1-oxopropyl)amino]-octahydro-6,10-dioxo-6H-pyridazino[1,2-a][1,2]diazepin-1-yl]carbonyl]amino]-5-oxohexanoate,
- 2-propenyl (4S)-6-diazo-4-[[[(1S,9S)-9-[(3-methoxybenzoyl)amino]-octahydro-6,10-dioxo-6H-pyridazino-[1,2-a][1,2]diazepin-1-yl]carbonyl]amino]-5-oxohexanoate,
- 2-propenyl (4S)-6-diazo-4-[[[(1S,9S)-9-[[(2-furanyl)-carbonyl]amino]-octahydro-6,10-dioxo-6H-pyridazino-[1,2-a][1,2]diazepin-1-yl]carbonyl]amino]-5-oxohexanoate,
- 2-propenyl (4S)-6-diazo-4-[[[(1S,9S)-9-[[(cyclohexylamino)carbonyl]amino]-octahydro-6,10-dioxo-6H-pyridazino[1,2-a][1,2]diazepin-1-yl]carbonyl]amino]-5-oxohexanoate,
- 2-propenyl (4S)-6-diazo-4-[[[(1S,9S)-9-[[[(4-phenoxyphenyl)amino]carbonyl]amino]-octahydro-6,10-dioxo-6H-pyridazino[1,2-a][1,2]diazepin-1-yl]carbonyl]amino]-5-oxohexanoate,
- 2-propenyl (4S)-6-diazo-4-[[[(1S,9S)-9-[[[[2-(p-2-yl)ethyl]amino]carbonyl]amino]-octahydro-6,10-dioxo-6H-pyridazino[1,2-a][1,2]diazepin-1-yl]carbonyl]amino]-5-oxohexanoate,
- 2-propenyl (4S)-6-diazo-4-[[[(1S,9S)-9-[(methylsulphonyl)amino]-octahydro-6,10-dioxo-6H-pyridazino-[1,2-a][1,2]diazepin-1-yl]carbonyl]amino]-5-oxohexanoate,
- 2-propenyl (4S)-6-diazo-4-[[[(1S,9S)-9-[[[4-(trifluoromethyl)phenyl]sulphonyl]amino]-octahydro-6,10-dioxo-6H-pyridazino[1,2-a][1,2]diazepin-1-yl]carbonyl]-amino]-5-oxohexanoate,
- 2-propenyl (4S)-6-diazo-4-[[[(1S,9S)-9-[[(2-naphthyl)sulphonyl]amino]-octahydro-6,10-dioxo-6H-pyridazino[1,2-a][1,2]diazepin-1-yl]carbonyl]amino]-5-oxohexanoate,
- 2-propenyl (4S)-6-diazo-4-[[[(1S,9S)-9-[[[5-(isoxazol-3-yl)-2-thienyl]sulphonyl]amino]-octahydro-6,10-dioxo-6H-pyridazino[1,2-a][1,2]diazepin-1-yl]-carbonyl]amino]-5-oxohexanoate,
- 9-[(9S)-[[(4-phenoxyphenyl)amino]carbonyl]amino]-N-[(2S)-4-diazo-1-[4-[(1,1-dimethyl)ethoxy]phenyl]-3-oxo-2-butyl]-6H-(1S)-pyridazino[1,2-a][1,2]diazepine-1-carboxamide,
- 2-propenyl (4S)-6-[(2,6-dichlorobenzoyl)oxy]-4-[[[(1S,9S)-9-[[(2-furanyl)carbonyl]amino]-octahydro-6,10-dioxo-6H-pyridazino[1,2-a][1,2]diazepin-1-yl]carbonyl]amino]-5-oxohexanoate,
- 2-propenyl (4S)-6-[(2,6-dichlorobenzoyl)oxy]-4-[[[(1S,9S)-9-[[[4-(trifluoromethyl)phenyl]sulphonyl]-amino]-octahydro-6,10-dioxo-6H-pyridazino[1,2-a][1,2]-diazepin-1-yl]carbonyl]amino]-5-oxohexanoate,
- 2-propenyl (4S)-6-[4-[2-oxo-2-(1-pyrrolidinyl)ethyl]-piperazin-1-yl]-4-[[[(1S,9S)-9-[[[4-(trifluoromethyl)-phenyl]sulphonyl]amino]-octahydro-6,10-dioxo-6H-pyridazino[1,2-a][1,2]diazepin-1-yl]carbonyl]amino]-5-oxohexanoate,
- 2-propenyl (4S)-6-[4-[2-oxo-2-(1-pyrrolidinyl)ethyl]-piperazin-1-yl]-4-[[[(1S,9S)-9-[[(2-naphthyl)-sulphonyl]amino]-octahydro-6,10-dioxo-6H-pyridazino-[1,2-a][1,2]diazepin-1-yl]carbonyl]amino]-5-oxohexanoate,
- 2-propenyl (4S)-6-[(2,6-dichlorobenzoyl)oxy]-4-[[[(1S,9S)-9-[[(cyclohexylamino)carbonyl]amino]-octahydro-6,10-dioxo-6H-pyridazino[1,2-a][1,2]diazepin-1-yl]carbonyl]amino]-5-oxohexanoate,
- 2-propenyl (4S)-6-[(1-phenyl-1H-tetrazol-5-yl)thio]-4-[[[(1S,9S)-9-[[[(4-phenoxyphenyl)amino]carbonyl]-amino]-octahydro-6,10-dioxo-6H-pyridazino-[1,2-a][1,2]diazepin-1-yl]carbonyl]amino]-5-oxohexanoate,
- 2-propenyl (4S)-6-[(benzothiazol-2-yl)thio]-4-[[[(1S,9S)-9-[[[(4-phenoxyphenyl)amino]carbonyl]amino]-octahydro-6,10-dioxo-6H-pyridazino[1,2-a][1,2]diazepin-1-yl]carbonyl]amino]-5-oxohexanoate,
- 2-propenyl (4S)-6-[[(4-methoxyphenyl)methyl]thio]-4-[[[(1S,9S)-9-[[[(4-phenoxyphenyl)amino]carbonyl]amino]-octahydro-6,10-dioxo-6H-pyridazino[1,2-a][1,2]diazepin-1-yl]carbonyl]amino]-5-oxohexanoate,
- 2-propenyl (4S)-6-[[(4-pyridinyl)carbonyl]oxy]-4-[[[(1S,9S)-9-[[[(4-phenoxyphenyl)a-mino]carbonyl]amino]-octahydro-6,10-dioxo-6H-pyridazino[1,2-a][1,2]diazepin-1-yl]carbonyl]amino]-5-oxohexanoate,
- 2-propenyl (4S)-6-[(2,6-dichlorobenzoyl)oxy]-4-[[[(1S,9S)-9-[[[(4-phenoxyphenyl)amino]carbonyl]amino]-octahydro-6,10-dioxo-6H-pyridazino[1,2-a][1,2]diazepin-1-yl]carbonyl]amino]-5-oxohexanoate,
- 2-propenyl (4S)-6-acetyloxy-4-[[[(1S,9S)-9-[[[(4-phenoxyphenyl)amino]carbonyl]amino]-octahydro-6,10-dioxo-6H-pyridazino[1,2-a][1,2]diazepin-1-yl]carbonyl]-amino]-5-oxohexanoate,
- 2-propenyl (4S)-6-[1-oxo-2-(thien-3-yl)ethoxy]-4-[[[(1S,9S)-9-[[[(4-phenoxyphenyl)amino]carbonyl]amino]-octahydro-6,10-dioxo-6H-pyridazino[1,2-a][1,2]diazepin-1-yl]carbonyl]amino]-5-oxohexanoate,
- 2-propenyl (4S)-6-[(1-phenyl-1H-tetrazol-5-yl)thio]-4-[[[(1S,9S)-9-[[[[2-(thien-2-yl)ethyl]amino]carbonyl]-amino]-octahydro-6,10-dioxo-6H-pyridazino[1,2-a][1,2]-diazepin-1-yl]carbonyl]amino]-5-oxohexanoate,
- 2-propenyl (4S)-6-[(2,6-dichlorobenzoyl)oxy]-4-[[[(1S,9S)-9-[[[[2-(thien-2-yl)ethyl]amino]carbonyl]-amino]-octahydro-6,10-dioxo-6H-pyridazino[1,2-a][1,2]-diazepin-1-yl]carbonyl]amino]-5-oxohexanoate,
- 2-propenyl (4S)-6-[(2,6-dichlorobenzoyl)oxy]-4-[[[(1S,9S)-9-[(methylsulphonyl)amino]-octahydro-6,10-dioxo-6H-pyridazino[1,2-a][1,2]diazepin-1-yl]carbonyl]amino]-5-oxohexanoate,
- 9-[(9S)-[[(4-phenoxyphenyl)amino]carbonyl]amino]-N-((2S)-4-diazo-1-phenyl-3-oxo-2-butyl)-octahydro-6,10-dioxo-6H-(1S)-pyridazino[2,2-a][1,2]diazepine-1-carboxamide,
- 9-[(9S)-[[[2-(thien-2-yl)ethyl]amino]carbonyl]amino]-N-((2S)-4-diazo-1-phenyl-3-oxo-2-butyl)-octahydro-6,10-dioxo-6H-(1S)-pyridazino[1,2-a][1,2]diazepine-1-carboxamide,
- 9-[(9S)-[(2-naphthyl)sulphonyl]amino]-N-((2S)-4-diazo-1-phenyl-3-oxo-2-butyl)-octahydro-6,10-dioxo-6H-(1S)-pyridazino[1,2-a][1,2]diazepine-1-carboxamide,
- 9-[(9S)-[[5-(isoxazol-3-yl)-2-thienyl]sulphonyl]-amino]-N-((2S)-4-diazo-1-phenyl-3-oxo-2-butyl)-octahydro-6,10-dioxo-6H-(1S)-pyridazino[1,2-a][1,2]-diazepine-1-carboxamide,
- 9-[(9S)-[[(4-phenoxyphenyl)amino]carbonyl]amino]-N-((2S)-4-diazo-3-oxo-2-butyl)-octahydro-6,10-dioxo-6H-(1S)-pyridazino[1,2-a][1,2]diazepine-1-carboxamide,
- 9-[(9S)-[[[3-(thien-2-yl)ethyl]amino]carbonyl]amino]-N-((2S)-4-diazo-3-oxo-2-butyl)-octahydro-6,10-dioxo-6H-(1S)-pyridazino[1,2-a][1,2]diazepine-1-carboxamide,
- 9-[(9S)-(3-methoxybenzoyl)amino]-N-[(2S)-4-[(2,6-dichlorobenzoyl)oxy]-1-phenyl-3-oxo-2-butyl]-octahydro-6,10-dioxo-6H-(1S)-pyridazino[1,2-a][1,2]diazepine-1-carboxamide,
- 9-[(9S)-[(2-furanyl)carbonyl]amino]-N-[(2S)-4-[(2,6-dichlorobenzoyl)oxy]-1-phenyl-3-oxo-2-butyl]-octahydro-6,10-dioxo-6H-(1S)-pyridazino[1,2-a][1,2]diazepine-1-carboxamide,
- 9-[(9S)-[(cyclohexylamino)carbonyl]amino]-N-[(2S)-4-[(2,6-dichlorobenzoyl)oxy]-1-phenyl-3-oxo-2-butyl]-octahydro-6,10-dioxo-6H-(1S)-pyridazino-[1,2-a][1,2]diazepine-2-carboxamide,
- 9-[(9S)-[[(4-phenoxyphenyl)amino]carbonyl]amino]-N-[(2S)-1-phenyl-4-[(1-phenyl-1H-tetrazol-5-yl)thio]-3-oxo-2-butyl]-octahydro-6,10-dioxo-6H-(1S)-pyridazino-[1,2-a][1,2]diazepine-1-carboxamide,
- 9-[(9S)-[[(4-phenoxyphenyl)amino]carbonyl]amino]-N-[(2S)-1-phenyl-4-[1-oxo-2-(3-thienyl)ethoxy]-3-oxo-2-butyl]-octahydro-6,10-dioxo-6H-(1S)-pyridazino-[1,2-a][1,2]diazepine-1-carboxamide, Methyl [2-oxo-4-phenyl-3-[(3S)-[[9-[(9S)-[[(4-phenoxyphenyl)amino]carbonyl]amino]-octahydro-6,10-dioxo-6H-(1S)-pyridazino[1,2-a][1,2]diazepin-1-yl]carbonyl]-amino]butyl] pentanedioate,
- 9-[(9S)-[[(4-phenoxyphenyl)amino]carbonyl]amino]-N-[(2S)-4-[(4-phenylmethyl)-1-piperidinyl]-1-phenyl-3-oxo-2-butyl]-octahydro-6,10-dioxo-6H-(1S)-pyridazino-[1,2-a][1,2]diazepine-1-carboxamide,
- 9-[(9S)-[[[2-(thien-2-yl)ethyl]amino]carbonyl]amino]-N-[(2S)-1-phenyl-4-[(1-phenyl-1H-tetrazol-5-yl)thio]-3-oxo-2-butyl]-octahydro-6,10-dioxo-6H-(1S)-pyridazino-[1,2-a][1,2]diazepine-1-carboxamide,
- 9-[(9S)-[[[2-(thien-2-yl)ethyl]amino]carbonyl]amino]-N-[(2S)-1-phenyl-4-[(2-benzothiazolyl)thio]-3-oxo-2-butyl]-octahydro-6,10-dioxo-6H-(1S)-pyridazino-[2,2-a][1,2]diazepine-1-carboxamide,
- 9-[(9S)-[[[2-(thien-2-yl)ethyl]amino]carbonyl]amino]-N-[(2S)-2-phenyl-4-[(2,6-dichlorobenzoyl)oxy]-3-oxo-2-butyl]-octahydro-6,10-dioxo-6H-(1S)-pyridazino-[1,2-a][1,2]diazepine-1-carboxamide,
- 9-[(9S)-[[[2-(thien-2-yl)ethyl]amino]carbonyl]amino]-N-[(2S)-1-phenyl-4-[4-[2-(1-pyrrolidinyl)-2-oxoethyl]-piperazin-1-yl]-3-oxo-2-butyl]-octahydro-6,10-dioxo-6H-(1S)-pyridazino[1,2-a][1,2]diazepine-1-carboxamide,
- 9-[(9S)-(methylsulphonyl)amino]-N-[(2S)-1-phenyl-4-[(2,6-dichlorobenzoyl)oxy]-3-oxo-2-butyl]-octahydro-6,10-dioxo-6H-(1S)-pyridazino[2,2-a][1,2]diazepine-1-carboxamide,
- 9-[(9S)-[[4-(trifluoromethyl)phenyl]sulphonyl]amino]-N-[(2S)-1-phenyl-4-[(1-phenyl-1H-tetrazol-5-yl)thio]-3-oxo-2-butyl]-octahydro-6,10-dioxo-6H-(1S)-pyridazino-[1,2-a][1,2]diazepine-1-carboxamide,
- 9-[(9S)-[[4-(trifluoromethyl)phenyl]sulphonyl]amino]-N-[(2S)-1-phenyl-4-[(2-benzothiazolyl)thio]-3-oxo-2-butyl]-octahydro-6,10-dioxo-6H-(1S)-pyridazino-[1,2-a][1,2]diazepine-1-carboxamide,
- 9-[(9S)-(methylsulphonyl)amino]-N-[(3S)-5-methyl-1-[(2,6-dichlorobenzoyl)oxy]-2-oxo-3-hexyl]-octahydro-6,10-dioxo-6H-(1S)-pyridazino[1,2-a][2,2]diazepine-1-carboxamide,
- 9-[(9S)-[[[4-(trifluoromethyl)phenyl]sulphonyl]-amino]]-N-[(3S)-5-methyl-1-[(2,6-dichlorobenzoyl)oxy]-2-oxo-3-hexyl]-octahydro-6,10-dioxo-6H-(1S)-pyridazino-[1,2-a][2,2]diazepine-1-carboxamide,
- 9-[(9S)-[(2-naphthyl)sulphonyl]amino]-N-[(3S)-5-methyl-1-[(2,6-dichlorobenzoyl)oxy]-2-oxo-3-hexyl]-octahydro-6,10-dioxo-6H-(1S)-pyridazino[1,2-a][1,2]-diazepine-1-carboxamide,
- 9-[(9S)-(2-methyl-1-oxopropyl)amino]-N-((3S)-1-diazo-5-methyl-2-oxo-3-hexyl)-octahydro-6,10-dioxo-6H-(1S)-pyridazino[1,2-a][1,2]diazepine-1-carboxamide,
- 9-[(9S)-(3-methoxybenzoyl)amino]-N-((3S)-1-diazo-5-methyl-2-oxo-3-hexyl)-octahydro-6,10-dioxo-6H-(1S)-pyridazino[1,2-a][1,2]diazepine-1-carboxamide,
- 9-[(9S)-[(2-furanyl)carbonyl]amino)-N-((3S)-1-diazo-5-methyl-2-oxo-3-hexyl)-octahydro-6,10-dioxo-6H-(1S)-pyridazino[1,2-a][1,2]diazepine-1-carboxamide,
- 9-[(9S)-[(cyclohexylamino)carbonyl]amino]-N-((3S)-1-diazo-5-methyl-2-oxo-3-hexyl)-octahydro-6,10-dioxo-6H-(1S)-pyridazino[1,2-a][1,2]diazepine-1-carboxamide,
- 9-[(9S)-[[(4-phenoxyphenyl)amino]carbonyl]amino]-N-((3S)-1-diazo-5-methyl-2-oxo-3-hexyl)-octahydro-6,10-dioxo-6H-(1S)-pyridazino[1,2-a][1,2]diazepine-1-carboxamide,
- 9-[(9S)-[[[2-(thien-2-yl)ethyl]amino]carbonyl]amino]-N-((3S)-1-diazo-5-methyl-2-oxo-3-hexyl)-octahydro-6,10-dioxo-6H-(1S)-pyridazino[1,2-a][1,2]diazepine-1-carboxamide,
- 9-[(9S)-(methylsulphonyl)amino]-N-((3S)-1-diazo-5-methyl-2-oxo-3-hexyl)-octahydro-6,10-dioxo-6H-(1S)-pyridazino[1,2-a][1,2]diazepine-1-carboxamide,
- 9-[(9S)-[[4-(trifluoromethyl)phenyl]sulphonyl]amino]-N-((3S)-1-diazo-5-methyl-2-oxo-3-hexyl)-octahydro-6,10-dioxo-6H-(1S)-pyridazino[1,2-a][1,2]diazepine-1-carboxamide,
- 9-[(9S)-[(2-naphthyl)sulphonyl]amino]-N-((3S)-1-diazo-5-methyl-2-oxo-3-hexyl)-octahydro-6,10-dioxo-6H-(1S)-pyridazino[1,2-a][1,2]diazepine-1-carboxamide,
- 9-[(9S)-(3-methoxybenzoyl)amino]-N-[(3S)-1-[(benzothiazol-2-yl)thio]-4-methyl-2-oxo-3-pentyl]-octahydro-6,10-dioxo-6H-(1S)-pyridazino[1,2-a][1,2]diazepine-1-carboxamide,
- 9-[(9S)-[(2-furanyl)carbonyl]amino]-N-[(3S)-1-[(benzothiazol-2-yl)thio]-4-methyl-2-oxo-3-pentyl]-octahydro-6,10-dioxo-6H-(1S)-pyridazino-[1,2-a][1,2]diazepine-1-carboxamide,
- 9-[(9S)-[(cyclohexylamino)carbonyl]amino]-N-[(3S)-1-[(benzothiazol-2-yl)thio]-4-methyl-2-oxo-3-pentyl]-octahydro-6,10-dioxo-6H-(1S)-pyridazino-[1,2-a][1,2]diazepine-2-carboxamide,
- 9-[(9S)-[[(4-phenoxyphenyl)amino]carbonyl]amino]-N-[(3S)-1-[(1-phenyl-1H-tetrazol-5-yl)thio]-4-methyl-2-oxo-3-pentyl]-octahydro-6,10-dioxo-6H-(1S)-pyridazino-[1,2-a][1,2]diazepine-1-carboxamide,
- 9-[(9S)-[[(4-phenoxyphenyl)amino]carbonyl]amino]-N-[(3S)-1-[(benzothiazol-2-yl)thio]-4-methyl-2-oxo-3-pentyl]-octahydro-6,10-dioxo-6H-(1S)-pyridazino-[1,2-a][1,2]diazepine-1-carboxamide,
- 9-[(9S)-[[[2-(thien-2-yl)ethyl]amino]carbonyl]amino]-N-[(3S)-1-[(1-phenyl-1H-tetrazol-5-yl)thio]-4-methyl-2-oxo-3-pentyl]-octahydro-6,10-dioxo-6H-(1S)-pyridazino-[1,2-a][1,2]diazepine-1-carboxamide,
- 9-[(9S)-[[[2-(thien-2-yl)ethyl]amino]carbonyl]amino]-N-[(3S)-1-[(benzothiazol-2-yl)thio]-4-methyl-2-oxo-3-pentyl]-octahydro-6,10-dioxo-6H-(1S)-pyridazino-[1,2-a][1,2]diazepine-1-carboxamide,
- 9-[(9S)-[(cyclohexylamino)carbonyl]amino]-N-[(2S)-4-[(2,6-dichlorobenzoyl)oxy]-3-oxo-2-butyl]-octahydro-6,10-dioxo-6H-(1S)-pyridazino[1,2-a][1,2)diazepine-1-carboxamide,
- 9-[(9S)-[(2-furanyl)carbonyl]amino]-N-[(2S)-4-[(2,6-dichlorobenzoyl)oxy]-3-oxo-2-butyl]-octahydro-6,10-dioxo-6H-(1S)-pyridazino[1,2-a][1,2]diazepine-1-carboxamide,
- 9-[(9S)-[[(4-phenoxyphenyl)amino]carbonyl]amino]-N-[(2S)-4-[(benzothiazol-2-yl)thio]-3-oxo-2-butyl]-octahydro-6,10-dioxo-6H-(1S)-pyridazino-[1,2-a][1,2]diazepine-1-carboxamide,
- 9-[(9S)-[[(4-phenoxyphenyl)amino]carbonyl]amino]-N-[(2S)-4-[(2,6-dichlorobenzoyl)oxy]-3-oxo-2-butyl]-octahydro-6,10-dioxo-6H-(1S)-pyridazino-[1,2-a][1,2]diazepine-1-carboxamide,
- 9-[(9S)-(3-methoxybenzoyl)amino)-N-[(2S)-4-[(2,6-dichlorobenzoyl)oxy]-3-oxo-2-butyl]-octahydro-6,10-dioxo-6H-(1S)-pyridazino[1,2-a][1,2]diazepine-1-carboxamide,
- 9-[(9S)-[[[2-(thien-2-yl)ethyl]amino]carbonyl]amino]-N-[(2S)-4-[(2,6-dichlorobenzoyl)oxy]-3-oxo-2-butyl]-octahydro-6,10-dioxo-6H-(1S)-pyridazino-[1,2-a][1,2]diazepine-1-carboxamide,
- 9-[(9S)-[[4-(trifluoromethyl)phenyl]sulphonyl]amino]-N-[(2S)-4-[(2,6-dichlorobenzoyl)oxy]-3-oxo-2-butyl]-octahydro-6,10-dioxo-6H-(1S)-pyridazino-[1,2-a][1,2]diazepine-1-carboxamide,
- 9-[(9S)-[(cyclohexylamino)carbonyl]amino]-N-[(2S)-4-[(benzothiazol-2-yl)thio]-1-[4-[(1,1-dimethyl)ethoxy]-phenyl]-3-oxo-2-butyl]-6H-(1S)-pyridazino[1,2-a][1,2]-diazepine-1-carboxamide.

12. Process for the preparation of the products of formula (I) as defined in Claim 1, **characterized in that** the compound of formula (II): 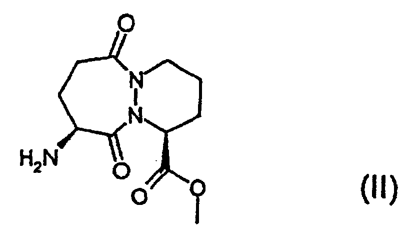 is reacted with a compound of formula (III):
R1'-X (III)
in which X represents a halogen atom and R1' has the meaning shown in Claim 1 for R1, in which the possible reactive functional groups are optionally protected by protective groups,
in order to obtain the product of formula (IV): 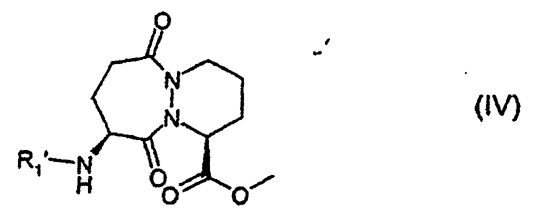 in which R1' has the meaning indicated above,
which product of formula (IV) is subjected to a saponification reaction in order to obtain the product of formula (V): 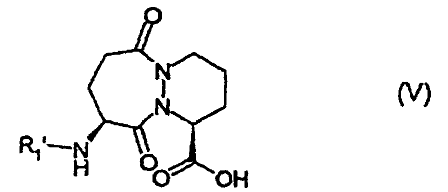 in which R1' has the meaning indicated above,
which product of formula (V) is reacted with a product of formula (VI): 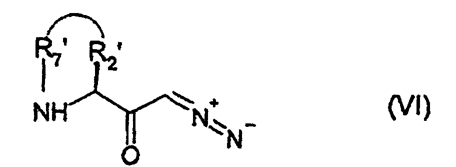 in which R2' and R7' have the meanings indicated in Claim 1 for R2 and R7 respectively, in which the possible reactive functional groups are optionally protected by protective groups,
in order to obtain a product of formula (Ix): 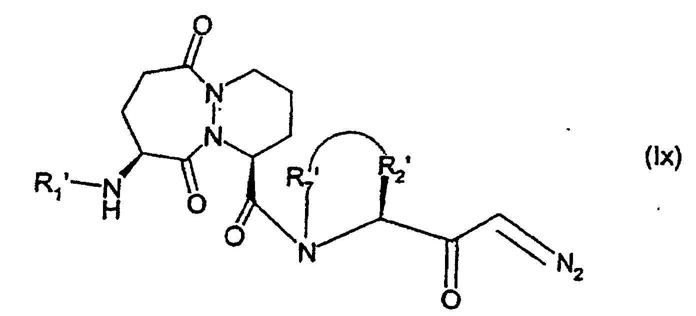 in which R1', R2' and R7' have the meanings indicated above,
which product of formula (Ix) can be subjected to a bromination reaction in order to obtain a product of formula (VII): 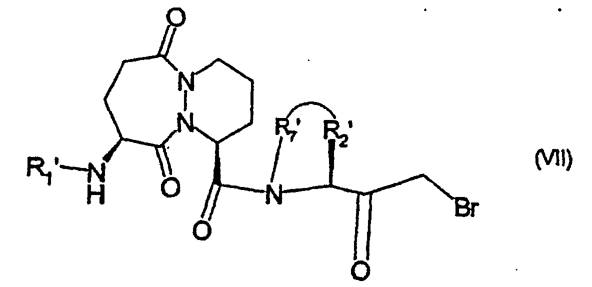 in which R1', R2' and R7' have the meanings indicated above,
which product of formula (VII) is reacted with a product of formula (VIII):
H'-L-R4' (VIII)
in which L has the meaning indicated in Claim 1 and R4' has the meaning indicated in Claim 1 for R4, in which the possible reactive functional groups are optionally protected by protective groups,
in order to obtain a product of formula (Iy): 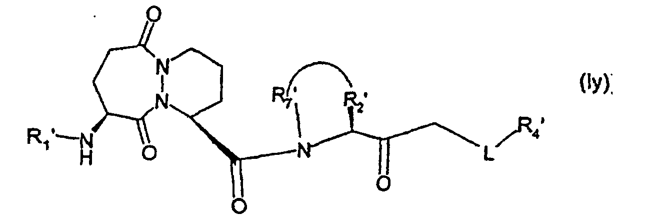 in which R1', R2', R4' and R7' have the meanings indicated above,
which products of formulae (Ix) and (Iy) may be products of formula (I) and, in order to obtain products or other products of formula (I), can be subjected, if desired and if necessary, to one or more of the following conversion reactions, in any order:
a) a reaction for the esterification of an acid functional group,
b) a reaction for the saponification of an ester functional group to an acid functional group,
c) a reaction for the oxidation of an alkylthio group to the corresponding sulphoxide or sulphone,
d) a reaction for the conversion of a ketone functional group to an oxime functional group,
e) a reaction for the reduction of the free or esterified carboxyl functional group to an alcohol functional group,
f) a reaction for the conversion of an alkoxy functional group to the hydroxyl functional group or alternatively of a hydroxyl functional group to an alkoxy functional group,
g) a reaction for the oxidation of an alcohol functional group to an aldehyde, acid or ketone functional group,
h) a reaction for the conversion of a nitrile radical to a tetrazolyl,
i) a reaction for the removal of the protective groups which may be carried by the protected reactive functional groups,
j) a reaction for salification by an inorganic or organic acid or by a base in order to obtain the corresponding salt,
k) a reaction for resolving the racemic forms into resolved products,
the said products of formula (I) thus obtained being in all the possible isomeric forms, racemic, enantiomeric and diastereoisomeric.

13. As medicaments, the products of formula (I) as defined in Claims 1 to 4 and the addition salts of the said products of formula (I) with pharmaceutically acceptable inorganic and organic acids or with pharmaceutically acceptable inorganic and organic bases.

14. As medicaments, the products of formulae (Ia) and (Ib) as defined in Claim 5 or 6 and the addition salts of the said products of formulae (Ia) and (Ib) with pharmaceutically acceptable inorganic and organic acids or with pharmaceutically acceptable inorganic and organic bases.

15. As medicaments, the products of formula (I) as defined in any one of Claims 7 to 9 and the addition salts of the said products of formula (I) with pharmaceutically acceptable inorganic and organic acids or with pharmaceutically acceptable inorganic and organic bases.

16. As medicaments, the products of formula (I) as defined in Claim 10 or 11 and the addition salts of the said products of formula (I) with pharmaceutically acceptable inorganic and organic acids or with pharmaceutically acceptable inorganic and organic bases.

17. Pharmaceutical compositions comprising, as active principle, at least one of the medicaments as defined in Claims 13 to 16.

18. Use of the products according to one of Claims 1 to 11 or of pharmaceutically acceptable salts of the said products in the preparation of medicaments intended for the prevention or treatment of diseases in which metabolic enzymes, such as proteases or kinases, are implicated.

19. Use according to Claim 18 in the preparation of medicaments intended for the prevention or treatment of diseases in which cathepsin K, cathepsin B or papain are implicated.

20. Use according to Claim 18, **characterized in that** the diseases to be prevented or treated are chosen from the following group of diseases: cardiovascular diseases, cancers, diseases of the central nervous system, inflammatory diseases, infectious diseases or bone diseases.

21. Use according to Claim 18, **characterized in that** the diseases to be prevented or treated are diseases of the central nervous system or bone diseases, such as, in particular, osteoporosis.
